# EUROPEAN PATENT APPLICATION

(11) **EP 4 632 074 A1**
(43) Date of publication of application: **15.10.2025**
(21) Application number: 23900075.5
(22) Date of filing: 08.12.2023
(51) Int. Cl.: C12N 15/87, C12N 5/10, C12N 15/113, C12N 15/11, C12N 5/078, C12N 5/0783, C12N 5/0784, C12N 5/0786, C12N 5/0787, A61K 35/17, C07K 14/725, A61P 35/00, A61P 35/04

(54) **MODIFIED CELL AND USE THEREOF**

(30) Priority: 09.12.2022 WO PCT/CN2022/137760; 09.12.2022 WO PCT/CN2022/137761; 09.12.2022 WO PCT/CN2022/137762; 09.12.2022 WO PCT/CN2022/137763; 08.08.2023 WO PCT/CN2023/111625
(71) Applicant: Suzhou Grit Biotechnology Co., Ltd., Shanghai 200120 (CN); Zhuhai Tuoyu Biotechnology Co., Ltd., Shanghai 200120 (CN); Shanghai Grit Biotechnology Co., Ltd., Shanghai 200120 (CN); Shenzhen Grit Biotechnology Co., Ltd., Shanghai 200120 (CN)
(72) Inventor: LIU, Yarong, Shanghai 200120 (CN); SUN, Jingwei, Shanghai 200120 (CN); SHENG, Yao, Shanghai 200120 (CN)
(74) Representative: Cabinet Becker et Associés
(86) International application number: PCT/CN2023/137333
(87) International publication number: WO 2024/120506

(57) **Abstract**

The present invention relates to the field of biomedicine, and provides a modified cell and its uses. Specifically, the present invention relates to a method of culturing cells, comprising reducing the expression and/or activity of target genes. The present invention also relates to methods of preventing and/or treating a tumor using the cultured cells.

## Description

### Technical Field

The present invention relates to the field of biomedicine, and specifically to a modified cell and its uses.

### Background

Currently, immunotherapy is an effective way to treat patients with poor prognosis. However, the immune cells used in immunotherapy have problems such as weak cell function or weak proliferation and survival capabilities after reinfusion into the body. Therefore, there is an urgent need for a modified immune cell and a robust and reliable immune cell culture method.

### Contents of the Invention

The invention provides a method for cultivating cells, which has one or more of the following advantages: enhanced target cell killing ability, enhanced cell proliferation ability, enhanced cytokine release ability, increased proportion of activated cells, reduced proportion of regulatory cells, decreased proportion of exhausted cells, increased proportion of central memory cells and/or naive cells, decreased proportion of apoptotic cells and increased proportion of stem cell-like cells.

In one aspect, the present invention provides a method of culturing cells, the method comprising: reducing the expression and/or activity of members selected from the peptidase C64 family, the ZC3H12 family, the STAT-induced STAT inhibitor (SSI) family, and the CBL family.

In another aspect, the present invention provides a cell produced by the method of the present invention.

In another aspect, the present invention provides a pharmaceutical composition comprising the cells of the present invention, and optionally a pharmaceutically acceptable carrier.

In another aspect, the invention provides a method of affecting cell growth, comprising administering the cells of the invention and/or pharmaceutical compositions of the invention.

In another aspect, the invention provides the use of the cells of the invention and/or the pharmaceutical composition of the invention in the preparation of medicaments for preventing and/or treating diseases and/or symptoms.

Other aspects and advantages of the present invention will be readily apparent to those skilled in the art from the detailed description that follows. Only exemplary embodiments of the invention are shown and described in the following detailed description.

As those skilled in the art will realize, the present disclosure enables those skilled in the art to make changes to the specific embodiments disclosed without departing from the spirit and scope of the invention to which the invention relates. Accordingly, the drawings and description of the present invention are illustrative only and not restrictive.

### Description of the Drawings

The features and advantages of the invention to which the present invention relates can be better understood by reference to the exemplary embodiments described in detail below and the accompanying drawings. A brief description of the drawings is as follows:
Figure 1A shows the human TNFAIP3 gene editing target segment relative to the start codon provided by the present invention. For example, it can be a continuous region with more than about 3 transcription factor binding numbers; and it can be the exosome of the gene. subregion or intron region approximately 20 bp from the exon.
Figure 1B shows the human ZC3H12A gene editing target segment relative to the start codon provided by the present invention. For example, it can be a continuous region with more than about 3 transcription factor binding numbers; and it can be the exosome of the gene. subregion or intronic region approximately 20 bp from the exon.
Figure 1C shows the human SOCS1 gene editing target segment relative to the start codon provided by the present invention. For example, it can be a continuous region with a transcription factor binding number of about 3 or more; and it can be the exosome of the gene. subregion or intronic region approximately 20 bp from the exon.
Figure 1D shows the human CBLB gene editing target segment relative to the start codon provided by the present invention. For example, it can be a continuous region with a transcription factor binding number of about 3 or more; and it can be the exosome of the gene. Subregion or intron approximately 20 bp from exon sub-region.
Figure 2A shows the TCR-T amplification fold of TNFAIP3 gene editing in the non-stimulating medium group.
Figures 2B-2C show the TCR-T amplification fold of TNFAIP3 gene editing in the TransACT stimulation group.
Figures 2D-2G show the target cell killing ability of TCR-T cells derived from different donors after TNFAIP3 gene editing. Figures 2D and 2F show the killing curves at each time point, and Figures 2E and 2G show the killing status of each test group at the end of the test, which are all higher than the unedited NT group.
Figures 2H-2K show the various cytokine release capabilities of TNFAIP3 gene-edited TCR-T cells.
Figure 3A shows the TCR-T amplification fold of ZC3H12A gene editing in the non-stimulating medium group.
Figure 3B shows the TCR-T amplification fold of ZC3H12A gene editing in the TransACT stimulation group.
Figures 3C-3F show the target cell killing ability of ZC3H12A gene-edited TCR-T cells. Figures 3C and 3E show the killing curves at each time point, and Figures 3D and 3F show the killing status of each test group at the end of the test, which are all higher than the unedited NT group.
Figures 3G-3J show the various cytokine release capabilities of ZC3H12A gene-edited TCR-T cells.
Figure 4A shows the TCR-T amplification fold of SOCS1 gene editing in the non-stimulating medium group.
Figure 4B shows the TCR-T amplification fold of SOCS1 gene editing in the TransACT stimulation group.
Figure 4C shows the amplification fold of SOCS1 gene-edited TILs derived from different donors in the non-stimulating medium group.
Figure 4D shows the amplification fold of TIL derived from donor 306 edited by the SOCS 1 gene in the TransACT stimulation group.
Figures 4E-4H show the target cell killing ability of SOCS1 gene-edited TCR-T cells.
Figure 4I shows the target cell killing ability of SOCS1 gene-edited TIL cells.
Figure 4J shows the cytokine expression of SOCS1 gene-edited TCR-T cells in the unstimulated group.
Figure 4K shows the cytokine expression of SOCS 1 gene-edited TCR-T cells in the CD3 antibody stimulation group.
Figure 4L shows the cytokine release ability of SOCS 1 gene-edited TCR-T cells co-cultured with A375 target cells.
Figure 4M shows the cytokine expression of SOCS1 gene-edited TIL cells in the unstimulated group.
Figure 4N shows the cytokine expression of SOCS 1 gene-edited TIL cells in the TransACT stimulation group.
Figure 4O shows that TIL cells after SOCS1 gene editing have a higher proportion of stem cells.
Figure 4P shows that TIL cells after SOCS1 gene editing have a lower proportion of exhausted T cells.
Figures 5A-5B show the TCR-T amplification fold of CBLB gene editing in the non-stimulating medium group.
Figures 5C-5D show the TCR-T amplification fold of CBLB gene editing in the TransACT stimulation group.
Figures 5E-5H show the target cell killing ability of CBLB gene-edited TCR-T cells. Figures 5E and 5G show the killing curves at each time point, and Figures 5F and 5H show the killing status of each test group at the end of the test, which are all higher than the unedited NT group.
Figures 5I-5L show various cytokine release capabilities of CBLB gene-edited TCR-T cells.
Figure 6A shows the TIL amplification fold of combined gene editing of CBLB and ZC3H12A in the non-stimulating medium group.
Figure 6B shows the TIL amplification fold of SOCS 1 and CBLB combined gene editing in the non-stimulating medium group.
Figure 6C shows the TIL amplification fold of SOCS 1 and TNFAIP3 combined gene editing in the non-stimulating medium group.
Figure 6D shows the TIL amplification fold of SOCS1 and TNFAIP3 combined gene editing in the CD3 antibody stimulation group.
Figure 6E shows the TIL amplification fold of combined gene editing of SOCS 1 and ZC3H12A in the non-stimulating medium group.
Figure 6F shows the TIL amplification fold of combined gene editing of SOCS 1 and ZC3H12A in the CD3 antibody stimulation group.
Figure 6G shows the TIL amplification fold of TNFAIP3 and CBLB combined gene editing in the non-stimulating medium group.
Figure 6H shows the TIL amplification fold of TNFAIP3 and ZC3H12A combined gene editing in the non-stimulating medium group.
Figure 6I shows the TIL amplification fold of TNFAIP3 and ZC3H12A combined gene editing in the CD3 antibody stimulation group.
Figure 6J shows the TIL amplification fold of TNFAIP3 and SOCS1 combined gene editing in the non-stimulating medium group.
Figure 6K shows the TIL amplification fold of TNFAIP3 and SOCS1 combined gene editing in the TransACT antibody stimulation group.
Figure 7A shows the target cell killing ability of TIL cells edited in both CBLB and ZC3H12A genes.
Figure 7B shows the target cell killing ability of TIL cells edited in both CBLB and ZC3H12A genes.
Figure 7C shows the target cell killing ability of TIL cells edited in both SOCS1 and CBLB genes.
Figure 7D shows the target cell killing ability of TIL cells edited in both SOCS1 and CBLB genes.
Figure 7E shows the target cell killing ability of TIL cells edited in both SOCS1 and CBLB genes.
Figure 7F shows the target cell killing ability of TIL cells edited in both SOCS1 and TNFAIP3 genes.
Figure 7G shows the target cell killing ability of TIL cells edited in both SOCS1 and TNFAIP3 genes.
Figure 7H shows the target cell killing ability of TIL cells edited in both SOCS1 and TNFAIP3 genes.
Figure 7I shows the target cell killing ability of TIL cells edited in both SOCS1 and TNFAIP3 genes.
Figure 7J shows the target cell killing ability of TIL cells edited in both SOCS1 and ZC3H12A genes.
Figure 7K shows the target cell killing ability of TIL cells edited in both SOCS1 and ZC3H12A genes.
Figure 7L shows the target cell killing ability of TIL cells edited in both SOCS1 and ZC3H12A genes.
Figure 7M shows the target cell killing ability of TIL cells edited in both TNFAIP3 and CBLB genes.
Figure 7N shows the target cell killing ability of TIL cells edited in both TNFAIP3 and CBLB genes.
Figure 7O shows the target cell killing ability of TIL cells edited in both TNFAIP3 and ZC3H12A genes.
Figure 7P shows the target cell killing ability of TIL cells edited in both TNFAIP3 and ZC3H12A genes.
Figure 7Q shows the target cell killing ability of TIL cells edited in both TNFAIP3 and ZC3H12A genes.
Figure 7R shows the target cell killing ability of TIL cells edited in both TNFAIP3 and ZC3H12A genes.
Figure 7S shows the target cell killing ability of TIL cells edited in both TNFAIP3 and SOCS1 genes.
Figure 7T shows the killing ability of TIL cells edited in both TNFAIP3 and SOCS1 genes against autologous tumor organoids.
Figure 8A shows that TIL cells edited in both CBLB and ZC3H12A genes have a lower proportion of exhausted T cells.
Figure 8B shows that TIL cells edited in both SOCS1 and CBLB genes have a higher proportion of central memory T cells.
Figure 8C shows that TIL cells edited in both SOCS1 and CBLB genes have a lower proportion of exhausted T cells.
Figure 8D shows that TIL cells edited in both SOCS1 and TNFAIP3 genes have a higher proportion of central memory T cells.
Figure 8E shows that TIL cells edited in both SOCS1 and TNFAIP3 genes have a lower proportion of exhausted T cells.
Figure 8F shows that TIL cells edited in both SOCS1 and ZC3H12A genes have a higher proportion of central memory T cells.
Figures 8G-8H show that TIL cells edited in both SOCS1 and ZC3H12A genes have a lower proportion of exhausted T cells.
Figure 8I shows that TIL cells edited in both TNFAIP3 and CBLB genes have a lower proportion of exhausted T cells.
Figure 8J shows that TIL cells edited in both TNFAIP3 and ZC3H12A genes have a higher proportion of naive T cells.
Figure 8K shows that TIL cells edited in both TNFAIP3 and ZC3H12A genes have a higher proportion of central memory T cells.
Figures 8L-8M show that TIL cells edited in both TNFAIP3 and ZC3H12A genes have a lower proportion of exhausted T cells.
Figure 8N shows that TIL cells edited in both TNFAIP3 and SOCS1 genes have a higher proportion of stem cells.
Figure 8O shows that TIL cells edited in both TNFAIP3 and SOCS1 genes have a lower proportion of exhausted T cells.
Figure 9A shows that the TIL cells edited in both CBLB and ZC3H12A genes in the CD3 antibody stimulation group had a higher proportion of cytokine expression.
Figure 9B shows that the TIL cells after gene editing of SOCS1 and CBLB combination in the non-stimulation Medium group had a higher proportion of cytokine expression.
Figure 9C shows that TIL cells after SOCS1 and CBLB combination gene editing in the CD3 antibody stimulation group had a higher proportion of cytokine expression.
Figure 9D shows that TIL cells after gene editing of SOCS 1 and TNFAIP3 combination in the non-stimulation Medium group had a higher proportion of cytokine expression.
Figure 9E shows that TIL cells after gene editing of SOCS1 and TNFAIP3 combination in the CD3 antibody stimulation group had a higher proportion of cytokine expression.
Figure 9F shows that the TIL cells after gene editing of SOCS1 and ZC3H12A combination in the non-stimulation Medium group had a higher proportion of cytokine expression.
Figure 9G shows that the TIL cells after gene editing of SOCS1 and ZC3H12A combination in the CD3 antibody stimulation group had a higher proportion of cytokine expression.
Figure 9H shows that the TIL cells after gene editing of TNFAIP3 and CBLB combination in the non-stimulation Medium group had a higher proportion of cytokine expression.
Figure 9I shows that the TIL cells after gene editing of TNFAIP3 and CBLB combination in the CD3 antibody stimulation group had a higher proportion of cytokine expression.
Figures 9J-9K show that the TIL cells after gene editing of TNFAIP3 and ZC3H12A combination in the non-stimulation Medium group have a higher proportion of cytokine expression.
Figure 9L shows that TIL cells after gene editing of TNFAIP3 and ZC3H12A combination in the CD3 antibody stimulation group had a higher proportion of cytokine expression.
Figure 9M shows that TIL cells genetically edited with the combination of TNFAIP3 and SOCS1 co-cultured with autologous tumor organoids have higher cytokine release capacity.
Figure 10 shows the results of apoptosis detection of TIL cells derived from donor 504.

### Specific Embodiments

The implementation of the present invention will be described below with specific examples. Those skilled in the art can easily understand other advantages and effects of the present invention from the content disclosed in this specification.

### Definitions

In the present invention, the term "CBL family member" generally refers to a family member protein having an SH3 domain or a functionally active fragment thereof. For example, a CBL family member may include CBLB. For example, the UniProt number for a CBL family member could be Q13191. The CBL family members of the present invention may also encompass functionally active fragments thereof, without being limited to human and mammalian forms, conservative amino acid substitutions, glycoform modifications or variants, active fragments thereof, or processing and/or modifications thereof that occur in cells Substances containing functionally active fragments produced later. For example, the CBL family members of the present invention may include functionally active fragments thereof and other optional structural domains.

**In** the present invention, the term "STAT-induced STAT inhibitor (SSI) family member" generally refers to a family member protein having an SH2 domain or a functionally active fragment thereof. For example, a member of the STAT-inducible STAT inhibitor (SSI) family may include SOCS1. For example, the UniProt number for a member of the STAT-induced STAT inhibitor (SSI) family may be 015524. The STAT-induced STAT inhibitor (SSI) family members of the present invention may also include functionally active fragments thereof, which are not limited to human and mammalian forms, conservative amino acid substitutions, glycoform modifications or variants, active fragments thereof, or their expression in cells. A substance containing the functionally active fragment resulting from the processing and/or modification that occurs in the substance. For example, the STAT-induced STAT inhibitor (SSI) family members of the present invention may include functionally active fragments thereof and other optional structural domains.

**In** the present invention, the term "peptidase C64 family member" generally refers to a family member protein with a ubiquitin-binding domain or a functionally active fragment thereof. For example, a peptidase C64 family member may include TNFAIP3. For example, the UniProt number for a member of the peptidase C64 family may be P21580. The peptidase C64 family members of the present invention can also include functionally active fragments thereof, which are not limited to human and mammalian forms, conservative amino acid substitutions, glycoform modifications or variants, active fragments thereof, or their processing and/or processes that occur in cells or a modified substance containing the functionally active fragment. For example, the peptidase C64 family member of the present invention may include its functionally active fragment and other optional structural domains.

**In** the present invention, the term "ZC3H12 family member" generally refers to a family member protein having a C3H1-type zinc finger domain or a functionally active fragment thereof. For example, a ZC3H12 family member may include ZC3H12A. For example, the UniProt number for a ZC3H12 family member could be Q5D1E8. The ZC3H12 family members of the present invention may also encompass functionally active fragments thereof, which are not limited to human and mammalian forms, conservative amino acid substitutions, glycoform modifications or variants, active fragments thereof, or processing and/or modifications thereof that occur in cells Substances containing functionally active fragments produced later. For example, the ZC3H12 family members of the present invention may include functionally active fragments thereof and other optional structural domains.

In the present invention, the term "IKAROS zinc finger protein family member" generally refers to family member proteins having zinc finger domains or functionally active fragments thereof. For example, a member of the zinc finger protein family including IKAROS may include IKZF1. For example, the UniProt number for a member of the IKAROS zinc finger protein family could be Q13422. The IKAROS zinc finger protein family members of the present invention may also include functionally active fragments thereof, without being limited to human and mammalian forms, conservative amino acid substitutions, glycoform modifications or variants, active fragments thereof, or processes and/or modifications thereof that occur in cells, including the functional activity fragments of matter. For example, the IKAROS zinc finger protein family members of the present invention may include functionally active fragments thereof and other optional structural domains.

In the present invention, the term "tumor necrosis factor alpha-induced protein 3 (TNFAIP3)" generally refers to an inhibitory molecule of a signaling pathway. For example, TNFAIP3 can ubiquitinate signal transduction substances of the NF-κB pathway. For example, the UniProt accession number for TNFAIP3 could be P21580. In the present invention, TNFAIP3 may encompass unprocessed TNFAIP3, any form of processed TNFAIP3, variants of TNFAIP3 or substances containing functionally active fragments of TNFAIP3.

In the present invention, the term "GTPase-activating protein 1 family member" generally refers to a family member protein having a GTPase-activating domain or a functionally active fragment thereof. For example, a GTPase-activating protein 1 family member may include RASA2. For example, the UniProt number for a member of the GTPase-activating protein 1 family may be Q15283. The GTPase-activating protein 1 family members of the present invention can also include functionally active fragments thereof, which are not limited to human and mammalian forms, conservative amino acid substitutions, glycoform modifications or variants, active fragments thereof, or their processing in cells. and/or modified substances containing the functionally active fragment. For example, the GTPase-activating protein 1 family member of the present invention may include its functionally active fragment and other optional structural domains.

In the present invention, the term "FGF binding protein family member" generally refers to a family member protein having an FGF binding domain or a functionally active fragment thereof. For example, an FGF binding protein family member may include FIBP. For example, the UniProt number for a member of the FGF binding protein family may be O43427. The FGF-binding protein family members of the present invention may also encompass functionally active fragments thereof, not limited to human and mammalian forms, conservative amino acid substitutions, glycoform modifications or variants, active fragments thereof, or their processing and/or processes that occur in cells. Or a modified substance containing the functionally active fragment. For example, the FGF-binding protein family members of the present invention may include functionally active fragments thereof and other optional structural domains.

In the present invention, the term "Mediator (MED) family member" generally refers to a family member protein having a CDK8 binding domain or a functionally active fragment thereof. For example, a family member containing Mediator (MED) could contain MED12. For example, the UniProt number of a Mediator (MED) family member may be Q93074. The Mediator (MED) family members of the present invention can also include functionally active fragments thereof, which are not limited to human and mammalian forms, conservative amino acid substitutions, glycoform modifications or variants, active fragments thereof, or their processing and processing in cells. /or modified substances containing the functionally active fragment. For example, the Mediator (MED) family members of the present invention may include functionally active fragments thereof and other optional structural domains.

In the present invention, the term "immune cells" generally refers to cells involved in conducting innate and adaptive immune responses. For example, may include, but are not limited to, lymphocytes (such as T cells (including thymocytes) and B cells), natural killer (NK) cells, NKT cells, macrophages, monocytes, eosinophils, basophils cells, neutrophils, dendritic cells, and mast cells. In some embodiments, the modified immune effector cells are T cells, such as CD4+ T cells, CD8+ T cells (also known as cytotoxic T cells or CTL), regulatory T cells (Treg), Th1 cells, Th2 cells, Th17 cells, αβ T cells and/or γδ T cells. For example, the immune cells of the present invention also include immune cells derived from differentiation of stem cells. For example, the immune cells of the present invention also include immune cells derived from differentiation of pluripotent stem cells. For example, the stem cells of the present invention can be obtained by induction. For example, the above-mentioned stem cells of the present invention may include induced pluripotent stem cells (iPSC).

In the present invention, the term "chimeric antigen receptor" generally refers to an engineered antigen receptor. For example, a CAR may comprise an extracellular antigen binding domain fused via a hinge and transmembrane domain to a cytoplasmic domain comprising a signaling domain. In some embodiments, the CAR extracellular domain can bind to an antigen expressed by a target cell in an MHC-independent manner, resulting in activation and proliferation of the cell. In some embodiments, the extracellular domain of the CAR can recognize a tag fused to an antibody or antigen-binding fragment thereof. For example, a single CAR construct can be made to target multiple different antigens by replacing one antibody with another. In some embodiments, the extracellular domain of the CAR may comprise an antibody derived from antigen-binding fragments. Antigen binding domains useful in the present disclosure may include, for example, scFv, antibodies, antigen binding regions of antibodies, variable regions of heavy/light chains, and/or single chain antibodies.

In the present invention, the term "T cell receptor" generally refers to an engineered antigen receptor. For example, a TCR may comprise TCRα and/or TCRβ chains that have been isolated and cloned from a population of T cells that recognize a specific target antigen. For example, TCRα and/or TCRβ genes (i.e., TRAC and TRBC) can be obtained from T cell populations isolated from individuals with specific malignancies or from T cells that have been isolated from humanized mice immunized with specific tumor antigens or tumor cells, cloned from the population. Engineered TCRs can recognize antigens through the same mechanism as their endogenous counterparts (e.g., by recognizing their cognate antigens presented in the context of major histocompatibility complex (MHC) proteins expressed on the target cell surface), thereby can lead to activation and proliferation of TCR-engineered cells.

In the present invention, the term "gene regulatory system" generally refers to a system that regulates the expression or activity of a target gene. For example, a gene regulatory system may include gene regulatory molecules. For example, a gene regulatory system can regulate the expression or activity of a gene, such as placing the gene in an inactivated or activated state, increasing or decreasing the amount of the gene, increasing or decreasing the amount of transcription of the gene, and/or causing the gene to be in a state of inactivation or activation. The transcription product of the gene is in an inactive or activated state; for example, the gene regulatory system can regulate the expression or activity of the gene, such as increasing or decreasing the amount of the expression product of the gene in a single cell and/or causing the number of cells expressing the product of the gene to increase or decrease.

In the present invention, the term "guide nucleic acid molecule" generally refers to a nucleic acid molecule that can be used for gene editing. For example, guide nucleic acid molecules can provide information about nucleotide insertion or deletion, guiding the editing process. For example, the guide nucleic acid molecule can be a guide RNA (gRNA). For example, "gRNA" may refer to an RNA molecule that binds to a Cas protein and targets the Cas protein to a specific location within target DNA. For example, where hybridization between the gRNA and the DNA targeting sequence promotes the formation of the CRISPR complex, perfect complementarity may not be required, for example, as long as sufficient complementarity exists to cause hybridization and promote the formation of the CRISPR complex.

In the present invention, the term "enzyme protein" generally refers to a protein with enzymatic activity. For example, the enzyme protein may refer to Cas protein. For example, Cas proteins can contain at least one RNA recognition or binding domain that can interact with gRNA. Cas proteins may also include nuclease domains (e.g., DNase or RNase domains), DNA binding domains, helicase domains, protein-protein interaction domains, dimerization domains, and/or other structural areas. The nuclease domain may have catalytic activity for nucleic acid cleavage. Cleavage can involve the cleavage of covalent bonds in the nucleic acid molecule. The Cas protein can be a wild-type protein (ie, a naturally occurring protein), a modified Cas protein (ie, a Cas protein variant), or a fragment of a wild-type or modified Cas protein. Cas proteins can also be active variants or fragments of wild-type or modified Cas proteins. In the present invention, Cas protein may include unprocessed Cas protein, any form of processed Cas protein, variants of Cas protein, or substances containing functionally active fragments of Cas protein.

In the present invention, the term "ribonucleoprotein complex" generally refers to a complex formed between a protein and a nucleic acid. For example, proteins in ribonucleoprotein complexes can have nuclease activity. For example, a ribonucleoprotein complex can cleave a target sequence under the guidance of the nucleic acid within it. For example, the ribonucleoprotein complex may be a complex formed by Cas protein and gRNA.

In the present invention, the term "lipid nanoparticle (LNP)" generally refers to a lipidnucleic acid particle or nucleic acid-lipid particle. For example, LNP represents particles made of lipids (e.g., cationic lipids, noncationic lipids, and conjugated lipids that prevent particle aggregation) and nucleic acids (e.g., mRNA, gRNA, siRNA, aiRNA, miRNA, ssDNA, dsDNA, ssRNA, short hairpin RNA (shRNA), dsRNA, self-amplifying RNA or plasmids, including plasmids from which interfering RNA or mRNA is transcribed, are encapsulated in lipids. For example, the protein can be encapsulated in LNP, for example, the Cas protein known in the art can be encapsulated in LNP. For example, lipids in LNP include (1) "simple lipids," which include fats and oils as well as waxes; (2) "complex lipids," which include phospholipids and glycolipids; and (3) "derivatized lipids," such as steroids. For example, lipids in LNPs may also include lipid derivatives, such as lipids that are covalently or non-covalently bound to proteins or polypeptides. For example, the components in the LNP may also include polypeptide components. The polypeptide component can replace one or more lipid components in traditional LNP to maintain or improve the delivery ability of LNP.

In the present invention, the term "exon" generally refers to the portion of a gene that can be expressed as a protein. For example, an exon may refer to the ability to be expressed as a protein during protein biosynthesis. For example, splicing exon sequences of a target gene can reduce the activity or function of the target gene.

In the present invention, the term "intron" generally refers to a segment of DNA that does not encode part or all of the expressed protein. Normally under endogenous conditions, introns are transcribed into RNA molecules, but they are cleaved from the endogenous RNA before being translated into proteins. For example, editing at the location of an intron can reduce the activity or function of a target gene. For example, editing can reduce the activity or function of the target gene by targeting the junction of introns and exons, such as the intron region from about 0bp to about 100bp upstream or downstream of the exon, preferably about 0bp to about 20bp.

In the present invention, the term "start codon" generally refers to the unit of adjacent nucleotides ('codon') that genetically defines the start of protein synthesis (mRNA translation). For example, targeting the region 0bp to 1500bp upstream of the start codon, preferably 0bp to 100bp upstream of the start codon, for editing can reduce the activity or function of the target gene.

In the present invention, the term "protospacer adjacent motif (PAM)" generally refers to the short sequence following the target sequence. For example, when Cas9 performs site-specific cleavage of target DNA, the PAM sequence can be used to determine the location of the cleavage. For example, once the region of PAM is determined, those skilled in the art can easily determine the location of a suitable target sequence, and can easily design a gRNA sequence for cleaving the target sequence.

In the present invention, the term "reduced expression" generally refers to a decrease in the expression level of a product or its gene and/or a decrease in the proportion of cells capable of expressing the product (eg, at least about 5-100%). For example, it can be that the amount of the product expressed by the gene in the cell is reduced or that it contains the product expressed by the gene. The proportion of cells producing the product decreases, or the proportion of cells secreting the product expressed by the gene decreases. For example, the reduced expression of the gene can be indirectly expressed by detecting the knockout amount of the gene in the genome of the cell. For example, the reduced expression of a gene can be indirectly expressed by detecting the proportion of cells in a cell population in which the gene has been knocked out.

In the present invention, the term "activity" generally refers to the biological function of a substance. For example, the activity of a gene may refer to the transcriptional and/or translational state of the gene. For example, the weakened activity of a gene (eg, at least about 5-100%) may mean that the transcriptional function of the gene is weakened, the gene cannot be transcribed normally, or the function of the gene's transcript product is inhibited.

In the present invention, the term "CD80" generally refers to a cell stimulating molecule. For example, CD80 can be a ligand for CD28. For example, CD80 can be found in GenBank accession number P33681. The CD80 protein of the present invention may also include functionally active fragments thereof, and is not limited to substances containing functionally active fragments of CD80 produced after processing and/or modification occurring in cells. For example, the CD80 of the present invention may include functionally active fragments of CD80 and other optional structural domains.

In the present invention, the term "CD86" generally refers to a cell stimulating molecule. For example, CD86 can be a ligand for CD28. For example, CD86 can be found in GenBank accession number P42081. The CD86 protein of the present invention may also include functionally active fragments thereof, and is not limited to substances containing functionally active fragments of CD86 produced after processing and/or modification occurring in cells. For example, the CD86 of the present invention may include functionally active fragments of CD86 and other optional domains.

In the present invention, the term "secreted" generally refers to a substance that can be localized extracellularly. For example, secreted substances can be synthesized within the cell and transported to the extracellular space of the cell. For example, whether a substance is a secreted substance can be tested by an enzyme-linked immunosorbent assay or other detection method.

In the present invention, the term "T cell receptor" or "TCR" generally refers to a complex of membrane proteins involved in the activation of T cells in response to presentation of antigen. TCRs can be responsible for recognizing antigens bound to major histocompatibility complex molecules. TCR can consist of heterodimers composed of alpha (α) and beta (β) chains, or of gamma (γ) and delta (δ) chains. TCRs can exist in alpha/beta and gamma/delta forms, which are structurally similar but have unique anatomical locations and functions. For example, the TCR can be a modified TCR on any cell that expresses the TCR. For example, the type of TCR can be analyzed using a TCR subtype analysis reagent.

In the present invention, the term "clonal diversity" generally refers to a substance having multiple clonotypes. For example, clonal diversity of TCRs may mean that TCRs may have different sequence structures and/or antigen recognition capabilities. For example, the diversity of TCRs is often distinguished by β chain subtypes, which can include Vβ23, Vβ7.2, Vβ5.2, Vβ11, Vβ16, Vβ3, etc. When a T cell population has more β chain subtypes, this T cell population is believed to have higher clonal diversity.

In the present invention, "CD4⁺ Cells" usually refer to CD4-positive cells, which may be T cells, for example. The term "CD4⁺ cells" and "CD4-positive cells" may be used synonymously. These cells can be identified by methods known in the art, such as by staining the cells with fluorescently labeled antibodies against CD4 and using fluorescence-activated cell sorting. For example, there are data that can prove that increasing the proportion of CD4 + cells can increase the ability of the cell population to secrete IFN and/or TNF, and can improve the tumorsuppressing effect of the T cell population. For example, see Tay, R.E., Richardson, E.K. et al. (2020). Cancer Gene Therapy, 1-13. However, the art lacks a method to improve the CD4 + cell ratio; the present invention can provide a method that affects the CD4⁺ cell proportion.

In the present invention, "CD8⁺ Cells" usually refer to CD8-positive cells, which may be T cells, for example. The term "CD8⁺ cells" and "CD8-positive cells" may be used synonymously. These cells may be identified by methods known in the art, such as by staining the cells with fluorescently labeled antibodies directed against CD8 and using fluorescence-activated cell sorting.

In the present invention, the term "IC₅₀ value" usually refers to the concentration of a target substance required to obtain 50% inhibition of a biological process. The Cheng-Prusoff equation (Biochem. Pharmacol. (1973) 22:3099) can be used to convert the IC50 value into an absolute inhibition constant (Ki).

In the present invention, the term "K_{D} value" or "KD value"usually refers to the dissociation constant, which can be determined by surface plasmon resonance. Typically, surface plasmon resonance analysis uses the BIAcore system (Pharmacia Biosensor, Piscataway, NJ), by surface plasmon resonance (SPR). Measuring real-time binding interactions between ligands (substances immobilized on the biosensor matrix) and analytes (substances in solution) can also be achieved by immobilizing the analyte (substances immobilized on the biosensor matrix) and presenting them to ligands for surface plasmon analysis.

In the present invention, the term "encoding" generally refers to the ability to directly or indirectly infer the structure or composition information of another type of molecule related to it from the structure or composition information of one molecule according to basically determined rules. For example, the nucleotide sequence can be inferred from the sequence of an amino acid, such as from the properties of DNA-transcribing complementary nucleic acids, including nucleic acids that can be translated into polypeptides. For example, deoxyribonucleic acid may encode RNA transcribed from the deoxyribonucleic acid. Deoxyribonucleic acid may similarly encode a polypeptide translated from the RNA transcribed from the deoxyribonucleic acid.

As used herein, the term "small molecule compound" generally refers to peptides, peptidomimetics, amino acids, amino acid analogs, polynucleotides, polynucleotide analogs, nucleotides, nucleotide analogs, organic or inorganic substances having a molecular weight of less than about 10,000 g/mol (i.e., including heterologous organic substances and organometallic compounds), organic or inorganic substances with a molecular weight of less than about 5,000 g/mol, organic or inorganic substances with a molecular weight of less than about 1,000 g/mol, organic or inorganic substances with a molecular weight of less than about 500 g/mol, as well as salts, esters and other pharmaceutically acceptable forms of such drugs.

In the present invention, the term "NK cell" is also called "natural killer cell" and generally refers to a cell with large granules in the cytoplasm. NK cells are developed from bone marrow lymphoid stem cells and can differentiate and develop depending on the bone marrow or thymus microenvironment. In the present invention, the ratio of NK cells in TIL cells can be changed by the method of the present invention.

In the present invention, the term "antibody" generally refers to an immunoglobulin or fragment thereof or derivative thereof, encompassing any polypeptide including an antigen-binding site, whether produced in vitro or in vivo. The term includes, but is not limited to, polyclonal, monoclonal, monospecific, multispecific, nonspecific, humanized, single chain, chimeric, synthetic, recombinant, hybrid, mutant and grafted antibodies. Unless otherwise modified by the term "intact", as in "intact antibody", for the purposes of the present invention, the term "antibody" also includes antibody fragments, such as Fab, F(ab')2, Fv, scFv, Fd, dAbs and other antibody fragments that retain antigen-binding functionality (e.g., specifically bind CD3). Typically, such fragments should include an antigen-binding domain. The basic 4-chain antibody unit is a heterotetrameric glycoprotein composed of two identical light (L) chains and two identical heavy (H) chains. IgM antibodies are composed of 5 basic heterotetramer units and another polypeptide called J chain, and contain 10 antigen-binding sites, while IgA antibodies include 2-5 basic units that can combine with the J chain and polymerize to form a multivalent combined basic 4-chain unit. For IgG, a 4-chain unit is typically about 150,000 daltons. Each L chain is connected to the H chain by a covalent disulfide bond, while the two H chains are connected to each other by one or more disulfide bonds depending on the H chain isotype. Each H and L chain also has regularly spaced intrachain disulfide bridges. Each H chain has a variable domain (VH) at the N-terminus, followed by three constant domains (CH) for each of the α and γ chains, and four CH domains for the µ and ε isoforms. Each L chain has a variable domain (VL) at the N-terminus and a constant domain at its other end. VL corresponds to VH, and CL corresponds to the first constant domain (CH1) of the heavy chain. Specific amino acid residues are thought to form the interface between the light and heavy chain variable domains. VH and VL pair together to form a single antigen binding site. L chains from any vertebrate species can be classified into one of two distinct types, termed kappa and lambda, based on the amino acid sequence of their constant domains. Immunoglobulins can be divided into different classes or isotypes based on the amino acid sequence of the heavy chain (CH) constant domain. There are currently five classes of immunoglobulins: IgA, IgD, IgE, IgG, and IgM, with heavy chains named α, δ, ε, γ and µ, respectively.

In the present invention, the term "antigen-binding fragment" generally refers to one or more polypeptide fragments that have the ability to specifically bind an antigen. In the present invention, the antigen-binding fragment may include Fab, Fab', F(ab)2, Fv, F(ab')2, scFv, di-scFv and/or dAb.

In the present invention, the term "expression" generally refers to the transcription and/or translation process that occurs within a cell of a gene encoding a target polypeptide. The level of transcription of a gene encoding a polypeptide of interest in a host cell can be determined by measuring the amount of corresponding mRNA present in the cell. For example, the mRNA transcribed from the gene encoding the polypeptide of interest is quantitatively measured by PCR or by RNA hybridization. The translation level of a gene encoding a polypeptide of interest can be measured by a variety of methods, such as by ELISA, by polypeptide biological activity testing, or by Western blotting or radioimmunoassays. In the present invention, the term "expression" may also generally refer to the process of transcription and/or translation in which a product occurs. For example, expression of a cytokine can be a process by which a cell transcribes and/or translates the cytokine. For example, the expression of a cytokine can be determined by detecting the amount of the corresponding mRNA present in the cell or detecting the amount of the cytokine produced by the cell, or both.

In the present invention, the "stage" in the terms "one stage of in vitro amplification", "single stage of in vitro amplification", or "first stage of in vitro amplification" generally refers to a period of amplification of TIL in vitro. In one embodiment, each stage can be divided by changes in the number of TIL cells. In one embodiment, when the number of TIL cells increases at least about 1-fold, the TIL cells can be considered to have entered the next stage of in vitro amplification. In some embodiments, when the number of TIL cells increases at least about 1-50 times, such as at least about 1-fold, at least about 2-fold, at least about 3-fold, at least about 4-fold, at least about 5-fold, at least about 6-fold, at least about 7 times, at least about 8 times, at least about 9 times, at least about 10 times, at least about 11 times, at least about 12 times, at least about 13 times, at least about 14 times, at least about 15 times, at least about 20 times, at least about 30 times, at least about 40 times, or at least about 50 times, the TIL cells can be considered to have entered the next stage of in vitro expansion. In one embodiment, each stage can also be divided by the conditions of TIL cell culture. In one embodiment, when T cell activators and/or T cell growth factors are added or supplemented in the cell culture medium, the TIL cells can be considered to have entered the next stage of in vitro expansion. In one embodiment, after the TIL cells have been centrifuged and/or washed, the TIL cells can be considered to have entered the next stage of in vitro expansion. In one embodiment, each stage can also be divided by the number of days of TIL cell culture. In one embodiment, when the TIL cells are cultured in vitro for about 1-100 days, such as about 1 day, about 2 days, about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20 days, about 30 days, about 40 days, about 50 days or about 100 days, it can be considered that TIL cells have entered the next stage of in vitro expansion.

In the present invention, the term "first-stage in vitro expansion" generally refers to the stage of expansion using T cell growth factors after primary TILs are obtained from tissues. In one embodiment, the tissue of the present invention can be selected from the following group: tumor tissue, tumor-associated lymph nodes with or without tumor metastasis, tumor metastasis, fragments of paracancerous tissue, pleural effusion and/or peritoneal effusion. The pleural effusion of the present invention may be the pleural effusion of patients with metastatic cancer. In one embodiment, the amplification of the present invention can be in vivo amplification performed autologously or allogeneically, or can be in vitro amplification. The first stage of in vitro amplification of the present invention may also be called the preREP (pre-rapid amplification) stage. For example, TILs derived from tumor tissue and not expanded in vitro may be referred to as the first TIL population. For example, the TILs obtained through the first stage of in vitro amplification in the two-step culture method of the present invention can be called the second TIL population.

In the present invention, the term "second-stage in vitro expansion" generally refers to the stage in which tissue is removed from the subject and expanded, and then expanded again. In one embodiment, compared with the TIL expanded in vitro in the first stage, the number of TIL cells expanded in vitro in the second stage of the present invention is increased, for example, it can be increased by at least about 10 times (or at least about 20, 30, 40, 50, 60, 70, 80 or 90-fold), or in one embodiment the number of cells can be increased at least about 100-fold. In one embodiment, the culture conditions of the second stage of in vitro amplification may be different from those of the first stage of in vitro amplification, for example, the added culture substances may be different. For example, in the two-step culture method of the present invention, the second stage of in vitro amplification may also be called the REP (rapid amplification) stage. For example, the TILs obtained through the second stage of in vitro amplification in the two-step culture method of the present invention can be called the third TIL group.

In the present invention, the term "in vivo" generally refers to events occurring within the body of a subject.

In the present invention, the term "in vitro" generally refers to events that occur outside the body of a subject.

In the present invention, the term "ex vivo" generally refers to events involving treatment or surgery on cells, tissues and/or organs that have been removed from the body of a subject. In one embodiment, the cells, tissues and/or organs can be returned to the subject's body through surgery or treatment.

In the present invention, the term "secretory capacity" generally refers to the ability of a cell to express a polypeptide or protein and to transfer the polypeptide or protein of the invention to the extracellular environment.

In the present invention, the term "irradiation" generally refers to the treatment of substances by radiation. For example, in one embodiment, irradiating may refer to irradiating a substance by X-rays, alpha-rays, beta-rays, or gamma-rays.

In the present invention, the term "engineered cell" generally refers to a cell that has been genetically modified by adding additional genetic material in the form of DNA or RNA to the total genetic material of the cell. In one embodiment, engineered cells can be genetically modified to express T cell activators and/or T cell growth factors of the invention.

In the present invention, the term "co-culture" generally refers to the cultivation of two or more different populations of cells with some degree of contact between them. The "contact" of two or more different populations of cells in the present invention can, in one embodiment, be through direct contact, that is, the cells of one population are in direct physical contact with the cells of another population. Or in one embodiment indirect contact may be mediated by a shared culture medium. The shared culture medium of the present invention may contain metabolites produced and released by at least one population of co-cultured cells and used to culture another population of cells.

In the present invention, the term "contacting" generally means that two or more substances of different types are brought into contact together in any order, in any manner and for any length of time. In one embodiment, by direct contact, for example, one or more feeder cells, T cell activators and/or T cell growth factors can be added to the culture medium of the TIL cells, for example, one or more feeder cells, T cell activators and/or T cell growth factors can be added to the culture medium of the TIL cells. The culture medium of TIL cells, T cell activators and/or T cell growth factors is added to and/or replaced with the culture medium of the TIL cells. For example, one or more feeder cells, T cell activators and/or T cell growth factors can be added to the culture medium of the TIL cells. Culture medium containing these factors is used for the culture of TIL cells; in one embodiment, it can be through indirect contact, for example, metabolic products from feeder cells can be produced and released for culturing TIL cells.

In the present invention, the terms "contact simultaneously", "contact together", "contact simultaneously with", "simultaneously" and "commonly" generally refer to the administration of two or more substances to a subject and/or cells such that the substances are both present in the subject and/or in the environment of the cell culture. Simultaneous exposure may include simultaneous administration in different compositions, administration in different compositions at different times, or administration in a composition in which more than two active pharmaceutical ingredients are present. For example, "contacting at the same time" in the present invention may generally mean contacting at substantially the same time.

In the present invention, the term "expansion" generally refers to a several-fold increase in the number of cells over a period of time. In one embodiment the number of cells can be increased at least about 3-fold (or 4, 5, 6, 7, 8 or 9-fold), in one embodiment the number of cells can be increased at least about 10-fold (or 20, 30, 40, 50, 60, 70, 80 or 90-fold), or in one embodiment the number of cells can be increased at least about 100-fold. In the present invention, the term "expanded" generally means that the cells of the invention have undergone one or more of the above-mentioned amplifications.

In the present invention, the term "polymer" generally refers to a molecule consisting of individual chemical moieties linked together. The polymer moieties of the present invention may be the same or different. In one embodiment, the term "polymer" may refer to individual chemical moieties joined end to end to form linear molecules, as well as individual chemical moieties linked together in branched (such as "multi-arm" or "star") structures. In one embodiment the polymer may include, for example, polysaccharides, dextran, hydrogels, polyethylene glycols, or poloxamer. Poloxamers are nonionic triblock copolymers with a central hydrophobic chain of polyoxypropylene (poly(propylene oxide)) flanked by two hydrophilic chains of polyoxyethylene (poly(ethylene oxide)). The materials encompassed by the present invention may be formulated with, or administered with, any polymer described herein or known in the art.

In the present invention, the term "chimeric antibody" generally refers to an antibody in which the variable region of a murine antibody is fused with the constant region of a human antibody, which can reduce the immune response induced by the murine antibody. To create chimeric antibodies, hybridomas that secrete mouse-derived specific monoclonal antibodies can be established, and then the variable region genes can be cloned from mouse hybridoma cells. To clone the constant region gene of a human antibody, the mouse variable region gene and the human constant region gene are connected to form a chimeric gene and then inserted into an expression vector. The chimeric antibody molecule can be expressed in a eukaryotic system or a prokaryotic system.

In the present invention, the term "humanized antibody", also known as CDR-grafted antibody, usually refers to transplanting the mouse CDR sequence into the human antibody variable region framework, that is, different types of antibodies produced within the framework sequences of human germline antibodies. It can overcome the heterologous reaction induced by chimeric antibodies carrying a large amount of mouse protein components. Such framework sequences can be obtained from public DNA databases or published references that include germline antibody gene sequences. For example, the germline DNA sequences of human heavy and light chain variable region genes are available in the "VBase" human germline sequence database.

In the present invention, the term "fully human antibody" is also called "fully human monoclonal antibody", and the variable region and constant region of the antibody are both of human source, eliminating immunogenicity and toxic side effects. The development of monoclonal antibodies has gone through four stages, namely: murine monoclonal antibodies, chimeric monoclonal antibodies, humanized monoclonal antibodies and fully human monoclonal antibodies. The antibody or ligand of the present invention may be a fully human monoclonal antibody. Relevant technologies for the preparation of fully human antibodies can include: human hybridoma technology, EBV-transformed B lymphocyte technology, phage display technology (phage display), transgenic mouse antibody preparation technology (transgenic mouse), and single B cell antibody preparation technology.

In the present invention, the term "CDR" generally refers to one of the six hypervariable regions within the variable domain of an antibody that primarily contribute to antigen binding. One of the most commonly used definitions of the 6 CDRs can be provided by Kabat E.A. et al., Chothia et al. and MacCallum et al. As used in the present invention, the Kabat definition of CDR can be applied to CDR1, CDR2 and CDR3 of the light chain variable domain (CDR L1, CDR L2, CDR L3 or L1, L2, L3), as well as the heavy chain variable domain CDR1, CDR2 and CDR3 (CDR H1, CDR H2, CDR H3 or H1, H2, H3).

In the present invention, the term "IL-2" or "IL2" generally refers to a T cell growth factor; it includes all forms of IL-2 and may include in one embodiment human and mammalian forms, conservative amino acid substitutions, glycoform modifications or variants, or active fragments thereof. The GeneID of the gene encoding IL-2 may be 3558.

In the present invention, the term "antigen-presenting cell" or "APC" generally refers to an immune cell that displays on its surface an exogenous antigen complexed with a major histocompatibility complex (MHC). Systemic cells, for example, include helper cells (e.g., B cells, dendritic cells, etc.). T cells can recognize these complexes using their T cell receptors (TCRs). APCs can process antigens and present them to T cells. In one embodiment, the antigen-presenting cells may include selected from the group consisting of peripheral mononuclear cells, dendritic cells, and artificial antigen-presenting cells.

In the present invention, the term "TIL characteristics" generally refers to the characteristics of TIL cells obtained by the culture method of the present invention. Changes in TIL characteristics can include: increased TIL cell number, increased proportion of viable cells, increased survival ability, improved proportion of T cell subpopulations, increased cytokine secretion ability, increased tumor cell killing ability in vitro, increased in vivo Tumor killing capacity, increased T cell receptor (TCR) clonal diversity and increased TIL cell numbers in tissues, or any combination thereof. Variations of the present invention may be improvements or decreases.

In the present invention, the term "persistence" generally refers to the presence of cells in vitro and/or in the body of a subject. For example, an increase in the survival ability of TIL cells may refer to an increase in the time that TIL cells exist in the body. For example, increased viability may refer to an increase in the time a cell exists within a subject's tissue, such as a tumor, spleen, bone marrow, lung tissue, and blood. For example, the increase in survival ability can be an increase in the survival ability of TIL cells after IL-2 is removed from the culture medium.

In the present invention, the term "artificial antigen-presenting cells" generally refers to artificially constructed immune cells for presenting exogenous antigens. For example, the way of presenting exogenous antigens can be that the surface of the artificial antigen-presenting cells contains exogenous antigens complexed with a major histocompatibility complex (MHC). In one embodiment, isolated artificial antigen presenting cells (aAPC) may be included, which may include expression of HLA-A/B/C (the GeneID of the gene encoding it may be 3105, 3106 or 3107), CD64 (the GeneID of the gene encoding it can be 2209), CD80 (the GeneID of the gene encoding it may be 941), ICOS-L (the GeneID of the gene encoding it may be 23308) and CD58 (the GeneID of the gene encoding it may be 965) cells, and may be modified to express more than one T cell activator.

As used herein, the term "fusion protein" generally refers to an amino acid sequence containing a first polypeptide or protein, or a fragment, analog or derivative thereof, and a heterologous polypeptide or protein (i.e., different from the first polypeptide or protein or of a second polypeptide or protein or a fragment, analog or derivative thereof, or that is generally not part of the first polypeptide or protein or a fragment, analog or derivative thereof) of the amino acid sequence Peptide or protein. In some cases, a fusion protein may comprise a prophylactic or therapeutic drug fused to a heterologous protein, polypeptide or peptide. Among others, the heterologous proteins, polypeptides or peptides of the invention may or may not be different types of preventive or therapeutic drugs. For example, two different proteins, polypeptides or peptides with immunomodulatory activity can be fused together to form a fusion protein. In some cases, the fusion protein may retain or have increased activity compared to the activity of the original polypeptide or protein prior to fusion of the heterologous protein, polypeptide, or protein.

In the present invention, the term "killing ability" generally refers to killing target cells by contacting the cells of the present invention with an effective amount of a substance. In one embodiment, the agent of the invention may be a TIL cell. Killing of the present invention may include killing cells by itself or by promoting CDC, apoptosis, ADCC and/or phagocytosis of other cells or substances, or by a combination of two or more of these mechanisms.

As used herein, the term "administration" or "administering" generally refers to the delivery of a substance to a subject in need thereof by any route known in the art. Pharmaceutical carriers and formulations or compositions are also well known in the art. Routes of administration may include: intravenous, intramuscular, intradermal, subcutaneous, transdermal, mucosal, intratumoral and/or mucosal.

In the present invention, the term "kit" generally refers to two or more components packaged together in a container, receptacle or other container, one of which corresponds to the substance of the present invention. For example, TIL cells of the invention are included.

In the present invention, the term "subject" generally refers to a cell or an animal, which may be a mammal, such as a human, a non-human primate (ape, gibbon, gorilla, chimpanzee, orangutan, macaque), a domestic animal (dog and cats), farm animals (poultry such as chickens and ducks, horses, cattle, goats, sheep, pigs) and laboratory animals (mice, rats, rabbits, guinea pigs). Human subjects include fetal, neonatal, infant, adolescent, and adult subjects. Subjects include animal disease models, such as tumor animal models, and other animal models known to those skilled in the art.

In the present invention, the term "feeder" generally refers to cultured cells that can be used to support the growth of cells for another purpose. For example, this can be achieved by growing in vitro and secreting at least one factor into the culture medium. In one embodiment, feeder cells may include antigen-presenting cells.

In the present invention, the term "specific binding" generally refers to a binding substance that recognizes a specific target substance but does not substantially recognize or bind to other molecules in the sample. For example, if a binding substance can specifically bind to a specific target substance of the invention from one species, the binding substance of the invention can also specifically bind to a target substance of the invention from one or more other species. or homologous target substances. This interspecies reactivity may not by itself alter the classification of the binding substance as specific. In some cases, a binding substance that specifically binds to a target substance may also bind to a different allelic form of the target substance.

In the present invention, the term "complete culture process" generally refers to the complete process starting from the isolation of cells from tumor tissue isolated from a patient, through one or more amplifications, and finally obtaining cells that can be administered to a subject..

In the present invention, the term "cell culture medium" generally refers to the nutrient solution in which cells, such as mammalian cells, are grown. The preparation of cell culture media is well known in the art. Typically, cell culture media includes buffers, salts, carbohydrates, amino acids, vitamins, and necessary trace elements. Cell culture media may or may not contain serum, peptone and/or protein. Cell culture media can be supplemented with additional components or increased concentrations of components such as amino

Acids, salts, sugars, vitamins, hormones, growth factors, buffers, antibiotics, lipids, trace elements, etc., depending on the requirements of the cells to be cultured and/or the desired cell culture parameters.

In the present invention, the term "pharmaceutical composition" or "pharmaceutical preparation" generally refers to a preparation that allows the biological activity of the active ingredient to be effective and that does not contain substances that are harmful to the recipient to whom the preparation is to be administered. Additional ingredients that are unacceptably toxic to testers. Such preparations are sterile. "Pharmaceutically acceptable" excipients (carriers, additives) are those which can be reasonably administered to a subject mammal to provide an effective dose of the active ingredient employed.

As used herein, the term "tumor-infiltrating lymphocytes" or "TILs" generally refers to a population of cells originally obtained as white blood cells that have left the subject's bloodstream and migrated into the tumor. TILs may include but are not limited to
CD8 "Cytotoxic T cells (lymphocytes), Th1 and Th17CD4⁺ T cells, natural killer cells, dendritic cells and M1 macrophages. TILs may include primary TILs and secondary TILs. "Primary TILs" can be those TIL cells obtained from a subject's tissue sample, and "secondary TILs" can be any population of TILs that have been expanded or expanded in the present invention. In some embodiments, the tumor-infiltrating lymphocytes of the present invention may not be isolated and purified, or may infiltrate with tumor cells. In one embodiment, the TIL of the present invention may refer to a TIL group.

In the present invention, the term "central memory T cells" generally refers to T cells with long-term memory and capable of receiving antigen restimulation. Central memory T cells can have CD45RO⁺ CD62L⁺ The phenotype can, for example, be expressed through CD45RO⁺ and CD62L⁺ to identify central memory T cells. Central memory T cells can have stronger anti-tumor growth capabilities than ordinary T cells.

In the present invention, the term "regulatory T cells" generally refers to a subset of T cells that control autoimmune reactivity in the body. Regulatory T cells can have CD4⁺ CD25⁺ Foxp3⁺ The phenotype can be, for example, via CD4⁺ , CD25⁺ and Foxp3⁺ to identify regulatory T cells. Regulatory T cells can have the ability to inhibit the anti-tumor growth of T cells.

In the present invention, the term "activated T cells" generally refers to T cells that have been activated to have the ability to resist tumor growth. Activated T cells can have PD-1⁺ (PD1⁺ ), LAG-3⁺ (LAG3⁺) or CD28⁺ The phenotype, for example, can be through PD-1⁺ ,LAG-3⁺ or CD28⁺ to identify activated T cells. Activated T cells can have the ability to fight tumor growth.

In the present invention, the term "tumor-specific T cells" generally refers to T cells that can specifically resist tumor growth. Tumor-specific T cells can have CD103⁺ CD39⁺ The phenotype, for example, can be determined by CD103⁺ and CD39⁺ to identify tumor-specific T cells. Tumor-specific T cells can have more specific anti-tumor growth capabilities than ordinary T cells.

In the present invention, the term "stem cell-like T cells" generally refers to a type of T cells that may have the potential to self-proliferate and/or differentiate. For example, cells with differentiation potential and/or sustained proliferation ability may be considered stem cell-like cells in the present invention. For example, naive T cells (CD45RO ⁻ CD62L ⁺) can be considered stem cell-like cells. For example, naive T cells can have CD45RO⁻
CD62L⁺ phenotype. For example via CD45RO⁻ CD62L⁺ to identify stem cell-like T cells. For example, it can be through CD39⁻ CD69⁻ to identify stem cell-like T cells. For example, stem cell-like T cells can have TCF1⁺ The phenotype can be, for example, via TCF1⁺ to identify stem cell-like T cells. Stem cell-like T cells can have stronger and/or longer-term anti-tumor growth capabilities than ordinary T cells.

In the present invention, the term tumor "fragments" generally refers to tumor fragments that can be formed by mechanical fragmentation, enzymatic hydrolysis and/or other fragmentation methods after tumor tissue is removed from the subject's body.

In the present invention, the term "composition" or "pharmaceutical composition" generally refers to at least one cell and at least one and optionally more than one other pharmaceutically acceptable chemical components such as carriers, stabilizers , diluents, dispersants, suspending agents, thickeners and/or excipients.

In the present invention, the term "pharmaceutically acceptable carrier" generally refers to one or more non-toxic materials that does not interfere with the active ingredient. For example, a pharmaceutically acceptable carrier may not interfere with the biological activity of the active ingredient; for example, a pharmaceutically acceptable carrier may not interfere with the effectiveness of the biological activity possessed by the active ingredient. Such preparations may conventionally contain salts, buffers, preservatives, compatible carriers, and optionally other therapeutic agents. Such pharmaceutically acceptable preparations may also contain compatible solid or liquid fillers, diluents or encapsulating materials suitable for administration to humans. Other contemplated carriers, excipients, and/or additives that may be used in the formulations described herein may include: for example, flavoring agents, antimicrobial agents, sweeteners, antioxidants, antistatic agents, lipids, Protein excipients (such as serum albumin, gelatin, casein), salt-forming counterions (such as sodium), etc. These and other known pharmaceutical carriers, excipients and/or additives suitable for use in the formulations described herein are known in the art. In the present invention, "pharmaceutically acceptable carrier" can be understood as a vector that does not include nucleic acid forms used in genetic engineering.

In the present invention, the term "functionally active fragment" generally refers to a fragment that has a partial region of a full-length protein or nucleic acid, but retains or partially retains the biological activity or function of the full-length protein or nucleic acid. For example, a functionally active fragment may retain or partially retain the ability of the full-length protein to bind another molecule.

In the present invention, the term "T cell activator" generally refers to a substance that binds to the corresponding binding receptor on T cells and mediates T cell costimulatory responses. T cell activators can be substances other than antigen receptors that are required by T cells to mount an effective immune response. T cell activators may refer to T cell costimulatory molecules. For example, the T cell activator of the present invention may comprise a variant, a homolog thereof, or any substance comprising a functionally active fragment thereof. T cell activators may include, but are not limited to, MHC class I molecules, TNF receptor proteins, immunoglobulin-like proteins, cytokine receptors, integrins, signaling lymphocyte activation molecules (SLAM proteins), NK cell activation receptors, BTLA (GeneID of the gene encoding it can be 151888), Toll ligand receptor, OX40 (GeneID of the gene encoding it can be 7293), CD2 (GeneID of the gene encoding it can be 914), CD7 (GeneID of the gene encoding it can be is 924), CD27 (the gene encoding it can have a GeneID of 939), CD28 (the gene encoding it can have a GeneID of 940), CD30 (the gene encoding it can have a GeneID of 943), CD40 (the gene encoding it can have a GeneID of 958), CDS, ICAM-1 (GeneID encoding it can be 3383), LFA-1 (CD11a/CD18) (GeneID encoding it can be 3689), 4-1BB (CD137) (GeneID encoding it The GeneID of the gene can be 3604), B7-H3 (The GeneID of the gene encoding it can be 80381), ICOS (CD278) (The GeneID of the gene encoding it can be 29851), GITR (The GeneID of the gene encoding it can be 8784), BAFFR (GeneID of the gene encoding it can be 115650), LIGHT (GeneID of the gene encoding it can be 8740), HVEM (LIGHTR) (GeneID of the gene encoding it can be 8764), KIRDS2 (GeneID of the gene encoding it can be 100132285), SLAMF7 (the gene encoding it can have a GeneID of 57823), NKp80 (KLRF1) (the gene encoding it can have a GeneID of 51348), NKp44 (the gene encoding it can have a GeneID of 9436), NKp30 (the gene encoding it can have a GeneID of 259197) , NKp46 (GeneID of the gene encoding it can be 9437), CD19 (GeneID of the gene encoding it can be 930), CD4 (GeneID of the gene encoding it can be 920), CD8α (GeneID of the gene encoding it can be 925), CD8β (GeneID of the gene encoding it can be 926), IL-2Rβ, IL-2Rγ, IL7Rα (GeneID of the gene encoding it can be 3575), ITGA4 (GeneID of the gene encoding it can be 3676), VLA1 (GeneID of the gene encoding it It can be 3672), CD49a (the gene GeneID encoding it can be 3672), IA4 (the gene encoding it GeneID can be 3732), CD49D (the gene encoding it GeneID can be 3676), ITGA6 (the gene encoding it GeneID can be 3655), VLA-6 (the gene GeneID encoding it can be 3655), CD49f (the gene encoding it GeneID can be 3655), ITGAD (the gene encoding it GeneID can be 3681), CD11d (the gene encoding it GeneID can be 3681), ITGAE (the gene encoding it can have a GeneID of 3682), CD103 (the gene encoding it can have a GeneID of 3682), ITGAL (the gene encoding it can have a GeneID of 3683), CD11a (the gene encoding it can have a GeneID of 3683) , LFA-1 (GeneID of the gene encoding it can be 3683), ITGAM (GeneID of the gene encoding it can be 3684), CD11b (GeneID of the gene encoding it can be 3684), ITGAX (GeneID of the gene encoding it can be 3687) , CD11c (GeneID of the gene encoding it can be 3687), ITGB1 (GeneID of the gene encoding it can be 3688), CD29 (GeneID of the gene encoding it can be 3688), ITGB2 (GeneID of the gene encoding it can be 3689), CD18 (GeneID of the gene encoding it can be 3689), LFA-1 (GeneID of the gene encoding it can be 3689), ITGB7 (the gene GeneID encoding it can be 3695), NKG2D (the gene encoding it GeneID can be 22914), NKG2C (the gene encoding it GeneID can be 3822), TNFR2 (the gene encoding it GeneID can be 7133), TRANCE/RANKL (the gene GeneID encoding it can be 8600), DNAM1 (CD226) (the gene encoding it GeneID can be 10666), SLAMF4 (CD244, 2B4) (the gene encoding it GeneID can be 51744), CD84 (GeneID of the gene encoding it can be 8832), CD96 (Tactile) (GeneID of the gene encoding it can be 10225), CEACAM1 (GeneID of the gene encoding it can be 634), CRTAM (GeneID of the gene encoding it can be 56253), Ly9 (CD229) (GeneID of the gene encoding it can be 4063), CD160 (BY55) (GeneID of the gene encoding it can be 11126), PSGL1 (GeneID of the gene encoding it can be 6404), CD100 (SEMA4D) (GeneID of the gene encoding it can be 6404), CD100 (SEMA4D) (GeneID of the gene encoding it can be 11126) The gene GeneID can be 10507), CD69 (the gene encoding it can have a GeneID of 969), SLAMF6 (NTB-A, Ly108) (the gene encoding it can have a GeneID of 114836), SLAM (SLAMF1, CD150, IPO-3) (encoding Its gene GeneID can be 6504), BLAME (SLAMF8) (the gene encoding it can have a GeneID of 56833), SELPLG (CD162) (the gene encoding it can have a GeneID of 6404), LTBR (the gene encoding it can have a GeneID of 4055) , LAT (the gene GeneID encoding it can be 27040), GADS (the gene encoding it GeneID can be 9402), SLP-76 (the gene encoding it GeneID can be 3937), PAG/Cbp (the gene encoding it GeneID can be 55824), CD19a, a ligand that specifically binds CD3, a ligand that specifically binds CD28, a ligand that specifically binds HVEM, a ligand that specifically binds CD40L, a ligand that specifically binds OX40, and a ligand that specifically binds 4- Ligand for 1BB. A costimulatory intracellular signaling domain may refer to the intracellular portion of a T cell activator. The intracellular signaling domain may comprise the entire intracellular portion of the molecule derived therefrom or the entire native intracellular signaling domain or a functional fragment thereof.

In the present invention, the term "T cell growth factor" generally refers to a biologically active polypeptide or small molecule compound that causes cell proliferation. For example, the T cell growth factors of the present invention may comprise variants, homologs or anything comprising functionally active fragments thereof. In one embodiment, the T cell growth factor may be selected from one or more of the following group: IL-2 (GeneID encoding it can be 3558), IL-4 (GeneID the gene encoding it can be 3565), IL-6 (GeneID encoding it can be 3569), IL-7 (GeneID encoding it can be 3574), IL -10 (GeneID encoding it can be 3586), IL-12 (GeneID encoding it can be 3592 or 3593), IL-15 (GeneID encoding it can be 3600), IL-21 (GeneID encoding it can be 59067), TNF-α (GeneID encoding it can be 100137091), gamma interferon (GeneID encoding it can be 3458), GZMB (GeneID encoding it can be 3002), CD107a (GeneID encoding it can be 6499) and so on.

In the present invention, the term "substantially simultaneously" generally means that TIL can be contacted with two or more substances simultaneously during a period of time during the contact process, but it is not limited to that TIL is always contacted with two or more substances simultaneously during the entire contact process. touch. In one embodiment, substantially simultaneously can mean that the TIL can be with at least 10-95%, such as at least 10%, 20%, 30%, 40%, 50%, 60%, 70%, 75% over a period of time , 80%, 85%, 90%, 95% of each substance of two or more substances are in contact at the same time.

In the present invention, the term "dendritic cells" generally refers to antigen-presenting cells present in vivo, in vitro, ex vivo or within a host or subject or which may be derived from hematopoietic stem cells or monocytes. Dendritic cells and their precursors can be isolated from various lymphoid organs such as spleen, lymph nodes, as well as bone marrow and peripheral blood. Dendritic cells of the present invention may have characteristic morphology, such as lamellae (lamellipodia) extending in multiple directions of the dendritic cell body. Typically, dendritic cells can express high levels of MHC and costimulatory (eg, B7-1 and B7-2) molecules. Dendritic cells can induce antigen-specific differentiation of T cells in vitro and can elicit primary T cell responses in vitro and in vivo.

In the present invention, the term "in vitro expansion" generally refers to being cultured to produce changes in the number of cells. The expanded cells can also produce changes in the number and/or proportion of cells, changes in secretion capacity, changes in killing capacity or expression. changes in abilities, or any combination thereof. Variations of the present invention may be improvements or decreases. In the present invention, in vitro amplification can be for the purpose of amplification; to detect the function of TIL cells, for example, to detect the release of cytokines by TIL cells. However, the steps performed on TIL cells (for example, adding more than one substance to the culture medium of TIL cells to detect the ability of TIL cells to release cytokines) may not belong to the in vitro expansion of the present invention.

In the present invention, the term "peripheral mononuclear cells" or "peripheral blood mononuclear cells" generally refers to cells with a single nucleus in peripheral blood. For example, in the present invention, the peripheral blood mononuclear cells of the present invention may include lymphocytes, monocytes and/or dendritic cells.

In the present invention, the term "cytokine" generally refers to a protein released by one cell population that acts as an intercellular regulator on another cell. Cytokines of the present invention may be lymphokines, monokines and polypeptide hormones. Cytokines of the present invention may include interleukins (ILs) such as IL-1, IL-1α, IL-2, IL-3, IL-4, IL-5, IL-6, IL-7, IL-8, IL-9, IL-10, IL-11, IL-15, IL-21 and/or IL-12. In the present invention, the term cytokine may include proteins from natural sources or from recombinant cell culture, biologically active equivalents of native sequence cytokines, and functionally active fragments thereof.

In the present invention, the term "diameter" generally refers to the diameter of a cross-section of the substance of the invention. For example, when the substance of the invention is not spherical, the term "diameter" generally refers to the maximum diameter and/or the mean diameter of the largest cross-section of the substance of the invention. The method for determining the diameter of a substance may be a method commonly used in the art, such as transmission electron microscopy.

In the present invention, the term "tumor" generally refers to any new pathological tissue proliferation. The tumors of the present invention may be benign or malignant. The tumors of the present invention may be solid or hematological. The term "tumor" may be selected from one or more of the following group: melanoma, ovarian cancer, cervical cancer, lung cancer, bladder cancer, breast cancer, head and neck cancer, pancreatic cancer, liver cancer, gastric cancer, colorectal cancer, and renal cancer.

In the present invention, the term "tumor tissue" generally refers to a sample from a tumor in a subject, including any solid tumor and/or any tissue that is not a solid tumor in the subject.

In the present invention, the term "T cell subset proportion" generally refers to the proportion of different T cell subsets in TIL cells or TIL populations. For example, different T cell subsets of the present invention have different immune activities and/or differentiation abilities. For example, T cell subsets of the invention can be distinguished based on T cell surface markers. For example, central memory T cells can have CD45RO⁺ CD62L⁺ phenotype. For example, naive T cells can have CD45RO⁻ CD62L⁺ phenotype. For example, regulatory T cells can have CD4⁺ CD25⁺ Foxp3⁺ phenotype. For example, activated T cells can have CD25⁺ , CD28⁺ , PD-1⁺ or 41BB⁺ phenotype. For example, tumor-specific T cells can have CD103⁺ CD39⁺ phenotype. For example, stem cell-like T cells can have TCF1⁺ phenotype.

In the present invention, the term "TIL cell number" generally refers to the number of cells in the TIL cells of the invention. In the present invention, the number of TIL cells may refer to the number of cells in the TIL population obtained at any stage of the present invention. For example, the number of TIL cells may refer to the number of cells of the first TIL population derived from tumor tissue and not expanded in vitro. For example, the number of TIL cells may refer to the number of cells of the second TIL population expanded in vitro in the first stage. For example, the number of TIL cells may refer to the number of cells of the third TIL population expanded in vitro in the second stage. For example, the number of TIL cells may refer to the TIL cells finally obtained by any culture method of the present invention. In the present invention, the number of TIL cells can be measured by methods commonly used in the art, which may include, but are not limited to, manual cell counting with a cell counting plate and/or counting with an automatic cell counter.

As used herein, the terms "about" and "approximately" generally refer to a statistically significant numerical range. Such a range may be within an order of magnitude of a given value or range and may be included within 50%, preferably within 20%, more preferably within 10%, and most preferably within 5%. Allowable variations encompassed by the terms "about" or "approximately" may depend on the particular system being studied, and will be readily understood by those of ordinary skill in the art.

In the present invention, the terms "above", "below", "at most" and "at least" include the present number.

### Detailed Description

### TNFAIP3 knockout

The present invention provides methods for reducing the expression and/or reducing the activity of peptidase C64 family members and/or functionally active fragments thereof in said cells.

In one aspect, the present invention provides a method for culturing cells so that the expression and/or activity of peptidase C64 family members and/or functionally active fragments thereof is reduced in the cells. For example, the peptidase C64 family member may comprise a ubiquitin binding domain. For example, the peptidase C64 family member may comprise TNFAIP3.

For example, the target gene of the present invention may be a gene encoding a member of the peptidase C64 family and/or a functionally active fragment thereof. For example, cells obtained by reducing the expression and/or attenuating the activity of at least one target gene may exhibit improved cell properties compared to cells in which the expression and/or activity of the target gene is unchanged. In one embodiment, the cells whose expression and/or activity of the target gene are unchanged may refer to cells derived from the same donor and which have not had the expression and/or activity of at least one target gene of the cells reduced.. In one embodiment, cells whose expression and/or activity of the target gene are unchanged may refer to cells derived from the same donor and in which other genes other than the target gene of the cells have not been altered (e.g., knocking out the other genes, Cells with reduced expression and/or weakened activity (basically no impact on cell function).

For example, the cells include immune cells. For example, the cells comprise immune effector cells. For example, the cells include immune effector T cells, immune effector NK cells, and immune effector NKT cells. For example, the cells include phagocytes, lymphocytes, neutrophils, eosinophils and/or basophils.

For example, the cells include monocytes, macrophages, and/or dendritic cells.

For example, the cells of the present invention also include cells derived from differentiation of stem cells. For example, the cells of the present invention also include cells derived from differentiation of pluripotent stem cells. For example, the stem cells of the present invention can be obtained by induction. For example, the above-mentioned stem cells of the present invention may include induced pluripotent stem cells (iPSC), embryonic stem cells, bone marrow stem cells, umbilical cord blood stem cells and/or peripheral blood stem cells.

For example, "stem cells" of the present invention also include pluripotent cells, multipotent cells, precursor cells and progenitor cells. For example, stem cells can be obtained from hematopoietic or mesenchymal stem cells obtained from bone marrow tissue, placental stem cells obtained from placental tissue, embryonic stem cells obtained from embryonic tissue, or embryonic germ cells obtained from the reproductive tissue of the fetus. Exemplary pluripotent stem cells can also be generated from somatic cells by reprogramming them to a pluripotent state through the expression of certain transcription factors associated with pluripotency; these cells are called "induced pluripotent stem cells" or "iPSCs" .

For example, the cells include B cells, T cells, natural killer cells and/or natural killer-like T cells (NKT). For example, "unmodified cells" or "unmodified cells" may refer to cells in which the genome has not been modified and does not contain a gene regulatory system or contains a control gene regulatory system (e.g., empty vector control, non-targeting gRNA, interfering siRNA, etc. ) cells or cell populations. For example, the cells comprise αβ T cells and/or γδ T cells. For example, the cells include tumor-infiltrating lymphocytes (TILs). For example, the TIL is a TIL derived from tumor tissue, tumor-associated lymph nodes with or without tumor metastasis, tumor metastasis, fragments of paracancerous tissue, pleural effusion and/or peritoneal effusion and/or derived from frozen Save the TIL after recovery.

For example, the TILs of the present invention may be TILs and/or sources derived from tumor tissue, tumor-associated lymph nodes with or without tumor metastasis, tumor metastasis foci, fragments of paracancerous tissue, pleural effusion and/or peritoneal effusion. TILs recovered after cryopreservation. For example, TILs of the present invention can be obtained by processing tumor tissue into tumor fragments. For example, the tumor fragments of the present invention have a volume of about 1-27 cubic millimeters. For example, the volume of the tumor fragments of the present invention is about 1 cubic millimeter, about 2 cubic millimeters, about 3 cubic millimeters, about 4 cubic millimeters, about 5 cubic millimeters, about 6 cubic millimeters, about 7 cubic millimeters, about 8 cubic millimeters. , about 9 cubic millimeters, about 10 cubic millimeters, about 11 cubic millimeters, about 12 cubic millimeters, about 13 cubic millimeters, about 14 cubic millimeters, about 15 cubic millimeters, about 16 cubic millimeters, about 17 cubic millimeters, about 18 cubic millimeters , about 19 cubic millimeters, about 20 cubic millimeters, about 21 cubic millimeters, about 23 cubic millimeters, about 24 cubic millimeters, about 25 cubic millimeters, about 26 cubic millimeters, or about 27 cubic millimeters.

For example, the cells comprise engineered immune receptors displayed on the cell surface. For example, the engineered immune receptor specifically binds to an antigen expressed on a target cell. For example, the cells comprise chimeric antigen receptors and/or T cell receptors.

In one aspect, the present invention provides a method for culturing tumor-infiltrating lymphocytes (TIL), which may include reducing the expression and/or activity of peptidase C64 family members and/or functionally active fragments thereof in the TIL.

For example, TILs derived from tumor tissue, pleural effusion and/or peritoneal effusion without in vitro amplification can be subjected to at least one stage of in vitro amplification, wherein in during at least one stage of in vitro amplification, the expression and/or activity of peptidase C64 family members and/or functionally active fragments thereof in the TIL is reduced.

For example, the TILs of the present invention derived from tumor tissue, pleural effusion and/or peritoneal effusion without in vitro amplification can be subjected to the first stage of in vitro amplification and the second stage of in vitro amplification, and in the second stage of the in vitro amplification, the expression and/or activity of peptidase C64 family members and/or functionally active fragments thereof in the TIL can be reduced.

For example, the TILs of the present invention derived from tumor tissue, pleural effusion and/or peritoneal effusion without in vitro amplification can be subjected to the first stage of in vitro amplification and the second stage of in vitro amplification, and in the first stage of the in vitro amplification, the expression and/or activity of peptidase C64 family members and/or functionally active fragments thereof in the TIL can be reduced.

For example, the TILs of the present invention derived from tumor tissue, pleural effusion and/or peritoneal effusion without in vitro amplification can be subjected to the first stage of in vitro amplification and the second stage of in vitro amplification, and in the first stage of the in vitro amplification, the expression and/or activity of the peptidase C64 family members and/or functionally active fragments thereof in the TIL can be reduced, and in the second stage of the in vitro amplification, the expression and/or activity of peptidase C64 family members and/or functionally active fragments thereof in the TIL can be reduced

For example, the TILs of the present invention derived from tumor tissue, pleural effusion and/or peritoneal effusion without in vitro amplification can be subjected to a first-stage in vitro amplification, a second-stage in vitro amplification and a third-stage in vitro amplification, and in the first stage of in vitro amplification of the present invention, the expression and/or activity of peptidase C64 family members and/or functionally active fragments thereof in the TIL can be reduced.

For example, the TILs of the present invention derived from tumor tissue, pleural effusion and/or peritoneal effusion without in vitro amplification can be subjected to a first-stage in vitro amplification, a second-stage in vitro amplification and a third-stage in vitro amplification, and in the second stage of in vitro amplification of the present invention, the expression and/or activity of peptidase C64 family members and/or functionally active fragments thereof in the TIL can be reduced.

For example, the TILs of the present invention derived from tumor tissue, pleural effusion and/or peritoneal effusion without in vitro amplification can be subjected to a first-stage in vitro amplification, a second-stage in vitro amplification and a third-stage in vitro amplification, and in the third stage of in vitro amplification of the present invention, the expression and/or activity of peptidase C64 family members and/or functionally active fragments thereof in the TIL can be reduced.

For example, the TILs of the present invention derived from tumor tissue, pleural effusion and/or peritoneal effusion without in vitro amplification can be subjected to a first-stage in vitro amplification, a second-stage in vitro amplification and a third-stage in vitro amplification, and in the first stage of in vitro expansion of the present invention, the expression and/or activity of peptidase C64 family members and/or functionally active fragments thereof in the TIL can be reduced, and in the second stage of in vitro expansion of the present invention, the expression and/or activity of peptidase C64 family members and/or functionally active fragments thereof in the TIL can be reduced.

For example, the TILs of the present invention derived from tumor tissue, pleural effusion and/or peritoneal effusion without in vitro amplification can be subjected to a first-stage in vitro amplification, a second-stage in vitro amplification and a third-stage in vitro amplification, and in the first stage of in vitro expansion of the present invention, the expression and/or activity of peptidase C64 family members and/or functionally active fragments thereof in the TIL can be reduced, and in the third stage of in vitro expansion of the present invention, the expression and/or activity of peptidase C64 family members and/or functionally active fragments thereof in the TIL can be reduced.

For example, the TILs of the present invention derived from tumor tissue, pleural effusion and/or peritoneal effusion without in vitro amplification can be subjected to a first-stage in vitro amplification, a second-stage in vitro amplification and a third-stage in vitro amplification, and in the second stage of in vitro expansion of the present invention, the expression and/or activity of peptidase C64 family members and/or functionally active fragments thereof in the TIL can be reduced, and in the third stage of in vitro expansion of the present invention, the expression and/or activity of peptidase C64 family members and/or functionally active fragments thereof in the TIL can be reduced.

For example, the TILs of the present invention derived from tumor tissue, pleural effusion and/or peritoneal effusion without in vitro amplification can be subjected to a first-stage in vitro amplification, a second-stage in vitro amplification and a third-stage in vitro amplification. , and in the first stage of in vitro expansion of the present invention, the expression and/or activity of peptidase C64 family members and/or functionally active fragments thereof in the TIL can be reduced, in the second stage of in vitro expansion of the present invention, the expression and/or activity of peptidase C64 family members and/or functionally active fragments thereof in the TIL can be reduced, and in the third stage of in vitro amplification of the present invention, the expression and/or activity of peptidase C64 family members and/or functionally active fragments thereof in the TIL can be reduced.

For example, cells obtained by reducing the expression and/or the activity of the peptidase C64 family member exhibit improved cellular properties compared to cells in which the expression and/or activity of the peptidase C64 family member is unchanged.

For example, improved cell numbers of the present invention refer to cells in which the expression and/or activity of the peptidase C64 family member is reduced in at least one in vitro expansion stage compared to cells in which the expression and/or activity of the peptidase C64 family member is unchanged. /or the cell number of the cells of the present invention with weakened activity can be increased by at least about 1 time, at least about 2 times, at least about 3 times, at least about 4 times, at least about 5 times, at least about 6 times, at least about 7 times, at least about 8 times, at least about 9 times, at least about 10 times, at least about 11 times, at least about 12 times, at least about 13 times, at least about 14 times, at least about 15 times, at least about 20 times, at least about 30 times, at least about 40 times, or at least about 50 times.

For example, an increased proportion of viable cells can manifest itself as an increase in cell viability. For example, the increased proportion of viable cells of the present invention may refer to increasing the expression of the peptidase C64 family member in at least one in vitro expansion stage compared to cells in which the expression and/or activity of the peptidase C64 family member is unchanged. The proportion of viable cells of the cells of the invention that is reduced and/or has reduced activity can be increased by at least about 100%, at least about 90%, at least about 80%, at least about 70%, at least about 60%, at least about 50%, at least about 40% , at least about 30%, at least about 20%, at least about 19%, at least about 18%, at least about 17%, at least about 16%, at least about 15%, at least about 14%, at least about 13%, at least about 12% , at least about 11%, at least about 10%, at least about 9%, at least about 8%, at least about 7%, at least about 6%, at least about 5%, at least about 4%, at least about 3%, at least about 2% , at least about 1%, at least about 0.5%, at least about 0.4%, at least about 0.3%, at least about 0.2%, or at least about 0.1%.

For example, the increased cytokine secretion ability of the present invention may refer to an increase in the cytokine secretion ability of a cell selected from the group consisting of: IL-2, IL-6, CD107a, GZMB, TNF-α, and IFN-γ. For example, the improved cytokine secretion capacity of the present invention may refer to increasing the expression and/or activity of the peptidase C64 family member in at least one in vitro expansion stage compared to cells in which the expression and/or activity of the peptidase C64 family member is unchanged. The proportion of cells secreting cytokines in the cells of the present invention with reduced expression and/or weakened activity can be increased by at least about 1 time, at least about 2 times, at least about 3 times, at least about 4 times, at least about 5 times, at least about 6 times, At least about 7 times, at least about 8 times, at least about 9 times, at least about 10 times, at least about 11 times, at least about 12 times, at least about 13 times, at least about 14 times, at least about 15 times, at least about 20 times, At least about 30 times, at least about 40 times, or at least about 50 times. For example, the improved cytokine secretion capacity of the present invention may refer to increasing the expression and/or activity of the peptidase C64 family member in at least one in vitro expansion stage compared to cells in which the expression and/or activity of the peptidase C64 family member is unchanged. The proportion of cells secreting cytokines in the cells of the present invention with reduced expression and/or weakened activity can be increased by at least about 100%, at least about 90%, at least about 80%, at least about 70%, at least about 60%, at least about 50%, At least about 40%, at least about 30%, at least about 20%, at least about 19%, at least about 18%, at least about 17%, at least about 16%, at least about 15%, at least about 14%, at least about 13%, At least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%.

For example, the improved tumor cell killing ability of the present invention may refer to increasing the expression and/or activity of the peptidase C64 family member in at least one in vitro expansion stage compared with cells in which the expression and/or activity of the peptidase C64 family member is unchanged. The tumor cell killing rate of the cells of the present invention with reduced expression and/or weakened activity can be increased by at least about 1 times, at least about 2 times, at least about 3 times, at least about 4 times, at least about 5 times, at least about 6 times, at least about 7 times, at least about 8 times, at least about 9 times, at least about 10 times, at least about 11 times, at least about 12 times, at least about 13 times, at least about 14 times, at least about 15 times, at least about 20 times, at least about 30 times, at least about 40 times, or at least about 50 times. For example, the improved tumor cell killing ability of the present invention may refer to increasing the expression and/or activity of the peptidase C64 family member in at least one in vitro expansion stage compared with cells in which the expression and/or activity of the peptidase C64 family member is unchanged. The tumor cell killing rate of the cells of the present invention with reduced expression and/or weakened activity can be increased by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%. For example, the tumor cell killing rate of the cells of the present invention can be measured by the IncuCyte system or CFSE and DAPI staining methods. For example, tumor cell killing of cells of the present invention may refer to the ability of the cells to kill solid tumor cells.

For example, the improved cell subpopulation ratio of the present invention may comprise one or more selected from the following group: increased CD8⁺ Cell proportion, increased central memory cell and/or naive cell proportion, decreased regulatory cell proportion, increased activated cell proportion, increased tumor-specific cell proportion, and stem cell-like cell proportion increased.

For example, in CD8⁺ cells, central memory cells and/or immature cells, activated cells, tumor-specific cells and/or stem cell-like cells can be increased by at least about 100%, at least about 90%, at least about 80%, at least about 70%, at least about 60%, at least about 50%, at least about 40%, At least about 30%, at least about 20%, at least about 19%, at least about 18%, at least about 17%, at least about 16%, at least about 15%, at least about 14%, at least about 13%, at least about 12%, At least about 11%, at least about 10%, at least about 9%, at least about 8%, at least about 7%, at least about 6%, at least about 5%, at least about 4%, at least about 3%, at least about 2%, At least about 1%, at least about 0.5%, at least about 0.4%, at least about 0.3%, at least about 0.2%, or at least about 0.1%.

For example, the reduced proportion of exhausted cells of the present invention can be increase of PD-1⁺ ,LAG-3⁺ ,TIM-3⁺ , and/or CD39⁺ cell proportion. For example, the reduced proportion of regulatory cells of the present invention can be reduction of CD4⁺ CD25⁺ Foxp3⁺ cell proportion. For example, the reduced proportion of apoptotic cells of the present invention can be reduction of CD95⁺ caspass3⁺ and/or CD95⁺ DR5⁺ cell proportion.

For example, the proportion of exhausted cells, regulatory cells and/or apoptotic cells in the cells can be reduced by at least about 100%, at least about 90%, at least about 80%, at least about 70%, at least about 60%, at least about 50%, At least about 40%, at least about 30%, at least about 20%, at least about 19%, at least about 18%, at least about 17%, at least about 16%, at least about 15%, at least about 14%, at least about 13%, At least about 12%, at least about 11%, at least about 10%, at least about 9%, at least about 8%, at least about 7%, at least about 6%, at least about 5%, at least about 4%, at least about 3%, At least about 2%, at least about 1%, at least about 0.5%, at least about 0.4%, at least about 0.3%, at least about 0.2%, or at least about 0.1%, or can be reduced by at least about 1 times, at least about 2 times, at least About 3 times, at least about 4 times, at least about 5 times, at least about 6 times, at least about 7 times, at least about 8 times, at least about 9 times, at least about 10 times, at least about 11 times, at least about 12 times, at least About 13 times, at least about 14 times, at least about 15 times, at least about 20 times, at least about 30 times, at least about 40 times, or at least about 50 times.

For example, the culture method of the present invention may include a gene editing step for cells. For example, it includes: subjecting said cells to at least one stage of in vitro expansion, wherein in at least one stage of the in vitro amplification, a gene regulatory system can be introduced into the cells.

For example, the gene regulatory system can destroy the target gene at the DNA level. For example, the gene regulatory system can disrupt a region of the target gene or a fragment thereof in the genome of the cell. For example, after using the gene regulation system, the DNA region or fragment thereof where the target gene is located in the cell is sheared, and the expression ability of the target gene is reduced or the activity of the target gene is inhibited. For example, the editing effect of the gene regulation system on the target gene can be long-term and sustained. The genomic region of the present invention is determined based on the hg38 version of the human reference genome.

For example, the gene regulatory system may include guide nucleic acid molecules and enzymatic proteins. For example, the enzyme protein can have nucleic acid shearing enzyme activity, and the guide nucleic acid molecule can guide the enzyme protein to specifically cut the region where the target gene is located or its fragments. For example, the guide nucleic acid molecule and the enzyme protein may exist in the form of a ribonucleoprotein complex (RNP), or each may exist independently. For example, the enzyme protein may comprise Cas protein. For example, polynucleotides encoding gRNA and Cas protein can be introduced into the target cell, or each independently.

For example, reducing the expression and/or weakening the activity of at least one target gene of a cell according to the present invention may include: introducing a ribonucleoprotein complex (RNP) containing the guide nucleic acid molecule and the enzyme protein into the cell. For example, the enzyme protein may comprise Cas protein, Cas protein homologues, or functionally active fragments thereof. For example, the guide nucleic acid molecule may comprise guide RNA (gRNA). For example, the guide nucleic acid molecule may comprise guide RNA (gRNA). For example, a complex comprising a polynucleotide encoding a gRNA and a Cas protein can be introduced into the cell. For example, a complex containing gRNA and Cas protein can be introduced into the cell.

For example, the gRNA can be used to bind to the sequence of the target gene. For example, the binding of the gRNA to the sequence of the target gene can be completely complementary, partially complementary, or can hybridize to the sequence of the target gene under moderately stringent or stringent conditions. For example, the binding of the gRNA to the sequence of the target gene can cause the CRISPR system gRNA to specifically cuts the target gene.

For example, the editing target region of the present invention may be a region before the promoter. For example, the editing target region of the present invention may be a region with high transcription factor binding capacity. For example, the editing target region of the present invention may be a region with a specific number of transcription factor binding numbers. For example, the editing target region of the present invention can be a continuous region with a binding number of about 3 or more transcription factors.

For example, when the gene editing system includes CRISPR/Cas9, there may be a protospacer adjacent motif (PAM) downstream of the region targeted by the guiding nucleic acid molecule of the present invention, and the protospacer adjacent motif (PAM) may be AGG, TGG, GGG or CGG. For example, when the PAM region of the target gene is determined, those skilled in the art can easily determine about 15 to about 25 (for example, about 15, about 16, about 17, about 18, about 19, about 20) upstream of the 5' end of the PAM of the target gene. , about 21, about 22, about 23, about 24, about 25) nucleotides, and a suitable gRNA can be designed for the target sequence. For example, the guide nucleic acid molecule is capable of binding to a sequence consisting of about 15 to about 25 nucleotides upstream of the 5' end of a protospacer adjacent motif (PAM) selected from the group consisting of: AGG, TGG, GGG, and CGG.

For example, when the gene editing system includes CRISPR/Cas12, there may be a protospacer adjacent motif (PAM) upstream of the region targeted by the guiding nucleic acid molecule of the present invention, and the protospacer adjacent motif (PAM) may be NTTN, TTYN, VTTV, TRTV, TTTV, TATV, TYCV, TNN, or NTN, where N is A, T, C, or G, Y is T or C, V is A, C, or G, and R is A or G. For example, when the PAM region of the target gene is determined, those skilled in the art can easily determine about 15 to about 25 (for example, about 15, about 16, about 17, about 18, about 19, about 20) downstream of the 3' end of the PAM of the target gene. , about 21, about 22, about 23, about 24, about 25) nucleotides, and a suitable gRNA can be designed for the target sequence. For example, the guide nucleic acid molecule can bind to a sequence consisting of about 15 to about 25 nucleotides downstream of the 3' end of the protospacer adjacent motif (PAM) selected from the group consisting of: NTTN, TTYN, VTTV, TRTV ,TTTV,TATV, TYCV, TNN, or NTN, where N is A, T, C, or G, Y is T or C, V is A, C, or G, and R is A or G.

For example, when the gene editing system of the present invention includes wild-type Cas12a (also known as Cpf1, such as AsCas12a, FnCas12a, LbCas12a, BbCas12a, CMaCas12a and OsCas12a), the upstream region of the guiding nucleic acid molecule targeting of the present invention can be selected from the following PAM sequence: NTTN, where N can be A, T, C or G. For example, when the PAM region of the target gene is determined, those skilled in the art can easily determine about 17 to about 25 (for example, about 17, about 18, about 19, about 20, about 21, about 22) downstream of the 3' end of the PAM of the target gene. , about 23, about 24, or about 25) nucleotides, and a suitable gRNA can be designed for the target sequence.

For example, when the gene editing system of the present invention includes mutant Cas12a, such as enAsCas12a (mutation sites E174R, S542R and K548R), the upstream of the region targeted by the guiding nucleic acid molecule of the present invention can have a PAM sequence selected from the following: TTYN (TTTN /TTCN), VTTV (ATTV/CTTV/GTTV), or TRTV (TATV/TGTV), where N can be A, T, C or G, Y can be T or C, V can be A, C or G, R Can be A or G. For example, when the PAM region of the target gene is determined, those skilled in the art can easily determine about 17 to about 25 (for example, about 17, about 18, about 19, about 20, about 21, about 22) downstream of the 3' end of the PAM of the target gene. , about 23, about 24, or about 25) nucleotides, and a suitable gRNA can be designed for the target sequence.

For example, when the gene editing system of the present invention includes mutant Cas12a, such as opAsCas12a (mutation sites: E174R and S542R), the upstream of the region targeted by the guiding nucleic acid molecule of the present invention can have a PAM sequence selected from the following: TTTV (TTTA, TTTC, or TTTG), where V can be A, C, or G. For example, when the PAM region of the target gene is determined, those skilled in the art can easily determine about 17 to about 25 (for example, about 17, about 18, about 19, about 20, about 21, about 22) downstream of the 3' end of the PAM of the target gene. , about 23, about 24, or about 25) nucleotides, and a suitable gRNA can be designed for the target sequence.

For example, when the gene editing system of the present invention includes mutant Cas12a, such as AsCas12a Ultra (mutation sites: M537R and F870L), the upstream of the region targeted by the guiding nucleic acid molecule of the present invention can have a PAM sequence selected from the following: TTTV, TATV, or TYCV, where V can be A, C or G and Y can be T or C. For example, when the PAM region of the target gene is determined, those skilled in the art can easily determine about 17 to about 25 (for example, about 17, about 18, about 19, about 20, about 21, about 22) downstream of the 3' end of the PAM of the target gene. , about 23, about 24, or about 25) nucleotides, and a suitable gRNA can be designed for the target sequence.

For example, when the gene editing system of the present invention includes mutant Cas12a, such as hfCas12Max (mutation site: N243R/E336R/D892R) and Cas12Max (mutation site: N243R), the upstream region of the guiding nucleic acid molecule targeting of the present invention can be A PAM sequence selected from: TNN, or NTN, where N can be A, T, C, or G. For example, when the PAM region of the target gene is determined, those skilled in the art can easily determine about 17 to about 25 (for example, about 17, about 18, about 19, about 20, about 21, about 22) downstream of the 3' end of the PAM of the target gene. , about 23, about 24, or about 25) nucleotides, and a suitable gRNA can be designed for the target sequence.

For example, the guide nucleic acid molecule may comprise a target sequence, before the PAM region represented by AGG, TGG, GGG and/or CGG in the DNA where the gene encoding the peptidase C64 family member and/or its functionally active fragment is located, of about 10 to about 30 nucleotides. For example, the guide nucleic acid molecule may comprise a target sequence, before the PAM region represented by AGG, TGG, GGG and/or CGG in the DNA where the gene encoding the peptidase C64 family member and/or its functionally active fragment is located, about 15 to about 25, about 17 to about 25, about 19 to about 25, about 20 to about 25, about 21 to about 25, about 23 to about 25, about 15 to about 23, about 17 to about 23, about 19 to about 23, about 20 to about 23, about 21 to about 23, about 15 to about 21, about 17 to about 21, about 19 to about 21, about 20 to about 21, about 15 to a target sequence consisting of about 20, about 17 to about 20, about 19 to about 21, about 15 to about 19, about 17 to about 19, or about 15 to about 17 nucleotides.

For example, the target sequence may be selected from a region defined by the genomic coordinates shown in Table 1A or a fragment thereof.

For example, the target sequence of the present invention can be the OUT structural functional domain of TNFAIP3. For example, the target sequence of the present invention can be the zinc finger structural domain of TNFAIP3. For example, the target sequence of the present invention can be chr6:137871529-137871637, chr6:137874734-137874807, chr6:137874895-137874943, chr6:137875008-137875040, chr6:137875046-137 875149, chr6:137875614-137875650, chr6:137875666-137875724 , chr6:137875789-137875816, chr6:137875844-137876075, chr6:137879031-137879264, chr6:137879337-137879460, chr6:137879958-137880090, chr6:137880155-137880268, chr6:137880275-137880377, chr6:137880940-137881384.

For example, the guide nucleic acid molecule can include a targeting domain that is complementary to a target sequence selected from the group consisting of: SEQ ID NOs: 107-212, 1562-2532.

For example, the guide nucleic acid molecule can include a targeting domain, which can include sequences as set forth in SEQ ID NOs: 1-106, 591-1561, 7267-7324, 7419, 7420.

For example, the guide nucleic acid molecule can include a targeting domain, which can include sequences as set forth in SEQ ID NOs: 7267-7324, 7419, 7420.

For example, the ratio of cells expressing the product of the gene of interest in cells obtained by reducing the expression and/or activity of at least one target gene of the cell can be reduced and/or the expression level of the target gene in a single cell can be reduced, compared to cells in which the expression and/or activity of the target gene is unchanged.

For example, in the method of the present invention, the target gene is expressed in cells obtained by reducing the expression and/or activity of at least one target gene in the cells compared with cells in which the expression and/or activity of the target gene is unchanged. The cell proportion of the product is reduced by at least about 5%. For example, the proportion of cells expressing the product of the gene encoding a peptidase C64 family member and/or a functionally active fragment thereof is reduced by at least about 100%, at least about 90%, at least about 80%, at least about 70%, at least about 60% , at least about 50%, at least about 40%, at least about 30%, at least about 20%, at least about 19%, at least about 18%, at least about 17%, at least about 16%, at least about 15%, at least about 14% , at least about 13%, at least about 12%, at least about 11%, at least about 10%, at least about 9%, at least about 8%, at least about 7%, at least about 6%, or at least about 5%. For example, the proportion of cells expressing the product of the gene encoding a member of the peptidase C64 family and/or a functionally active fragment thereof may range from an observable proportion of cells to 0%. For example, the proportion of cells expressing the product of the gene encoding a peptidase C64 family member and/or a functionally active fragment thereof can be reduced to at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, or at least about 1%. For example, the proportion of cells expressing the product of the gene encoding a peptidase C64 family member and/or a functionally active fragment thereof can be detected by cell flow cytometry.

For example, in the method of the present invention, the expression of at least one target gene of the cells is reduced and/or the activity is weakened, and the cells obtained express the product of the gene encoding the peptidase C64 family member and/or its functionally active fragment. The ratio can be up to about 95%. For example, the proportion of cells expressing the product of the gene encoding the peptidase C64 family member and/or a functionally active fragment thereof may be at most about 95%, at most about 90%, at most about 80%, at most about 70%, At most about 60%, at most about 50%, at most about 40%, at most about 30%, at most about 20%, at most about 19%, at most about 18%, at most about 17%, at most about 16%, at most about 15%, Up to about 14%, up to about 13%, up to about 12%, up to about 11%, up to about 10%, up to about 9%, up to about 8%, up to about 7%, up to about 6%, or up to about 5%. For example, the cellular proportion expressing the encoded peptidase C64 family member and/or its functionally active fragment can be measured by cell flow cytometry.

For example, in the method of the present invention, compared with cells in which the expression and/or activity of the target gene are unchanged, the cells obtained by reducing the expression and/or activity of at least one target gene are described in a single cell. The expression level of the target gene can be reduced by at least about 5%. For example, the expression level of the gene of interest in a single cell can be reduced by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least About 11%, at least about 10%, at least about 9%, at least about 8%, at least about 7%, at least about 6%, or at least about 5%. For example, the expression level of the target gene in a single cell can range from an observable amount to 0%. For example, the expression level of the gene of interest in a single cell can be reduced to at least about 100%, at least about 90%, at least about 80%, at least about 70%, at least about 60%, at least about 50%, at least about 40% , at least about 30%, at least about 20%, at least about 19%, at least about 18%, at least about 17%, at least about 16%, at least about 15%, at least about 14%, at least about 13%, at least about 12% , at least about 11%, at least about 10%, at least about 9%, at least about 8%, at least about 7%, at least about 6%, at least about 5%, or at least about 1%.

For example, in the method of the present invention, the expression amount of the target gene in a single cell in the cells obtained by reducing the expression and/or activity of at least one target gene can be the expression and/or activity of the target gene. At most about 95% of the cells are unchanged. For example, the expression level of the gene encoding the peptidase C64 family member and/or the functionally active fragment thereof (eg, the gene encoding TNFAIP3) in a single cell may be the expression level of the gene encoding the peptidase C64 family member and/or the functionally active fragment thereof. At most about 95%, at most about 90%, at most about 80%, at most about 70%, at most about 60%, at most about 50%, at most about 40%, at most about 30% of the cells whose expression and/or activity is unchanged , at most about 20%, at most about 19%, at most about 18%, at most about 17%, at most about 16%, at most about 15%, at most about 14%, at most about 13%, at most about 12%, at most about 11% , up to about 10%, up to about 9%, up to about 8%, up to about 7%, up to about 6%, or up to about 5%.

For example, the method of the present invention comprises: subjecting said cell to at least one stage of in vitro expansion, wherein in at least one stage of said in vitro expansion, the expression of a member of the peptidase C64 family of the cell is reduced and/ or reduced activity.

For example, the TILs derived from tumor tissue, tumor-related lymph nodes with or without tumor metastasis, tumor metastasis lesions, fragments of para-cancerous tissue, pleural effusion and/or peritoneal effusion and have not been amplified in vitro are subjected to The first stage of in vitro amplification and the second stage of in vitro amplification, and in the second stage of in vitro amplification, the expression of the peptidase C64 family members of the TILs that have been amplified in the first stage of in vitro is reduced and/or Reduced activity.

For example, the first stage of in vitro expansion is performed for at least about 7 days. For example, the second stage of in vitro expansion is performed for at least about 7 days.

For example, the cells may be contacted with the one or more cell activating agents and the peptidase C64 family members and/or functionally active fragments thereof may be included in the cells in a single stage of in vitro expansion of the invention. reduced expression and/or activity. For example, the cell activator may comprise an agonist for one or more targets selected from the group consisting of: CD3, CD28, HVEM, CD40L, OX40, and 4-1BB. For example, in a single stage of said in vitro expansion, cells of the invention are exposed to reduced expression and/or attenuated activity of members of the peptidase C64 family and are contacted with one or more cell activators of the invention. For example, in the first stage of in vitro expansion of the present invention, the expression and/or activity of the TIL of the present invention and the peptidase C64 family member of the present invention can be reduced and contacted with one or more cell activators of the present invention.. For example, in the second stage of in vitro expansion of the present invention, the expression and/or activity of the peptidase C64 family member of the TIL of the present invention can be reduced and contacted with one or more cell activators of the present invention. For example, in the third stage of in vitro expansion of the present invention, the expression and/or activity of the peptidase C64 family member of the TIL of the present invention can be reduced and contacted with one or more cell activators of the present invention.

For example, in a single stage of in vitro expansion of the invention, the expression and/or activity of the peptidase C64 family member in the cells of the invention can be reduced substantially simultaneously with exposure to one or more cellular activators of the invention. For example, in a single stage of in vitro expansion of the present invention, the cells of the present invention can be made to reduce the expression and/or activity of peptidase C64 family members first, for example, 2 hours in advance, 4 hours in advance, or 8 hours in advance. , 12 hours in advance, 24 hours in advance, or 48 hours in advance, etc., and then contacted with one or more cell activators of the present invention. For example, in a single stage of in vitro expansion of the present invention, the cells of the present invention can be contacted with one or more cell activators of the present invention first, for example, 2 hours in advance, 4 hours in advance, 8 hours in advance, 12 hours in advance, 24 hours in advance, or 48 hours in advance, etc., to reduce the expression and/or activity of the peptidase C64 family members.

For example, in the first stage of in vitro expansion of the present invention, the TIL of the present invention can be caused to substantially simultaneously reduce the expression and/or activity of peptidase C64 family members and contact one or more cell activators of the present invention. For example, in the second stage of in vitro expansion of the invention, the TIL of the invention can substantially simultaneously reduce the expression and/or activity of peptidase C64 family members and contact one or more cell activators of the invention. For example, in the third stage of in vitro expansion of the invention, the TIL of the invention can substantially simultaneously reduce the expression and/or activity of peptidase C64 family members and contact one or more cell activators of the invention.

In another aspect, the invention provides a method for culturing tumor infiltrating lymphocytes (TIL), which may comprise: (A) making cells derived from tumor tissue, pleural effusion and/or peritoneal effusion without in vitro expansion The first TIL population is contacted with one or more cell growth factors; wherein the second TIL population is obtained through the step (A); (B) the expression of the peptidase C64 family members of the second TIL population is reduced and/ Or the activity is weakened; wherein, the third TIL group is obtained through the step (B).

In the terminology of one embodiment, the first stage in vitro amplification of the present invention can be used arbitrarily interchangeably with step (A) in the method of the above aspect. In the terminology of one embodiment, the second stage in vitro amplification of the present invention can be used arbitrarily interchangeably with step (B) in the method of the above aspect. In one embodiment of the term, the amplified TIL in vitro in the first stage of the present invention can be used interchangeably with the second TIL population obtained in step (A) of the above method. In one embodiment, the TILs expanded in vitro in the second stage of the present invention can be used interchangeably with the third TIL population obtained in step (B) of the above method. In the terminology of one embodiment, if necessary, the third stage in vitro amplification of the present invention can be used interchangeably with any additional step (C) in the method of the above aspect. In the terminology of one embodiment, if necessary, the third-stage in vitro expanded TIL of the present invention can be used interchangeably with the fourth TIL group obtained by any additional step (C) in the above method.

In another aspect, the present invention provides a method for culturing tumor infiltrating lymphocytes (TIL), which may include: (A) contacting the first TIL population cells derived from tumor tissue, pleural effusion and/or peritoneal effusion without in vitro amplification with a variety of cell growth factors; wherein, the second TIL population is obtained through step (A); (B) the second TIL population can be contacted with a variety of cell growth factors and cell activator to reduce the expression and/or activity of the peptidase C64 family members, and the TILs are co-cultured with feeder cells; wherein, the third TIL population is obtained through step (B).

In another aspect, the present invention provides a method for culturing tumor infiltrating lymphocytes (TIL), which may include: (A) culturing cells derived from tumor tissue, pleural effusion and/or peritoneal effusion without in vitro amplification The first TIL population is contacted with cell growth factors; wherein, the second TIL population is obtained through the step (A); (B) the second TIL population can be contacted with cell growth factors, with cell activators, and the expression and/or activity of the peptidase C64 family members is reduced and the TILs are co-cultured with feeder cells. The peptidase C64 family members may include TNFAIP3; wherein, the third TIL population is obtained through the step (B).

In another aspect, the invention provides a method of culturing tumor-infiltrating lymphocytes (TIL). The method of obtaining TIL cells from subject tissue samples can be to obtain orthotopic tumor samples or metastatic tumor samples from the patient during surgery, and the weight can be at least about 1g, or multiple pieces of tissue can be merged. Tumor tissue, pleural effusion and/or peritoneal effusion are stored in a sample transport fluid, which may be, for example, a commercially available tumor tissue transport fluid, tumor tissue preservation fluid or tumor tissue transfer fluid, shipping at about 2-8 degrees, processed within 48 hours. The tissue pieces can be mechanically broken to a size of about 1-27 cubic millimeters per piece, transferred into a breathable culture bag or Grex, and cell serum-free medium added with a concentration of 300-9000IU/mL (for example, it can be 1000-9000IU/mL, e.g. it can be cultured with IL-2 (6000IU/mL) for about 3-14 days. Collect the cells in the culture medium and transfer them to a breathable culture bag, or Grex, or Xuri equipment. The cell serum-free culture medium can be added with the CD28 antibody, CD3 antibody and CD28 antibody of the present invention, and magnetic beads containing CD3 antibody and CD28 antibody (such as Dynabeads ) and/or a nanomatrix (such as transACT) containing CD3 antibodies and CD28 antibodies, IL-2 at a concentration of 300-9000IU/mL (such as 1000-9000IU/mL, such as 6000IU/mL), and reduced expression and/or activity of peptidase C64 family members (peptidase C64 family members may include TNFAIP3, for example, may be transduced by carrying a ribonucleoprotein complex (RNP) formed by a gRNA containing the present invention and a Cas protein. The cell ratio of genes encoding peptidase C64 family members in TIL is about 95% or less), after activating the TIL of the present invention for a certain period of time, add irradiated PBMC (the ratio of TIL to PBMC is about 1:40 to about 1:400) , amplification culture takes about 3-14 days. Cell processing systems can be used to collect cells in the culture medium, wash, freeze, and detect. The CD3 ratio of the final product can be greater than 80%, the cell viability rate can be greater than 50%, and the cells greater than 80% can be memory effector cells and effector cells. IFN-γ can be secreted after stimulation, and/or can be characterized by an increase in the proportion of activated cells.

### ZC3H12A knockout

1. A method for culturing cells, the method comprising: reducing the expression and/or weakening the activity of ZC3H12 family members and/or functionally active fragments thereof of the cells.
2. The method of embodiment 1, wherein the cells comprise immune cells.
3. The method of embodiment 2, wherein the immune cells comprise phagocytes, lymphocytes, neutrophils, eosinophils and/or basophils.
4. The method according to any one of embodiments 2-3, wherein the immune cells comprise monocytes, macrophages and/or dendritic cells.
5. The method according to any one of embodiments 2-4, wherein the immune cells are derived from stem cell differentiated immune cells.
6. The method of embodiment 5, wherein the stem cells comprise induced pluripotent stem cells (iPSCs).
7. The method according to any one of embodiments 2-6, wherein the immune cells comprise B cells, T cells, natural killer cells and/or natural killer-like T cells (NKT).
8. The method according to any one of embodiments 2-7, wherein the immune cells comprise αβ T cells and/or γδ T cells.
9. The method of any one of embodiments 2-8, wherein the immune cells comprise tumor-infiltrating lymphocytes (TIL).
10. The method of embodiment 9, wherein the TIL is a TIL derived from fragments of tumor tissue, pleural effusion and/or peritoneal effusion and/or TIL derived from resuscitation after cryopreservation.
11. The method of embodiment 10, wherein the fragment has a volume from about 1 cubic millimeter to about 27 cubic millimeters.
12. The method of any one of embodiments 2-11, wherein the immune cell comprises an engineered immune receptor displayed on the cell surface.
13. The method of embodiment 12, wherein the engineered immune receptor specifically binds to an antigen expressed on a target cell.
14. The method of any one of embodiments 2-13, wherein the immune cell contains chimeric antigen receptors and/or T cell receptors.
15. The method according to any one of embodiments 1-14, wherein reducing the expression and/or activity of the ZC3H12 family member of the cell comprises inhibiting the function of a nuclease.
16. The method according to any one of embodiments 1-15, wherein the expression of the ZC3H12 family member is reduced and/or the activity is attenuated compared to cells in which the expression and/or activity of the ZC3H12 family member is unchanged. of cells showed improved cellular properties.
17. The method of embodiment 16, wherein the improved cell properties comprise one or more selected from the group consisting of: improved cell proliferation capacity, increased proportion of viable cells, improved proportion of cell subpopulations, the increased ability to secrete cytokines and improve the killing ability of tumor cells.
18. The method of embodiment 17, wherein the improved proportion of cell subpopulations comprises one or more selected from the group consisting of an increased proportion of activated cells, a reduced proportion of regulatory cells, a reduced proportion of exhausted cells, increased proportion of central memory cells and/or naive cells, decreased proportion of apoptotic cells and increased proportion of stem cell-like cells.
19. The method of any one of embodiments 1-18, wherein the ZC3H12 family member comprises a C3H1-type zinc finger domain.
20. The method of any one of embodiments 1-19, wherein the ZC3H12 family member comprises ZC3H12A.
21. The method of any one of embodiments 1-20, wherein reducing the expression and/or attenuating the activity of a ZC3H12 family member of the cell comprises introducing a gene regulatory system into the cell.
22. The method of embodiment 21, wherein the gene regulatory system is capable of Destruction of the ZC3H12 family members at the DNA level.
23. The method of any one of embodiments 21-22, wherein the gene regulatory system comprises a guide nucleic acid molecule and an enzymatic protein.
24. The method of embodiment 23, wherein reducing the expression and/or activity of the ZC3H12 family member comprises: converting a ribonucleoprotein complex (RNP) comprising the guide nucleic acid molecule and the enzyme protein, An LNP comprising a gRNA and a Cas protein, or an LNP comprising a nucleic acid encoding a gRNA and a Cas protein, is introduced into the cell.
25. The method of any one of embodiments 23-24, wherein the enzyme protein comprises a Cas protein, a Cas protein homolog, or a functionally active fragment thereof.
26. The method of any one of embodiments 23-25, wherein the guide nucleic acid molecule comprises a guide RNA (gRNA).
27. The method of any one of embodiments 23-26, wherein the guide nucleic acid molecule is capable of binding to the sequence of the ZC3H12 family member.
28. The method according to any one of embodiments 23-27, wherein the guide nucleic acid molecule is capable of binding to a region selected from the group defined by the genomic coordinates shown in Table 1B, or a fragment thereof.
29. The method of any one of embodiments 23-28, wherein the guide nucleic acid molecule is capable of binding to a region selected from the group consisting of: SEQ ID NOs: 281-348, 3116-3698, or fragments thereof.
30. The method of any one of embodiments 23-29, wherein the guide nucleic acid molecule is capable of being about 15 to about 25 upstream of the 5' end of a protospacer adjacent motif (PAM) selected from the group consisting of: Sequence combinations of nucleotides: AGG, TGG, CGG and GGG.
31. The method according to any one of embodiments 23-30, wherein the guide nucleic acid molecule comprises a targeting domain comprising SEQ ID NOs: 213-280, 2533-3115, 7325 - 7345, 7416, and 7417.
32. The method according to any one of embodiments 1-31, wherein the expression of the ZC3H12 family member is reduced and/or the activity is attenuated compared to cells in which the expression and/or activity of the ZC3H12 family member is unchanged. The proportion of cells expressing the product of the target gene among the cells decreases and/or the expression level of the target gene in a single cell decreases.
33. The method according to any one of embodiments 1-32, wherein among the cells obtained by reducing the expression and/or weakening the activity of the ZC3H12 family member, the proportion of cells expressing the gene of interest is about 95% or the following.
34. A cell obtained by the method of any one of embodiments 1-33.
35. A composition comprising the cell of embodiment 34.
36. A pharmaceutical composition comprising the cell of embodiment 34 and/or the composition of embodiment 35, and optionally a pharmaceutically acceptable carrier.
37. A method of affecting cell growth, comprising administering the cell of embodiment 34, the composition of embodiment 35, and/or the pharmaceutical composition of embodiment 36.
38. Use of the cell of embodiment 34, the composition of embodiment 35 and/or the pharmaceutical composition of embodiment 36 in the preparation of a medicament, wherein the medicament is used to prevent and/or treat diseases and /or symptoms.
39. Use according to embodiment 38, wherein the disease and/or condition comprises a tumor.
40. Use according to any one of embodiments 38-39, wherein the disease and/or condition comprises a solid tumor.
41. Use according to any one of embodiments 38-40, wherein the disease and/or condition comprises one or more selected from the group consisting of: melanoma, ovarian cancer, cervical cancer, lung cancer, bladder cancer , breast cancer, head and neck cancer, pancreatic cancer, liver cancer, stomach cancer, colorectal cancer and kidney cancer.

The present invention provides methods for reducing the expression and/or reducing the activity of ZC3H12 family members and/or functionally active fragments thereof in said cells.

In one aspect, the present invention provides a method for culturing cells so that the expression and/or activity of ZC3H12 family members and/or functionally active fragments thereof is reduced in the cells. For example, the ZC3H12 family member may comprise a C3H1-type zinc finger domain. For example, the ZC3H12 family member may comprise ZC3H12A.

For example, the target gene of the present invention may be a gene encoding a ZC3H12 family member and/or a functionally active fragment thereof. For example, a cell obtained by reducing the expression and/or attenuating the activity of at least one target gene of the cell may exhibit improved cell properties compared to a cell in which the expression and/or activity of the target gene is unchanged. In one embodiment, the cells whose expression and/or activity of the target gene are unchanged may refer to cells derived from the same donor and which have not had the expression and/or activity of at least one target gene of the cells reduced. In one embodiment, cells whose expression and/or activity of the target gene are unchanged may refer to cells derived from the same donor and in which the expression and/or activity of other genes other than the target gene of the cells have not been altered (e.g., knocking out the other genes has basically no impact on cell function).

For example, the cells include immune cells. For example, the cells comprise immune effector cells. For example, the cells include immune effector T cells, immune effector NK cells, and immune effector NKT cells. For example, the cells include phagocytes, lymphocytes, neutrophils, eosinophils and/or basophils.

For example, the cells include monocytes, macrophages, and/or dendritic cells.

For example, the cells of the present invention also include cells derived from differentiation of stem cells. For example, the cells of the present invention also include cells derived from differentiation of pluripotent stem cells. For example, the stem cells of the present invention can be obtained by induction. For example, the above-mentioned stem cells of the present invention may include induced pluripotent stem cells (iPSCs), embryonic stem cells, bone marrow stem cells, umbilical cord blood stem cells and/or peripheral blood stem cells.

For example, "stem cells" of the present invention also include pluripotent cells, multipotent cells, precursor cells and progenitor cells. For example, stem cells can be obtained from hematopoietic or mesenchymal stem cells obtained from bone marrow tissue, placental stem cells obtained from placental tissue, embryonic stem cells obtained from embryonic tissue, or embryonic germ cells obtained from the reproductive tissue of the fetus. Exemplary pluripotent stem cells can also be generated from somatic cells by reprogramming them to a pluripotent state through the expression of certain transcription factors associated with pluripotency; these cells are called "induced pluripotent stem cells" or "iPSCs" .

For example, the cells include B cells, T cells, natural killer cells and/or natural killer-like T cells (NKT). For example, "unmodified cells" may refer to cells in which the genome has not been modified and does not contain a gene regulatory system or contains a control gene regulatory system (e.g., empty vector control, non-targeting gRNA, interfering siRNA, etc. ) cells or cell populations. For example, the cells comprise αβ T cells and/or γδ T cells. For example, the cells include tumor-infiltrating lymphocytes (TILs). For example, the TIL is a TIL derived from tumor tissue, tumor-associated lymph nodes with or without tumor metastasis, tumor metastasis, fragments of paracancerous tissue, pleural effusion and/or peritoneal effusion and/or TILs recovered from frozen cells.

For example, the TILs of the present invention may be TILs and/or TILs recovered after cryopreservation derived from tumor tissue, tumor-associated lymph nodes with or without tumor metastasis, tumor metastasis foci, fragments of paracancerous tissue, pleural effusion and/or peritoneal effusion. For example, tumor tissues can be processed into tumor fragments to obtain TILs of the present invention. For example, the tumor fragments of the present invention have a volume of about 1 to 27 cubic millimeters. For example, the volume of the tumor fragments of the present invention is about 1 cubic millimeter, about 2 cubic millimeters, about 3 cubic millimeters, about 4 cubic millimeters, about 5 cubic millimeters, about 6 cubic millimeters, about 7 cubic millimeters, about 8 cubic millimeters. , about 9 cubic millimeters, about 10 cubic millimeters, about 11 cubic millimeters, about 12 cubic millimeters, about 13 cubic millimeters, about 14 cubic millimeters, about 15 cubic millimeters, about 16 cubic millimeters, about 17 cubic millimeters, about 18 cubic millimeters , about 19 cubic millimeters, about 20 cubic millimeters, about 21 cubic millimeters, about 23 cubic millimeters, about 24 cubic millimeters, about 25 cubic millimeters, about 26 cubic millimeters or about 27 cubic millimeters.

For example, the cells comprise engineered immune receptors displayed on the cell surface. For example, the engineered immune receptor specifically binds to an antigen expressed on a target cell. For example, the cells comprise chimeric antigen receptors and/or T cell receptors.

In one aspect, the present invention provides a method for culturing tumor-infiltrating lymphocytes (TIL), which may include: reducing the expression and/or activity of ZC3H12 family members and/or functionally active fragments thereof in the TIL.

For example, TILs derived from tumor tissue, pleural effusion and/or peritoneal effusion without in vitro amplification can be subjected to at least one stage of in vitro amplification, wherein in at least one stage of in vitro amplification, the expression and/or activity of ZC3H12 family members and/or functionally active fragments thereof are reduced in the TIL.

For example, the TILs of the present invention derived from tumor tissue, pleural effusion and/or peritoneal effusion without in vitro amplification can be subjected to the first stage of in vitro amplification and the second stage of in vitro amplification, and in the second stage of the in vitro amplification, the expression and/or activity of peptidase ZC3H12 family members and/or functionally active fragments thereof in the TIL can be reduced.

For example, the TILs of the present invention derived from tumor tissue, pleural effusion and/or peritoneal effusion without in vitro amplification can be subjected to the first stage of in vitro amplification and the second stage of in vitro amplification, and in the first stage of the in vitro amplification, the expression and/or activity of peptidase ZC3H12 family members and/or functionally active fragments thereof in the TIL can be reduced.

For example, the TILs of the present invention derived from tumor tissue, pleural effusion and/or peritoneal effusion without in vitro amplification can be subjected to the first stage of in vitro amplification and the second stage of in vitro amplification, and in the first stage of the in vitro amplification, the expression and/or activity of the peptidase ZC3H12 family members and/or functionally active fragments thereof in the TIL can be reduced, and in the second stage of the in vitro amplification, the expression and/or activity of peptidase ZC3H12 family members and/or functionally active fragments thereof in the TIL can be reduced

For example, the TILs of the present invention derived from tumor tissue, pleural effusion and/or peritoneal effusion without in vitro amplification can be subjected to a first-stage in vitro amplification, a second-stage in vitro amplification and a third-stage in vitro amplification, and in the first stage of in vitro amplification of the present invention, the expression and/or activity of peptidase ZC3H12 family members and/or functionally active fragments thereof in the TIL can be reduced.

For example, the TILs of the present invention derived from tumor tissue, pleural effusion and/or peritoneal effusion without in vitro amplification can be subjected to a first-stage in vitro amplification, a second-stage in vitro amplification and a third-stage in vitro amplification, and in the second stage of in vitro amplification of the present invention, the expression and/or activity of peptidase ZC3H12 family members and/or functionally active fragments thereof in the TIL can be reduced.

For example, the TILs of the present invention derived from tumor tissue, pleural effusion and/or peritoneal effusion without in vitro amplification can be subjected to a first-stage in vitro amplification, a second-stage in vitro amplification and a third-stage in vitro amplification, and in the third stage of in vitro amplification of the present invention, the expression and/or activity of peptidase ZC3H12 family members and/or functionally active fragments thereof in the TIL can be reduced.

For example, the TILs of the present invention derived from tumor tissue, pleural effusion and/or peritoneal effusion without in vitro amplification can be subjected to a first-stage in vitro amplification, a second-stage in vitro amplification and a third-stage in vitro amplification, and in the first stage of in vitro expansion of the present invention, the expression and/or activity of peptidase ZC3H12 family members and/or functionally active fragments thereof in the TIL can be reduced, and in the second stage of in vitro expansion of the present invention, the expression and/or activity of peptidase ZC3H12 family members and/or functionally active fragments thereof in the TIL can be reduced.

For example, the TILs of the present invention derived from tumor tissue, pleural effusion and/or peritoneal effusion without in vitro amplification can be subjected to a first-stage in vitro amplification, a second-stage in vitro amplification and a third-stage in vitro amplification, and in the first stage of in vitro expansion of the present invention, the expression and/or activity of peptidase ZC3H12 family members and/or functionally active fragments thereof in the TIL can be reduced, and in the third stage of in vitro expansion of the present invention, the expression and/or activity of peptidase ZC3H12 family members and/or functionally active fragments thereof in the TIL can be reduced.

For example, the TILs of the present invention derived from tumor tissue, pleural effusion and/or peritoneal effusion without in vitro amplification can be subjected to a first-stage in vitro amplification, a second-stage in vitro amplification and a third-stage in vitro amplification, and in the second stage of in vitro expansion of the present invention, the expression and/or activity of peptidase ZC3H12 family members and/or functionally active fragments thereof in the TIL can be reduced, and in the third stage of in vitro expansion of the present invention, the expression and/or activity of peptidase ZC3H12 family members and/or functionally active fragments thereof in the TIL can be reduced.

For example, the TILs of the present invention derived from tumor tissue, pleural effusion and/or peritoneal effusion without in vitro amplification can be subjected to a first-stage in vitro amplification, a second-stage in vitro amplification and a third-stage in vitro amplification. , and in the first stage of in vitro expansion of the present invention, the expression and/or activity of peptidase ZC3H12 family members and/or functionally active fragments thereof in the TIL can be reduced, in the second stage of in vitro expansion of the present invention, the expression and/or activity of peptidase ZC3H12 family members and/or functionally active fragments thereof in the TIL can be reduced, and in the third stage of in vitro amplification of the present invention, the expression and/or activity of peptidase ZC3H12 family members and/or functionally active fragments thereof in the TIL can be reduced.

For example, cells obtained by reducing the expression and/or attenuating the activity of the ZC3H12 family member exhibit improved cellular properties compared to cells in which the expression and/or activity of the ZC3H12 family member is unchanged.

For example, the improved cell numbers of the present invention are related to the expression of ZC3H12 family members.

The cell number of the cells of the present invention that reduces the expression and/or weakens the activity of the ZC3H12 family member in at least one in vitro expansion stage can be increased by at least about 1 times, at least about 2 times, compared to cells with unchanged activity. times, at least about 3 times, at least about 4 times, at least about 5 times, at least about 6 times, at least about 7 times, at least about 8 times, at least about 9 times, at least about 10 times, at least about 11 times, at least about 12 times, at least about 13 times, at least about 14 times, at least about 15 times, at least about 20 times, at least about 30 times, at least about 40 times, or at least about 50 times.

For example, an increased proportion of viable cells can manifest itself as an increase in cell viability. For example, the increased proportion of viable cells of the present invention may refer to reducing the expression and/or activity of the ZC3H12 family member in at least one in vitro expansion stage compared to cells in which the expression and/or activity of the ZC3H12 family member is unchanged. The proportion of viable cells of the cells of the invention with reduced activity can be increased by at least about 100%, at least about 90%, at least about 80%, at least about 70%, at least about 60%, at least about 50%, at least about 40%, at least about 30 %, at least about 20%, at least about 19%, at least about 18%, at least about 17%, at least about 16%, at least about 15%, at least about 14%, at least about 13%, at least about 12%, at least about 11 %, at least about 10%, at least about 9%, at least about 8%, at least about 7%, at least about 6%, at least about 5%, at least about 4%, at least about 3%, at least about 2%, at least about 1 %, at least about 0.5%, at least about 0.4%, at least about 0.3%, at least about 0.2%, or at least about 0.1%.

For example, the increased cytokine secretion ability of the present invention may refer to an increase in the cytokine secretion ability of a cell selected from the group consisting of: IL-2, IL-6, CD107a, GZMB, TNF-α, and IFN-γ. For example, the improved cytokine secretion ability of the present invention may refer to reducing the expression and/or activity of the ZC3H12 family member in at least one in vitro expansion stage compared to cells in which the expression and/or activity of the ZC3H12 family member is unchanged. Or the proportion of cells secreting cytokines in the cells of the present invention with weakened activity can be increased by at least about 1 time, at least about 2 times, at least about 3 times, at least about 4 times, at least about 5 times, at least about 6 times, at least about 7 times , at least about 8 times, at least about 9 times, at least about 10 times, at least about 11 times, at least about 12 times, at least about 13 times, at least about 14 times, at least about 15 times, at least about 20 times, at least about 30 times , at least about 40 times, or at least about 50 times. For example, the improved cytokine secretion ability of the present invention may refer to secretion in cells of the invention in which the expression and/or activity of the ZC3H12 family member is reduced in at least one in vitro expansion stage compared to cells in which the expression and/or activity of the ZC3H12 family member is unchanged. The cellular proportion of the cytokine can be increased by at least about 100%, at least about 90%, at least about 80%, at least about 70%, at least about 60%, at least about 50%, at least about 40%, at least about 30%, at least about 20 %, at least about 19%, at least about 18%, at least about 17%, at least about 16%, at least about 15%, at least about 14%, at least about 13%, at least about 12%, at least about 11%, at least about 10 %, at least about 9%, at least about 8%, at least about 7%, at least about 6%, at least about 5%, at least about 4%, at least about 3%, at least about 2%, at least about 1%, at least about 0.5 %, at least about 0.4%, at least about 0.3%, at least about 0.2%, or at least about 0.1%.

For example, the improved tumor cell killing ability of the present invention may refer to reducing the expression and/or activity of the ZC3H12 family member in at least one in vitro expansion stage compared with cells in which the expression and/or activity of the ZC3H12 family member is unchanged. Or the tumor cell killing rate of the cells of the present invention with weakened activity can be increased by at least about 1 time, at least about 2 times, at least about 3 times, at least about 4 times, at least about 5 times, at least about 6 times, at least about 7 times, at least About 8 times, at least about 9 times, at least about 10 times, at least about 11 times, at least about 12 times, at least about 13 times, at least about 14 times, at least about 15 times, at least about 20 times, at least about 30 times, at least About 40 times, or at least about 50 times. For example, the improved tumor cell killing ability of the present invention may refer to reducing the expression and/or activity of the ZC3H12 family member in at least one in vitro expansion stage compared with cells in which the expression and/or activity of the ZC3H12 family member is unchanged. Or the tumor cell killing rate of the cells of the present invention with weakened activity can be increased by at least about 100%, at least about 90%, at least about 80%, at least about 70%, at least about 60%, at least about 50%, at least about 40%, at least About 30%, at least about 20%, at least about 19%, at least about 18%, at least about 17%, at least about 16%, at least about 15%, at least about 14%, at least about 13%, at least about 12%, at least About 11%, at least about 10%, at least about 9%, at least about 8%, at least about 7%, at least about 6%, at least about 5%, at least about 4%, at least about 3%, at least about 2%, at least About 1%, at least about 0.5%, at least about 0.4%, at least about 0.3%, at least about 0.2%, or at least about 0.1%. For example, the tumor cell killing rate of the cells of the present invention can be measured by the IncuCyte system or CFSE and DAPI staining methods. For example, the tumor cell killing of the present invention may refer to the ability of cells to kill solid tumor cells.

For example, the improved cell subpopulation ratio of the present invention may comprise one or more selected from the following group: increased CD8⁺ cell proportion, increased central memory cell and/or naive cell proportion, decreased regulatory cell proportion, increased activated cell proportion, increased tumor-specific cell proportion, and stem cell-like cell proportion increased.

For example, the proportion of CD8⁺ cells, central memory cells and/or naive cells, activated cells, tumor-specific cells and/or stem cell-like cells can be increased by at least about 100%, at least about 90%, at least about 80%, at least about 70%, at least about 60 %, at least about 50%, at least about 40%, at least about 30%, at least about 20%, at least about 19%, at least about 18%, at least about 17%, at least about 16%, at least about 15%, at least about 14 %, at least about 13%, at least about 12%, at least about 11%, at least about 10%, at least about 9%, at least about 8%, at least about 7%, at least about 6%, at least about 5%, at least about 4 %, at least about 3%, at least about 2%, at least about 1%, at least about 0.5%, at least about 0.4%, at least about 0.3%, at least about 0.2%, or at least about 0.1%.

For example, the reduced proportion of exhausted cells of the present invention can be increase of PD-1⁺ ,LAG-3⁺ ,TIM-3⁺ , and/or CD39⁺ cell proportion. For example, the reduced proportion of regulatory cells of the present invention can be reduction of CD4⁺ CD25⁺ Foxp3⁺ cell proportion. For example, the reduced proportion of apoptotic cells of the present invention can be reduction of CD95⁺ caspass3⁺ and/or CD95⁺ DR5⁺ cell proportion.

For example, the proportion of exhausted cells, regulatory cells and/or apoptotic cells in the cells can be reduced by at least about 100%, at least about 90%, at least about 80%, at least about 70%, at least about 60%, at least about 50%, At least about 40%, at least about 30%, at least about 20%, at least about 19%, at least about 18%, at least about 17%, at least about 16%, at least about 15%, at least about 14%, at least about 13%, At least about 12%, at least about 11%, at least about 10%, at least about 9%, at least about 8%, at least about 7%, at least about 6%, at least about 5%, at least about 4%, at least about 3%, At least about 2%, at least about 1%, at least about 0.5%, at least about 0.4%, at least about 0.3%, at least about 0.2%, or at least about 0.1%, or can be reduced by at least about 1 times, at least about 2 times, at least about 3 times, at least about 4 times, at least about 5 times, at least about 6 times , at least about 7 times, at least about 8 times, at least about 9 times, at least about 10 times, at least about 11 times, at least about 12 times, at least about 13 times, at least about 14 times, at least about 15 times, at least about 20 times , at least about 30 times, at least about 40 times, or at least about 50 times.

For example, the culture method of the present invention may include a gene editing step for cells. For example, it includes: subjecting the cells to at least one stage of in vitro expansion, wherein a gene regulatory system can be introduced into the cells during at least one stage of the in vitro expansion.

For example, the gene regulatory system can destroy the target gene at the DNA level. For example, the gene regulatory system can disrupt a region of the target gene or a fragment thereof in the genome of the cell. For example, after using the gene regulation system, the DNA region or fragment thereof where the target gene is located in the cell is sheared, and the expression ability of the target gene is reduced or the activity of the target gene is inhibited. For example, the editing effect of the gene regulation system on the target gene can be long-term and sustained. The genomic region of the present invention is determined based on the hg38 version of the human reference genome.

For example, the gene regulatory system may include guide nucleic acid molecules and enzymatic proteins. For example, the enzyme protein can have nucleic acid shearing enzyme activity, and the guide nucleic acid molecule can guide the enzyme protein to specifically cut the region where the target gene is located or its fragments. For example, the guide nucleic acid molecule and the enzyme protein may exist in the form of a ribonucleoprotein complex (RNP), or each may exist independently. For example, the enzyme protein may comprise Cas protein. For example, polynucleotides encoding gRNA and Cas protein can be introduced into the target cell, or each independently.

For example, reducing the expression and/or weakening the activity of at least one target gene of a cell according to the present invention may include: introducing a ribonucleoprotein complex (RNP) containing the guide nucleic acid molecule and the enzyme protein into the cell. For example, the enzyme protein may comprise Cas protein, Cas protein homologues, or functionally active fragments thereof. For example, the guide nucleic acid molecule may comprise guide RNA (gRNA). For example, the guide nucleic acid molecule may comprise guide RNA (gRNA). For example, a complex comprising a polynucleotide encoding a gRNA and a Cas protein can be introduced into the cell. For example, a complex containing gRNA and Cas protein can be introduced into the cell.

For example, the gRNA can be used to bind to the sequence of the target gene. For example, the binding of the gRNA to the sequence of the target gene can be completely complementary, partially complementary, or can hybridize to the sequence of the target gene under moderately stringent or stringent conditions. For example, the binding of the gRNA to the sequence of the target gene can cause the CRISPR system of the gRNA to specifically cleave the target gene.

For example, the editing target region of the present invention may be a region before the promoter. For example, the editing target region of the present invention may be a region with high transcription factor binding capacity. For example, the editing target region of the present invention may be a region with a specific number of transcription factor binding numbers. For example, the editing target region of the present invention can be a continuous region with a binding number of about 3 or more transcription factors.

For example, when the gene editing system includes CRISPR/Cas9, there may be a protospacer adjacent motif (PAM) downstream of the region targeted by the guiding nucleic acid molecule of the present invention, and the protospacer adjacent motif (PAM) may be AGG, TGG, GGG or CGG. For example, when the PAM region of the target gene is determined, those skilled in the art can easily determine about 15 to about 25 (for example, about 15, about 16, about 17, about 18, about 19, about 20) upstream of the 5' end of the PAM of the target gene. , about 21, about 22, about 23, about 24, about 25) nucleotides, and a suitable gRNA can be designed for the target sequence. For example, the guide nucleic acid molecule is capable of binding to a sequence consisting of about 15 to about 25 nucleotides upstream of the 5' end of a protospacer adjacent motif (PAM) selected from the group consisting of: AGG, TGG, GGG, and CGG.

For example, when the gene editing system includes CRISPR/Cas12, there may be a protospacer adjacent motif (PAM) upstream of the region targeted by the guiding nucleic acid molecule of the present invention, and the protospacer adjacent motif (PAM) may be NTTN, TTYN, VTTV, TRTV, TTTV, TATV, TYCV, TNN, or NTN, where N is A, T, C, or G, Y is T or C, V is A, C, or G, and R is A or G. For example, when the PAM region of the target gene is determined, those skilled in the art can easily determine about 15 to about 25 (for example, about 15, about 16, about 17, about 18, about 19, about 20) downstream of the 3' end of the PAM of the target gene. , about 21, about 22, about 23, about 24, about 25) nucleotides, and a suitable gRNA can be designed for the target sequence. For example, the guide nucleic acid molecule can bind to a sequence consisting of about 15 to about 25 nucleotides downstream of the 3' end of the protospacer adjacent motif (PAM) selected from the group consisting of: NTTN, TTYN, VTTV, TRTV , TTTV, TATV, TYCV, TNN, or NTN, where N is A, T, C, or G, Y is T or C, V is A, C, or G, and R is A or G.

For example, when the gene editing system of the present invention includes wild-type Cas12a (also known as Cpf1, such as AsCas12a, FnCas12a, LbCas12a, BbCas12a, CMaCas12a and OsCas12a), the upstream region of the guiding nucleic acid molecule targeting of the present invention can be selected from the following PAM sequence: NTTN, where N can be A, T, C or G. For example, when the PAM region of the target gene is determined, those skilled in the art can easily determine about 17 to about 25 (for example, about 17, about 18, about 19, about 20, about 21, about 22) downstream of the 3' end of the PAM of the target gene. , about 23, about 24, or about 25) nucleotides, and a suitable gRNA can be designed for the target sequence.

For example, when the gene editing system of the present invention includes mutant Cas12a, such as enAsCas12a (mutation sites E174R, S542R and K548R), the upstream of the region targeted by the guiding nucleic acid molecule of the present invention can have a PAM sequence selected from the following: TTYN (TTTN /TTCN), VTTV (ATTV/CTTV/GTTV), or TRTV (TATV/TGTV), where N can be A, T, C or G, Y can be T or C, V can be A, C or G, R Can be A or G. For example, when the PAM region of the target gene is determined, those skilled in the art can easily determine about 17 to about 25 (for example, about 17, about 18, about 19, about 20, about 21, about 22) downstream of the 3' end of the PAM of the target gene. , about 23, about 24, or about 25) nucleotides, and a suitable gRNA can be designed for the target sequence.

For example, when the gene editing system of the present invention includes mutant Cas12a, such as opAsCas12a (mutation sites: E174R and S542R), the upstream of the region targeted by the guiding nucleic acid molecule of the present invention can have a PAM sequence selected from the following: TTTV (TTTA, TTTC, or TTTG), where V can be A, C, or G. For example, when the PAM region of the target gene is determined, those skilled in the art can easily determine about 17 to about 25 (for example, about 17, about 18, about 19, about 20, about 21, about 22) downstream of the 3' end of the PAM of the target gene. , about 23, about 24, or about 25) nucleotides, and a suitable gRNA can be designed for the target sequence.

For example, when the gene editing system of the present invention includes mutant Cas12a, such as AsCas12a Ultra (mutation sites: M537R and F870L), the upstream of the region targeted by the guiding nucleic acid molecule of the present invention can have a PAM sequence selected from the following: TTTV, TATV, Or TYCV, where V can be A, C or G and Y can be T or C. For example, when the PAM region of the target gene is determined, those skilled in the art can easily determine about 17 to about 25 (for example, about 17, about 18, about 19, about 20, about 21, about 22) downstream of the 3' end of the PAM of the target gene. , about 23, about 24, or about 25) nucleotides, and a suitable gRNA can be designed for the target sequence.

For example, when the gene editing system of the present invention includes mutant Cas12a, such as hfCas12Max (mutation site: N243R/E336R/D892R) and Cas12Max (mutation site: N243R), the upstream region of the guiding nucleic acid molecule targeting of the present invention can be A PAM sequence selected from: TNN, or NTN, where N can be A, T, C, or G. For example, when the PAM region of the target gene is determined, those skilled in the art can easily determine about 17 to about 25 (for example, about 17, about 18, about 19, about 20, about 21, about 22) downstream of the 3' end of the PAM of the target gene. , about 23, about 24, or about 25) nucleotides, and a suitable gRNA can be designed for the target sequence.

For example, the guide nucleic acid molecule may comprise a target sequence, before the PAM region represented by AGG, TGG, GGG and/or CGG in the DNA where the gene encoding the ZC3H12 family member and/or its functionally active fragment is located, of about 10 to about 30 nucleotides.

For example, the guide nucleic acid molecule may comprise a target sequence, before the PAM region represented by AGG, TGG, GGG and/or CGG in the DNA where the gene encoding the ZC3H12 family member and/or its functionally active fragment is located, of about 15 to about 25 and about 17 to about 25, about 19 to about 25, about 20 to about 25, about 21 to about 25, about 23 to about 25, about 15 to about 23, about 17 to about 23, about 19 to about 23 , about 20 to about 23, about 21 to about 23, about 15 to about 21, about 17 to about 21, about 19 to about 21, about 20 to about 21, about 15 to about 20, A target sequence consisting of about 17 to about 20, about 19 to about 21, about 15 to about 19, about 17 to about 19, or about 15 to about 17 nucleotides.

For example, the target sequence may be selected from a region defined by the genomic coordinates shown in Table 1B or a fragment thereof.

For example, the target sequence of the present invention can be the C3H1 zinc finger structural functional domain of ZC3H12A. For example, the target sequence of the present invention may be R_22_chr1:37482635-37482714.

For example, the guide nucleic acid molecule can include a targeting domain that is complementary to a target sequence selected from the group consisting of: SEQ ID NOs: 281-348, 3116-3698.

For example, the guide nucleic acid molecule can include a targeting domain, which can include sequences as set forth in SEQ ID NOs: 213-280, 2533-3115, 7325-7345, 7416, 7417.

For example, the guide nucleic acid molecule can include a targeting domain, which can include sequences as set forth in SEQ ID NOs: 7325-7345, 7416, 7417.

For example, the ratio of cells expressing the product of the gene of interest in cells obtained by reducing the expression and/or activity of at least one target gene of the cell can be reduced and/or the expression level of the target gene in a single cell can be reduced, compared to cells in which the expression and/or activity of the target gene is unchanged.

For example, in the method of the present invention, the target gene is expressed in cells obtained by reducing the expression and/or activity of at least one target gene in the cells compared with cells in which the expression and/or activity of the target gene is unchanged. The cell proportion of the product is reduced by at least about 5%. For example, the proportion of cells expressing the product of the gene encoding the ZC3H12 family member and/or a functionally active fragment thereof is reduced by at least about 100%, at least about 90%, at least about 80%, at least about 70%, at least about 60%, at least About 50%, at least about 40%, at least about 30%, at least about 20%, at least about 19%, at least about 18%, at least about 17%, at least about 16%, at least about 15%, at least about 14%, at least About 13%, at least about 12%, at least about 11%, at least about 10%, at least about 9%, at least about 8%, at least about 7%, at least about 6%, or at least about 5%. For example, the proportion of cells expressing the product of the gene encoding a ZC3H12 family member and/or a functionally active fragment thereof can range from an observable proportion of cells to 0%. For example, the proportion of cells expressing the product of the gene encoding the ZC3H12 family member and/or a functionally active fragment thereof can be reduced to at least about 100%, at least about 90%, at least about 80%, at least about 70%, at least about 60% , at least about 50%, at least about 40%, at least about 30%, at least about 20%, at least about 19%, at least about 18%, at least about 17%, at least about 16%, at least about 15%, at least about 14% , at least about 13%, at least about 12%, at least about 11%, at least about 10%, at least about 9%, at least about 8%, at least about 7%, at least about 6%, at least about 5%, or at least about 1 %. For example, the proportion of cells expressing the product of the gene encoding a ZC3H12 family member and/or a functionally active fragment thereof can be detected by cell flow cytometry.

For example, the proportion of cells expressing the product of the gene encoding the ZC3H12 family member and/or its functionally active fragment among the cells obtained by reducing the expression and/or weakening the activity of at least one target gene of the cells in the method of the present invention can be is at most about 95%. For example, the proportion of cells expressing the product of the gene encoding the ZC3H12 family member and/or a functionally active fragment thereof may be at most about 95%, at most about 90%, at most about 80%, at most about 70%, at most about 60%, up to about 50%, up to about 40%, up to about 30%, up to about 20%, up to about 19%, up to about 18%, up to about 17%, up to about 16%, up to about 15%, up to about 14%, up to about 13%, up to about 12%, up to about 11%, up to about 10%, up to about 9%, up to about 8%, up to about 7%, up to about 6%, or up to about 5%. For example, the proportion of cells expressing the product of the gene encoding a peptidase ZC3H12 family member and/or a functionally active fragment thereof can be detected by cell flow cytometry.

For example, in the method of the present invention, compared with cells in which the expression and/or activity of the target gene are unchanged, the cells obtained by reducing the expression and/or activity of at least one target gene are described in a single cell. The expression level of the target gene can be reduced by at least about 5%. For example, the expression level of the gene of interest in a single cell can be reduced by at least about 100%, at least about 90%, at least about 80%, at least about 70%, at least about 60%, at least about 50%, at least about 40%, at least About 30%, at least about 20%, at least about 19%, at least about 18%, at least about 17%, at least about 16%, at least about 15%, at least about 14%, at least about 13%, at least about 12%, at least About 11%, at least about 10%, at least about 9%, at least about 8%, at least about 7%, at least about 6%, or at least about 5%. For example, the expression level of the target gene in a single cell can range from an observable amount to 0%. For example, the expression level of the gene of interest in a single cell can be reduced to at least about 100%, at least about 90%, at least about 80%, at least about 70%, at least about 60%, at least about 50%, at least about 40% , at least about 30%, at least about 20%, at least about 19%, at least about 18%, at least about 17%, at least about 16%, at least about 15%, at least about 14%, at least about 13%, at least about 12% , at least about 11%, at least about 10%, at least about 9%, at least about 8%, at least about 7%, at least about 6%, at least about 5%, or at least about 1%.

For example, in the method of the present invention, the expression amount of the target gene in a single cell in the cells obtained by reducing the expression and/or activity of at least one target gene can be the expression and/or activity of the target gene. At most about 95% of the cells are unchanged. For example, the expression level of the gene encoding the ZC3H12 family member and/or the functionally active fragment thereof (e.g., the gene encoding ZC3H12A) in a single cell in the cell may be the expression and/or the expression level of the gene encoding the ZC3H12 family member and/or the functionally active fragment thereof. or up to about 95%, up to about 90%, up to about 80%, up to about 70%, up to about 60%, up to about 50%, up to about 40%, up to about 30%, up to about 20% of the cells with unchanged activity %, up to about 19%, up to about 18%, up to about 17%, up to about 16%, up to about 15%, up to about 14%, up to about 13%, up to about 12%, up to about 11%, up to about 10 %, up to about 9%, up to about 8%, up to about 7%, up to about 6%, or up to about 5%.

For example, the methods of the invention comprise: subjecting the cells to at least one stage of in vitro expansion, wherein the expression and/or activity of ZC3H12 family members of the cells is reduced during at least one stage of the in vitro expansion.

For example, the TILs derived from tumor tissue, tumor-related lymph nodes with or without tumor metastasis, tumor metastasis lesions, fragments of para-cancerous tissue, pleural effusion and/or peritoneal effusion and have not been amplified in vitro are subjected to The first stage of in vitro amplification and the second stage of in vitro amplification, and in the second stage of in vitro amplification, the expression and/or activity of the ZC3H12 family members of the TILs that have been amplified in the first stage of in vitro are reduced.

For example, the first stage of in vitro expansion is performed for at least about 7 days. For example, the second stage of in vitro expansion is performed for at least about 7 days.

For example, the cells may be contacted with the one or more cellular activators and the cells may comprise expression of ZC3H12 family members and/or functionally active fragments thereof in a single stage of in vitro expansion of the invention. and/or reduced activity. For example, the cell activator may comprise an agonist for one or more targets selected from the group consisting of: CD3, CD28, HVEM, CD40L, OX40, and 4-1BB. For example, in a single stage of said in vitro expansion, cells of the invention are made to have reduced expression and/or attenuated activity of ZC3H12 family members and are contacted with one or more cell activators of the invention. For example, in the first stage of in vitro expansion of the present invention, the expression and/or activity of the TIL of the present invention and the ZC3H12 family member of the present invention can be reduced and contacted with one or more cell activators of the present invention. For example, in the second stage of in vitro expansion of the present invention, the expression and/or activity of the ZC3H12 family members of the TIL of the present invention can be reduced and contacted with one or more cell activators of the present invention. For example, in the third stage of in vitro expansion of the present invention, the expression and/or activity of the ZC3H12 family members of the TIL of the present invention can be reduced and contacted with one or more cell activators of the present invention.

For example, in a single stage of in vitro expansion of the invention, the expression and/or activity of the peptidase ZC3H12 family member in the cells of the invention can be reduced substantially simultaneously with exposure to one or more cellular activators of the invention. For example, in a single stage of in vitro amplification of the present invention, the cells of the present invention can be made to reduce the expression and/or activity of ZC3H12 family members first, for example, 2 hours in advance, 4 hours in advance, 8 hours in advance, or 8 hours in advance. 12 hours, 24 hours in advance, or 48 hours in advance, etc., and then contacted with one or more cell activators of the present invention. For example, in a single stage of in vitro expansion of the present invention, the cells of the present invention can be contacted with one or more cell activators of the present invention first, for example, 2 hours in advance, 4 hours in advance, 8 hours in advance, 12 hours in advance, 24 hours in advance, or 48 hours in advance, etc., to reduce the expression and/or activity of ZC3H12 family members.

For example, in the first stage of in vitro expansion of the invention, the TIL of the invention can substantially simultaneously reduce the expression and/or activity of ZC3H12 family members and contact one or more cell activators of the invention. For example, in the second stage of in vitro expansion of the present invention, the TIL of the present invention can substantially simultaneously reduce the expression and/or activity of ZC3H12 family members and contact one or more cell activators of the present invention. For example, in the third stage of in vitro expansion of the present invention, the TIL of the present invention can substantially simultaneously reduce the expression and/or activity of ZC3H12 family members and contact one or more cell activators of the present invention.

In another aspect, the invention provides a method for culturing tumor infiltrating lymphocytes (TIL), which may comprise: (A) making cells derived from tumor tissue, pleural effusion and/or peritoneal effusion without in vitro expansion The first TIL population is contacted with one or more cell growth factors; wherein the second TIL population is obtained through the step (A); (B) the expression of the peptidase ZC3H12 family members of the second TIL population is reduced and/ Or the activity is weakened; wherein, the third TIL group is obtained through the step (B).

In the terminology of one embodiment, the first stage in vitro amplification of the present invention can be used arbitrarily interchangeably with step (A) in the method of the above aspect. In the terminology of one embodiment, the second stage in vitro amplification of the present invention can be used interchangeably with step (B) in the method of the above aspect. In one embodiment, the TILs expanded in vitro in the first stage body of the present invention can be optionally replaced with the second TIL population obtained in step (A) of the above method. In one embodiment, the TILs expanded in vitro in the second stage of the present invention can be used arbitrarily interchangeably with the third TIL population obtained in step (B) of the above method. In one embodiment, if necessary, the third stage in vitro amplification of the present invention can be used interchangeably with any additional step (C) in the method of the above aspect. In the one embodiment, if necessary, the third-stage in vitro expanded TIL of the present invention can be used interchangeably with the fourth TIL group obtained by any additional step (C) in the above method.

In another aspect, the present invention provides a method for culturing tumor infiltrating lymphocytes (TIL), which may include: (A) culturing cells derived from tumor tissue, pleural effusion and/or peritoneal effusion without in vitro amplification The first TIL population is contacted with a variety of cell growth factors; wherein, the second TIL population is obtained through the step (A); (B) the second TIL population can be contacted with a variety of cell growth factors, and with a variety of cell growth factors, and the cell activator is contacted to reduce the expression and/or activity of ZC3H12 family members, and the TILs are co-cultured with feeder cells; wherein, the third TIL population is obtained through the step (B).

In another aspect, the present invention provides a method for culturing tumor infiltrating lymphocytes (TIL), which may include: (A) culturing cells derived from tumor tissue, pleural effusion and/or peritoneal effusion without in vitro amplification The first TIL population is contacted with cell growth factors; wherein, the second TIL population is obtained through the step (A); (B) the second TIL population can be contacted with cell growth factors, with cell activators, and the expression and/or activity of ZC3H12 family members is reduced and the TILs are co-cultured with feeder cells. The ZC3H12 family members may include ZC3H12A; wherein the third TIL population is obtained through the step (B).

In another aspect, the invention provides a method of culturing tumor-infiltrating lymphocytes (TIL). The method of obtaining TIL cells from subject tissue samples can be to obtain orthotopic tumor samples or metastatic tumor samples from the patient during surgery, and the weight can be at least about 1g, or multiple pieces of tissue can be merged. Tumor tissue, pleural effusion and/or peritoneal effusion are stored in a sample transport fluid, which may be, for example, a commercially used tumor tissue transport fluid, tumor tissue preservation fluid or tumor tissue transfer fluid, shipped at about 2-8 degrees, processed within 48 hours. The tissue pieces can be mechanically broken to a size of about 1-27 cubic millimeters per piece, transferred into a breathable culture bag or Grex, and added with cell serum-free medium and a concentration of 300-9000IU/mL (for example, it can be 1000-9000IU/mL, for example It can be cultured with IL-2 (6000IU/mL) for about 3-14 days. Collect the cells in the culture medium and transfer them to a breathable culture bag, or Grex, or Xuri equipment. The cell serum-free culture medium can be added with the CD28 antibody, CD3 antibody and CD28 antibody of the present invention, and magnetic beads containing CD3 antibody and CD28 antibody (such as Dynabeads ) and/or a nanomatrix (such as transACT) containing CD3 antibodies and CD28 antibodies, IL-2 at a concentration of 300-9000IU/mL (for example, it can be 1000-9000IU/mL, for example, it can be 6000IU/mL), and the expression and/or activity of the ZC3H12 family members is reduced (ZC3H12 family members can include ZC3H12A, for example, ZC3H12 can be encoded in the TIL by transduction with a ribonucleoprotein complex (RNP) formed by carrying the gRNA of the present invention and the Cas protein) The cell ratio of genes of family members is about 95% or less), after activating the TIL of the present invention for a certain period of time, add irradiated PBMC (the ratio of TIL to PBMC is about 1:40 to about 1:400), and expand and culture for about 3 -14 days. Cell processing systems can be used to collect cells in the culture medium, wash, freeze, and detect. The CD3 ratio of the final product can be greater than 80%, the cell viability rate can be greater than 50%, and the cells greater than 80% can be memory effector cells and effector cells. IFN-γ can be secreted after stimulation, and/or can be characterized by an increase in the proportion of activated cells.

### SOCS1 knockout

1. A method for culturing cells, the method comprising: reducing the expression and/or weakening the activity of STAT-induced STAT inhibitor (SSI) family members and/or functionally active fragments thereof of the cells.
2. The method of embodiment 1, wherein the cells comprise immune cells.
3. The method of embodiment 2, wherein the immune cells comprise phagocytes, lymphocytes, neutrophils, eosinophils and/or basophils.
4. The method according to any one of embodiments 2-3, wherein the immune cells comprise monocytes, macrophages, and/or dendritic cells.
5. The method according to any one of embodiments 2-4, wherein the immune cells are derived from stem cell differentiated immune cells.
6. The method of embodiment 5, wherein the stem cells comprise induced pluripotent stem cells (iPSCs).
7. The method according to any one of embodiments 2-6, wherein the immune cells comprise B cells, T cells, natural killer cells and/or natural killer-like T cells (NKT).
8. The method according to any one of embodiments 2-7, wherein the immune cells comprise αβ T cells and/or γδ T cells.
9. The method of any one of embodiments 2-8, wherein the immune cells comprise tumor-infiltrating lymphocytes (TIL).
10. The method of embodiment 9, wherein the TIL is a TIL derived from fragments of tumor tissue, pleural effusion and/or peritoneal effusion and/or TIL derived from resuscitation after cryopreservation.
11. The method of embodiment 10, wherein the fragment has a volume from about 1 cubic millimeter to about 27 cubic millimeters.
12. The method of any one of embodiments 2-11, wherein the immune cell comprises an engineered immune receptor displayed on the cell surface.
13. The method of embodiment 12, wherein the engineered immune receptor specifically binds to an antigen expressed on a target cell.
14. The method according to any one of embodiments 2-13, wherein the immune cell comprises a chimeric antigen receptor and/or a T cell receptor.
15. The method of any one of embodiments 1-14, wherein reducing expression and/or attenuating activity of a STAT-induced STAT inhibitor (SSI) family member of the cell comprises the function of inhibiting negative regulation of cytokine signaling.
16. The method of any one of embodiments 1-15, wherein the STAT-induced STAT is compared to cells in which the expression and/or activity of STAT-induced STAT inhibitor (SSI) family members is unchanged. Cells obtained with reduced expression and/or attenuated activity of inhibitor (SSI) family members display improved cellular properties.
17. The method of embodiment 16, wherein the improved cell properties comprise one or more selected from the group consisting of: improved cell proliferation capacity, increased proportion of viable cells, improved proportion of cell subpopulations, increased ability to secrete cytokines and improve the killing ability of tumor cells.
18. The method of embodiment 17, wherein the improved proportion of cell subpopulations comprises one or more selected from the group consisting of an increased proportion of activated cells, a reduced proportion of regulatory cells, a reduced proportion of exhausted cells. proportion, increased proportion of central memory cells and/or naive cells, decreased proportion of apoptotic cells and increased proportion of stem cell-like cells.
19. The method of any one of embodiments 1-18, wherein the STAT-induced STAT inhibitor (SSI) family member comprises an SH2 domain.
20. The method of any one of embodiments 1-19, wherein the STAT-induced STAT inhibitor (SSI) family member comprises SOCS1.
21. The method of any one of embodiments 1-20, wherein reducing the expression and/or activity of a STAT-induced STAT inhibitor (SSI) family member comprises introducing a gene regulatory system into the cell.
22. The method of embodiment 21, wherein the gene regulatory system is capable of disrupting the STAT-induced STAT inhibitor (SSI) family member at the DNA level.
23. The method of any one of embodiments 21-22, wherein the gene regulatory system comprises a guide nucleic acid molecule and an enzymatic protein.
24. The method of embodiment 23, wherein reducing the expression and/or attenuating the activity of the STAT-induced STAT inhibitor (SSI) family member comprises: converting a ribose containing the guide nucleic acid molecule and the enzyme protein A nucleoprotein complex (RNP), an LNP comprising a gRNA and a Cas protein, or an LNP comprising a nucleic acid encoding a gRNA and a Cas protein is introduced into the cell.
25. The method of any one of embodiments 23-24, wherein the enzyme protein comprises a Cas protein, a Cas protein homologue, or a functionally active fragment thereof.
26. The method of any one of embodiments 23-25, wherein the guide nucleic acid molecule comprises a guide RNA (gRNA).
27. The method of any one of embodiments 23-26, wherein the guide nucleic acid molecule is capable of binding to a sequence of a member of the STAT-induced STAT inhibitor (SSI) family.
28. The method according to any one of embodiments 23-27, wherein the guide nucleic acid molecule is capable of binding to a region selected from the group defined by the genomic coordinates shown in Table 1C, or a fragment thereof.
29. The method of any one of embodiments 23-28, wherein the guide nucleic acid molecule is capable of binding to a region selected from the group consisting of: SEQ ID NOs: 399-448, 4393-5086, or fragments thereof.
30. The method of any one of embodiments 23-29, wherein the guide nucleic acid molecule is capable of being located about 15 to about 25 upstream of the 5' end of a protospacer adjacent motif (PAM) selected from the group consisting of: Sequence combinations of nucleotides: AGG, TGG, CGG and GGG.
31. The method of any one of embodiments 23-30, wherein the guide nucleic acid molecule comprises a targeting domain comprising SEQ ID NOs: 349-398, 3699-4392, 7346 -The sequence shown in any one of 7375 and 7418.
32. The method of any one of embodiments 1-31, wherein the STAT-induced STAT is compared to cells in which expression and/or activity of STAT-induced STAT inhibitor (SSI) family members is unchanged. The expression and/or activity of inhibitor (SSI) family members is reduced and the proportion of cells expressing the product of the target gene in the cells obtained is reduced and/or the expression level of the target gene in a single cell is reduced.
33. The method according to any one of embodiments 1-32, wherein the gene of interest is expressed in cells obtained by reducing the expression and/or attenuating the activity of the STAT-induced STAT inhibitor (SSI) family member. The proportion of cells is about 95% or less.
34. A cell obtained by the method of any one of embodiments 1-33.
35. A composition comprising the cell of embodiment 34.
36. A pharmaceutical composition comprising the cell of embodiment 34 and/or the composition of embodiment 35, and optionally a pharmaceutically acceptable carrier.
37. A method of affecting cell growth, comprising administering the cell of embodiment 34, the composition of embodiment 35, and/or the pharmaceutical composition of embodiment 36.
38. Use of the cell of embodiment 34, the composition of embodiment 35 and/or the pharmaceutical composition of embodiment 36 in the preparation of a medicament, wherein the medicament is used to prevent and/or treat diseases and /or symptoms.
39. Use according to embodiment 38, wherein the disease and/or condition comprises a tumor.
40. Use according to any one of embodiments 38-39, wherein the disease and/or condition comprises a solid tumor.
41. Use according to any one of embodiments 38-40, wherein the disease and/or condition comprises one or more selected from the group consisting of: melanoma, ovarian cancer, cervical cancer, lung cancer, bladder cancer , breast cancer, head and neck cancer, pancreatic cancer, liver cancer, stomach cancer, colorectal cancer and kidney cancer.

The present invention provides methods for reducing the expression and/or attenuating the activity of STAT-induced STAT inhibitor (SSI) family members and/or functionally active fragments thereof in said cells.

In one aspect, the present invention provides a method for culturing cells such that the expression and/or activity of STAT-induced STAT inhibitor (SSI) family members and/or functionally active fragments thereof is reduced in the cells. For example, the STAT-induced STAT inhibitor (SSI) family member may comprise an SH2 domain. For example, the STAT-induced STAT inhibitor (SSI) family member may comprise SOCS1.

For example, the target gene of the present invention may be a gene encoding a STAT-induced STAT inhibitor (SSI) family member and/or a functionally active fragment thereof. For example, a cell obtained by reducing the expression and/or attenuating the activity of at least one target gene of the cell may exhibit improved cell properties compared to a cell in which the expression and/or activity of the target gene is unchanged. In one embodiment, cells whose expression and/or activity of the target gene are unchanged may refer to cells originating from the same donor and in which the expression and/or activity of at least one target gene of the cell has not been reduced. In one embodiment, cells whose expression and/or activity of the target gene are unchanged may refer to cells derived from the same donor and in which the expression and/or activity of other genes other than the target gene of the cells have not been altered (e.g., knocking out the other genes has basically no impact on cell function).

For example, the cells include immune cells. For example, the cells comprise immune effector cells. For example, the cells include immune effector T cells, immune effector NK cells, and immune effector NKT cells. For example, the cells include phagocytes, lymphocytes, neutrophils, eosinophils, and/or basophils.

For example, the cells include monocytes, macrophages, and/or dendritic cells.

For example, the cells of the present invention also include cells derived from differentiation of stem cells. For example, the cells of the present invention also include cells derived from differentiation of pluripotent stem cells. For example, the stem cells of the present invention can be obtained by induction. For example, the above-mentioned stem cells of the present invention may include induced pluripotent stem cells (iPSCs), embryonic stem cells, bone marrow stem cells, umbilical cord blood stem cells and/or peripheral blood stem cells.

For example, "stem cells" of the present invention also include pluripotent cells, multipotent cells, precursor cells and progenitor cells. For example, stem cells can be obtained from hematopoietic or mesenchymal stem cells obtained from bone marrow tissue, placental stem cells obtained from placental tissue, embryonic stem cells obtained from embryonic tissue, or embryonic germ cells obtained from the reproductive tissue of the fetus. Exemplary pluripotent stem cells can also be generated from somatic cells by reprogramming them to a pluripotent state through the expression of certain transcription factors associated with pluripotency; these cells are called "induced pluripotent stem cells" or "iPSCs" .

For example, the cells include B cells, T cells, natural killer cells and/or natural killer-like T cells (NKT). For example, "unmodified cells" or "unmodified cells" may refer to cells in which the genome has not been modified and does not contain a gene regulatory system or contains a control gene regulatory system (e.g., empty vector control, non-targeting gRNA, interfering siRNA, etc. ) cells or cell populations. For example, the cells comprise αβ T cells and/or γδ T cells. For example, the cells include tumor-infiltrating lymphocytes (TILs). For example, the TIL is a TIL derived from tumor tissue, tumor-associated lymph nodes with or without tumor metastasis, tumor metastasis, fragments of paracancerous tissue, pleural effusion and/or peritoneal effusion and/or TILs recovered from cryopreservation.

For example, the TILs of the present invention may be TILs and/or sources derived from tumor tissue, tumor-associated lymph nodes with or without tumor metastasis, tumor metastasis foci, fragments of paracancerous tissue, pleural effusion and/or peritoneal effusion. TILs recovered after cryopreservation. For example, TILs of the present invention can be obtained by processing tumor tissue into tumor fragments. For example, the tumor fragments of the present invention have a volume of about 1-27 cubic millimeters. For example, the volume of the tumor fragments of the present invention is about 1 cubic millimeter, about 2 cubic millimeters, about 3 cubic millimeters, about 4 cubic millimeters, about 5 cubic millimeters, about 6 cubic millimeters, about 7 cubic millimeters, about 8 cubic millimeters. , about 9 cubic millimeters, about 10 cubic millimeters, about 11 cubic millimeters, about 12 cubic millimeters, about 13 cubic millimeters, about 14 cubic millimeters, about 15 cubic millimeters, about 16 cubic millimeters, about 17 cubic millimeters, about 18 cubic millimeters , about 19 cubic millimeters, about 20 cubic millimeters, about 21 cubic millimeters, about 23 cubic millimeters, about 24 cubic millimeters, about 25 cubic millimeters, about 26 cubic millimeters or about 27 cubic millimeters.

For example, the cells comprise engineered immune receptors displayed on the cell surface. For example, the engineered immune receptor specifically binds to an antigen expressed on a target cell. For example, the cells comprise chimeric antigen receptors and/or T cell receptors.

In one aspect, the present invention provides a method for culturing tumor infiltrating lymphocytes (TIL), which may include: reducing expression and/or activity of STAT-induced STAT inhibitor (SSI) family members and/or functionally active fragments thereof.

For example, TILs derived from tumor tissue, pleural effusion and/or peritoneal effusion without in vitro amplification can be subjected to at least one stage of in vitro amplification, wherein in at least one stage of in vitro amplification, the expression and/or activity of STAT-induced STAT inhibitor (SSI) family members and/or functionally active fragments thereof are reduced in the TIL.

For example, the TILs of the present invention derived from tumor tissue, pleural effusion and/or peritoneal effusion without in vitro amplification can be subjected to the first stage of in vitro amplification and the second stage of in vitro amplification, and in the second stage of the in vitro amplification, the expression and/or activity of STAT-induced STAT inhibitor (SSI) family members and/or functionally active fragments thereof in the TIL can be reduced.

For example, the TILs of the present invention derived from tumor tissue, pleural effusion and/or peritoneal effusion without in vitro amplification can be subjected to the first stage of in vitro amplification and the second stage of in vitro amplification, and in the first stage of the in vitro amplification, the expression and/or activity of STAT-induced STAT inhibitor (SSI) family members and/or functionally active fragments thereof in the TIL can be reduced.

For example, the TILs of the present invention derived from tumor tissue, pleural effusion and/or peritoneal effusion without in vitro amplification can be subjected to the first stage of in vitro amplification and the second stage of in vitro amplification, and in the first stage of the in vitro amplification, the expression and/or activity of the STAT-induced STAT inhibitor (SSI) family members and/or functionally active fragments thereof in the TIL can be reduced, and in the second stage of the in vitro amplification, the expression and/or activity of STAT-induced STAT inhibitor (SSI) family members and/or functionally active fragments thereof in the TIL can be reduced

For example, the TILs of the present invention derived from tumor tissue, pleural effusion and/or peritoneal effusion without in vitro amplification can be subjected to a first-stage in vitro amplification, a second-stage in vitro amplification and a third-stage in vitro amplification, and in the first stage of in vitro amplification of the present invention, the expression and/or activity of STAT-induced STAT inhibitor (SSI) family members and/or functionally active fragments thereof in the TIL can be reduced.

For example, the TILs of the present invention derived from tumor tissue, pleural effusion and/or peritoneal effusion without in vitro amplification can be subjected to a first-stage in vitro amplification, a second-stage in vitro amplification and a third-stage in vitro amplification, and in the second stage of in vitro amplification of the present invention, the expression and/or activity of STAT-induced STAT inhibitor (SSI) family members and/or functionally active fragments thereof in the TIL can be reduced.

For example, the TILs of the present invention derived from tumor tissue, pleural effusion and/or peritoneal effusion without in vitro amplification can be subjected to a first-stage in vitro amplification, a second-stage in vitro amplification and a third-stage in vitro amplification, and in the third stage of in vitro amplification of the present invention, the expression and/or activity of STAT-induced STAT inhibitor (SSI) family members and/or functionally active fragments thereof in the TIL can be reduced.

For example, the TILs of the present invention derived from tumor tissue, pleural effusion and/or peritoneal effusion without in vitro amplification can be subjected to a first-stage in vitro amplification, a second-stage in vitro amplification and a third-stage in vitro amplification, and in the first stage of in vitro expansion of the present invention, the expression and/or activity of STAT-induced STAT inhibitor (SSI) family members and/or functionally active fragments thereof in the TIL can be reduced, and in the second stage of in vitro expansion of the present invention, the expression and/or activity of STAT-induced STAT inhibitor (SSI) family members and/or functionally active fragments thereof in the TIL can be reduced.

For example, the TILs of the present invention derived from tumor tissue, pleural effusion and/or peritoneal effusion without in vitro amplification can be subjected to a first-stage in vitro amplification, a second-stage in vitro amplification and a third-stage in vitro amplification, and in the first stage of in vitro expansion of the present invention, the expression and/or activity of STAT-induced STAT inhibitor (SSI) family members and/or functionally active fragments thereof in the TIL can be reduced, and in the third stage of in vitro expansion of the present invention, the expression and/or activity of STAT-induced STAT inhibitor (SSI) family members and/or functionally active fragments thereof in the TIL can be reduced.

For example, the TILs of the present invention derived from tumor tissue, pleural effusion and/or peritoneal effusion without in vitro amplification can be subjected to a first-stage in vitro amplification, a second-stage in vitro amplification and a third-stage in vitro amplification, and in the second stage of in vitro expansion of the present invention, the expression and/or activity of STAT-induced STAT inhibitor (SSI) family members and/or functionally active fragments thereof in the TIL can be reduced, and in the third stage of in vitro expansion of the present invention, the expression and/or activity of STAT-induced STAT inhibitor (SSI) family members and/or functionally active fragments thereof in the TIL can be reduced.

For example, the TILs of the present invention derived from tumor tissue, pleural effusion and/or peritoneal effusion without in vitro amplification can be subjected to a first-stage in vitro amplification, a second-stage in vitro amplification and a third-stage in vitro amplification. , and in the first stage of in vitro expansion of the present invention, the expression and/or activity of STAT-induced STAT inhibitor (SSI) family members and/or functionally active fragments thereof in the TIL can be reduced, in the second stage of in vitro expansion of the present invention, the expression and/or activity of STAT-induced STAT inhibitor (SSI) family members and/or functionally active fragments thereof in the TIL can be reduced, and in the third stage of in vitro amplification of the present invention, the expression and/or activity of STAT-induced STAT inhibitor (SSI) family members and/or functionally active fragments thereof in the TIL can be reduced.

For example, cells obtained by reducing the expression and/or attenuating the activity of the STAT-induced STAT inhibitor (SSI) family member exhibit improved cellular properties compared to cells in which the expression and/or activity of the STAT-induced STAT inhibitor (SSI) family member is unchanged.

For example, improved cell numbers of the present invention refer to STAT-induced STAT inhibitor (SSI) family members whose expression and/or activity are unchanged compared to cells in which said STAT is induced during at least one in vitro expansion stage. The cell number of cells of the invention with reduced expression and/or weakened activity of STAT inhibitor (SSI) family members can be increased by at least about 1 time, at least about 2 times, at least about 3 times, at least about 4 times, at least about 5 times, At least about 6 times, at least about 7 times, at least about 8 times, at least about 9 times, at least about 10 times, at least about 11 times, at least about 12 times, at least about 13 times, at least about 14 times, at least about 15 times, At least about 20 times, at least about 30 times, at least about 40 times, or at least about 50 times.

For example, an increased proportion of viable cells can manifest itself as an increase in cell viability. For example, the increased proportion of viable cells of the present invention may refer to STAT-induced STAT inhibitor (SSI) family members in which the expression and/or activity of the STAT inhibitor (SSI) family members is unchanged compared to cells that increase the proportion of viable cells in at least one in vitro expansion stage. Decreased expression of induced STAT inhibitor (SSI) family members and/or the proportion of viable cells of the cells of the invention with reduced activity can be increased by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%.

For example, the increased cytokine secretion ability of the present invention may refer to an increase in the cytokine secretion ability of a cell selected from the group consisting of: IL-2, IL-6, CD107a, GZMB, TNF-α, and IFN-γ. For example, the improved cytokine secretion capacity of the present invention may refer to the STAT-induced STAT inhibitor (SSI) family member's expression and/or activity compared to cells whose expression and/or activity is unchanged in at least one in vitro expansion stage. The proportion of cells secreting cytokines in the cells of the present invention in which the expression and/or activity of STAT-induced STAT inhibitor (SSI) family members is reduced can be increased by at least about 1 times, at least about 2 times, at least about 3 times, at least about 4 times. times, at least about 5 times, at least about 6 times, at least about 7 times, at least about 8 times, at least about 9 times, at least about 10 times, at least about 11 times, at least about 12 times, at least about 13 times, at least about 14 times, at least about 15 times, at least about 20 times, at least about 30 times, at least about 40 times, or at least about 50 times. For example, the improved cytokine secretion capacity of the present invention may refer to the STAT-induced STAT inhibitor (SSI) family member's expression and/or activity compared to cells whose expression and/or activity is unchanged in at least one in vitro expansion stage. The proportion of cells secreting cytokines in the cells of the invention in which the expression and/or activity of STAT-induced STAT inhibitor (SSI) family members is reduced can be increased by at least about 100%, at least about 90%, at least about 80%, at least about 70 %, at least about 60%, at least about 50%, at least about 40%, at least about 30%, at least about 20%, at least about 19%, at least about 18%, at least about 17%, at least about 16%, at least about 15 %, at least about 14%, at least about 13%, at least about 12%, at least about 11%, at least about 10%, at least about 9%, at least about 8%, at least about 7%, at least about 6%, at least about 5 %, at least about 4%, at least about 3%, at least about 2%, at least about 1%, at least about 0.5%, at least about 0.4%, at least about 0.3%, at least about 0.2%, or at least about 0.1%.

For example, the improved tumor cell killing ability of the present invention may refer to the improvement of the STAT-induced STAT inhibitor (SSI) family member's expression and/or activity compared to cells in which the expression and/or activity of the STAT-induced STAT inhibitor (SSI) family members is not changed in at least one in vitro expansion stage. The tumor cell killing rate of the cells of the present invention with reduced expression and/or weakened activity of STAT-induced STAT inhibitor (SSI) family members can be increased by at least about 1 time, at least about 2 times, at least about 3 times, at least about 4 times, At least about 5 times, at least about 6 times, at least about 7 times, at least about 8 times, at least about 9 times, at least about 10 times, at least about 11 times, at least about 12 times, at least about 13 times, at least about 14 times, At least about 15 times, at least about 20 times, at least about 30 times, at least about 40 times, or at least about 50 times. For example, the improved tumor cell killing ability of the present invention may refer to the improvement of the STAT-induced STAT inhibitor (SSI) family member's expression and/or activity compared to cells in which the expression and/or activity of the STAT-induced STAT inhibitor (SSI) family members is not changed in at least one in vitro expansion stage. The tumor cell killing rate of the cells of the present invention with reduced expression and/or activity of STAT-induced STAT inhibitor (SSI) family members can be increased by at least about 100%, at least about 90%, at least about 80%, at least about 70%, At least about 60%, at least about 50%, at least about 40%, at least about 30%, at least about 20%, at least about 19%, at least about 18%, at least about 17%, at least about 16%, at least about 15%, At least about 14%, at least about 13%, at least about 12%, at least about 11%, at least about 10%, at least about 9%, at least about 8%, at least about 7%, at least about 6%, at least about 5%, At least about 4%, at least about 3%, at least about 2%, at least about 1%, at least about 0.5%, at least about 0.4%, at least about 0.3%, at least about 0.2%, or at least about 0.1%. For example, the tumor cell killing rate of the cells of the present invention can be measured by the IncuCyte system or CFSE and DAPI staining methods. For example, tumor cell killing of cells of the present invention may refer to the ability of the cells to kill solid tumor cells.

For example, the improved cell subpopulation ratio of the present invention may comprise one or more selected from the following group: increased CD8⁺ Cell proportion, increased central memory cell and/or naive cell proportion, decreased regulatory cell proportion, increased activated cell proportion, increased tumor-specific cell proportion, and stem cell-like cell proportion increased.

For example, the proportion of CD8⁺ cells, central memory cells and/or immature cells, activated cells, tumor-specific cells and/or stem cell-like cells can be increased by at least about 100%, at least about 90%, at least about 80%, at least about 70%, at least about 60%, at least about 50%, at least about 40%, At least about 30%, at least about 20%, at least about 19%, at least about 18%, at least about 17%, at least about 16%, at least about 15%, at least about 14%, at least about 13%, at least about 12%, At least about 11%, at least about 10%, at least about 9%, at least about 8%, at least about 7%, at least about 6%, at least about 5%, at least about 4%, at least about 3%, at least about 2%, At least about 1%, at least about 0.5%, at least about 0.4%, at least about 0.3%, at least about 0.2%, or at least about 0.1%.

For example, the reduced proportion of exhausted cells of the present invention can be increase of PD-1⁺ ,LAG-3⁺ ,TIM-3⁺ , and/or CD39⁺ cell proportion. For example, the reduced proportion of regulatory cells of the present invention can be reduction of CD4⁺ CD25⁺ Foxp3⁺ cell proportion. For example, the reduced proportion of apoptotic cells of the present invention can be reduction of CD95⁺ caspass3⁺ and/or CD95⁺ DR5⁺ cell proportion.

For example, the proportion of exhausted cells, regulatory cells and/or apoptotic cells in the cells can be reduced by at least about 100%, at least about 90%, at least about 80%, at least about 70%, at least about 60%, at least about 50%, At least about 40%, at least about 30%, at least about 20%, at least about 19%, at least about 18%, at least about 17%, at least about 16%, at least about 15%, at least about 14%, at least about 13%, At least about 12%, at least about 11%, at least about 10%, at least about 9%, at least about 8%, at least about 7%, at least about 6%, at least about 5%, at least about 4%, at least about 3%, At least about 2%, at least about 1%, at least about 0.5%, at least about 0.4%, at least about 0.3%, at least about 0.2%, or at least about 0.1%, or can be reduced by at least about 1 times, at least about 2 times, at least about 3 times, at least about 4 times, at least about 5 times, at least about 6 times, at least about 7 times, at least about 8 times, at least about 9 times, at least about 10 times, at least about 11 times, at least about 12 times, at least about 13 times, at least about 14 times, at least about 15 times, at least about 20 times, at least about 30 times, at least about 40 times, or at least about 50 times.

For example, the culture method of the present invention may include a gene editing step for cells. For example, it includes: subjecting the cells to at least one stage of in vitro expansion, wherein a gene regulatory system can be introduced into the cells during at least one stage of the in vitro expansion.

For example, the gene regulatory system can destroy the target gene at the DNA level. For example, the gene regulatory system can disrupt a region of the target gene or a fragment thereof in the genome of the cell. For example, after using the gene regulation system, the DNA region or fragment thereof where the target gene is located in the cell is sheared, and the expression ability of the target gene is reduced or the activity of the target gene is inhibited. For example, the editing effect of the gene regulation system on the target gene can be long-term and sustained. The genomic region of the present invention is determined based on the hg38 version of the human reference genome.

For example, the gene regulatory system may include guide nucleic acid molecules and enzymatic proteins. For example, the enzyme protein can have nucleic acid shearing enzyme activity, and the guide nucleic acid molecule can guide the enzyme protein to specifically cut the region where the target gene is located or its fragments. For example, the guide nucleic acid molecule and the enzyme protein may exist in the form of a ribonucleoprotein complex (RNP), or each may exist independently. For example, the enzyme protein may comprise Cas protein. For example, polynucleotides encoding gRNA and Cas protein can be introduced into the target cell, or each independently.

For example, reducing the expression and/or weakening the activity of at least one target gene of a cell according to the present invention may include: introducing a ribonucleoprotein complex (RNP) containing the guide nucleic acid molecule and the enzyme protein into the cell. For example, the enzyme protein may comprise Cas protein, Cas protein homologues, or functionally active fragments thereof. For example, the guide nucleic acid molecule may comprise guide RNA (gRNA). For example, the guide nucleic acid molecule may comprise guide RNA (gRNA). For example, a complex comprising a polynucleotide encoding a gRNA and a Cas protein can be introduced into the cell. For example, a complex containing gRNA and Cas protein can be introduced into the cell.

For example, the gRNA can be used to bind to the sequence of the target gene. For example, the binding of the gRNA to the sequence of the target gene may be completely complementary, partially complementary, or may hybridize to the sequence of the target gene under moderately stringent or stringent conditions. For example, the binding of the gRNA to the sequence of the target gene can cause the CRISPR system to specifically cut the target gene.

For example, the editing target region of the present invention may be a region before the promoter. For example, the editing target region of the present invention may be a region with high transcription factor binding capacity. For example, the editing target region of the present invention may be a region with a specific number of transcription factor binding numbers. For example, the editing target region of the present invention may be a continuous region with a binding number of about 3 or more transcription factors.

For example, when the gene editing system includes CRISPR/Cas9, there may be a protospacer adjacent motif (PAM) downstream of the region targeted by the guiding nucleic acid molecule of the present invention, and the protospacer adjacent motif (PAM) may be AGG, TGG, GGG or CGG. For example, when the PAM region of the target gene is determined, those skilled in the art can easily determine about 15 to about 25 (for example, about 15, about 16, about 17, about 18, about 19, about 20) upstream of the 5' end of the PAM of the target gene. , about 21, about 22, about 23, about 24, about 25) nucleotides, and a suitable gRNA can be designed for the target sequence. For example, the guide nucleic acid molecule is capable of binding to a sequence consisting of about 15 to about 25 nucleotides upstream of the 5' end of a protospacer adjacent motif (PAM) selected from the group consisting of: AGG, TGG, GGG, and CGG.

For example, when the gene editing system includes CRISPR/Cas12, there may be a protospacer adjacent motif (PAM) upstream of the region targeted by the guiding nucleic acid molecule of the present invention, and the protospacer adjacent motif (PAM) may be NTTN, TTYN, VTTV, TRTV, TTTV, TATV, TYCV, TNN, or NTN, where N is A, T, C, or G, Y is T or C, V is A, C, or G, and R is A or G. For example, when the PAM region of the target gene is determined, those skilled in the art can easily determine about 15 to about 25 (for example, about 15, about 16, about 17, about 18, about 19, about 20) downstream of the 3' end of the PAM of the target gene. , about 21, about 22, about 23, about 24, about 25) nucleotides, and a suitable gRNA can be designed for the target sequence. For example, the guide nucleic acid molecule can bind to a sequence consisting of about 15 to about 25 nucleotides downstream of the 3' end of the protospacer adjacent motif (PAM) selected from the group consisting of: NTTN, TTYN, VTTV, TRTV ,TTTV,TATV, TYCV, TNN, or NTN, where N is A, T, C, or G, Y is T or C, V is A, C, or G, and R is A or G.

For example, when the gene editing system of the present invention includes wild-type Cas12a (also known as Cpf1, such as AsCas12a, FnCas12a, LbCas12a, BbCas12a, CMaCas12a and OsCas12a), the upstream region of the guiding nucleic acid molecule targeting of the present invention can be selected from the following PAM sequence: NTTN, where N can be A, T, C or G. For example, when the PAM region of the target gene is determined, those skilled in the art can easily determine about 17 to about 25 (for example, about 17, about 18, about 19, about 20, about 21, about 22) downstream of the 3' end of the PAM of the target gene. , about 23, about 24, or about 25) nucleotides, and a suitable gRNA can be designed for the target sequence.

For example, when the gene editing system of the present invention includes mutant Cas12a, such as enAsCas12a (mutation sites E174R, S542R and K548R), the upstream of the region targeted by the guiding nucleic acid molecule of the present invention can have a PAM sequence selected from the following: TTYN (TTTN /TTCN), VTTV (ATTV/CTTV/GTTV), or TRTV (TATV/TGTV), where N can be A, T, C or G, Y can be T or C, V can be A, C or G, R Can be A or G. For example, when the PAM region of the target gene is determined, those skilled in the art can easily determine about 17 to about 25 (for example, about 17, about 18, about 19, about 20, about 21, about 22) downstream of the 3' end of the PAM of the target gene. , about 23, about 24, or about 25) nucleotides, and a suitable gRNA can be designed for the target sequence.

For example, when the gene editing system of the present invention includes mutant Cas12a, such as opAsCas12a (mutation sites: E174R and S542R), the upstream of the region targeted by the guiding nucleic acid molecule of the present invention can have a PAM sequence selected from the following: TTTV (TTTA, TTTC, or TTTG), where V can be A, C, or G. For example, when the PAM region of the target gene is determined, those skilled in the art can easily determine about 17 to about 25 (for example, about 17, about 18, about 19, about 20, about 21, about 22) downstream of the 3' end of the PAM of the target gene. , about 23, about 24, or about 25) nucleotides, and a suitable gRNA can be designed for the target sequence.

For example, when the gene editing system of the present invention includes mutant Cas12a, such as AsCas12a Ultra (mutation sites: M537R and F870L), the upstream of the region targeted by the guiding nucleic acid molecule of the present invention can have a PAM sequence selected from the following: TTTV, TATV, Or TYCV, where V can be A, C or G and Y can be T or C. For example, when the PAM region of the target gene is determined, those skilled in the art can easily determine about 17 to about 25 (for example, about 17, about 18, about 19, about 20, about 21, about 22) downstream of the 3' end of the PAM of the target gene. , about 23, about 24, or about 25) nucleotides, and a suitable gRNA can be designed for the target sequence.

For example, when the gene editing system of the present invention includes mutant Cas12a, such as hfCas12Max (mutation site: N243R/E336R/D892R) and Cas12Max (mutation site: N243R), the upstream region of the guiding nucleic acid molecule targeting of the present invention can be A PAM sequence selected from: TNN, or NTN, where N can be A, T, C, or G. For example, when the PAM region of the target gene is determined, those skilled in the art can easily determine about 17 to about 25 (for example, about 17, about 18, about 19, about 20, about 21, about 22) downstream of the 3' end of the PAM of the target gene. , about 23, about 24, or about 25) nucleotides, and a suitable gRNA can be designed for the target sequence.

For example, the guide nucleic acid molecule may comprise a target sequence, before the PAM region represented by AGG, TGG, GGG and/or CGG in the DNA where the gene encoding the STAT-induced STAT inhibitor (SSI) family member and/or its functionally active fragment is located, of about 10 to about 30 nucleotides. For example, the guide nucleic acid molecule may comprise a target sequence, before the PAM region represented by AGG, TGG, GGG and/or CGG in the DNA where the gene encoding the STAT-induced STAT inhibitor (SSI) family member and/or its functionally active fragment is located, of about 15 to about 25 and about 17 to about 25, about 19 to about 25, about 20 to about 25, about 21 to about 25, about 23 to about 25, about 15 to about 23, about 17 to about 23, about 19 to about 23 , about 20 to about 23, about 21 to about 23, about 15 to about 21, about 17 to about 21, about 19 to about 21, about 20 to about 21, about 15 to about 20, A target sequence consisting of about 17 to about 20, about 19 to about 21, about 15 to about 19, about 17 to about 19, or about 15 to about 17 nucleotides.

For example, the target sequence may be selected from the region defined by the genomic coordinates shown in Table 1C or a fragment thereof.

For example, the guide nucleic acid molecule can include a targeting domain that is complementary to a target sequence selected from the group consisting of: SEQ ID NOs: 399-448, 4393-5086.

For example, the guide nucleic acid molecule can include a targeting domain, which can include sequences as set forth in SEQ ID NOs: 349-398, 3699-4392, 7346-7375, 7418.

For example, the guide nucleic acid molecule can include a targeting domain, which can include sequences as shown in SEQ ID NOs: 7346-7375, 7418.

For example, the ratio of cells expressing the product of the gene of interest in cells obtained by reducing the expression and/or activity of at least one target gene of the cell compared to cells in which the expression and/or activity of the target gene is unchanged. can be reduced and/or the expression level of the target gene in a single cell can be reduced.

For example, in the method of the present invention, the target gene is expressed in cells obtained by reducing the expression and/or activity of at least one target gene in the cells compared with cells in which the expression and/or activity of the target gene is unchanged. The cell proportion of the product is reduced by at least about 5%. For example, the proportion of cells expressing the product of the gene encoding a STAT-induced STAT inhibitor (SSI) family member and/or a functionally active fragment thereof is reduced by at least about 100%, at least about 90%, at least about 80%, at least about 70 %, at least about 60%, at least about 50%, at least about 40%, at least about 30%, at least about 20%, at least about 19%, at least about 18%, at least about 17%, at least about 16%, at least about 15 %, at least about 14%, at least about 13%, at least about 12%, at least about 11%, at least about 10%, at least about 9%, at least about 8%, at least about 7%, at least about 6%, or at least about 5%. For example, the proportion of cells expressing the product of the gene encoding a STAT-induced STAT inhibitor (SSI) family member and/or a functionally active fragment thereof can range from an observable proportion of cells to 0%. For example, the proportion of cells expressing the product of the gene encoding a STAT-induced STAT inhibitor (SSI) family member and/or a functionally active fragment thereof can be reduced to at least about 100%, at least about 90%, at least about 80%, at least About 70%, at least about 60%, at least about 50%, at least about 40%, at least about 30%, at least about 20%, at least about 19%, at least about 18%, at least about 17%, at least about 16%, at least About 15%, at least about 14%, at least about 13%, at least about 12%, at least about 11%, at least about 10%, at least about 9%, at least about 8%, at least about 7%, at least about 6%, at least About 5%, or at least about 1%. For example, the proportion of cells expressing the product of the gene encoding a STAT-induced STAT inhibitor (SSI) family member and/or a functionally active fragment thereof can be detected by cell flow cytometry.

For example, in the method of the present invention, the expression of at least one target gene of the cells is reduced and/or the activity is weakened, and the cells obtained express the STAT-induced STAT inhibitor (SSI) family member and/or its functionally active fragment. The cellular proportion of the gene's product may be up to about 95%. For example, the proportion of cells expressing the product of the gene encoding a STAT-induced STAT inhibitor (SSI) family member and/or a functionally active fragment thereof may be at most about 95%, at most about 90%, at most about 80% , at most about 70%, at most about 60%, at most about 50%, at most about 40%, at most about 30%, at most about 20%, at most about 19%, at most about 18%, at most about 17%, at most about 16% , at most about 15%, at most about 14%, at most about 13%, at most about 12%, at most about 11%, at most about 10%, at most about 9%, at most about 8%, at most about 7%, at most about 6% , or at most about 5%. For example, the proportion of cells expressing the product of the gene encoding a STAT-induced STAT inhibitor (SSI) family member and/or a functionally active fragment thereof can be detected by cell flow cytometry.

For example, in the method of the present invention, compared with cells in which the expression and/or activity of the target gene are unchanged, the cells obtained by reducing the expression and/or activity of at least one target gene are described in a single cell. The expression level of the target gene can be reduced by at least about 5%. For example, the expression level of the gene of interest in a single cell can be reduced by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, or at least about 5%. For example, the expression level of the target gene in a single cell can range from an observable amount to 0%. For example, the expression level of the gene of interest in a single cell can be reduced to at least about 100%, at least about 90%, at least about 80%, at least about 70%, at least about 60%, at least about 50%, at least about 40% , at least about 30%, at least about 20%, at least about 19%, at least about 18%, at least about 17%, at least about 16%, at least about 15%, at least about 14%, at least about 13%, at least about 12% , at least about 11%, at least about 10%, at least about 9%, at least about 8%, at least about 7%, at least about 6%, at least about 5%, or at least about 1%.

For example, in the method of the present invention, the expression amount of the target gene in a single cell in the cells obtained by reducing the expression and/or activity of at least one target gene can be the expression and/or activity of the target gene. At most about 95% of the cells are unchanged. For example, the expression level of a gene encoding a STAT-induced STAT inhibitor (SSI) family member and/or a functionally active fragment thereof (e.g., a gene encoding SOCS1) in a single cell in a cell can be the amount of the gene encoding a STAT-induced STAT inhibitor. Up to about 95%, up to about 90%, up to about 80%, up to about 70%, up to about 60%, up to about 60% of cells in which the expression and/or activity of (SSI) family members and/or functionally active fragments thereof is unchanged. 50%, up to about 40%, up to about 30%, up to about 20%, up to about 19%, up to about 18%, up to about 17%, up to about 16%, up to about 15%, up to about 14%, up to about 13%, up to about 12%, up to about 11%, up to about 10%, up to about 9%, up to about 8%, up to about 7%, up to about 6%, or up to about 5%.

For example, methods of the present invention comprise: subjecting said cells to at least one stage of in vitro expansion, wherein in at least one stage of said in vitro expansion, the STAT-induced STAT inhibitor (SSI) family member of said cells is expression is reduced and/or activity is reduced.

For example, the TILs derived from tumor tissue, tumor-associated lymph nodes, with or without tumor metastasis, tumor metastasis, fragments of paracancerous tissue, pleural effusion and/or peritoneal effusion and has not been amplified in vitro have undergone the first stage of in vitro amplification and the second stage of in vitro amplification, and in the second stage In the staged in vitro amplification, the expression and/or activity of the STAT-induced STAT inhibitor (SSI) family members of the TILs amplified in the first stage in vitro is reduced.

For example, the first stage of in vitro expansion is performed for at least about 7 days. For example, the second stage of in vitro expansion is performed for at least about 7 days.

For example, the cells may be contacted with the one or more cellular activators and the cells may comprise STAT-inducing STAT inhibitor (SSI) family members and /or reduced expression and/or activity of functionally active fragments thereof. For example, the cell activator may comprise an agonist for one or more targets selected from the group consisting of: CD3, CD28, HVEM, CD40L, OX40, and 4-1BB. For example, in a single stage of said in vitro expansion, the expression and/or activity of STAT-induced STAT inhibitor (SSI) family members of the cells of the invention is reduced and is associated with the activation of one or more cells of the invention. agent contact. For example, in the first stage of in vitro amplification of the present invention, the expression and/or activity of the TIL of the present invention and the STAT-induced STAT inhibitor (SSI) family member of the present invention can be reduced and combined with one or more of the present invention. Exposure to multiple cell activators. For example, in the second stage of in vitro expansion of the present invention, the expression and/or activity of the STAT-induced STAT inhibitor (SSI) family members of the TIL of the present invention can be reduced and combined with one or more cells of the present invention. Activator contact. For example, in the third stage of in vitro expansion of the present invention, the expression and/or activity of the STAT-induced STAT inhibitor (SSI) family members of the TIL of the present invention can be reduced and contacted with one or more cell activators of the present invention.

For example, in a single stage of in vitro expansion of the present invention, the cells of the present invention can be caused to substantially simultaneously reduce the expression and/or activity of STAT-induced STAT inhibitor (SSI) family members and one or more of the present invention. Exposure to multiple cell activators. For example, in a single stage of in vitro expansion of the present invention, the cells of the present invention can be made to first reduce the expression and/or activity of STAT-induced STAT inhibitor (SSI) family members, for example, 2 hours in advance, 2 hours in advance, 4 hours, 8 hours in advance, 12 hours in advance, 24 hours in advance, or 48 hours in advance, etc., and then contact with one or more cell activators of the present invention. For example, in a single stage of in vitro expansion of the present invention, the cells of the present invention can be contacted with one or more cell activators of the present invention first, for example, 2 hours in advance, 4 hours in advance, 8 hours in advance, 12 hours in advance, 24 hours in advance, or 48 hours in advance, etc., to reduce the expression and/or activity of STAT-induced STAT inhibitor (SSI) family members.

For example, in the first stage of in vitro expansion of the invention, the TIL of the invention can substantially simultaneously reduce the expression and/or activity of STAT-induced STAT inhibitor (SSI) family members and contact one or more cell activators of the invention. For example, in the second stage of in vitro expansion of the present invention, the TIL of the present invention can substantially simultaneously reduce the expression and/or activity of STAT-induced STAT inhibitor (SSI) family members and contact one or more cell activators of the present invention. For example, in the third stage of in vitro expansion of the present invention, the TIL of the present invention can substantially simultaneously reduce the expression and/or activity of STAT-induced STAT inhibitor (SSI) family members and contact one or more cell activators of the present invention.

In another aspect, the invention provides a method for culturing tumor infiltrating lymphocytes (TIL), which may comprise: (A) making cells derived from tumor tissue, pleural effusion and/or peritoneal effusion without in vitro expansion. The first TIL population is contacted with one or more cell growth factors; wherein the second TIL population is obtained through the step (A); (B) the second TIL population STAT-induced STAT inhibitor (SSI) family The expression and/or activity of the members is reduced; wherein, the third TIL group is obtained through the step (B).

In one embodiment, the first stage in vitro amplification of the present invention can be used arbitrarily interchangeably with step (A) in the method of the above aspect. In one embodiment, the second stage in vitro amplification of the present invention can be used interchangeably with step (B) in the method of the above aspect. In one embodiment, the TILs expanded in vitro in the first stage of the present invention can be used interchangeably with the second TIL population obtained in step (A) of the above method. In one embodiment, the second stage body of the present invention

The externally amplified TIL can be optionally replaced with the third TIL group obtained in step (B) of the above method. In one embodiment, if necessary, the third stage in vitro amplification of the present invention can be used interchangeably with any additional step (C) in the method of the above aspect. In one embodiment, if necessary, the third-stage in vitro expanded TIL of the present invention can be used interchangeably with the fourth TIL group obtained by any additional step (C) in the above method.

In another aspect, the present invention provides a method for culturing tumor infiltrating lymphocytes (TIL), which may include: (A) culturing cells derived from tumor tissue, pleural effusion and/or peritoneal effusion without in vitro amplification The first TIL population is contacted with a variety of cell growth factors; wherein, the second TIL population is obtained through the step (A); (B) the second TIL population can be contacted with a variety of cell growth factors, and with a variety of cell growth factors, and the cell activator is contacted to reduce the expression and/or activity of STAT-induced STAT inhibitor (SSI) family members, and the TILs are co-cultured with feeder cells; wherein, the third TIL population is obtained through the step (B).

In another aspect, the present invention provides a method for culturing tumor infiltrating lymphocytes (TIL), which may include: (A) culturing cells derived from tumor tissue, pleural effusion and/or peritoneal effusion without in vitro amplification The first TIL population is contacted with cell growth factors; wherein, the second TIL population is obtained through the step (A); (B) the second TIL population can be contacted with cell growth factors, with cell activators, and the expression and/or activity of STAT-induced STAT inhibitor (SSI) family members is reduced and the TILs are co-cultured with feeder cells. The STAT-induced STAT inhibitor (SSI) family members may include SOC1; wherein the third TIL population is obtained through the step (B).

In another aspect, the invention provides a method of culturing tumor-infiltrating lymphocytes (TIL). The method of obtaining TIL cells from subject tissue samples can be to obtain orthotopic tumor samples or metastatic tumor samples from the patient during surgery, and the weight can be at least about 1g, or multiple pieces of tissue can be merged. Tumor tissue, pleural effusion and/or peritoneal effusion are transported in a sample transport fluid, such as a commercially available tumor tissue transport fluid, tumor tissue preservation fluid or tumor tissue transfer fluid, shipped at about 2-8 degrees Celsius, and processed within 48 hours. Tissue blocks can be mechanically broken down to approximately 1-27 cubic mm size, transfer to a breathable culture bag or Grex, add cell serum-free medium and IL-2 with a concentration of 300-9000IU/mL (for example, it can be 1000-9000IU/mL, for example, it can be 6000IU/mL) and culture for about 3 -14 days. Collect the cells in the culture medium and transfer them to a breathable culture bag, or Grex, or Xuri equipment. The cell serum-free culture medium can be added with the CD28 antibody, CD3 antibody and CD28 antibody of the present invention, and magnetic beads containing CD3 antibody and CD28 antibody (such as Dynabeads ) and/or a nanomatrix (such as transACT) containing CD3 antibodies and CD28 antibodies, IL-2 at a concentration of 300-9000IU/mL (for example, it can be 1000-9000IU/mL, for example, it can be 6000IU/mL), and STAT induction The STAT inhibitor (SSI) family member whose expression is reduced and/or activity is weakened (STAT-induced STAT inhibitor (SSI) family member can include SOCSI1, for example, can be obtained by carrying a ribonucleoside formed with the Cas protein containing the gRNA of the present invention. The protein complex (RNP) is transduced so that the proportion of cells in the TIL encoding genes encoding STAT-induced STAT inhibitor (SSI) family members is about 95% or less), and after activating the TIL of the present invention for a certain period of time, radiation is added. According to PBMC (the ratio of TIL to PBMC is about 1:40-about 1:400), expand and culture for about 3-14 days. Cell processing systems can be used to collect cells in the culture medium, wash, freeze, and detect. The CD3 ratio of the final product can be greater than 80%, the cell viability rate can be greater than 50%, and the cells greater than 80% can be memory effector cells and effector cells. IFN-γ can be secreted after stimulation, and/or can be characterized by an increase in the proportion of activated cells.

### CBLB knockout

1. A method for culturing cells, the method comprising: reducing the expression and/or weakening the activity of CBL family members and/or functionally active fragments thereof of the cells.
2. The method of embodiment 1, wherein the cells comprise immune cells.
3. The method of embodiment 2, wherein the immune cells comprise phagocytes, lymphocytes, neutrophils, eosinophils and/or basophils.
4. The method according to any one of embodiments 2-3, wherein the immune cells comprise monocytes, macrophages and/or dendritic cells.
5. The method according to any one of embodiments 2-4, wherein the immune cells are derived from stem cell differentiated immune cells.
6. The method of embodiment 5, wherein the stem cells comprise induced pluripotent stem cells (iPSCs).
7. The method according to any one of embodiments 2-6, wherein the immune cells comprise B cells, T cells, natural killer cells and/or natural killer-like T cells (NKT).
8. The method according to any one of embodiments 2-7, wherein the immune cells comprise αβ T cells and/or γδ T cells.
9. The method of any one of embodiments 2-8, wherein the immune cells comprise tumor-infiltrating lymphocytes (TIL).
10. The method of embodiment 9, wherein the TIL is a TIL derived from fragments of tumor tissue, pleural effusion and/or peritoneal effusion and/or TIL derived from resuscitation after cryopreservation.
11. The method of embodiment 10, wherein the fragment has a volume from about 1 cubic millimeter to about 27 cubic millimeters.
12. The method of any one of embodiments 2-11, wherein the immune cell comprises an engineered immune receptor displayed on the cell surface.
13. The method of embodiment 12, wherein the engineered immune receptor specifically binds to an antigen expressed on a target cell.
14. The method according to any one of embodiments 2-13, wherein the immune cell comprises chimeric antigen receptors and/or T cell receptors.
15. The method of any one of embodiments 1-14, wherein reducing the expression and/or activity of a CBL family member of the cell comprises inhibiting the function of an E3 ubiquitin protein ligase.
16. The method according to any one of embodiments 1-15, wherein the expression of the CBL family member is reduced and/or the activity is attenuated compared to cells in which the expression and/or activity of the CBL family member is unchanged. of cells showed improved cellular properties.
17. The method of embodiment 16, wherein the improved cell characteristics comprise one or more selected from the group consisting of: improved cell proliferation capacity, increased proportion of viable cells, improved proportion of cell subpopulations, increased ability to secrete cytokines and improve the killing ability of tumor cells.
18. The method of embodiment 17, wherein the improved proportion of cell subpopulations comprises one or more selected from the group consisting of: an increased proportion of activated cells, a reduced proportion of regulatory cells, a reduced proportion of exhausted cells. proportion, increased proportion of central memory cells and/or naive cells, decreased proportion of apoptotic cells and increased proportion of stem cell-like cells.
19. The method of any one of embodiments 1-18, wherein the CBL family member comprises an SH3 domain.
20. The method of any one of embodiments 1-19, wherein the CBL family member comprises CBLB.
21. The method of any one of embodiments 1-20, wherein reducing the expression and/or the activity of a CBL family member of the cell comprises introducing a gene regulatory system into the cell.
22. The method of embodiment 21, wherein the gene regulatory system is capable of disrupting the CBL family member at the DNA level.
23. The method of any one of embodiments 21-22, wherein the gene regulatory system comprises a guide nucleic acid molecule and an enzymatic protein.
24. The method of embodiment 23, wherein reducing the expression and/or weakening the activity of the CBL family member comprises: converting a ribonucleoprotein complex (RNP) comprising the guide nucleic acid molecule and the enzyme protein, an LNP comprising a gRNA and a Cas protein, or an LNP comprising a nucleic acid encoding a gRNA and a Cas protein, is introduced into the cell.
25. The method of any one of embodiments 23-24, wherein the enzyme protein comprises a Cas protein, a Cas protein homolog, or a functionally active fragment thereof.
26. The method of any one of embodiments 23-25, wherein the guide nucleic acid molecule comprises a guide RNA (gRNA).
27. The method of any one of embodiments 23-26, wherein the guide nucleic acid molecule is capable of binding to a sequence of the CBL family member.
28. The method according to any one of embodiments 23-27, wherein the guide nucleic acid molecule is capable of binding to a region selected from the group defined by the genomic coordinates shown in Table 1D, or a fragment thereof.
29. The method of any one of embodiments 23-28, wherein the guide nucleic acid molecule is capable of binding to a region selected from the group consisting of: SEQ ID NOs: 520-590, 6177-7266, or fragments thereof.
30. The method of any one of embodiments 23-29, wherein the guide nucleic acid molecule is capable of being about 15 to about 25 upstream of the 5' end of a protospacer adjacent motif (PAM) selected from the group consisting of: Sequence combinations of nucleotides: AGG, TGG, CGG and GGG.
31. The method of any one of embodiments 23-30, wherein the guide nucleic acid molecule comprises a targeting domain comprising SEQ ID NOs: 449-519, 5087-6176, 7376 -The sequence shown in any one of 7413, 7414, and 7415.
32. The method according to any one of embodiments 1-31, wherein the expression of the CBL family member is reduced and/or the activity is attenuated compared to cells in which the expression and/or activity of the CBL family member is unchanged. The proportion of cells expressing the product of the target gene among the cells decreases and/or the expression level of the target gene in a single cell decreases.
33. The method according to any one of embodiments 1-32, wherein among the cells obtained by reducing the expression and/or weakening the activity of the CBL family member, the proportion of cells expressing the gene of interest is about 95% or lower.
34. A cell obtained by the method of any one of embodiments 1-33.
35. A composition comprising the cell of embodiment 34.
36. A pharmaceutical composition comprising the cell of embodiment 34 and/or the composition of embodiment 35, and optionally a pharmaceutically acceptable carrier.
37. A method of affecting cell growth, comprising administering the cell of embodiment 34, the composition of embodiment 35, and/or the pharmaceutical composition of embodiment 36.
38. Use of the cell of embodiment 34, the composition of embodiment 35 and/or the pharmaceutical composition of embodiment 36 in the preparation of a medicament, wherein the medicament is used to prevent and/or treat diseases and /or symptoms.
39. Use according to embodiment 38, wherein the disease and/or condition comprises a tumor.
40. Use according to any one of embodiments 38-39, wherein the disease and/or condition comprises a solid tumor.
41. Use according to any one of embodiments 38-40, wherein the disease and/or condition comprises one or more selected from the group consisting of: melanoma, ovarian cancer, cervical cancer, lung cancer, bladder cancer , breast cancer, head and neck cancer, pancreatic cancer, liver cancer, stomach cancer, colorectal cancer and kidney cancer.

The present invention provides methods for reducing the expression and/or weakening the activity of CBL family members and/or functionally active fragments thereof in said cells.

In one aspect, the present invention provides a method for culturing cells so that the expression and/or activity of CBL family members and/or functionally active fragments thereof is reduced in the cells. For example, the CBL family member may comprise an SH3 domain. For example, the CBL family member may include CBLB.

For example, the target gene of the present invention may be a gene encoding a CBL family member and/or a functionally active fragment thereof. For example, a cell obtained by reducing the expression and/or attenuating the activity of at least one target gene of the cell may exhibit improved cell properties compared to a cell in which the expression and/or activity of the target gene is unchanged. In one embodiment, the cells whose expression and/or activity of the target gene are unchanged may refer to cells derived from the same donor and which have not had the expression and/or activity of at least one target gene of the cells reduced. In one embodiment, cells whose expression and/or activity of the target gene are unchanged may refer to cells derived from the same donor and in which the expression and/or activity of other genes other than the target gene of the cells have not been altered (e.g., knocking out the other genes has basically no impact on cell function).

For example, the cells include immune cells. For example, the cells comprise immune effector cells. For example, the cells include immune effector T cells, immune effector NK cells, immune effector NKT cells. For example, the cells include phagocytes, lymphocytes, neutrophils, eosinophils and/or basophils.

For example, the cells include monocytes, macrophages, and/or dendritic cells.

For example, the cells of the present invention also include cells derived from differentiation of stem cells. For example, the cells of the present invention also include cells derived from differentiation of pluripotent stem cells. For example, the stem cells of the present invention can be obtained by induction. For example, the above-mentioned stem cells of the present invention may include induced pluripotent stem cells (iPSCs), embryonic stem cells, bone marrow stem cells, umbilical cord blood stem cells and/or peripheral blood stem cells.

For example, "stem cells" of the present invention also include pluripotent cells, multipotent cells, precursor cells and progenitor cells. For example, stem cells can be obtained from hematopoietic or mesenchymal stem cells obtained from bone marrow tissue, placental stem cells obtained from placental tissue, embryonic stem cells obtained from embryonic tissue, or embryonic germ cells obtained from the reproductive tissue of the fetus. Exemplary pluripotent stem cells can also be generated from somatic cells by reprogramming them to a pluripotent state through the expression of certain transcription factors associated with pluripotency; these cells are called "induced pluripotent stem cells" or "iPSCs" .

For example, the cells include B cells, T cells, natural killer cells and/or natural killer-like T cells (NKT). For example, "unmodified cells" or "unmodified cells" may refer to cells in which the genome has not been modified and does not contain a gene regulatory system or contains a control gene regulatory system (e.g., empty vector control, non-targeting gRNA, interfering siRNA, etc. ) cells or cell populations. For example, the cells comprise αβ T cells and/or γδ T cells. For example, the cells include tumor-infiltrating lymphocytes (TILs). For example, the TIL is a TIL derived from tumor tissue, tumor-associated lymph nodes with or without tumor metastasis, tumor metastasis, fragments of paracancerous tissue, pleural effusion and/or peritoneal effusion and/or derived from frozen Save the TIL after recovery.

For example, the TIL of the present invention can be derived from tumor tissue, tumor-associated lymph nodes with or without tumor metastasis, tumor metastasis, fragments of paracancerous tissue, pleural effusion and/or TILs from peritoneal effusion and/or TILs derived from cryopreservation and resuscitation. For example, TILs of the present invention can be obtained by processing tumor tissue into tumor fragments. For example, the tumor fragments of the present invention have a volume of approximately 1 to 27 cubic millimeters. For example, the volume of the tumor fragments of the present invention is about 1 cubic millimeter, about 2 cubic millimeters, about 3 cubic millimeters, about 4 cubic millimeters, about 5 cubic millimeters, about 6 cubic millimeters, about 7 cubic millimeters, about 8 cubic millimeters. , about 9 cubic millimeters, about 10 cubic millimeters, about 11 cubic millimeters, about 12 cubic millimeters, about 13 cubic millimeters, about 14 cubic millimeters, about 15 cubic millimeters, about 16 cubic millimeters, about 17 cubic millimeters, about 18 cubic millimeters , about 19 cubic millimeters, about 20 cubic millimeters, about 21 cubic millimeters, about 23 cubic millimeters, about 24 cubic millimeters, about 25 cubic millimeters, about 26 cubic millimeters or about 27 cubic millimeters.

For example, the cells comprise engineered immune receptors displayed on the cell surface. For example, the engineered immune receptor specifically binds to an antigen expressed on a target cell. For example, the cells comprise chimeric antigen receptors and/or T cell receptors.

In one aspect, the present invention provides a method for culturing tumor-infiltrating lymphocytes (TIL), which may include reducing the expression and/or activity of CBL family members and/or functionally active fragments thereof in the TIL.

For example, TILs derived from tumor tissue, pleural effusion and/or peritoneal effusion without in vitro amplification can be subjected to at least one stage of in vitro amplification, wherein in at least one stage of in vitro amplification, the expression and/or activity of CBL family members and/or functionally active fragments thereof are reduced in the TIL.

For example, the TILs of the present invention derived from tumor tissue, pleural effusion and/or peritoneal effusion without in vitro amplification can be subjected to the first stage of in vitro amplification and the second stage of in vitro amplification, and in the second stage of the in vitro amplification, the expression and/or activity of CBL family members and/or functionally active fragments thereof in the TIL can be reduced.

For example, the TILs of the present invention derived from tumor tissue, pleural effusion and/or peritoneal effusion without in vitro amplification can be subjected to the first stage of in vitro amplification and the second stage of in vitro amplification, and in the first stage of the in vitro amplification, the expression and/or activity of peptidase CBL family members and/or functionally active fragments thereof in the TIL can be reduced.

For example, the TILs of the present invention derived from tumor tissue, pleural effusion and/or peritoneal effusion without in vitro amplification can be subjected to the first stage of in vitro amplification and the second stage of in vitro amplification, and in the first stage of the in vitro amplification, the expression and/or activity of the CBL family members and/or functionally active fragments thereof in the TIL can be reduced, and in the second stage of the in vitro amplification, the expression and/or activity of CBL family members and/or functionally active fragments thereof in the TIL can be reduced

For example, the TILs of the present invention derived from tumor tissue, pleural effusion and/or peritoneal effusion without in vitro amplification can be subjected to a first-stage in vitro amplification, a second-stage in vitro amplification and a third-stage in vitro amplification, and in the first stage of in vitro amplification of the present invention, the expression and/or activity of CBL family members and/or functionally active fragments thereof in the TIL can be reduced.

For example, the TILs of the present invention derived from tumor tissue, pleural effusion and/or peritoneal effusion without in vitro amplification can be subjected to a first-stage in vitro amplification, a second-stage in vitro amplification and a third-stage in vitro amplification, and in the second stage of in vitro amplification of the present invention, the expression and/or activity of CBL family members and/or functionally active fragments thereof in the TIL can be reduced.

For example, the TILs of the present invention derived from tumor tissue, pleural effusion and/or peritoneal effusion without in vitro amplification can be subjected to a first-stage in vitro amplification, a second-stage in vitro amplification and a third-stage in vitro amplification, and in the third stage of in vitro amplification of the present invention, the expression and/or activity of CBL family members and/or functionally active fragments thereof in the TIL can be reduced.

For example, the TILs of the present invention derived from tumor tissue, pleural effusion and/or peritoneal effusion without in vitro amplification can be subjected to a first-stage in vitro amplification, a second-stage in vitro amplification and a third-stage in vitro amplification, and in the first stage of in vitro expansion of the present invention, the expression and/or activity of CBL family members and/or functionally active fragments thereof in the TIL can be reduced, and in the second stage of in vitro expansion of the present invention, the expression and/or activity of CBL family members and/or functionally active fragments thereof in the TIL can be reduced.

For example, the TILs of the present invention derived from tumor tissue, pleural effusion and/or peritoneal effusion without in vitro amplification can be subjected to a first-stage in vitro amplification, a second-stage in vitro amplification and a third-stage in vitro amplification, and in the first stage of in vitro expansion of the present invention, the expression and/or activity of CBL family members and/or functionally active fragments thereof in the TIL can be reduced, and in the third stage of in vitro expansion of the present invention, the expression and/or activity of CBL family members and/or functionally active fragments thereof in the TIL can be reduced.

For example, the TILs of the present invention derived from tumor tissue, pleural effusion and/or peritoneal effusion without in vitro amplification can be subjected to a first-stage in vitro amplification, a second-stage in vitro amplification and a third-stage in vitro amplification, and in the second stage of in vitro expansion of the present invention, the expression and/or activity of CBL family members and/or functionally active fragments thereof in the TIL can be reduced, and in the third stage of in vitro expansion of the present invention, the expression and/or activity of CBL family members and/or functionally active fragments thereof in the TIL can be reduced.

For example, the TILs of the present invention derived from tumor tissue, pleural effusion and/or peritoneal effusion without in vitro amplification can be subjected to a first-stage in vitro amplification, a second-stage in vitro amplification and a third-stage in vitro amplification. , and in the first stage of in vitro expansion of the present invention, the expression and/or activity of CBL family members and/or functionally active fragments thereof in the TIL can be reduced, in the second stage of in vitro expansion of the present invention, the expression and/or activity of CBL family members and/or functionally active fragments thereof in the TIL can be reduced, and in the third stage of in vitro amplification of the present invention, the expression and/or activity of CBL family members and/or functionally active fragments thereof in the TIL can be reduced.

For example, cells obtained by reducing the expression and/or attenuating the activity of a CBL family member exhibit improved cellular properties compared to cells in which the expression and/or activity of the CBL family member is unchanged.

For example, the improved cell number of the present invention refers to reducing the expression and/or activity of the CBL family member in at least one in vitro expansion stage compared to cells in which the expression and/or activity of the CBL family member is unchanged. The cell number of the cells of the invention can be increased by at least about 1 time, at least about 2 times, at least about 3 times, at least about 4 times, at least about 5 times, at least about 6 times, at least about 7 times, at least about 8 times, at least About 9 times, at least about 10 times, at least about 11 times, at least about 12 times, at least about 13 times, at least about 14 times, at least about 15 times, at least about 20 times, at least about 30 times, at least about 40 times, or At least about 50 times.

For example, an increased proportion of viable cells can manifest itself as an increase in cell viability. For example, the increased proportion of viable cells of the present invention may refer to reducing the expression and/or activity of the CBL family member in at least one in vitro expansion stage compared to cells in which the expression and/or activity of the CBL family member is unchanged. The proportion of viable cells of the cells of the invention with reduced activity can be increased by at least about 100%, at least about 90%, at least about 80%, at least about 70%, at least about 60%, at least about 50%, at least about 40%, at least about 30 %, at least about 20%, at least about 19%, at least about 18%, at least about 17%, at least about 16%, at least about 15%, at least about 14%, at least about 13%, at least about 12%, at least about 11 %, at least about 10%, at least about 9%, at least about 8%, at least about 7%, at least about 6%, at least about 5%, at least about 4%, at least about 3%, at least about 2%, at least about 1 %, at least about 0.5%, at least about 0.4%, at least about 0.3%, at least about 0.2%, or at least about 0.1%.

For example, the increased cytokine secretion ability of the present invention may refer to an increase in the cytokine secretion ability of a cell selected from the group consisting of: IL-2, IL-6, CD107a, GZMB, TNF-α, and IFN-γ. For example, the improved cytokine secretion ability of the present invention may refer to reducing the expression and/or activity of the CBL family member in at least one in vitro expansion stage compared with cells in which the expression and/or activity of the CBL family member is unchanged. Or the proportion of cells secreting cytokines in the cells of the present invention with weakened activity can be increased by at least about 1 time, at least about 2 times, at least about 3 times, at least about 4 times, at least about 5 times, at least about 6 times, at least about 7 times , at least about 8 times, at least about 9 times, at least about 10 times, at least about 11 times, at least about 12 times, at least about 13 times, at least about 14 times, at least about 15 times, at least about 20 times, at least about 30 times , at least about 40 times, or at least about 50 times. For example, the enhanced cytokine secretion of the present invention

Ability may refer to secretory cells in cells of the invention that have reduced expression and/or attenuated activity of said CBL family member during at least one stage of in vitro expansion compared to cells in which the expression and/or activity of the CBL family member is unchanged. The cellular proportion of the factor can be increased by at least about 100%, at least about 90%, at least about 80%, at least about 70%, at least about 60%, at least about 50%, at least about 40%, at least about 30%, at least about 20% , at least about 19%, at least about 18%, at least about 17%, at least about 16%, at least about 15%, at least about 14%, at least about 13%, at least about 12%, at least about 11%, at least about 10% , at least about 9%, at least about 8%, at least about 7%, at least about 6%, at least about 5%, at least about 4%, at least about 3%, at least about 2%, at least about 1%, at least about 0.5% , at least about 0.4%, at least about 0.3%, at least about 0.2%, or at least about 0.1%.

For example, the improved tumor cell killing ability of the present invention may refer to reducing the expression and/or activity of the CBL family member in at least one in vitro expansion stage compared with cells in which the expression and/or activity of the CBL family member is unchanged. Or the tumor cell killing rate of the cells of the present invention with weakened activity can be increased by at least about 1 time, at least about 2 times, at least about 3 times, at least about 4 times, at least about 5 times, at least about 6 times, at least about 7 times, at least About 8 times, at least about 9 times, at least about 10 times, at least about 11 times, at least about 12 times, at least about 13 times, at least about 14 times, at least about 15 times, at least about 20 times, at least about 30 times, at least About 40 times, or at least about 50 times. For example, the improved tumor cell killing ability of the present invention may refer to reducing the expression and/or activity of the CBL family member in at least one in vitro expansion stage compared with cells in which the expression and/or activity of the CBL family member is unchanged. Or the tumor cell killing rate of the cells of the present invention with weakened activity can be increased by at least about 100%, at least about 90%, at least about 80%, at least about 70%, at least about 60%, at least about 50%, at least about 40%, at least About 30%, at least about 20%, at least about 19%, at least about 18%, at least about 17%, at least about 16%, at least about 15%, at least about 14%, at least about 13%, at least about 12%, at least About 11%, at least about 10%, at least about 9%, at least about 8%, at least about 7%, at least about 6%, at least about 5%, at least about 4%, at least about 3%, at least about 2%, at least About 1%, at least about 0.5%, at least about 0.4%, at least about 0.3%, at least about 0.2%, or at least about 0.1%. For example, the tumor cell killing rate of the cells of the present invention can be measured by the IncuCyte system or CFSE and DAPI staining methods. For example, the improved tumor cell killing ability may refer to the ability of cells to kill solid tumor cells.

For example, the improved cell subpopulation ratio of the present invention may comprise one or more selected from the following group: increased CD8 ⁺ Cell proportion, increased central memory cell and/or naive cell proportion, decreased regulatory cell proportion, increased activated cell proportion, increased tumor-specific cell proportion, and stem cell-like cell proportion increased.

For example, the proportion of CD8⁺ cells, central memory cells and/or immature cells, activated cells, tumor-specific cells and/or stem cell-like cells can be increased by at least about 100%, at least about 90%, at least about 80%, at least about 70%, at least about 60%, at least about 50%, at least about 40%, At least about 30%, at least about 20%, at least about 19%, at least about 18%, at least about 17%, at least about 16%, at least about 15%, at least about 14%, at least about 13%, at least about 12%, At least about 11%, at least about 10%, at least about 9%, at least about 8%, at least about 7%, at least about 6%, at least about 5%, at least about 4%, at least about 3%, at least about 2%, At least about 1%, at least about 0.5%, at least about 0.4%, at least about 0.3%, at least about 0.2%, or at least about 0.1%.

For example, the reduced proportion of exhausted cells of the present invention can be increase of PD-1⁺ ,LAG-3⁺ ,TIM-3⁺ , and/or CD39⁺ cell proportion. For example, the reduced proportion of regulatory cells of the present invention can be reduction of CD4⁺ CD25⁺ Foxp3⁺ cell proportion. For example, the reduced proportion of apoptotic cells of the present invention can be reduction of CD95⁺ caspass3⁺ and/or CD95⁺ DR5⁺ cell proportion.

For example, the proportion of exhausted cells, regulatory cells and/or apoptotic cells in the cells can be reduced by at least about 100%, at least about 90%, at least about 80%, at least about 70%, at least about 60%, at least about 50%, At least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%, or can be reduced by at least about 1 times, at least about 2 times, at least about 3 times, at least about 4 times, at least about 5 times, at least about 6 times , at least about 7 times, at least about 8 times, at least about 9 times, at least about 10 times, at least about 11 times, at least about 12 times, at least about 13 times, at least about 14 times, at least about 15 times, at least about 20 times , at least about 30 times, at least about 40 times, or at least about 50 times.

For example, the culture method of the present invention may include a gene editing step for cells. For example, it includes subjecting the cells to at least one stage of in vitro expansion, wherein in at least one stage of the in vitro expansion, a gene regulatory system can be introduced into the cells.

For example, the gene regulatory system can destroy the target gene at the DNA level. For example, the gene regulatory system can disrupt a region of the target gene or a fragment thereof in the genome of the cell. For example, after using the gene regulation system, the DNA region or fragment thereof where the target gene is located in the cell is sheared, and the expression ability of the target gene is reduced or the activity of the target gene is inhibited. For example, the editing effect of the gene regulation system on the target gene can be long-term and sustained. The genomic region of the present invention is determined based on the hg38 version of the human reference genome.

For example, the gene regulatory system may include guide nucleic acid molecules and enzymatic proteins. For example, the enzyme protein can have nucleic acid shearing enzyme activity, and the guide nucleic acid molecule can guide the enzyme protein to specifically cut the region where the target gene is located or its fragments. For example, the guide nucleic acid molecule and the enzyme protein may exist in the form of a ribonucleoprotein complex (RNP), or each may exist independently. For example, the enzyme protein may comprise Cas protein. For example, polynucleotides encoding gRNA and Cas protein can be introduced into the target cell, or each independently.

For example, reducing the expression and/or weakening the activity of at least one target gene of a cell according to the present invention may include: introducing a ribonucleoprotein complex (RNP) containing the guide nucleic acid molecule and the enzyme protein into the cell. For example, the enzyme protein may comprise Cas protein, Cas protein homologues, or functionally active fragments thereof. For example, the guide nucleic acid molecule may comprise guide RNA (gRNA). For example, the guide nucleic acid molecule may comprise guide RNA (gRNA). For example, a complex comprising a polynucleotide encoding a gRNA and a Cas protein can be introduced into the cell. For example, a complex containing gRNA and Cas protein can be introduced into the cell.

For example, the gRNA can be used to bind to the sequence of the target gene. For example, the binding of the gRNA to the sequence of the target gene can be completely complementary, partially complementary, or can hybridize to the sequence of the target gene under moderately stringent or stringent conditions. For example, the binding of the gRNA to the sequence of the target gene can cause the CRISPR system of the gRNA to specifically cleave the target gene.

For example, the editing target region of the present invention may be a region before the promoter. For example, the editing target region of the present invention may be a region with high transcription factor binding capacity. For example, the editing target region of the present invention may be a region with a specific number of transcription factor binding numbers. For example, the editing target region of the present invention can be a continuous region with a binding number of about 3 or more transcription factors.

For example, when the gene editing system includes CRISPR/Cas9, there may be a protospacer adjacent motif (PAM) downstream of the region targeted by the guiding nucleic acid molecule of the present invention, and the protospacer adjacent motif (PAM) may be AGG, TGG, GGG or CGG. For example, when the PAM region of the target gene is determined, those skilled in the art can easily determine about 15 to about 25 (for example, about 15, about 16, about 17, about 18, about 19, about 20) upstream of the 5' end of the PAM of the target gene. , about 21, about 22, about 23, about 24, about 25) nucleotides, and a suitable gRNA can be designed for the target sequence. For example, the guide nucleic acid molecule is capable of binding to a sequence consisting of about 15 to about 25 nucleotides upstream of the 5' end of a protospacer adjacent motif (PAM) selected from the group consisting of: AGG, TGG, GGG, and CGG.

For example, when the gene editing system includes CRISPR/Cas12, there may be a protospacer adjacent motif (PAM) upstream of the region targeted by the guiding nucleic acid molecule of the present invention, and the protospacer adjacent motif (PAM) may be NTTN, TTYN, VTTV, TRTV, TTTV, TATV, TYCV, TNN, or NTN, where N is A, T, C, or G, Y is T or C, V is A, C, or G, and R is A or G. For example, when the PAM region of the target gene is determined, those skilled in the art can easily determine about 15 to about 25 (for example, about 15, about 16, about 17, about 18, about 19, about 20) downstream of the 3' end of the PAM of the target gene. , about 21, about 22, about 23, about 24, about 25) nucleotides, and a suitable gRNA can be designed for the target sequence. For example, the guide nucleic acid molecule can bind to a sequence consisting of about 15 to about 25 nucleotides downstream of the 3' end of the protospacer adjacent motif (PAM) selected from the group consisting of: NTTN, TTYN, VTTV, TRTV , TTTV, TATV, TYCV, TNN, or NTN, where N is A, T, C, or G, Y is T or C, V is A, C, or G, and R is A or G.

For example, when the gene editing system of the present invention includes wild-type Cas12a (also known as Cpf1, such as AsCas12a, FnCas12a, LbCas12a, BbCas12a, CMaCas12a and OsCas12a), the upstream region of the guiding nucleic acid molecule targeting of the present invention can be selected from the following PAM sequence: NTTN, where N can be A, T, C or G. For example, when the PAM region of the target gene is determined, those skilled in the art can easily determine about 17 to about 25 (for example, about 17, about 18, about 19, about 20, about 21, about 22) downstream of the 3' end of the PAM of the target gene. , about 23, about 24, or about 25) nucleotides, and a suitable gRNA can be designed for the target sequence.

For example, when the gene editing system of the present invention includes mutant Cas12a, such as enAsCas12a (mutation sites E174R, S542R and K548R), the upstream of the region targeted by the guiding nucleic acid molecule of the present invention can have a PAM sequence selected from the following: TTYN (TTTN /TTCN), VTTV (ATTV/CTTV/GTTV), or TRTV (TATV/TGTV), where N can be A, T, C or G, Y can be T or C, V can be A, C or G, R Can be A or G. For example, when the PAM region of the target gene is determined, those skilled in the art can easily determine about 17 to about 25 (for example, about 17, about 18, about 19, about 20, about 21, about 22) downstream of the 3' end of the PAM of the target gene. , about 23, about 24, or about 25) nucleotides, and a suitable gRNA can be designed for the target sequence.

For example, when the gene editing system of the present invention includes mutant Cas12a, such as opAsCas12a (mutation sites: E174R and S542R), the upstream of the region targeted by the guiding nucleic acid molecule of the present invention can have a PAM sequence selected from the following: TTTV (TTTA, TTTC, or TTTG), where V can be A, C, or G. For example, when the PAM region of the target gene is determined, those skilled in the art can easily determine about 17 to about 25 (for example, about 17, about 18, about 19, about 20, about 21, about 22) downstream of the 3' end of the PAM of the target gene. , about 23, about 24, or about 25) nucleotides, and a suitable gRNA can be designed for the target sequence.

For example, when the gene editing system of the present invention includes mutant Cas12a, such as AsCas12a Ultra (mutation sites: M537R and F870L), the upstream of the region targeted by the guiding nucleic acid molecule of the present invention can have a PAM sequence selected from the following: TTTV, TATV, Or TYCV, where V can be A, C or G and Y can be T or C. For example, when the PAM region of the target gene is determined, those skilled in the art can easily determine about 17 to about 25 (for example, about 17, about 18, about 19, about 20, about 21, about 22) downstream of the 3' end of the PAM of the target gene. , about 23, about 24, or about 25) nucleotides, and a suitable gRNA can be designed for the target sequence.

For example, when the gene editing system of the present invention includes mutant Cas12a, such as hfCas12Max (mutation site: N243R/E336R/D892R) and Cas12Max (mutation site: N243R), the upstream region of the guiding nucleic acid molecule targeting of the present invention can be A PAM sequence selected from: TNN, or NTN, where N can be A, T, C, or G. For example, when the PAM region of the target gene is determined, those skilled in the art can easily determine about 17 to about 25 (for example, about 17, about 18, about 19, about 20, about 21, about 22) downstream of the 3' end of the PAM of the target gene. , about 23, about 24, or about 25) nucleotides, and a suitable gRNA can be designed for the target sequence.

For example, the guide nucleic acid molecule may comprise about 10 to about 30 nucleotides before the PAM region represented by AGG, TGG, GGG and/or CGG in the DNA where the gene encoding the CBL family member and/or its functionally active fragment is located. composed of target sequences. For example, the guidance

The nucleic acid molecule may include about 15 to about 25, about 17 to about 25, and about 17 to about 25 before the PAM region represented by AGG, TGG, GGG and/or CGG in the DNA where the gene encoding the CBL family member and/or its functionally active fragment is located. About 19 to about 25, about 20 to about 25, about 21 to about 25, about 23 to about 25, about 15 to about 23, about 17 to about 23, about 19 to about 23, about 20 to about 23, about 21 to about 23, about 15 to about 21, about 17 to about 21, about 19 to about 21, about 20 to about 21, about 15 to about 20, about 17 to a target sequence consisting of about 20, about 19 to about 21, about 15 to about 19, about 17 to about 19, or about 15 to about 17 nucleotides.

For example, the target sequence may be selected from the region defined by the genomic coordinates shown in Table 1D or a fragment thereof. For example, the target sequence of the present invention can be the PTB structural and functional domain of CBLB. For example, the target sequence of the present invention can be the zinc finger structural domain of CBLB. For example, the target sequence of the present invention can be the UBA structural and functional domain of CBLB. For example, the target sequences of the present invention can be chr3:105658893-105659215, chr3:105720184-105720231, chr3:105720234-105720278, chr3:105720300-105720327, chr3:105720333-105 720375, chr3:105723918-105723956, chr3:105724025-105724052 , chr3:105724100-105724131, chr3:105724134-105724219, chr3:105724232-105724310, chr3:105733918-105733970, chr3:105733988-105734073, chr3:105734082-105734109, chr3:105737162-105737268, chr3:105740404-105740449, chr3 :105740470-105740551, chr3:105740563-105740646, chr3:105740707-105740734, chr3:105745796-105745885, chr3:105745896-105745947, chr3:1 05745963-105746060, chr3:105749514-105749557, chr3:105749570-105749653, chr3:105749657 -105749701, chr3:105749751-105749827, chr3:105749860-105749909, chr3:105751528-105751641, chr3:105751674-105751705, chr3:105754228-1 05754312, chr3:105754319-105754376, chr3:105754386-105754419, chr3:105754544-105754599 , chr3:105776307-105776394, chr3:105776402-105776557, chr3:105776632-105776662, chr3:105824008-105824362, chr3:105839348-105839393, chr3:105839418-105839485, chr3:105839533-105839704, chr3 :105853309-105853347, chr3:105853445-105853472, chr3:105853571-105853598, chr3: 105867293-105867352, chr3:105867358-105867389.

For example, the guide nucleic acid molecule can include a targeting domain that is complementary to a target sequence selected from the group consisting of: SEQ ID NOs: 520-590, 6177-7266.

For example, the guide nucleic acid molecule can include a targeting domain, which can include sequences as set forth in SEQ ID NOs: 449-519, 5087-6176, 7376-7413, 7414, 7415.

For example, the guide nucleic acid molecule can include a targeting domain, which can include sequences as set forth in SEQ ID NOs: 7376-7413, 7414, 7415.

For example, the ratio of cells expressing the product of the gene of interest in cells obtained by reducing the expression and/or activity of at least one target gene of the cell compared to cells in which the expression and/or activity of the target gene is unchanged. can be reduced and/or the expression level of the target gene in a single cell can be reduced.

For example, in the method of the present invention, the target gene is expressed in cells obtained by reducing the expression and/or activity of at least one target gene in the cells compared with cells in which the expression and/or activity of the target gene is unchanged. The cell proportion of the product is reduced by at least about 5%. For example, the proportion of cells expressing the product of the gene encoding a CBL family member and/or a functionally active fragment thereof is reduced by at least about 100%, at least about 90%, at least about 80%, at least about 70%, at least about 60%, at least About 50%, at least about 40%, at least about 30%, at least about 20%, at least about 19%, at least about 18%, at least about 17%, at least about 16%, at least about 15%, at least about 14%, at least About 13%, at least about 12%, at least about 11%, at least about 10%, at least about 9%, at least about 8%, at least about 7%, at least about 6%, or at least about 5%. For example, the proportion of cells expressing the product of the gene encoding a CBL family member and/or a functionally active fragment thereof can range from an observable proportion of cells to 0%. For example, the proportion of cells expressing the product of the gene encoding a CBL family member and/or a functionally active fragment thereof can be reduced to at least about 100%, at least about 90%, at least about 80%, at least about 70%, at least about 60% , at least about 50%, at least about 40%, at least about 30%, at least about 20%, at least about 19%, at least about 18%, at least about 17%, at least about 16%, at least about 15%, at least about 14% , at least about 13%, at least about 12%, at least about 11%, at least about 10%, at least about 9%, at least about 8%, at least about 7%, at least about 6%, at least about 5%, or at least about 1 %. For example, the proportion of cells expressing the product of the gene encoding a CBL family member and/or a functionally active fragment thereof can be detected by cell flow cytometry.

For example, the proportion of cells expressing the product of the gene encoding the CBL family member and/or its functionally active fragment among the cells obtained by reducing the expression and/or weakening the activity of at least one target gene of the cells in the method of the present invention can be is at most about 95%. For example, the proportion of cells expressing the product of the gene encoding a CBL family member and/or a functionally active fragment thereof may be at most about 95%, at most about 90%, at most about 80%, at most about 70%, at most about 60%, up to about 50%, up to about 40%, up to about 30%, up to about 20%, up to about 19%, up to about 18%, up to about 17%, up to about 16%, up to about 15%, up to about 14%, up to about 13%, up to about 12%, up to about 11%, up to about 10%, up to about 9%, up to about 8%, up to about 7%, up to about 6%, or up to about 5%. For example, the proportion of cells expressing the product of the gene encoding a CBL family member and/or a functionally active fragment thereof can be detected by cell flow cytometry.

For example, in the method of the present invention, compared with cells in which the expression and/or activity of the target gene are unchanged, the cells obtained by reducing the expression and/or activity of at least one target gene are described in a single cell. The expression level of the target gene can be reduced by at least about 5%. For example, the expression level of the gene of interest in a single cell can be reduced by at least about 100%, at least about 90%, at least about 80%, at least about 70%, at least about 60%, at least about 50%, at least about 40%, at least About 30%, at least about 20%, at least about 19%, at least about 18%, at least about 17%, at least about 16%, at least about 15%, at least about 14%, at least about 13%, to about 12% less, at least about 11%, at least about 10%, at least about 9%, at least about 8%, at least about 7%, at least about 6%, or at least about 5%. For example, the expression level of the target gene in a single cell can range from an observable amount to 0%. For example, the expression level of the gene of interest in a single cell can be reduced to at least about 100%, at least about 90%, at least about 80%, at least about 70%, at least about 60%, at least about 50%, at least about 40% , at least about 30%, at least about 20%, at least about 19%, at least about 18%, at least about 17%, at least about 16%, at least about 15%, at least about 14%, at least about 13%, at least about 12% , at least about 11%, at least about 10%, at least about 9%, at least about 8%, at least about 7%, at least about 6%, at least about 5%, or at least about 1%.

For example, in the method of the present invention, the expression amount of the target gene in a single cell in the cells obtained by reducing the expression and/or activity of at least one target gene can be the expression and/or activity of the target gene. At most about 95% of the cells are unchanged. For example, the expression level of the gene encoding the CBL family member and/or the functionally active fragment thereof (e.g., the gene encoding CBLB) in a single cell in the cell may be the expression and/or the expression of the gene encoding the CBL family member and/or the functionally active fragment thereof. or up to about 95%, up to about 90%, up to about 80%, up to about 70%, up to about 60%, up to about 50%, up to about 40%, up to about 30%, up to about 20% of the cells with unchanged activity %, up to about 19%, up to about 18%, up to about 17%, up to about 16%, up to about 15%, up to about 14%, up to about 13%, up to about 12%, up to about 11%, up to about 10 %, up to about 9%, up to about 8%, up to about 7%, up to about 6%, or up to about 5%.

For example, the methods of the invention comprise: subjecting the cells to at least one stage of in vitro expansion, wherein the expression and/or activity of the CBL family members of the cells is reduced during at least one stage of the in vitro expansion.

For example, the TILs derived from tumor tissue, tumor-related lymph nodes with or without tumor metastasis, tumor metastasis lesions, fragments of para-cancerous tissue, pleural effusion and/or peritoneal effusion and have not been amplified in vitro are subjected to the first stage of in vitro amplification and the second stage of in vitro amplification, and in the second stage of in vitro amplification, the expression and/or activity of family members CBL is reduced.

For example, the first stage of in vitro expansion is performed for at least about 7 days. For example, the second stage of in vitro expansion is performed for at least about 7 days.

For example, the cells may be contacted with the one or more cellular activators and the cells may comprise expression of CBL family members and/or functionally active fragments thereof in a single stage of in vitro expansion of the invention. and/or reduced activity. For example, the cell activator may comprise an agonist for one or more targets selected from the group consisting of: CD3, CD28, HVEM, CD40L, OX40, and 4-1BB. For example, in a single stage of said in vitro expansion, cells of the invention are exposed to reduced expression and/or attenuated activity of CBL family members and to one or more cell activators of the invention. For example, in the first stage of in vitro expansion of the present invention, the expression and/or activity of the TIL of the present invention and the CBL family member of the present invention can be reduced and contacted with one or more cell activators of the present invention. For example, in the second stage of in vitro expansion of the present invention, the expression and/or activity of the CBL family members of the TIL of the present invention can be reduced and contacted with one or more cell activators of the present invention. For example, in the third stage of in vitro expansion of the present invention, the expression and/or activity of the CBL family members of the TIL of the present invention can be reduced and contacted with one or more cell activators of the present invention.

For example, in a single stage of in vitro expansion of the invention, cells of the invention can be exposed to substantially simultaneously reduced expression and/or activity of CBL family members and one or more cell activators of the invention. For example, in a single stage of in vitro expansion of the present invention, the cells of the present invention can be made to reduce the expression and/or activity of CBL family members first, for example, 2 hours in advance, 4 hours in advance, 8 hours in advance, or 8 hours in advance. 12 hours, 24 hours in advance, or 48 hours in advance, etc., and then contacted with one or more cell activators of the present invention. For example, in a single stage of in vitro expansion of the present invention, the cells of the present invention can be contacted with one or more cell activators of the present invention first, for example, 2 hours in advance, 4 hours in advance, 8 hours in advance, 12 hours in advance, 24 hours in advance, or 48 hours in advance, etc., to reduce the expression and/or activity of CBL family members.

For example, in the first stage of in vitro expansion of the invention, the TIL of the invention can substantially simultaneously reduce the expression and/or activity of CBL family members and contact one or more cell activators of the invention. For example, in the second stage of in vitro expansion of the invention, the TIL of the invention can substantially simultaneously reduce the expression and/or activity of CBL family members and contact one or more cell activators of the invention. For example, in the third stage of in vitro expansion of the invention, the TIL of the invention can substantially simultaneously reduce the expression and/or activity of CBL family members and contact one or more cell activators of the invention.

In another aspect, the invention provides a method for culturing tumor infiltrating lymphocytes (TIL), which may comprise: (A) making cells derived from tumor tissue, pleural effusion and/or peritoneal effusion without in vitro expansion. The first TIL population is contacted with one or more cell growth factors; wherein the second TIL population is obtained through the step (A); (B) the expression and/or activity of CBL family members of the second TIL population is reduced weaken; wherein, the third TIL group is obtained through the step (B).

In one embodiment, the first stage in vitro amplification of the present invention can be used interchangeably with step (A) in the method of the above aspect. In the one embodiment, the second stage in vitro amplification of the present invention can be used interchangeably with step (B) in the method of the above aspect. In one embodiment, the TILs expanded in vitro in the first stage of the present invention can be used interchangeably with the second TIL population obtained in step (A) of the above method. In one embodiment, the TILs expanded in vitro in the second stage of the present invention can be used interchangeably with the third TIL population obtained in step (B) of the above method. In one embodiment, if necessary, the third stage in vitro amplification of the present invention can be used interchangeably with any additional step (C) in the method of the above aspect. In one embodiment, if necessary, the third-stage in vitro expanded TIL of the present invention can be used arbitrarily interchangeably with the fourth TIL group obtained by any additional step (C) in the above method..

In another aspect, the present invention provides a method for culturing tumor infiltrating lymphocytes (TIL), which may include: (A) culturing cells derived from tumor tissue, pleural effusion and/or peritoneal effusion without in vitro amplification The first TIL population is contacted with a variety of cell growth factors; wherein, the second TIL population is obtained through the step (A); (B) the second TIL population can be contacted with a variety of cell growth factors, and with a variety of cell growth factors, and the cell activator is contacted to reduce the expression and/or activity of CBL family members, and the TILs are co-cultured with feeder cells; wherein, the third TIL population is obtained through the step (B).

In another aspect, the present invention provides a method for culturing tumor infiltrating lymphocytes (TIL), which may include: (A) culturing cells derived from tumor tissue, pleural effusion and/or peritoneal effusion without in vitro amplification The first TIL population is contacted with cell growth factors; wherein, the second TIL population is obtained through the step (A); (B) the second TIL population can be contacted with cell growth factors, with cell activators, and the expression and/or activity of CBL family members is reduced and the TILs are co-cultured with feeder cells. The CBL family members may include CBLB; wherein the third TIL population is obtained through the step (B).

In another aspect, the invention provides a method of culturing tumor-infiltrating lymphocytes (TIL). The method of obtaining TIL cells from subject tissue samples can be to obtain orthotopic tumor samples or metastatic tumor samples from the patient during surgery, and the weight can be at least about 1g, or multiple pieces of tissue can be merged. Tumor tissue, pleural effusion and/or peritoneal effusion are transported in a sample transport fluid, such as a commercially available tumor tissue transport fluid, tumor tissue preservation fluid or tumor tissue transfer fluid, shipped at about 2-8 degrees Celsius, and processed within 48 hours.. The tissue pieces can be mechanically broken to a size of about 1-27 cubic millimeters per piece, transferred into a breathable culture bag or Grex, and added with cell serum-free medium and a concentration of 300-9000IU/mL (for example, it can be 1000-9000IU/mL, for example It can be cultured with IL-2 (6000IU/mL) for about 3-14 days. Collect the cells in the culture medium and transfer them to a breathable culture bag, or Grex, or Xuri equipment. The cell serum-free culture medium can be added with the CD28 antibody, CD3 antibody and CD28 antibody of the present invention, and magnetic beads containing CD3 antibody and CD28 antibody (such as Dynabeads ) and/or a nanomatrix (such as transACT) containing CD3 antibodies and CD28 antibodies, IL-2 at a concentration of 300-9000IU/mL (for example, it can be 1000-9000IU/mL, for example, it can be 6000IU/mL), and a CBL family The expression and/or activity of members is reduced (CBL family members may include CBLB, for example, by carrying a ribonucleoprotein complex (RNP) formed by the gRNA of the present invention and Cas protein.

The TIL is transduced so that the proportion of cells encoding genes encoding CBL family members is about 95% or less), and after activating the TIL of the present invention for a certain period of time, irradiated PBMC are added (TIL to PBMC at a ratio of about 1:40 to about 1 :400), amplification culture takes about 3-14 days. Cell processing systems can be used to collect cells in the culture medium, wash, freeze, and detect. The CD3 ratio of the final product can be greater than 80%, the cell viability rate can be greater than 50%, and the cells greater than 80% can be memory effector cells and effector cells. IFN-γ can be secreted after stimulation, and/or can be characterized by an increase in the proportion of activated cells.

### Target combination knockout

1. A method of culturing cells, the method comprising: causing the reduction of expression and/or activity of at least two family members selected from the group consisting of the peptidase C64 family, the ZC3H12 family, the STAT-induced STAT inhibitor (SSI) family, and the CBL family, or their functional fragments.
2. The method of embodiment 1, wherein the cells comprise immune cells.
3. The method of embodiment 2, wherein the immune cells comprise phagocytes, lymphocytes, neutrophils, eosinophils and/or basophils.
4. The method according to any one of embodiments 2-3, wherein the immune cells comprise monocytes, macrophages and/or dendritic cells.
5. The method according to any one of embodiments 2-4, wherein the immune cells are derived from stem cell differentiated immune cells.
6. The method of embodiment 5, wherein the stem cells comprise induced pluripotent stem cells (iPSCs), embryonic stem cells, bone marrow stem cells, umbilical cord blood stem cells and/or peripheral blood stem cells.
7. The method according to any one of embodiments 2-6, wherein the immune cells comprise Contains B cells, T cells, natural killer cells and/or natural killer-like T cells (NKT).
8. The method according to any one of embodiments 2-7, wherein the immune cells comprise αβ T cells and/or γδ T cells.
9. The method of any one of embodiments 2-8, wherein the immune cells comprise tumor-infiltrating lymphocytes (TIL).
10. The method of embodiment 9, wherein the TIL is derived from tumor tissue, tumor-associated lymph nodes with or without tumor metastasis, tumor metastasis, fragments of paracancerous tissue, pleural effusion and/or peritoneal cavity. TIL from effusion and/or from TIL recovered after cryopreservation.
11. The method of embodiment 10, wherein the fragment has a volume from about 1 cubic millimeter to about 27 cubic millimeters.
12. The method of any one of embodiments 2-11, wherein the immune cell comprises an engineered immune receptor displayed on the cell surface.
13. The method of embodiment 12, wherein the engineered immune receptor specifically binds to an antigen expressed on a target cell.
14. The method of any one of embodiments 2-13, wherein the immune cell comprises a chimeric antigen receptor and/or a T cell receptor.
15. The method according to any one of embodiments 1-14, wherein said causing the cell to have at least 2 members selected from the group consisting of the peptidase C64 family, the ZC3H12 family, the STAT-inducing STAT inhibitor (SSI) family, and the CBL family. The reduced expression and/or weakened activity of a family member and/or its functionally active fragment includes at least 2 effects selected from the following group: inhibiting the function of deubiquitinating enzymes and/or zinc finger nucleases, inhibiting the function of nucleases, inhibiting the negative regulation of cytokine signaling and inhibiting E3 ubiquitin protein ligase.
16. The method according to any one of embodiments 1-15, wherein at least 2 family members selected from the group consisting of peptidase C64 family, ZC3H12 family, STAT-induced STAT inhibitor (SSI) family and CBL family and compared with cells whose expression of functionally active fragments is reduced and/or whose activity is unchanged, at least 2 selected from the group consisting of peptidase C64 family, ZC3H12 family, STAT-induced STAT inhibitor (SSI) family and CBL family Cells obtained by reducing expression and/or attenuating activity of each family member and/or functionally active fragment thereof exhibit improved cellular properties.
17. The method of embodiment 16, wherein the improved cell characteristics comprise one or more selected from the group consisting of: improved cell proliferation capacity, increased proportion of viable cells, improved proportion of cell subpopulations, increased ability to secrete cytokines and improve the killing ability of tumor cells.
18. The method of embodiment 17, wherein the improved proportion of cell subpopulations comprises one or more selected from the group consisting of: an increased proportion of activated cells, a reduced proportion of regulatory cells, a reduced proportion of exhausted cells. proportion, increased proportion of central memory cells and/or naive cells, decreased proportion of apoptotic cells and increased proportion of stem cell-like cells.
19. The method according to any one of embodiments 1-18, wherein the family members selected from the group consisting of the peptidase C64 family, the ZC3H12 family, the STAT-induced STAT inhibitor (SSI) family, and the CBL family comprise ubiquitin binding domain, C3H1 type zinc finger domain, SH2 domain and SH3 domain, respectively.
20. The method according to any one of embodiments 1-19, wherein the family members selected from the group consisting of the peptidase C64 family, the ZC3H12 family, the STAT-induced STAT inhibitor (SSI) family, and the CBL family respectively comprise TNFAIP3, ZC3H12A, SOCS1 and CBLB.
21. The method of any one of embodiments 1-20, wherein the cell is treated with a STAT inhibitor (SSI) selected from the group consisting of peptidase C64 family, ZC3H12 family, the STAT-induced STAT inhibitor (SSI) family and the CBL family involves the introduction of a gene regulatory system into the cells.
22. The method of embodiment 21, wherein the gene regulatory system destroys the family member selected from the group consisting of the peptidase C64 family, the ZC3H12 family, the STAT-induced STAT inhibitor (SSI) family and the CBL family at the DNA level; and optionally, the cells are selected from the group consisting of BRD4, FAS, FIBP, IKZF1, LAG3, MED12, PD1, RASA2, TIGIT, TIM3, ADNP, NFKBIA, PTPN6 and TNIP1 with reduced expression and/or attenuated activity.
23. The method of any one of embodiments 21-22, wherein the gene regulatory system comprises a guide nucleic acid molecule and an enzymatic protein.
24. The method of embodiment 23, wherein reducing the expression and/or attenuating the activity of the family member selected from the group consisting of the peptidase C64 family, the ZC3H12 family, the STAT-induced STAT inhibitor (SSI) family and the CBL family comprises : a complex containing the guide nucleic acid molecule and the enzyme protein or a complex containing the guide nucleic acid molecule and a nucleic acid encoding the enzyme protein, an LNP including gRNA and Cas protein, or an LNP containing encoding gRNA and encoding Cas The protein nucleic acid LNP is introduced into the cells.
25. The method according to any one of embodiments 23-24, wherein the enzyme protein comprises a Cas protein, a Cas protein homologue, or a functionally active fragment thereof, preferably selected from Cas 9 and Cas 12.
26. The method of any one of embodiments 23-25, wherein the guide nucleic acid molecule comprises a guide RNA (gRNA).
27. The method according to any one of embodiments 23-26, wherein the guide nucleic acid molecule is selected from the group consisting of the peptidase C64 family, the ZC3H12 family, the STAT-induced STAT inhibitor (SSI) family, and the CBL family. Sequence combination of at least 2 family members.
28. The method of any one of embodiments 23-27, wherein the guide nucleic acid molecule is about 15 to about 25 upstream of the 5' end of a protospacer adjacent motif (PAM) selected from the group consisting of: A sequence consisting of nucleotides: AGG, TGG, CGG, and GGG, or a sequence consisting of about 15 to about 25 nucleotides downstream of the 3' end of a protospacer adjacent motif (PAM) selected from the group shown below Combined with: NTTN, TTYN, VTTV, TRTV, TTTV, TATV, TYCV, TNN, and NTN, where N is A, T, C, or G, Y is T or C, V is A, C, or G, and R is A or G.
29. The method according to any one of embodiments 23-28, wherein the guide nucleic acid molecule binds to a region selected from the group defined by genomic coordinates shown in Tables 2A-2D or Tables 3A-3D, or a fragment thereof.
30. The method according to any one of embodiments 23-29, wherein the guide nucleic acid molecule binds to at least 2 regions or fragments thereof selected from the group consisting of: SEQ ID NO: 107-212, 1562- 2532, SEQ ID NOs: 281-348, 3116-3698, SEQ ID NOs: 399-448, 4393-5086, and SEQ ID NOs: 520-590, 6177-7266.
31. The method of any one of embodiments 23-30, wherein the guide nucleic acid molecule comprises a targeting domain comprising SEQ ID NOs: 1-106, 591-1561, 7267 -7324, 7419, 7420, SEQ ID NO: 213-280, 2533-3115, 7325-7345, 7416, 7417, SEQ ID NO: 349-398, 3699-4392, 7346-7375, 7418, and SEQ ID NO: At least 2 of the sequences shown in 449-519, 5087-6176, 7376-7413, 7414, and 7415.
32. The method according to any one of embodiments 1-31, wherein the expression and/or Expression in cells obtained by reducing the expression and/or weakening the activity of the family members selected from the group consisting of the peptidase C64 family, the ZC3H12 family, the STAT-induced STAT inhibitor (SSI) family and the CBL family compared to cells whose activity remains unchanged. The cell proportion of the target gene decreases and/or the expression level of the target gene in a single cell decreases.
33. The method of any one of embodiments 1-32, wherein the expression of the family member selected from the group consisting of the peptidase C64 family, the ZC3H12 family, the STAT-induced STAT inhibitor (SSI) family, and the CBL family is reduced And/or the proportion of cells expressing the target gene among the cells obtained with weakened activity is about 95% or less.
34. A cell obtained by the method of any one of embodiments 1-33.
35. A pharmaceutical composition comprising the cell of embodiment 34, and optionally a pharmaceutically acceptable carrier.
36. A method of affecting cell growth, comprising administering the cells of embodiment 34 and/or the pharmaceutical composition of embodiment 35.
37. Use of the cells of embodiment 34 and/or the pharmaceutical composition of embodiment 35 in the preparation of a medicament for preventing and/or treating diseases and/or symptoms.
38. A medicament for preventing and/or treating diseases and/or symptoms, comprising the cell of embodiment 34 and/or the pharmaceutical composition of embodiment 35 as an active ingredient.
39. A method for preventing and/or treating diseases and/or symptoms, comprising administering the cells of embodiment 34 and/or the pharmaceutical composition of embodiment 35 to a subject in need thereof.
40. The cell of embodiment 34 and/or the pharmaceutical composition of embodiment 35, for use in preventing and/or treating diseases and/or symptoms.
41. The use according to embodiment 37, the medicament according to embodiment 38, the method according to embodiment 39, and/or the cells and/or pharmaceutical compositions according to the use according to embodiment 40, wherein the the diseases and/or symptoms described above include tumors.
42. The use according to embodiment 37, the medicament according to embodiment 38, the method according to embodiment 39, and/or the cells and/or pharmaceutical compositions according to the use according to embodiment 40, wherein the the diseases and/or symptoms described include solid tumors.
43. The use according to embodiment 37, the medicament according to embodiment 38, the method according to embodiment 39, and/or the cells and/or pharmaceutical compositions according to the use according to embodiment 40, wherein the the diseases and/or symptoms include one or more selected from the group consisting of melanoma, ovarian cancer, cervical cancer, lung cancer, bladder cancer, breast cancer, head and neck cancer, pancreatic cancer, liver cancer, stomach cancer, colorectal cancer, and kidney cancer.

The present invention provides a method for reducing the expression of members of at least two families selected from the peptidase C64 family, the ZC3H12 family, the STAT-induced STAT inhibitor (SSI) family and the CBL family and/or functionally active fragments thereof in the cells. and/or activity reduction methods.

On the one hand, the present invention provides a method of culturing cells, so that the expression and/or activity of at least two families members selected from the group consisting of the peptidase C64 family, the ZC3H12 family, the STAT-induced STAT inhibitor (SSI) family, and the CBL family, or their functional fragments is reduced.

For example, the cells may further comprise reduced expression and/or attenuated activity optionally selected from the group consisting of BRD4, FAS, FIBP, IKZF1, LAG3, MED12, PD1, RASA2, TIGIT, TIM3, ADNP, NFKBIA, PTPN6 and TNIP1.

For example, the peptidase C64 family member may comprise a ubiquitin binding domain. For example, the peptidase C64 family member may comprise TNFAIP3.

For example, the ZC3H12 family member may comprise a C3H1-type zinc finger domain. For example, the ZC3H12 family member may comprise ZC3H12A.

For example, the STAT-induced STAT inhibitor (SSI) family member may comprise an SH2 domain. For example, the STAT-induced STAT inhibitor (SSI) family member may comprise SOCS1.

For example, the CBL family member may comprise an SH3 domain. For example, the CBL family member may include CBLB.

For example, in the cells of the present invention, the expression of peptidase C64 family and ZC3H12 family members and/or functionally active fragments thereof is reduced and/or the activity is weakened. For example, the expression and/or activity of TNFAIP3 and ZC3H12A are reduced.

For example, in the cells of the present invention, the expression of the peptidase C64 family and the STAT-induced STAT inhibitor (SSI) family members and/or functionally active fragments thereof is reduced and/or the activity is weakened. For example, the expression and/or activity of TNFAIP3 and SOCS1 are reduced.

For example, in the cells of the present invention, the expression of peptidase C64 family and CBL family members and/or functionally active fragments thereof is reduced and/or the activity is weakened. For example, the expression and/or activity of TNFAIP3 and CBLB are reduced.

For example, in the cells of the present invention, the expression of ZC3H12 family and STAT-induced STAT inhibitor (SSI) family members and/or functionally active fragments thereof is reduced and/or the activity is weakened. For example, the expression and/or activity of ZC3H12A and SOCS1 are reduced.

For example, in the cells of the present invention, the expression of ZC3H12 family and CBL family members and/or functionally active fragments thereof is reduced and/or the activity is weakened. For example, the expression and/or activity of ZC3H12A and CBLB are reduced.

For example, in the cells of the present invention, the expression of STAT-induced STAT inhibitor (SSI) family and CBL family members and/or functionally active fragments thereof is reduced and/or the activity is weakened. For example, the expression and/or activity of SOCS1 and CBLB are reduced.

For example, in the cells of the present invention, the expression of peptidase C64 family, ZC3H12 family and STAT-induced STAT inhibitor (SSI) family members and/or functionally active fragments thereof is reduced and/or the activity is weakened. For example, the expression and/or activity of TNFAIP3, ZC3H12A and SOCS1 are reduced.

For example, in the cells of the present invention, the expression of peptidase C64 family, ZC3H12 family and CBL family members and/or functionally active fragments thereof is reduced and/or the activity is weakened. For example, the expression and/or activity of TNFAIP3, ZC3H12A and CBLB are reduced.

For example, in the cells of the present invention, the expression of peptidase C64 family, STAT-induced STAT inhibitor (SSI) family and CBL family members and/or functionally active fragments thereof is reduced and/or the activity is weakened. For example, the expression and/or activity of TNFAIP3, SOCS1 and CBLB are reduced.

For example, in the cells of the present invention, the expression of ZC3H12 family, STAT-induced STAT inhibitor (SSI) family and CBL family members and/or functionally active fragments thereof is reduced and/or the activity is weakened. For example, the expression and/or activity of ZC3H12A, SOCS1 and CBLB are reduced.

For example, in the cells of the present invention, the expression of peptidase C64 family, ZC3H12 family, STAT-induced STAT inhibitor (SSI) family and CBL family members and/or functionally active fragments thereof is reduced and/or the activity is weakened. For example, the expression and/or activity of TNFAIP3, ZC3H12A, SOCS1 and CBLB are reduced.

For example, the target gene of the present invention may be a gene encoding members and/or functionally active fragments of at least two families selected from the peptidase C64 family, the ZC3H12 family, the STAT-induced STAT inhibitor (SSI) family, and the CBL family.. For example, cells obtained by reducing the expression and/or activity of at least two genes of interest in the cells may show improved cell properties compared to cells whose expression and/or activity of the genes of interest are unchanged. In one embodiment, the cells whose expression and/or activity of the target gene are unchanged may refer to cells derived

from the same donor and in which the expression and/or activity of at least two target genes of the cells have not been reduced. In one embodiment, cells whose expression and/or activity of the target gene are unchanged may refer to cells derived from the same donor and in which the expression and/or activity of other genes other than the target gene of the cells have not been altered (e.g., knocking out the other genes has basically no impact on cell function).

In one embodiment, the corresponding cells that have not reduced the expression and/or weakened the activity of at least two target genes of the cells may refer to cells derived from the same donor that have been isolated in the same way and that have not caused the cells to Cells with reduced expression and/or weakened activity of at least 2 target genes. In one embodiment, the corresponding cells that have not reduced the expression and/or weakened the activity of at least two target genes of the cells may refer to the same tumor origin from the same donor and have not caused at least two of the cells with reduced expression and/or weakened activity of 2 target genes. In one embodiment, the corresponding cells that have not reduced the expression and/or weakened the activity of at least two target genes of the cells may refer to dividing cells from the same tumor origin from the same donor into two groups, wherein a group of cells in which the expression and/or activity of at least two target genes of the cells have not been reduced may be corresponding cells in which the expression of at least two target genes of the cells has not been reduced and/or the activity has been weakened. For example, the reduced expression and/or weakened activity of at least two target genes may mean that the target genes in natural cells are in a certain degree of expression. After the treatment of the present invention, the expression level of the target genes in the cells can be reduced. Reducing, that is, reducing the expression level of the target gene, can be caused by converting natural cells from expressing the target gene to essentially not expressing the target gene or reducing the amount of expression of the target gene.

For example, the cells include immune cells. For example, the cells comprise immune effector cells. For example, the cells include immune effector T cells, immune effector NK cells, and immune effector NKT cells. For example, the cells include phagocytes, lymphocytes, neutrophils, eosinophils and/or basophils.

For example, the cells include monocytes, macrophages, and/or dendritic cells.

For example, the cells of the present invention also include cells derived from differentiation of stem cells. For example, the cells of the present invention also include cells derived from differentiation of pluripotent stem cells. For example, the stem cells of the present invention can be obtained by induction. For example, the above-mentioned stem cells of the present invention may include induced pluripotent stem cells (iPSCs), embryonic stem cells, bone marrow stem cells, umbilical cord blood stem cells and/or peripheral blood stem cells.

For example, "stem cells" of the present invention also include pluripotent cells, multipotent cells, precursor cells and progenitor cells. For example, stem cells can be obtained from hematopoietic or mesenchymal stem cells obtained from bone marrow tissue, placental stem cells obtained from placental tissue, embryonic stem cells obtained from embryonic tissue, or embryonic germ cells obtained from the reproductive tissue of the fetus. Exemplary pluripotent stem cells can also be generated from somatic cells by reprogramming them to a pluripotent state through the expression of certain transcription factors associated with pluripotency; these cells are called "induced pluripotent stem cells" or "iPSCs" .

For example, the cells include B cells, T cells, natural killer cells and/or natural killer-like T cells (NKT). For example, "unmodified cells" or "unmodified cells" may refer to cells in which the genome has not been modified and does not contain a gene regulatory system or contains a control gene regulatory system (e.g., empty vector control, non-targeting gRNA, interfering siRNA, etc. ) cells or cell populations. For example, the cells comprise αβ T cells and/or γδ T cells. For example, the cells include tumor-infiltrating lymphocytes (TILs). For example, the TIL is a TIL derived from tumor tissue, tumor-associated lymph nodes with or without tumor metastasis, tumor metastasis, fragments of paracancerous tissue, pleural effusion and/or peritoneal effusion and/or derived from frozen Save the TIL after recovery.

For example, the TILs of the present invention may be TILs and/or sources derived from tumor tissue, tumor-associated lymph nodes with or without tumor metastasis, tumor metastasis foci, fragments of paracancerous tissue, pleural effusion and/or peritoneal effusion. TILs recovered after cryopreservation. For example, TILs of the present invention can be obtained by processing tumor tissue into tumor fragments. For example, the tumor fragments of the present invention have a volume of approximately 1 to 27 cubic millimeters. For example, the volume of the tumor fragments of the present invention is about 1 cubic millimeter, about 2 cubic millimeters, about 3 cubic millimeters, about 4 cubic millimeters, about 5 cubic millimeters, about 6 cubic millimeters, about 7 cubic millimeters, about 8 cubic millimeters, about 9 cubic millimeters, about 10 cubic millimeters, about 11 cubic millimeters, about 12 cubic millimeters, about 13 cubic millimeters, about 14 cubic millimeters, about 15 cubic millimeters mm, about 16 cubic millimeters, about 17 cubic millimeters, about 18 cubic millimeters, about 19 cubic millimeters, about 20 cubic millimeters, about 21 cubic millimeters, about 23 cubic millimeters, about 24 cubic millimeters, about 25 cubic millimeters, about 26 cubic millimeters mm or approximately 27 cubic millimeters.

For example, the cells comprise engineered immune receptors displayed on the cell surface. For example, the engineered immune receptor specifically binds to an antigen expressed on a target cell. For example, the cells comprise chimeric antigen receptors and/or T cell receptors.

In one aspect, the present invention provides a method for culturing tumor infiltrating lymphocytes (TIL), which may include: making the TIL comprise a protein selected from the group consisting of the peptidase C64 family, the ZC3H12 family, the STAT-induced STAT inhibitor (SSI) family, and the CBL family. The expression and/or activity of at least 2 family members and/or functionally active fragments thereof are reduced.

For example, TILs derived from tumor tissue, tumor-associated lymph nodes with or without tumor metastasis, tumor metastasis lesions, fragments of paracancerous tissue, pleural effusion and/or peritoneal effusion and have not been amplified in vitro can be subjected to at least One stage of in vitro amplification, wherein in at least one stage of the in vitro amplification, the TIL may be selected from the group consisting of peptidase C64 family, ZC3H12 family, STAT-induced STAT inhibitor (SSI) family, and the CBLfamily. The expression and/or activity of at least 2 family members and/or functionally active fragments thereof are reduced.

For example, the present invention can be derived from tumor tissue, tumor-related lymph nodes with or without tumor metastasis, tumor metastasis foci, fragments of paracancerous tissue, pleural effusion and/or peritoneal effusion without in vitro amplification. TIL undergoes the first stage of in vitro amplification and the second stage of in vitro amplification, and in the second stage of in vitro amplification of the present invention, the TIL can be selected from the group consisting of peptidase C64 family, ZC3H12 family, and STAT-induced STAT inhibition. The expression and/or activity of at least 2 members of the agent (SSI) family and the CBL family and/or functionally active fragments thereof is reduced. For example, the tumor tissue or tumor-related lymph node derived from the present invention can be accompanied or not accompanied by tumor metastasis, tumor metastasis, fragments of adjacent cancer tissue, pleural effusion and/or peritoneal effusion and have not been amplified in vitro have undergone the first stage of in vitro amplification and the second stage of in vitro amplification, and In the first stage of in vitro amplification of the present invention, members of at least two families selected from the peptidase C64 family, the ZC3H12 family, the STAT-induced STAT inhibitor (SSI) family and the CBL family can be used in the TIL. /or reduced expression and/or activity of functionally active fragments thereof.

For example, the present invention can be derived from tumor tissue, tumor-related lymph nodes with or without tumor metastasis, tumor metastasis foci, fragments of paracancerous tissue, pleural effusion and/or peritoneal effusion without in vitro amplification. TIL undergoes the first stage of in vitro amplification and the second stage of in vitro amplification, and in the first stage of in vitro amplification of the present invention, the TIL can be selected from the peptidase C64 family, the ZC3H12 family, and STAT-induced STAT inhibition The expression and/or activity of at least 2 members of the agent (SSI) family and the CBL family and/or functionally active fragments thereof are reduced, and in the second stage of in vitro expansion of the present invention, the TIL can be The expression and/or activity of at least two family members and/or functionally active fragments thereof selected from the peptidase C64 family, the ZC3H12 family, the STAT-induced STAT inhibitor (SSI) family, and the CBL family is reduced.

For example, the present invention can be derived from tumor tissue, tumor-related lymph nodes with or without tumor metastasis, tumor metastasis foci, fragments of paracancerous tissue, pleural effusion and/or peritoneal effusion without in vitro amplification. TIL undergoes the first stage of in vitro amplification, the second stage of in vitro amplification and the third stage of in vitro amplification, and in the first stage of in vitro amplification of the present invention, the TIL can be selected from the group consisting of peptidase C64 family, ZC3H12 The expression and/or activity of at least 2 members of the STAT family, the STAT-induced STAT inhibitor (SSI) family and the CBL family and/or functionally active fragments thereof is reduced.

For example, the present invention can be derived from tumor tissue, tumor-related lymph nodes with or without tumor metastasis, tumor metastasis foci, fragments of paracancerous tissue, pleural effusion and/or peritoneal effusion without in vitro amplification. TIL undergoes the first stage of in vitro amplification, the second stage of in vitro amplification and the third stage of in vitro amplification, and in the second stage of in vitro amplification of the present invention, the TIL has reduced expression and/or activity of members of at least two families selected from the peptidase C64 family, ZC3H12 family, STAT-induced STAT inhibitor (SSI) family and CBL family and/or functionally active fragments thereof.

For example, the present invention can be derived from tumor tissue, tumor-related lymph nodes with or without tumor metastasis, tumor metastasis foci, fragments of paracancerous tissue, pleural effusion and/or peritoneal effusion without in vitro amplification. TIL undergoes the first stage of in vitro amplification, the second stage of in vitro amplification and the third stage of in vitro amplification, and in the third stage of in vitro amplification of the present invention, the expression and/or activity of at least 2 members of peptidase C64 family, the ZC3H12 family, the STAT-induced STAT inhibitor (SSI) family and the CBL family and/or functionally active fragments thereof is reduced.

For example, the present invention can be derived from tumor tissue, tumor-related lymph nodes with or without tumor metastasis, tumor metastasis foci, fragments of paracancerous tissue, pleural effusion and/or peritoneal effusion without in vitro amplification. TIL undergoes the first stage of in vitro amplification, the second stage of in vitro amplification and the third stage of in vitro amplification, and in the first stage of in vitro amplification of the present invention, the expression and/or activity of at least 2 members of peptidase C64 family, the ZC3H12 family, the STAT-induced STAT inhibitor (SSI) family and the CBL family and/or functionally active fragments thereof is reduced, and in the second stage in vitro amplification of the present invention , the expression and/or activity of at least 2 members of peptidase C64 family, the ZC3H12 family, the STAT-induced STAT inhibitor (SSI) family and the CBL family and/or functionally active fragments thereof is reduced.

For example, the present invention can be derived from tumor tissue, tumor-related lymph nodes with or without tumor metastasis, tumor metastasis foci, fragments of paracancerous tissue, pleural effusion and/or peritoneal effusion without in vitro amplification. TIL undergoes the first stage of in vitro amplification, the second stage of in vitro amplification and the third stage of in vitro amplification, and in the first stage of in vitro amplification of the present invention, the expression and/or activity of at least 2 members of peptidase C64 family, the ZC3H12 family, the STAT-induced STAT inhibitor (SSI) family and the CBL family and/or functionally active fragments thereof is reduced, and in the third stage in vitro amplification of the present invention, the expression and/or activity of at least 2 members of peptidase C64 family, the ZC3H12 family, the STAT-induced STAT inhibitor (SSI) family and the CBL family and/or functionally active fragments thereof is reduced.

For example, the present invention can be derived from tumor tissue, tumor-related lymph nodes with or without tumor metastasis, tumor metastasis foci, fragments of paracancerous tissue, pleural effusion and/or peritoneal effusion without in vitro amplification. TIL undergoes the first stage of in vitro amplification, the second stage of in vitro amplification and the third stage of in vitro amplification, and in the second stage of in vitro amplification of the present invention, the expression and/or activity of at least 2 members of peptidase C64 family, the ZC3H12 family, the STAT-induced STAT inhibitor (SSI) family and the CBL family and/or functionally active fragments thereof is reduced, and in the third stage in vitro amplification of the present invention , the expression and/or activity of at least 2 members of peptidase C64 family, the ZC3H12 family, the STAT-induced STAT inhibitor (SSI) family and the CBL family and/or functionally active fragments thereof is reduced.

For example, TILs derived from tumor tissue, tumor-related lymph nodes with or without tumor metastasis, tumor metastasis foci, fragments of paracancerous tissue, pleural effusion and/or peritoneal effusion without in vitro amplification undergoe the first stage of in vitro amplification, the second stage of in vitro amplification and the third stage of in vitro amplification, and in the first stage of in vitro amplification of the present invention, the expression and/or activity of at least 2 members of peptidase C64 family, the ZC3H12 family, the STAT-induced STAT inhibitor (SSI) family and the CBL family and/or functionally active fragments thereof is reduced, and in the second stage in vitro amplification of the present invention , the expression and/or activity of at least 2 members of peptidase C64 family, the ZC3H12 family, the STAT-induced STAT inhibitor (SSI) family and the CBL family and/or functionally active fragments thereof is reduced, and in the third stage of in vitro expansion of the present invention, the expression and/or activity of at least 2 members of peptidase C64 family, the ZC3H12 family, the STAT-induced STAT inhibitor (SSI) family and the CBL family and/or functionally active fragments thereof is reduced.

For example, changes in the number of TIL cells between each stage of in vitro expansion can be used to define each stage of in vitro expansion. For example, when the number of TIL cells increases at least approximately 1-fold, the TIL cells can be considered to have entered the next stage of in vitro expansion. In some embodiments, when the number of TIL cells increases at least about 1-1000 times, for example, at least about 1 times, at least about 2 times, at least about 3 times, at least about 4 times, at least about 5 times, at least about 6 times , at least about 7 times, at least about 8 times, at least about 9 times, at least about 10 times, at least about 11 times, at least about 12 times, at least about 13 times, at least about 14 times, at least about 15 times, at least about 20 times , at least about 30 times, at least about 40 times, at least about 50 times, at least about 100 times, at least about 200 times, at least about 500 times, or at least about 1000 times, it can be considered that the TIL cells have entered the next stage of in vitro expansion. For example, each stage of in vitro expansion can also be divided by changes in TIL cell culture conditions. For example, when cell activators and/or cell growth factors are added or supplemented in the cell culture medium, the TIL cells can be considered to have entered the next stage of in vitro expansion. For example, when IL-2 is added or supplemented to the cell culture medium, TIL cells can be considered to have entered the next stage of in vitro expansion. For example, when one or more gene regulatory systems are added or supplemented to the cell culture medium, TIL cells can be considered to have entered the next stage of in vitro expansion. For example, when feeder cells are added or supplemented to the cell culture medium, TIL cells can be considered to have entered the next stage of in vitro expansion. For example, when TIL cells undergo centrifugation and/or cell washing, the TIL cells can be considered to have entered the next stage of in vitro expansion. For example, each stage can also be divided by the number of days in which TIL cells are cultured. For example, when TIL cells are cultured in vitro for about 1-100 days, such as about 1 day, about 2 days, about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20 days, about 30 days, about 40 days Days, approximately 50 days, or approximately 100 days later, the TIL cells can be considered to have entered the next stage of in vitro expansion.

For example, the reducing the expression and/or weakening the activity of the peptidase C64 family member of the cell includes inhibiting the function of a deubiquitinating enzyme and/or a zinc finger nuclease.

For example, the reducing the expression and/or weakening the activity of the ZC3H12 family member of the cell includes inhibiting the function of a nuclease.

For example, wherein said reducing the expression and/or attenuating the activity of a member of the STAT-induced STAT inhibitor (SSI) family in said cell comprises inhibiting the function of negative regulation of cytokine signaling.

For example, the reducing the expression and/or weakening the activity of the CBL family member of the cell includes inhibiting the function of E3 ubiquitin protein ligase.

For example, compared with cells in which the expression and/or activity of members of at least 2 families selected from the peptidase C64 family, the ZC3H12 family, the STAT-induced STAT inhibitor (SSI) family, and the CBL family is unchanged, the Cells obtained by reducing the expression and/or attenuating the activity of members of at least 2 families selected from the group consisting of the peptidase C64 family, the ZC3H12 family, the STAT-induced STAT inhibitor (SSI) family and the CBL family show improved cell properties.

For example, the improved cell characteristics include one or more selected from the group consisting of: improved cell proliferation (i.e., cell number), increased proportion of viable cells, improved proportion of cell subpopulations, increased cytokine secretion capacity , improved tumor cell killing ability in vitro and improved tumor killing ability in vivo.

For example, the improved cell subpopulation ratio includes one or more selected from the group consisting of an increased activated cell ratio, a reduced regulatory cell ratio, a reduced exhausted cell ratio, an increased central memory cell, and/or Proportion of immature cells, decreased proportion of apoptotic cells, and increased proportion of stem cell-like cells.

For example, the improved cell number of the present invention refers to the expression and/or activity of at least two families selected from the peptidase C64 family, the ZC3H12 family, the STAT-induced STAT inhibitor (SSI) family, and the CBL family. Compared with cells, the expression of at least two families selected from the peptidase C64 family, the ZC3H12 family, the STAT-induced STAT inhibitor (SSI) family and the CBL family is reduced in at least one in vitro expansion stage. and/or the cell number of the cells of the invention with reduced activity can be increased by at least about 1-50 times, for example, about 1 times less, at least about 2 times, at least about 3 times, at least about 4 times, at least about 5 times, at least about 6 times, at least about 7 times, at least about 8 times, at least about 9 times, at least about 10 times, at least about 11 times, at least about 12 times, at least about 13 times, at least about 14 times, at least about 15 times, at least about 20 times, at least about 30 times, at least about 40 times, or at least about 50 times.

For example, an increased proportion of viable cells can manifest itself as an increase in cell viability. For example, the increased proportion of living cells in the present invention may refer to the expression and/or activity of members of at least two families selected from the group consisting of the peptidase C64 family, the ZC3H12 family, the STAT-induced STAT inhibitor (SSI) family, and the CBL family. Compared with unchanged cells, the members of at least 2 families selected from the group consisting of the peptidase C64 family, the ZC3H12 family, the STAT-induced STAT inhibitor (SSI) family and the CBL family are used in at least one in vitro expansion stage. The proportion of viable cells of the cells of the present invention with reduced expression and/or weakened activity can be increased by at least about 100-0.1%, such as at least about 100%, at least about 90%, at least about 80%, at least about 70%, at least about 60%, At least about 50%, at least about 40%, at least about 30%, at least about 20%, at least about 19%, at least about 18%, at least about 17%, at least about 16%, at least about 15%, at least about 14%, At least about 13%, at least about 12%, at least about 11%, at least about 10%, at least about 9%, at least about 8%, at least about 7%, at least about 6%, at least about 5%, at least about 4%, At least about 3%, at least about 2%, at least about 1%, at least about 0.9%, at least about 0.8%, at least about 0.7%, at least about 0.6%, at least about 0.5%, at least about 0.4%, at least about 0.3%, At least about 0.2%, or at least about 0.1%.

For example, the increased cytokine secretion ability of the present invention may refer to an increase in the cytokine secretion ability of a cell selected from the group consisting of: IL-2, IL-6, CD107a, GZMB, TNF-α, and IFN-γ. For example, the improved cytokine secretion ability of the present invention may refer to the expression and/or expression of members of at least two families selected from the group consisting of the peptidase C64 family, the ZC3H12 family, the STAT-induced STAT inhibitor (SSI) family, and the CBL family. Compared to cells with unchanged activity, members of at least 2 families selected from the group consisting of the peptidase C64 family, the ZC3H12 family, the STAT-induced STAT inhibitor (SSI) family and the CBL family are used in at least one in vitro expansion stage. The proportion of cells secreting cytokines in the cells of the present invention with reduced expression and/or weakened activity can be increased by at least about 1-50 times, such as at least about 1 time, at least about 2 times, at least about 3 times, at least about 4 times, at least about 5 times, at least about 6 times, at least about 7 times, at least about 8 times, at least about 9 times, at least about 10 times, at least about 11 times, at least about 12 times, at least about 13 times, at least about 14 times, at least about 15 times, at least about 20 times, at least about 30 times, at least about 40 times, or at least about 50 times. For example, the improved cytokine secretion ability of the present invention may refer to the expression and/or expression of members of at least two families selected from the group consisting of the peptidase C64 family, the ZC3H12 family, the STAT-induced STAT inhibitor (SSI) family, and the CBL family. Or compared with cells whose activity has not changed, in at least one in vitro expansion stage, at least 2 families selected from the peptidase C64 family, the ZC3H12 family, the STAT-induced STAT inhibitor (SSI) family and the CBL family are The proportion of cells secreting cytokines in cells of the invention with reduced expression and/or weakened activity of members can be increased by at least about 100-0.1%, for example, at least about 100%, at least about 90%, at least about 80%, at least about 70%, At least about 60%, at least about 50%, at least about 40%, at least about 30%, at least about 20%, at least about 19%, at least about 18%, at least about 17%, at least about 16%, at least about 15%, At least about 14%, at least about 13%, at least about 12%, at least about 11%, at least about 10%, at least about 9%, at least about 8%, at least about 7%, at least about 6%, at least about 5%, At least about 4%, at least about 3%, at least about 2%, at least about 1%, at least about 0.9%, at least about 0.8%, at least about 0.7%, at least about 0.6%, at least about 0.5%, at least about 0.4%, At least about 0.3%, at least about 0.2%, or at least about 0.1%. For example, the cytokine secretion ability of the cells of the present invention is measured by flow cytometry or CBA method (Cytometric Bead Array).

For example, the improved in vitro tumor cell killing ability and/or the improved in vivo tumor killing ability of the present invention may refer to at least one selected from the group consisting of the peptidase C64 family, the ZC3H12 family, the STAT-induced STAT inhibitor (SSI) family, and the CBL family. Cells with unchanged expression and/or activity of members of 2 families selected from the group consisting of the peptidase C64 family, ZC3H12 family, STAT-induced STAT inhibitor (SSI) family and the tumor cell killing rate of the cells of the present invention with reduced expression and/or weakened activity of at least two members of the CBL family can be increased by at least about 1-50 times, for example, at least about 1 time, at least about 2 times, at least about 3 times. times, at least about 4 times, at least about 5 times, at least about 6 times, at least about 7 times, at least about 8 times, at least about 9 times, at least about 10 times, at least about 11 times, at least about 12 times, at least about 13 times times, at least about 14 times, at least about 15 times, at least about 20 ∂times, at least about 30 times, at least about 40 times, or at least about 50 times. For example, the improved in vitro tumor cell killing ability and/or the improved in vivo tumor killing ability of the present invention may refer to at least one selected from the group consisting of the peptidase C64 family, the ZC3H12 family, the STAT-induced STAT inhibitor (SSI) family, and the CBL family. Cells with unchanged expression and/or activity of members of 2 families selected from the group consisting of the peptidase C64 family, ZC3H12 family, STAT-induced STAT inhibitor (SSI) family and The tumor cell killing rate of the cells of the present invention with reduced expression and/or weakened activity of at least two members of the CBL family can be increased by at least about 100-0.1%, such as at least about 100%, at least about 90%, at least about 80 %, at least about 70%, at least about 60%, at least about 50%, at least about 40%, at least about 30%, at least about 20%, at least about 19%, at least about 18%, at least about 17%, at least about 16 %, at least about 15%, at least about 14%, at least about 13%, at least about 12%, at least about 11%, at least about 10%, at least about 9%, at least about 8%, at least about 7%, at least about 6 %, at least about 5%, at least about 4%, at least about 3%, at least about 2%, at least about 1%, at least about 0.9%, at least about 0.8%, at least about 0.7%, at least about 0.6%, at least about 0.5 %, at least about 0.4%, at least about 0.3%, at least about 0.2%, or at least about 0.1%. For example, the tumor cell killing rate of the cells of the present invention can be measured by the IncuCyte system or CFSE and DAPI staining methods. For example, tumor cell killing of the cells of the present invention may refer to the ability of the cells to kill solid tumor cells.

For example, the improved cell subpopulation ratio of the present invention may comprise one or more selected from the following group: increased CD8 ⁺ Cell proportion, increased proportion of central memory cells and/or naive cells, decreased proportion of regulatory cells, increased proportion of activated cells, increased proportion of tumor-specific cells (with CD103 ⁺ CD39 ⁺ phenotype), increased proportion of stem cell-like cells, decreased proportion of exhausted cells, and decreased proportion of apoptotic cells.

For example, the CD8 increased by the present invention ⁺ The cell ratio may be an increase in the proportion of CD8 positive cells among the cells. For example, in cells CD8 ⁺ The cell proportion can be increased by at least about 100-0.1%, such as at least about 100%, at least about 90%, at least about 80%, at least about 70%, at least about 60%, at least about 50%, at least about 40%, at least about 30 %, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, At least about 4%, at least about 3%, at least about 2%, at least about 1%, at least about 0.9%, at least about 0.8%, at least about 0.7%, at least about 0.6%, at least about 0.5%, at least about 0.4%, At least about 0.3%, at least about 0.2%, or at least about 0.1%.

For example, the increased proportion of activated cells by the present invention can be the increased proportion of CD28 ⁺ , CD25 ⁺ and/or 41BB ⁺ cells. For example, the proportion of activated cells in the cells can be increased by at least about 100-0.1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least About 0.1%, or can be increased by at least about 1-50 times, such as at least about 1 time, at least about 2 times, at least about 3 times, at least about 4 times, at least about 5 times, at least about 6 times, at least about 7 times, At least about 8 times, at least about 9 times, at least about 10 times, at least about 11 times, at least about 12 times, at least about 13 times, at least about 14 times, at least about 15 times, at least about 20 times, at least about 30 times, At least about 40 times, or at least about 50 times.

For example, the proportion of exhausted cells reduced by the present invention can be the increased proportion of PD-1 ⁺ ,LAG-3 ⁺ ,TIM-3 ⁺ , CD39 ⁺ , CD38 ⁺ and/or CD101 ⁺ cells. For example, the proportion of depleted cells in the cells can be reduced by at least about 100-0.1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or At least about 0.1%, or can be reduced by at least about 1-50 times, such as at least about 1 times, at least about 2 times, at least about 3 times, at least about 4 times, at least about 5 times, at least about 6 times, at least about 7 times , at least about 8 times, at least about 9 times, at least about 10 times, at least about 11 times, at least about 12 times, at least about 13 times, at least about 14 times, at least about 15 times, at least about 20 times, at least about 30 times , at least about 40 times, or at least about 50 times.

For example, the proportion of regulatory cells reduced by the present invention can be the decreased proportion of CD4 ⁺ CD25 ⁺ Foxp3 ⁺ cells. For example, the proportion of regulatory cells in the cells can be reduced by at least about 100-0.1%, such as at least about 100%, at least about 90%, at least about 80%, at least about 70%, at least about 60%, at least about 50%, at least About 40%, at least about 30%, at least about 20%, at least about 19%, at least about 18%, at least about 17%, at least about 16%, at least about 15%, at least about 14%, at least about 13%, at least About 12%, at least about 11%, at least about 10%, at least about 9%, at least about 8%, at least about 7%, at least about 6%, at least about 5%, at least about 4%, at least about 3%, at least About 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the proportion of decreased apoptotic cells in the present invention can be the decreased proportion of Annexin V ⁺ 7-AAD ⁺ Cells and/or Annexin V ⁺ 7-AAD ⁻ cells. For example, the proportion of apoptotic cells in the cells can be reduced by at least about 100-0.1%, such as at least about 100%, at least about 90%, at least about 80%, at least about 70%, at least about 60%, at least about 50%, at least About 40%, at least about 30%, at least about 20%, at least about 19%, at least about 18%, at least about 17%, at least about 16%, at least about 15%, at least about 14%, at least about 13%, at least About 12%, at least about 11%, at least about 10%, at least about 9%, at least about 8%, at least about 7%, at least about 6%, at least about 5%, at least about 4%, at least about 3%, at least About 2%, at least about 1%, at least about 0.9%, at least about 0.8%, at least about 0.7%, at least about 0.6%, at least about 0.5%, at least about 0.4%, at least about 0.3%, at least about 0.2%, or At least about 0.1%.

For example, the increased proportion of cells with stemness by the present invention can be the increased proportion of CD69 ⁻ CD39 ⁻ cells and/or increased proportion of TCF1 ⁺ cells. For example, the proportion of cells with stemness in the cells can be increased by at least about 100-0.1%, such as at least about 100%, at least about 90%, at least about 80%, at least about 70%, at least about 60%, at least about 50% , at least about 40%, at least about 30%, at least about 20%, at least about 19%, at least about 18%, at least about 17%, at least about 16%, at least about 15%, at least about 14%, at least about 13% , at least about 12%, at least about 11%, at least about 10%, at least about 9%, at least about 8%, at least about 7%, at least about 6%, at least about 5%, at least about 4%, at least about 3% , at least about 2%, at least about 1%, at least about 0.9%, at least about 0.8%, at least about 0.7%, at least about 0.6%, at least about 0.5%, at least about 0.4%, at least about 0.3%, at least about 0.2% , or at least about 0.1%.

For example, the increased proportion of central memory cells by the present invention can be the increased proportion of CD45RA ⁻ CCR7 ⁺ or CD45RO ⁺ CD62L ⁺ cells. For example, the proportion of central memory cells in the cells can be increased by at least about 100-0.1%, such as at least about 100%, at least about 90%, at least about 80%, at least about 70%, at least about 60%, at least about 50%, at least About 40%, at least about 30%, at least about 20%, at least about 19%, at least about 18%, at least about 17%, at least about 16%, at least about 15%, at least about 14%, at least about 13%, at least About 12%, at least about 11%, at least about 10%, at least about 9%, at least about 8%, at least about 7%, at least about 6%, at least about 5%, at least about 4%, at least about 3%, at least About 2%, at least about 1%, at least about 0.9%, at least about 0.8%, at least about 0.7%, at least about 0.6%, at least about 0.5%, at least about 0.4%, at least about 0.3%, at least about 0.2%, or At least about 0.1%.

For example, the increased proportion of naive T cells by the present invention can be the increased proportion of CD45RO ⁻ CD62L ⁺ cells. For example, the proportion of naive cells in the cells can be increased by at least about 100-0.1%, such as at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least About 0.5%, at least about 0.4%, at least about 0.3%, at least about 0.2%, or at least about 0.1%.

For example, the culture method of the present invention may include a gene editing step for cells. For example, it includes: subjecting the cells to at least one stage of in vitro expansion, wherein a gene regulatory system can be introduced into the cells during at least one stage of the in vitro expansion.

For example, the gene regulation system can destroy the target gene at the DNA level. For example, the gene regulatory system can destroy the region of the gene of interest or a fragment thereof in the genome of the cell. For example, after using the gene control system, the DNA region or fragment thereof in the cell where the target gene is located is sheared, and the expression ability of the target gene is reduced or the activity of the target gene is inhibited. For example, the editing effect of the gene regulation system on the target gene can be long-term and sustained. For example, the activity of members of at least two families selected from the peptidase C64 family, the ZC3H12 family, the STAT-induced STAT inhibitor (SSI) family, and the CBL family is inhibited in the cells of the present invention.

The genomic region of the present invention is determined based on the hg38 version of the human reference genome.

For example, the activities of TNFAIP3 and ZC3H12A are inhibited in the cells of the present invention. For example, preferred subregions of TNFAIP3 and ZC3H12A are knocked out and/or inhibited in the cells shown in Tables 1A and 1B of the present invention respectively. For example, the preferred targeting subregions shown in TNFAIP3 R_1 to 30 in Table 1A of the present invention are combined with those shown in ZC3H12A R_1 to 9 in Table 1B to form a combination with combination numbers 1 to 270 that is knocked out and/or inhibited.

For example, the activities of TNFAIP3 and SOCS1 are inhibited in the cells of the present invention. For example, the preferred subtypes of TNFAIP3 and SOCS1 in the cells shown in Tables 1A and 1C of the present invention are respectively

Regions are knocked out and/or suppressed. For example, the preferred targeting subregions shown in TNFAIP3 R_1 to 30 in Table 1A of the present invention are combined with those shown in SOCS1 R_1 to 10 in Table 1C to form a combination with combination numbers 271 to 570 that is knocked out and/or inhibited.

For example, the activities of TNFAIP3 and CBLB are inhibited in the cells of the present invention. For example, preferred subregions of TNFAIP3 and CBLB are knocked out and/or inhibited in the cells shown in Tables 1A and 1D of the present invention, respectively. For example, two combinations of the preferred targeting subregions shown in TNFAIP3 R_1 to 30 in Table 1A of the present invention and CBLB R_1 to 44 in Table 1D are combined to form combination numbers 571 to 1890 that are knocked out and/or inhibited.

For example, the activities of ZC3H12A and SOCS1 are inhibited in the cells of the present invention. For example, preferred subregions of ZC3H12A and SOCS1 are knocked out and/or inhibited in the cells shown in Tables 1B and 1C of the present invention respectively. For example, the preferred targeting subregions shown in ZC3H12A R_1 to 9 in Table 1B of the present invention are combined with those shown in SOCS1 R_1 to 10 in Table 1C to form a combination with combination numbers 1891 to 1980 that is knocked out and/or inhibited.

For example, the activities of ZC3H12A and CBLB are inhibited in the cells of the present invention. For example, preferred subregions of ZC3H12A and CBLB are knocked out and/or inhibited in the cells shown in Tables 1B and 1D of the present invention, respectively. For example, the preferred targeting subregions shown in ZC3H12A R_1 to 9 in Table 1B of the present invention and CBLB R_1 to 44 in Table 1D are combined to form a combination with combination numbers 1981 to 2376 that is knocked out and/or inhibited.

For example, the activities of SOCS1 and CBLB are inhibited in the cells of the present invention. For example, preferred subregions of SOCS1 and CBLB are knocked out and/or inhibited in the cells shown in Tables 1C and 1D of the present invention, respectively. For example, the preferred targeting subregions shown in SOCS1 R_1 to 10 in Table 1C of the present invention and CBLB R_1 to 44 in Table 1D are combined to form a combination with combination numbers 2377 to 2816 that is knocked out and/or inhibited.

For example, the gene regulatory system may include guide nucleic acid molecules and enzymatic proteins. For example, the enzyme protein can have nucleic acid shearing enzyme activity, and the guide nucleic acid molecule can guide the

The enzyme protein specifically cuts the region where the target gene is located or its fragment. For example, the guide nucleic acid molecule and the enzyme protein may exist in the form of a ribonucleoprotein complex (RNP), or each may exist independently. For example, the enzyme protein may comprise Cas protein. For example, polynucleotides encoding gRNA and Cas protein can be introduced into the target cell, or each independently.

For example, reducing the expression and/or weakening the activity of at least two target genes in a cell according to the present invention may include: introducing a ribonucleoprotein complex (RNP) containing the guide nucleic acid molecule and the enzyme protein into the cell. For example, the enzyme protein may comprise Cas protein, Cas protein homologues, or functionally active fragments thereof. For example, the guide nucleic acid molecule may comprise guide RNA (gRNA). For example, a complex comprising a polynucleotide encoding a gRNA and a Cas protein can be introduced into the cell. For example, a complex containing gRNA and Cas protein can be introduced into the cell.

For example, the gRNA can be used to bind to the sequence of the gene of interest. For example, the binding of the gRNA to the sequence of the target gene may be completely complementary, partially complementary, or may hybridize to the sequence of the target gene under moderately stringent or stringent conditions. For example, the binding of the gRNA to the sequence of the target gene can cause the CRISPR system of the gRNA to specifically cleave the target gene.

For example, the editing target region of the present invention may be a region before the start codon. For example, the editing target region of the present invention may be a region with high transcription factor binding capacity. For example, the editing target region of the present invention may be a region with a specific number of transcription factor binding numbers. For example, the editing target region of the present invention can be a continuous region with a binding number of transcription factors of about 3 or more. For example, the genomic coordinates of the editing target region of the present invention can be selected from the preferred targeting subregions shown in Tables 1A to 1D.

For example, the guide nucleic acid molecule targeting TNFAIP3 of the present invention can be combined with a region selected from the following group or a fragment thereof: SEQ ID NO: 107-212, 1562-2532. For example, the ZC3H12A-targeting guide nucleic acid molecule of the present invention can be combined with a region selected from the following group or a fragment thereof: SEQ ID NO: 281-348, 3116-3698. For example, the present invention

The guide nucleic acid molecule targeting SOCS1 may be combined with a region or fragment thereof selected from the group consisting of: SEQ ID NO: 399-448, 4393-5086. For example, the guidance nucleic acid molecule targeting CBLB of the present invention can be combined with a region selected from the following group or a fragment thereof: SEQ ID NO: 520-590, 6177-7266.

For example, when the gene editing system includes CRISPR/Cas9, there may be a protospacer adjacent motif (PAM) downstream of the region targeted by the guiding nucleic acid molecule of the present invention, and the protospacer adjacent motif (PAM) may be AGG, TGG, GGG or CGG. For example, when the PAM region of the target gene is determined, those skilled in the art can easily determine about 15 to about 25 (for example, about 15, about 16, about 17, about 18, about 19, about 20) upstream of the 5' end of the PAM of the target gene. , about 21, about 22, about 23, about 24, about 25) nucleotides, and a suitable gRNA can be designed for the target sequence. For example, the guide nucleic acid molecule is capable of binding to a sequence consisting of about 15 to about 25 nucleotides upstream of the 5' end of a protospacer adjacent motif (PAM) selected from the group consisting of: AGG, TGG, GGG, and CGG.

For example, when the gene editing system includes CRISPR/Cas12, there may be a protospacer adjacent motif (PAM) upstream of the region targeted by the guiding nucleic acid molecule of the present invention, and the protospacer adjacent motif (PAM) may be NTTN, TTYN, VTTV, TRTV, TTTV, TATV, TYCV, TNN, or NTN, where N is A, T, C, or G, Y is T or C, V is A, C, or G, and R is A or G. For example, when the PAM region of the target gene is determined, those skilled in the art can easily determine about 15 to about 25 (for example, about 15, about 16, about 17, about 18, about 19, about 20) downstream of the 3' end of the PAM of the target gene. , about 21, about 22, about 23, about 24, about 25) nucleotides, and a suitable gRNA can be designed for the target sequence. For example, the guide nucleic acid molecule can bind to a sequence consisting of about 15 to about 25 nucleotides downstream of the 3' end of the protospacer adjacent motif (PAM) selected from the group consisting of: NTTN, TTYN, VTTV, TRTV , TTTV, TATV, TYCV, TNN, or NTN, where N is A, T, C, or G, Y is T or C, V is A, C, or G, and R is A or G.

For example, when the gene editing system of the present invention includes wild-type Cas12a (also known as Cpf1, such as AsCas12a, FnCas12a, LbCas12a, BbCas12a, CMaCas12a and OsCas12a), the upstream region of the guiding nucleic acid molecule targeting of the present invention can be selected from the following PAM sequence: NTTN, where N can be A, T, C or G. For example, when the PAM region of the target gene is determined, those skilled in the art can easily determine about 17 to about 25 (for example, about 17, about 18, about 19, about 20, about 21, about 22) downstream of the 3' end of the PAM of the target gene. , about 23, about 24, or about 25) nucleotides, and a suitable gRNA can be designed for the target sequence.

For example, when the gene editing system of the present invention includes mutant Cas12a, such as enAsCas12a (mutation sites E174R, S542R and K548R), the upstream of the region targeted by the guiding nucleic acid molecule of the present invention can have a PAM sequence selected from the following: TTYN (TTTN /TTCN), VTTV (ATTV/CTTV/GTTV), or TRTV (TATV/TGTV), where N can be A, T, C or G, Y can be T or C, V can be A, C or G, R Can be A or G. For example, when the PAM region of the target gene is determined, those skilled in the art can easily determine about 17 to about 25 (for example, about 17, about 18, about 19, about 20, about 21, about 22) downstream of the 3' end of the PAM of the target gene. , about 23, about 24, or about 25) nucleotides, and a suitable gRNA can be designed for the target sequence.

For example, when the gene editing system of the present invention includes mutant Cas12a, such as opAsCas12a (mutation sites: E174R and S542R), the upstream of the region targeted by the guiding nucleic acid molecule of the present invention can have a PAM sequence selected from the following: TTTV (TTTA, TTTC, or TTTG), where V can be A, C, or G. For example, when the PAM region of the target gene is determined, those skilled in the art can easily determine about 17 to about 25 (for example, about 17, about 18, about 19, about 20, about 21, about 22) downstream of the 3' end of the PAM of the target gene. , about 23, about 24, or about 25) nucleotides, and a suitable gRNA can be designed for the target sequence.

For example, when the gene editing system of the present invention includes mutant Cas12a, such as AsCas12a Ultra (mutation sites: M537R and F870L), the guiding nucleic acid molecule of the present invention targets

The region upstream can have a PAM sequence selected from: TTTV, TATV, or TYCV, where V can be A, C, or G, and Y can be T or C. For example, when the PAM region of the target gene is determined, those skilled in the art can easily determine about 17 to about 25 (for example, about 17, about 18, about 19, about 20, about 21, about 22) downstream of the 3' end of the PAM of the target gene. , about 23, about 24, or about 25) nucleotides, and a suitable gRNA can be designed for the target sequence.

For example, when the gene editing system of the present invention includes mutant Cas12a, such as hfCas12Max (mutation site: N243R/E336R/D892R) and Cas12Max (mutation site: N243R), the upstream region of the guiding nucleic acid molecule targeting of the present invention can be A PAM sequence selected from: TNN, or NTN, where N can be A, T, C, or G. For example, when the PAM region of the target gene is determined, those skilled in the art can easily determine about 17 to about 25 (for example, about 17, about 18, about 19, about 20, about 21, about 22) downstream of the 3' end of the PAM of the target gene. , about 23, about 24, or about 25) nucleotides, and a suitable gRNA can be designed for the target sequence.

For example, the guide nucleic acid molecule may comprise a member and/or a functionally active fragment thereof capable of binding and encoding at least 2 families selected from the group consisting of the peptidase C64 family, the ZC3H12 family, the STAT-induced STAT inhibitor (SSI) family and the CBL family. The target sequence consists of about 15 to about 25 nucleotides before the PAM region represented by AGG, TGG, GGG and/or CGG in the DNA where the gene is located. For example, the guide nucleic acid molecule may comprise a member and/or a functionally active fragment thereof capable of binding and encoding at least 2 families selected from the group consisting of the peptidase C64 family, the ZC3H12 family, the STAT-induced STAT inhibitor (SSI) family and the CBL family. In the DNA where the gene is located, there are about 15 to about 25, about 17 to about 25, about 19 to about 25, about 20 to about 25, about 21 to About 25, about 23 to about 25, about 15 to about 23, about 17 to about 23, about 19 to about 23, about 20 to about 23, about 21 to about 23, about 22 to about 23, about 15 to about 21, about 17 to about 21, about 19 to about 21, about 20 to about 21, about 15 to about 20, about 17 to about 20, about 19 to about 20 A target sequence consisting of, about 15 to about 19, about 17 to about 19, or about 15 to about 17 nucleotides. For example, target

The sequence may be selected from the region defined by the genomic coordinates shown in Tables 2A-2D or a fragment thereof. In the present invention, only for the convenience of display and limited display space, some target sequences are displayed as 2A-2D as target sequences for double knockout; however, two or two are selected from Tables 3A-3D in the present invention. The target sequences of more types of targets can be combined with each other and can also be used to double-knock or multiple-knock the target.

For example, the guide nucleic acid molecule may comprise a targeting domain of a sgRNA targeting TNFAIP3 as shown in any one of SEQ ID NOs: 1-106, 591-1561, 7267-7324, 7419, 7420, such as SEQ ID NO : The targeting domain of the sgRNA targeting ZC3H12A shown in any one of 213-280, 2533-3115, 7325-7345, 7416, and 7417, such as SEQ ID NO: 349-398, 3699-4392, 7346-7375, The targeting domain of the sgRNA targeting SOCS1 as shown in any one of 7418, or the sgRNA targeting CBLB as shown in any one of SEQ ID NOs: 449-519, 5087-6176, 7376-7413, 7414, and 7415 targeting domain.

For example, the activities of TNFAIP3 and ZC3H12A are inhibited in the cells of the present invention. For example, SEQ ID NOs: 1-106 of the present invention and the guide sequences shown in SEQ ID NOs: 213-280 are combined in pairs to form editing combination numbers 1 to 7208, which are knocked out and/or inhibited.

For example, the activities of TNFAIP3 and SOCS1 are inhibited in the cells of the present invention. For example, SEQ ID NOs: 1-106 of the present invention and the guide sequences shown in SEQ ID NOs: 349-398 are combined in pairs to form editing combination numbers 7209 to 12508, which are knocked out and/or suppressed.

For example, the activities of TNFAIP3 and CBLB are inhibited in the cells of the present invention. For example, SEQ ID NOs: 1-106 of the present invention and the guide sequences shown in SEQ ID NOs: 449-519 are combined in pairs to form editing combination numbers 12509 to 20034, which are knocked out and/or suppressed.

For example, the activities of ZC3H12A and SOCS1 are inhibited in the cells of the present invention. For example, the guide shown in SEQ ID NO: 213-280 and SEQ ID NO: 349-398 of the present invention were combined in pairs to form editing combination serial numbers 20035 to 23434, which were knocked out and/or suppressed.

For example, the activities of ZC3H12A and CBLB are inhibited in the cells of the present invention. For example, SEQ ID NOs: 213-280 of the present invention and the guide sequences shown in SEQ ID NOs: 449-519 are combined in pairs to form editing combination numbers 23435 to 28262, which are knocked out and/or inhibited.

For example, the activities of SOCS1 and CBLB are inhibited in the cells of the present invention. For example, SEQ ID NOs: 349-398 of the present invention and the guide sequences shown in SEQ ID NOs: 449-519 are combined in pairs to form editing combination numbers 28263 to 31812, which are knocked out and/or suppressed.

For example, the ratio of cells expressing the product of the target gene in cells obtained by reducing the expression and/or weakening the activity of at least two target genes in the cells compared with cells in which the expression and/or activity of the target gene is unchanged. can be reduced and/or the expression level of the target gene in a single cell can be reduced.

For example, in the method of the present invention, the target genes are expressed in cells obtained by reducing the expression and/or activity of at least two target genes in the cells compared with cells in which the expression and/or activity of the target genes are unchanged. The cell proportion of the product is reduced by at least about 5%. For example, a cell expressing the product of a gene encoding members and/or functionally active fragments of at least 2 families selected from the peptidase C64 family, the ZC3H12 family, the STAT-induced STAT inhibitor (SSI) family, and the CBL family. The proportion is reduced by at least about 100-5%, such as at least about 100%, at least about 90%, at least about 80%, at least about 70%, at least about 60%, at least about 50%, at least about 40%, at least about 30%, At least about 20%, at least about 19%, at least about 18%, at least about 17%, at least about 16%, at least about 15%, at least about 14%, at least about 13%, at least about 12%, at least about 11%, At least about 10%, at least about 9%, at least about 8%, at least about 7%, at least about 6%, or at least about 5%. For example, expression of the cellular proportion of the products of genes of at least 2 family members and/or functionally active fragments thereof of the STAT inhibitor (SSI), peptidase C64 family, the ZC3H12 family, and STAT-induced the CBL family can range from an observable cellular proportion to 1%. For example, a cell expressing the product of a gene encoding members and/or functionally active fragments of at least 2 families selected from the peptidase C64 family, the ZC3H12 family, the STAT-induced STAT inhibitor (SSI) family, and the CBL family. The ratio may be reduced to at least about 100-1%, such as at least about 100%, at least about 90%, at least about 80%, at least about 70%, at least about 60%, at least about 50%, at least about 40%, at least about 30 %, at least about 20%, at least about 19%, at least about 18%, at least about 17%, at least about 16%, at least about 15%, at least about 14%, at least about 13%, at least about 12%, at least about 11 %, at least about 10%, at least about 9%, at least about 8%, at least about 7%, at least about 6%, at least about 5%, or at least about 1%. For example, a cell expressing the product of a gene encoding members and/or functionally active fragments of at least 2 families selected from the peptidase C64 family, the ZC3H12 family, the STAT-induced STAT inhibitor (SSI) family, and the CBL family. The ratio can be measured by cell flow cytometry.

For example, in the method of the present invention, the expression of at least two target genes of the cells is reduced and/or the activity is weakened, and the cells expressing the genes selected from the group consisting of peptidase C64 family, ZC3H12 family, STAT-induced STAT inhibitor (SSI) are obtained. The cellular proportion of products of genes of at least 2 families in the ) family and the CBL family and/or functionally active fragments thereof may be at most about 95%. For example, a cell expressing the product of a gene encoding members and/or functionally active fragments of at least 2 families selected from the peptidase C64 family, the ZC3H12 family, the STAT-induced STAT inhibitor (SSI) family, and the CBL family. The proportion of cells in the ratio may be at most about 95-5%, such as at most about 95%, at most about 90%, at most about 80%, at most about 70%, at most about 60%, at most about 50%, at most about 40%, at most About 30%, at most about 20%, at most about 19%, at most about 18%, at most about 17%, at most about 16%, at most about 15%, at most about 14%, at most about 13%, at most about 12%, at most About 11%, up to about 10%, up to about 9%, up to about 8%, up to about 7%, up to about 6%, or up to about 5%. For example, a cell expressing the product of a gene encoding members and/or functionally active fragments thereof of at least 2 families selected from the peptidase C64 family, the ZC3H12 family, the STAT-induced STAT inhibitor (SSI) family, and the CBL family. The ratio can be measured by cell flow cytometry.

For example, in the method of the present invention, compared with cells in which the expression and/or activity of the target genes are unchanged, the cells obtained by reducing the expression and/or activity of at least two target genes are described in a single cell. The expression level of the target gene can be reduced by at least about 5%. For example, the expression level of the gene of interest in a single cell can be reduced by at least about 100-5%, such as at least about 100%, at least about 90%, at least about 80%, at least about 70%, at least about 60%, at least about 50 %, at least about 40%, at least about 30%, at least about 20%, at least about 19%, at least about 18%, at least about 17%, at least about 16%, at least about 15%, at least about 14%, at least about 13 %, at least about 12%, at least about 11%, at least about 10%, at least about 9%, at least about 8%, at least about 7%, at least about 6%, or at least about 5%. For example, the expression level of the target gene in a single cell can range from the observable expression level to 1%. For example, the expression level of the gene of interest in a single cell can be reduced to at least about 100-1%, such as at least about 100%, at least about 90%, at least about 80%, at least about 70%, at least about 60%, at least about 50% %, at least about 40%, at least about 30%, at least about 20%, at least about 19%, at least about 18%, at least about 17%, at least about 16%, at least about 15%, at least about 14%, at least about 13 %, at least about 12%, at least about 11%, at least about 10%, at least about 9%, at least about 8%, at least about 7%, at least about 6%, at least about 5%, at least about 4%, at least about 3 %, at least about 2%, or at least about 1%.

For example, in the method of the present invention, the expression level of the target genes in a single cell obtained by reducing the expression and/or activity of at least two target genes in the cells can be the expression and/or activity of the target genes. At most about 95% of the cells are unchanged. For example, the genes encoding members and/or functionally active fragments thereof of at least 2 families selected from the peptidase C64 family, the ZC3H12 family, the STAT-induced STAT inhibitor (SSI) family and the CBL family in a single cell (e.g. The expression level of genes encoding TNFAIP3, ZC3H12A, SOCS1, and CBLB may be the genes encoding at least two families selected from the group consisting of the peptidase C64 family, the ZC3H12 family, the STAT-induced STAT inhibitor (SSI) family, and the CBL family, and At most about 95-5% of the cells in which the expression and/or activity of the functionally active fragments thereof is unchanged, for example, at most about 95%, at most about 90%, at most about 80%, at most about 70%, at most about 60%, At most about 50%, at most about 40%, at most about 30%, at most about 20%,

At most about 19%, at most about 18%, at most about 17%, at most about 16%, at most about 15%, at most about 14%, at most about 13%, at most about 12%, at most about 11%, at most about 10%, Up to about 9%, up to about 8%, up to about 7%, up to about 6%, or up to about 5%.

For example, the method of the present invention includes: causing the cells to undergo at least one stage of in vitro expansion, wherein in at least one stage of the in vitro expansion, the cells are selected from the group consisting of peptidase C64 family, ZC3H12 family, STAT Decreased expression and/or attenuated activity of members of at least 2 families of the induced STAT inhibitor (SSI) family and the CBL family.

For example, the TILs derived from tumor tissue, tumor-related lymph nodes with or without tumor metastasis, tumor metastasis lesions, fragments of para-cancerous tissue, pleural effusion and/or peritoneal effusion and have not been amplified in vitro are subjected to The first stage of in vitro amplification and the second stage of in vitro amplification, and in the second stage of in vitro amplification, the TILs amplified in the first stage of in vitro are selected from the group consisting of peptidase C64 family, ZC3H12 family, STAT Decreased expression and/or attenuated activity of members of at least 2 families of the induced STAT inhibitor (SSI) family and the CBL family.

For example, the first stage of in vitro expansion is performed for at least about 7 days. For example, the second stage of in vitro expansion is performed for at least about 7 days.

For example, in a single stage of in vitro expansion of the present invention, the cells can be contacted with the one or more cell activators and the cells can contain a protein selected from the group consisting of peptidase C64 family, ZC3H12 family, STAT-inducing The expression and/or activity of at least 2 members of the STAT inhibitor (SSI) family and the CBL family and/or functionally active fragments thereof is reduced. For example, the cell activator may comprise an agonist for one or more targets selected from the group consisting of: CD3, CD28, HVEM, CD40L, OX40, and 4-1BB. For example, in a single stage of the in vitro expansion, the cells of the present invention are made to contain members of at least two families selected from the peptidase C64 family, the ZC3H12 family, the STAT-induced STAT inhibitor (SSI) family, and the CBL family. Expression and/or activity is reduced and the cell is contacted with one or more cell activating agents of the invention. For example, in the first stage of in vitro amplification of the present invention, the TIL of the present invention can be selected from the group consisting of peptidase C64 family, ZC3H12 family, and STAT-induced STAT inhibitor.

The expression and/or activity of at least 2 members of the (SSI) family and the CBL family is reduced and the TIL is contacted with one or more cell activators of the invention. For example, in the second stage of in vitro amplification of the present invention, the TIL of the present invention can be selected from at least two families selected from the peptidase C64 family, the ZC3H12 family, the STAT-induced STAT inhibitor (SSI) family and the CBL family. The expression and/or activity of the TIL is reduced and the TIL is contacted with one or more cell activators of the invention. For example, in the third stage of in vitro amplification of the present invention, the TIL of the present invention can be selected from at least two families selected from the peptidase C64 family, the ZC3H12 family, the STAT-induced STAT inhibitor (SSI) family and the CBL family. The expression and/or activity of the TIL is reduced and the TIL is contacted with one or more cell activators of the invention.

For example, in a single stage of in vitro expansion, the cells of the present invention substantially simultaneously use members of at least 2 families selected from the peptidase C64 family, the ZC3H12 family, the STAT-induced STAT inhibitor (SSI) family, and the CBL family. The expression and/or activity is reduced and contact with one or more cell activators of the invention. For example, in a single stage of in vitro expansion, the cells of the present invention first express members of at least 2 families selected from the peptidase C64 family, the ZC3H12 family, the STAT-induced STAT inhibitor (SSI) family, and the CBL family. Reduction and/or activity weakening, for example, can be carried out 2-48 hours in advance, such as 2 hours in advance, 4 hours in advance, 8 hours in advance, 12 hours in advance, 24 hours in advance, or 48 hours in advance, etc., and then combined with a method of the present invention or contact with multiple cell activators. For example, in a single stage of in vitro expansion, the cells of the present invention are first contacted with one or more cell activators of the present invention, for example, 2-48 hours in advance, such as 2 hours in advance, 4 hours in advance, 8 hours in advance. hours, 12 hours in advance, 24 hours in advance, or 48 hours in advance, etc., and then use members of at least two families selected from the peptidase C64 family, the ZC3H12 family, the STAT-induced STAT inhibitor (SSI) family and the CBL family. Reduced expression and/or weakened activity.

For example, in the first stage of in vitro amplification of the present invention, the TIL of the present invention substantially simultaneously activates at least 2 families selected from the peptidase C64 family, the ZC3H12 family, the STAT-induced STAT inhibitor (SSI) family and the CBL family. The expression and/or activity of the members is reduced and contact with one or more cell activators of the invention. For example, in the second stage of in vitro amplification of the present invention, the expression and/or activity of at least 2 members of peptidase C64 family, the ZC3H12 family, the STAT-induced STAT inhibitor (SSI) family and the CBL family and/or functionally active fragments thereof is reduced and contact with one or more cell activators of the invention. For example, in the third stage of in vitro amplification of the present invention, the TIL of the present invention substantially simultaneously activates at least 2 families selected from the group consisting of the peptidase C64 family, the ZC3H12 family, the STAT-induced STAT inhibitor (SSI) family and the CBL family. the expression and/or activity of at least 2 members of peptidase C64 family, the ZC3H12 family, the STAT-induced STAT inhibitor (SSI) family and the CBL family and/or functionally active fragments thereof is reduced.

For example, the second stage of in vitro expansion of the invention is conducted for at least about 7 days. For example, the second stage in vitro amplification of the present invention can be performed for at least about 7 days, at least about 8 days, at least about 9 days, at least about 10 days, at least about 11 days, at least about 12 days, at least about 13 days, or at least About 14 days. For example, the second stage in vitro amplification of the present invention can be carried out for about 9 days to about 14 days. For example, the second stage in vitro amplification of the present invention can be carried out for about 9 days to about 14 days, about 10 days to about 14 days, About 11 days to about 14 days, about 12 days to about 14 days, about 13 days to about 14 days, about 9 days to about 13 days, about 10 days to about 13 days, about 11 days to about 13 days, about 12 days to about 13 days, about 9 days to about 12 days, about 10 days to about 12 days, about 11 days to about 12 days, or about 10 days to about 11 days. For example, the second stage of in vitro amplification of the present invention can be considered as the REP (rapid expansion protocol) stage. For example, the first stage of in vitro amplification of the present invention can be considered as the preREP stage.

For example, the first stage of in vitro expansion of the invention is conducted for at least about 7 days. For example, the second stage in vitro amplification of the present invention can be performed for at least about 7 days, at least about 8 days, at least about 9 days, at least about 10 days, at least about 11 days, at least about 12 days, at least about 13 days, or at least About 14 days. For example, the second stage in vitro amplification of the present invention can be carried out for about 9 days to about 14 days. For example, the second stage in vitro amplification of the present invention can be carried out for about 9 days to about 14 days, about 10 days to about 14 days, About 11 days to about 14 days, about 12 days to about 14 days, about 13 days to about 14 days, about 9 days to about 13 days, about 10 days to about 13 days, about 11 days to about 13 days, about 12 days to about 13 days, about 9 days to about 12 days, about 10 days to about 12 days, about 11 days to about 12 days, or about 10 days to about 11 days.

For example, the number of days during the second stage of in vitro amplification of the present invention can be calculated from the start time of the second stage of in vitro amplification. For example, when the second-stage in vitro amplification starts, it can be considered that approximately 0 hour has passed in the second-stage in vitro amplification. For example, if the second stage of in vitro amplification is carried out for about 24 hours after the start, it can be considered that the second stage of in vitro amplification is carried out for about 1 day. For example, the day on which the second stage of in vitro amplification begins can be considered to be about 0 days of the second stage of in vitro amplification. For example, the number of days for the second stage of in vitro amplification of the present invention can be calculated based on the number of days for the second stage of in vitro amplification. For example, the day after the second stage of in vitro amplification starts, it can be considered that the second stage of in vitro amplification has been carried out for about one day.

For example, the cell activator of the invention may comprise one or more selected from the group consisting of: CD80, CD86, B7-H3, 4-1BBL, CD27, CD30, CD134, B7h, CD40, LIGHT, and their functional activities fragment. For example, a cell activator of the invention may comprise an agonist for one or more targets selected from the group consisting of: CD3, CD28, HVEM, CD40L, OX40 and 4-1BB. For example, the cell activator of the invention may comprise antibodies selected from the group consisting of CD3, CD28, HVEM, CD40L, OX40 and 4-1BB and antigen-binding fragments thereof. For example, the cell activating agent of the present invention may comprise a CD3 agonist. For example, the cell activator of the present invention may include an anti-CD3 antibody and/or an antigen-binding fragment thereof, such as OKT3 from Miltenyi Biotech or SP34 from BD. For example, a cell activator of the invention may comprise a CD28 agonist. For example, the cell activator of the present invention may comprise an anti-CD28 antibody and/or an antigen-binding fragment thereof, such as Merck's 15E8.

For example, the cell activator of the present invention may comprise an anti-CD3 antibody and/or an antigen-binding fragment thereof, for example, it may comprise the light chain VL and heavy chain VH of OKT3 from Miltenyi Biotech, and it may comprise the light chain VL and heavy chain of BD's SP34. VH. For example, a cell activator of the invention may comprise a CD28 agonist. For example, the cell activator of the present invention may comprise an anti-CD28 antibody and/or an antigen-binding fragment thereof, such as the light chain VL and heavy chain VH of Merck's 15E8. For example, the cell activator of the present invention may comprise an anti-CD3 antibody and/or an antigen-binding fragment thereof, for example, it may comprise the light chain LCDR1-3 and the heavy chain HCDR1-3 of Miltenyi Biotech's OKT3, and it may comprise the light chain of BD's SP34.

LCDR1-3 and heavy chain HCDR1-3, the anti-CD3 antibody and/or antigen-binding fragment thereof of the present invention may have CD3-binding ability. For example, a cell activator of the invention may comprise a CD28 agonist. For example, the cell activator of the present invention may comprise an anti-CD28 antibody and/or an antigen-binding fragment thereof, for example, it may comprise the light chain LCDR1-3 and heavy chain HCDR1-3 of Merck's 15E8, the anti-CD28 antibody of the present invention and/or its antigen-binding fragment. Or its antigen-binding fragment may have CD28-binding ability. In the present invention, the antibody of the present invention or its antigen-binding protein comprises at least one CDR in the variable region VH of the heavy chain of the antibody and/or at least one CDR in the variable region VL of the light chain of the antibody. The CDRs of the present invention may be defined according to the IMGT nomenclature, the CDRs of the present invention may be defined according to Chothia, or the CDRs of the present invention may be defined according to Kabat.

For example, contacting a cell of the invention with one or more cell activators of the invention may comprise one or more means selected from the following group: (1) adding a cell activator of the invention to the cell of the invention in the cell culture medium; (2) adding engineered cells expressing the cell activator of the present invention to the cell culture medium of the cells of the present invention; (3) adding a solid phase medium containing the cell activator of the present invention to the cell culture medium of the present invention. cells in the cell culture medium. For example, contacting a cell of the invention with one or more cell activators of the invention may comprise adding a solid phase medium comprising a cell activator of the invention to the cell culture medium of the cells of the invention. For example, contacting a cell of the invention with one or more cell activators of the invention may comprise adding a solid phase medium comprising a CD28 antibody and a CD3 antibody of the invention to the cell culture medium of the cells of the invention.

For example, the initial concentration of the cell activator in the cell culture medium of the cells of the invention can be at least about 30 ng/mL. For example, the initial concentration of the CD28 antibody of the invention in the cell culture medium of the cells of the invention can be at least about 30 ng/mL; for example, the initial concentration of the CD3 antibody of the invention in the cell culture medium of the cells of the invention can be at least about 30ng/mL. For example, the selection of the initial concentration of the CD28 antibody of the present invention can be independent of the selection of the initial concentration of the CD3 antibody of the present invention; for example, the initial concentration of the CD28 antibody of the present invention and the CD3 antibody of the present invention in the cell culture medium of the cells of the present invention Can be combined in any way. For example, the initial concentration of the CD28 antibody of the invention in the cell culture medium of the cells of the invention can be from about 30ng/mL to about 300ng/mL. For example, the initial concentration of the CD3 antibody of the present invention in the cell culture medium of the cells of the present invention can be arbitrarily selected from about 30 ng/mL to about 300 ng/mL. For example, the initial concentration of the CD28 antibody of the invention in the cell culture medium of the cells of the invention can be arbitrarily selected from about 30ng/mL to about 300ng/mL, and the initial concentration of the CD3 antibody of the invention in the cell culture medium of the cells of the invention The concentration can be arbitrarily selected from about 30ng/mL to about 300ng/mL, and the selection of the initial concentration of the CD28 antibody of the present invention can be independent of the selection of the initial concentration of the CD3 antibody of the present invention. For example, the diameter of the solid media of the present invention may range from about 500 nanometers to about 10 micrometers. For example, the diameter of the solid medium of the present invention can be measured by transmission electron microscopy. For example, the diameter of the solid media of the present invention can range from about 1 nanometer to about 500 nanometers. For example, the diameter of the solid media of the present invention can range from about 100 nanometers to about 500 nanometers. For example, the diameter of the solid media of the present invention may range from about 200 nanometers to about 500 nanometers. For example, the diameter of the solid medium of the present invention can be measured by transmission electron microscopy.

For example, the solid phase media of the present invention may contain polymers. For example, the solid phase medium of the present invention may contain dextran.

For example, the solid phase medium of the invention contains at least about 25 µg of the cell activator of the invention per mg of the solid phase medium of the invention.

For example, the solid phase medium containing the cell activator of the present invention is added to the present invention at a ratio of about 100: 1 to about 1:2000, preferably about 1:100 to about 1:2000. In the cell culture medium of the invented cells. For example, the solid phase medium containing the cell activator of the invention is added to the cell culture medium of the cells of the invention at a ratio of the solid phase medium of the invention to the cells of the invention of about 2:1 to about 1:2.

For example, when the diameter of the solid phase medium of the present invention is about 100 nanometers to about 500 nanometers, the cells of the present invention can be included in a ratio of about 2:1 to about 1:2 of the solid phase medium of the present invention and the cells of the present invention. A solid medium of activator is added to the cell culture medium of the cells of the invention. For example, when the diameter of the solid phase medium of the present invention is about 100 nanometers to about 500 nanometers, it can be about 2:1 to about 1:2, about 2:1 to about 1:1, or about 1:1 to about 1:2 in the ratio of the solid phase medium of the present invention to the cells of the present invention, the activation of cells containing the present invention will be A solid phase medium of an agent, such as a CD3 agonist and/or a CD28 agonist, is added to the cell culture medium of the cells of the invention.

For example, when the diameter of the solid phase medium of the present invention is about 100 nanometers to about 500 nanometers, the cells of the present invention can be included in a ratio of the solid phase medium of the present invention to the cells of the present invention of about 1:100 to about 1:2000. A solid medium of activator is added to the cell culture medium of the cells of the invention. For example, when the diameter of the solid phase medium of the present invention is about 100 nanometers to about 500 nanometers, the ratio can be about 1:100 to about 1:2000, about 1:200 to about 1:2000, about 1:300 to about 1:300. About 1:2000, about 1:400-about 1:2000, about 1:500-about 1:2000, about 1:600-about 1:2000, about 1:700-about 1:2000, about About 1:800-about 1:2000, about 1:900-about 1:2000, about 1:1000-about 1:2000, about 1:1200-about 1:2000, about 1:1400-about 1 :2000, at a ratio of about 1:1600 to about 1:2000, or at a ratio of about 1:1800 to about 1:2000 of the solid phase medium of the present invention and the cells of the present invention, for example, the CD28 agonist of the present invention and the CD3 agonist can be The solid phase medium of the agent is added to the cell culture medium of the cells of the present invention.

For example, the methods of the invention may further comprise contacting the cells of the invention with one or more cell growth factors in at least one stage of in vitro expansion of the invention.

For example, in a single stage of in vitro expansion of the invention, cells of the invention can be contacted with a cell activator of the invention and with one or more cell growth factors of the invention. For example, in the first stage of in vitro expansion of the invention, the TIL of the invention can be contacted with the cell activator of the invention and with one or more cell growth factors of the invention. For example, in the second stage of in vitro expansion of the invention, the TIL of the invention can be contacted with the cell activator of the invention and with one or more cell growth factors of the invention. For example, in the third stage of in vitro expansion of the invention, the TIL of the invention can be contacted with the cell activator of the invention and with one or more cell growth factors of the invention.

For example, in a single stage of in vitro expansion of the invention, cells of the invention are contacted with a cell activator of the invention and one or more cell growth factors of the invention substantially simultaneously. For example, in a single stage of in vitro expansion of the invention, the cells of the invention can be contacted with one or more cell growth factors of the invention and one or more cell activators of the invention substantially simultaneously. For example, in a single stage of in vitro expansion of the present invention, the cells of the present invention can be contacted with one or more cell growth factors of the present invention first, for example, 2-48 hours in advance, such as 2 hours in advance, 4 hours, 8 hours in advance, 12 hours in advance, 24 hours in advance, or 48 hours in advance, etc., and then contacted with one or more cell activators of the present invention. For example, in a single stage of in vitro expansion of the present invention, the cells of the present invention can be contacted with one or more cell activators of the present invention first, for example, 2-48 hours in advance, such as 2 hours in advance, 2 hours in advance, 4 hours, 8 hours in advance, 12 hours in advance, 24 hours in advance, or 48 hours in advance, etc., and then contacted with one or more cell growth factors of the present invention.

For example, in the first stage of in vitro expansion of the invention, the cells of the invention can be contacted with the cell activator of the invention and one or more cell growth factors of the invention substantially simultaneously. For example, in the second stage of in vitro expansion of the invention, the TIL of the invention can be contacted with the cell activator of the invention and one or more cell growth factors of the invention substantially simultaneously. For example, in the third stage of in vitro expansion of the invention, the TIL of the invention can be contacted with the cell activator of the invention and one or more cell growth factors of the invention substantially simultaneously.

For example, the cell growth factor of the present invention can be selected from one or more of the following groups: IL-2, IL-7, IL-12, IL-15, IL-21, gamma interferon, and functionally active fragments thereof . For example, the cell growth factors of the invention may comprise IL-2 and/or functionally active fragments thereof. For example, a functionally active fragment of IL-2 may comprise a fragment of IL-2 known in the art that can bind to a cell's IL-2 receptor. For example, the cell growth factors of the invention may comprise IL-2 and/or functionally active fragments thereof, IL-7 and/or functionally active fragments thereof, and IL-15 and/or functionally active fragments thereof.

For example, contacting a cell of the invention with one or more cell growth factors of the invention may comprise adding a cell growth factor of the invention to the cell culture medium of the cells of the invention. For example, the initial concentration of the cell growth factor of the invention in the cell culture medium of the cells of the invention can be at least about 300IU/mL. For example, the initial concentration of IL-2 of the present invention in the cell culture medium of the cells of the present invention can be at least about 300-9000 IU/mL, such as at least about 300 IU/mL, at least about 350 IU/mL, at least about 400 IU/mL, at least about 500IU/mL, at least about 600IU/mL, at least about 700IU/mL, at least about 800IU/mL, at least about 900IU/mL, at least about 1000IU/mL, at least about 1100IU/mL, at least about 1200IU/mL, at least about 1300IU/mL mL, at least about 1400IU/mL, at least about 1500IU/mL, at least about 2000IU/mL, at least about 2500IU/mL, at least about 2600IU/mL, at least about 2700IU/mL, at least about 2800IU/mL, at least about 2900IU/mL, At least about 3000IU/mL, at least about 3100IU/mL, at least about 3200IU/mL, at least about 3300IU/mL, at least about 3400IU/mL, at least about 3500IU/mL, at least about 4000IU/mL, at least about 4500IU/mL, at least about 5000IU/mL, at least about 5500IU/mL, at least about 6000IU/mL, at least about 6500IU/mL, at least about 7000IU/mL, at least about 7500IU/mL, at least about 8000IU/mL, at least about 8500IU/mL, or at least about 9000IU /mL.

For example, when the cells of the present invention are in contact with IL-2, IL-7 and IL-15, the dosage of cytokines can be reduced compared to when they are in contact with IL-2 only. For example, adding IL-7 and IL-15 can reduce the amount of IL-2 added. For example, the concentration of IL-7 may be about 1 to 1000 ng/mL, preferably about 1 to 100 ng/mL. For example, the concentration of IL-15 may be about 1 to 1000 ng/mL, preferably about 1 to 100 ng/mL. For example, the amount of IL-2 added can be reduced to the range commonly used in the art for various immune cells, for example, to 50-10% of the range commonly used in the art, such as 50%, 20% or 10%. For example, the IL-2 addition amount of TCR-T can be in the range of 30-300IU/mL commonly used in this field. For example, the amount of IL-2 added to TIL can range from 300 to 9000 IU/mL (for example, 1000 to 9000 IU/mL) commonly used in the field.

For example, the method of the present invention may further comprise: in at least one stage of in vitro expansion of the present invention, the cells of the present invention may be co-cultured with feeder cells.

For example, in a single stage of in vitro expansion of the invention, cells of the invention can be contacted with one or more cell activators and/or one or more cell growth factors and co-cultured with feeder cells of the invention, e.g. A single stage of in vitro amplification of the present invention may refer to the same stage

For example, the in vitro amplification of the present invention can be carried out in the first stage of the present invention, the second stage of in vitro amplification of the present invention, or the third stage of the present invention.

For example, in the first stage of in vitro expansion of the present invention, the TIL of the present invention can be contacted with one or more cell activators and/or one or more cell growth factors and co-cultured with the feeder cells of the present invention. For example, in the second stage of in vitro expansion of the present invention, the TIL of the present invention can be contacted with one or more cell activators and/or one or more cell growth factors of the present invention and with the feeder cells of the present invention. Co-culture. For example, in the third stage of in vitro expansion of the present invention, the TIL of the present invention can be contacted with one or more cell activators and/or one or more cell growth factors of the present invention and with the feeder cells of the present invention.

For example, in a single stage of in vitro expansion of the present invention, the cells of the present invention can be contacted with one or more cell activators and/or one or more cell growth factors of the present invention for a certain period of time, and then co-cultured with feeder cells of the present invention. For example, in the first stage of in vitro expansion of the present invention, the TIL of the present invention can be contacted with one or more cell activators and/or one or more cell growth factors of the present invention for a certain period of time, and then co-cultured with feeder cells of the present invention. For example, in the second stage of in vitro expansion of the present invention, the TIL of the present invention can be contacted with one or more cell activators and/or one or more cell growth factors of the present invention for a certain period of time, and then co-cultured with feeder cells the present invention. For example, in the third stage of in vitro expansion of the present invention, the TIL of the present invention can be contacted with one or more cell activators and/or one or more cell growth factors of the present invention for a certain period of time, and then co-cultured with feeder cells of the present invention.

For example, in a single stage of in vitro expansion of the present invention, the cells of the present invention can be contacted with one or more cell activators and/or one or more cell growth factors of the present invention for a certain period of time, and then co-cultured with feeder cells of the present invention. For example, a certain amount of time for the present invention may be at least about 1 hour. For example, the certain time of the present invention can be at least about 1-72 hours, such as at least about 1 hour, at least about 2 hours, at least about 3 hours, at least about 4 hours, at least about 5 hours, at least about 6 hours, at least about 7 hours, at least about 8 hours, at least about 9 hours, at least about 10 hours, at least about 11 hours, at least about 12 hours, at least about 13 hours, at least about 14 hours, at least about 15 hours, at least about 16 hours, at least about 17 hours, at least about 18 hours, at least about 19 hours, at least about 20 hours, at least about 21 hours, at least about 22 hours, at least about 23 hours, at least about 24 hours, at least about 36 hours, at least about 48 hours, at least about 60 hours, or at least about 72 hours. For example, a certain period of time in the present invention may range from about 2 hours to about 72 hours. For example, the certain time of the present invention can be about 6 hours to about 7 hours, about 6 hours to about 8 hours, about 6 hours to about 9 hours, about 6 hours to about 10 hours, about 6 hours to about 11 hours, about 6 hours to about 12 hours, about 6 hours to about 13 hours, about 6 hours to about 14 hours, about 6 hours to about 15 hours, about 6 hours to about 16 hours, about 6 hours to about 17 hours, about 6 hours to about 18 hours, about 6 hours to about 19 hours, about 6 hours to about 20 hours, about 6 hours to about 21 hours, about 6 hours to about 22 hours, about 6 hours to about 23 hours, about 6 hours to about 24 hours, about 6 hours to about 36 hours, about 6 hours to about 48 hours, about 6 hours to about 60 hours, or about 6 hours to about 72 hours. For example, the certain time of the present invention can be about 12 hours to about 13 hours, about 12 hours to about 14 hours, about 12 hours to about 15 hours, about 12 hours to about 16 hours, about 12 hours to about 17 hours, about 12 hours to about 18 hours, about 12 hours to about 19 hours, about 12 hours to about 20 hours, about 12 hours to about 21 hours, about 12 hours to about 22 hours, about 12 hours to about 23 hours, about 12 hours to about 24 hours, about 12 hours to about 36 hours, about 12 hours to about 48 hours, about 12 hours to about 60 hours, or about 12 hours to about 72 hours. For example, the certain time of the present invention can be about 1 hour, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours , about 24 hours, about 36 hours, about 48 hours, about 60 hours or about 72 hours.

For example, the feeder cells of the present invention may comprise antigen-presenting cells. For example, the feeder cells of the present invention may comprise one or more selected from the group consisting of peripheral mononuclear cells, dendritic cells, and artificial antigen-presenting cells. For example, the feeder cells of the present invention may be peripheral mononuclear cells. For example, the feeder cells of the present invention may be irradiated feeder cells.

For example, the feeder cells of the present invention can be isolated artificial antigen-presenting cells (aAPC), and the artificial antigen-presenting cells of the present invention can include cells expressing HLA-A/B/C, CD64, CD80, ICOS-L and/or CD58 , and can be modified to express more than one cell activator of the invention. For example, the feeder cells of the present invention can be irradiated, for example, they can be irradiated with gamma rays, or they can be irradiated with X-rays.

For example, co-culture of the cells of the present invention and the feeder cells of the present invention may include contacting the surface of the feeder cells of the present invention with the surface of the cells of the present invention. For example, co-culture of the cells of the present invention and the feeder cells of the present invention includes adding the feeder cells of the present invention to the cell culture medium of the cells of the present invention.

For example, the present invention can add the feeder cells of the present invention to the cell culture medium of the cells of the present invention at a ratio of the feeder cells of the present invention to the cells of the present invention of about 40:1 to about 400:1. For example, the present invention can be used in a ratio of about 40:1 to about 400:1, in a ratio of about 40:1 to about 300:1, in a ratio of about 40:1 to about 200:1, in a ratio of about 40:1 to about 100:1, in a ratio of about 40:1 to about 100:1. About 40:1 to about 90:1, about 40:1 to about 80:1, about 40:1 to about 70:1, about 40:1 to about 60:1, about 40:1 to about 60:1 50:1, about 50:1 to about 400:1, about 60:1 to about 400:1, about 70:1 to about 400:1, about 80:1 to about 400:1, about 90:1 to about 400:1, about 100:1 to about 400:1, about 200:1 to about 400:1, or about 300:1 to about 400:1 between the feeder cells of the present invention and the present invention The proportion of cells, the feeder cells of the invention are added to the cell culture medium of the cells of the invention.

In another aspect, the present invention provides a method for culturing tumor infiltrating lymphocytes (TIL), which may include: (A) making cells derived from tumor tissue, tumor-associated lymph nodes with or without tumor metastasis, tumor metastasis lesions, The fragments of paracancerous tissue, pleural effusion and/or peritoneal effusion and the first TIL population that has not been amplified in vitro are contacted with one or more cell growth factors; wherein the second TIL is obtained through the step (A) group; (B) reducing the expression and/or activity of members of at least two families of the second TIL group selected from the group consisting of the peptidase C64 family, the ZC3H12 family, the STAT-induced STAT inhibitor (SSI) family, and the CBL family. weaken; wherein, the third TIL group is obtained through the step (B).

In another aspect, the present invention provides a method for culturing tumor infiltrating lymphocytes (TIL), which includes: (A) making cells derived from tumor tissue, tumor-associated lymph nodes with or without tumor metastasis, tumor metastasis lesions, cancer Debris from nearby tissue, pleural effusion and/or peritoneal effusion and the first TIL population that has not been expanded in vitro are contacted with one or more T cell growth factors, wherein the second TIL is obtained through the step (A) group; (B) reducing the expression and/or activity of members of at least two families of the second TIL group selected from the group consisting of the peptidase C64 family, the ZC3H12 family, the STAT-induced STAT inhibitor (SSI) family, and the CBL family, and contacting the second TIL population with a T cell activator and/or a T cell growth factor, wherein a third TIL population is obtained through the step (B); (C) the third TIL population is contacted with a T cell activator and/or a T cell growth factor Feeder cells are co-cultured, wherein the fourth TIL population is obtained through the step (C).

In one embodiment, the first stage of in vitro amplification of the present invention can be used arbitrarily interchangeably with step (A) in the method of the above aspect. In one embodiment, the second stage in vitro amplification of the present invention can be used arbitrarily interchangeably with step (B) in the method of the above aspect. In one embodiment, the TILs amplified in vitro in the first stage of the present invention can be used arbitrarily interchangeably with the second TIL population obtained in step (A) of the above method. In one embodiment, the TILs expanded in vitro in the second stage of the present invention can be used arbitrarily interchangeably with the third TIL group obtained in step (B) of the above method. In one embodiment, if necessary, the third stage of in vitro amplification of the present invention can be used in any replacement with any additional step (C) in the method of the above aspect. In one embodiment, if necessary, the third-stage in vitro amplified TIL of the present invention can be used arbitrarily interchangeably with the fourth TIL group obtained through any additional step (C) in the above method.

In another aspect, the present invention provides a method for culturing tumor infiltrating lymphocytes (TIL), which may include: (A) making cells derived from tumor tissue, tumor-associated lymph nodes with or without tumor metastasis, and tumor metastasis lesions, fragments of paracancerous tissue, pleural effusion and/or peritoneal effusion and the first TIL population that has not been amplified in vitro are contacted with a variety of cell growth factors; wherein, the second TIL population is obtained through the step (A); (B) The second TIL population can be contacted with a variety of cell growth factors, contacted with a variety of cell activators, selected from the group consisting of the peptidase C64 family, the ZC3H12 family, the STAT-induced STAT inhibitor (SSI) family, and CBL in the family

The expression and/or activity of at least two family members is reduced, and the TIL is co-cultured with feeder cells; wherein, a third TIL population is obtained through the step (B).

In another aspect, the present invention provides a method for culturing tumor infiltrating lymphocytes (TIL), which may include: (A) making cells derived from tumor tissue, tumor-associated lymph nodes with or without tumor metastasis, and tumor metastasis lesions, fragments of paracancerous tissue, pleural effusion and/or peritoneal effusion and the first TIL population that has not been amplified in vitro are contacted with cell growth factors; wherein, the second TIL population is obtained through the step (A); (B ) The second TIL population may be contacted with a cell growth factor, a cell activator, or at least two selected from the group consisting of the peptidase C64 family, the ZC3H12 family, the STAT-induced STAT inhibitor (SSI) family, and the CBL family. The expression and/or activity of family members is reduced and the TIL is co-cultured with feeder cells, at least 2 of which are selected from the group consisting of the peptidase C64 family, the ZC3H12 family, the STAT-induced STAT inhibitor (SSI) family and the CBL family. Members of the family may include TNFAIP3, ZC3H12A, SOCS1, and CBLB respectively; wherein, the third TIL group is obtained through the step (B).

In another aspect, the invention provides a method of culturing tumor-infiltrating lymphocytes (TIL). The method of obtaining TIL cells from a subject's tissue sample can be to obtain an orthotopic tumor sample or a metastatic tumor sample from the patient through surgery, and the weight can be at least about 1g, or multiple pieces of tissue can be merged. Tumor tissue, tumor-related lymph nodes with or without tumor metastasis, tumor metastasis, fragments of adjacent tumor tissue, pleural effusion and/or peritoneal effusion in the sample transport fluid, which can be, for example, commercially used tumor tissue transport fluid, Transport in tumor tissue preservation solution or tumor tissue transport solution at approximately 2-8°C and process within 48 hours. The tissue pieces can be mechanically broken to a size of about 1-27 cubic millimeters each, transferred into a breathable culture bag or Grex, and added with cell serum-free medium and a concentration of 300-9000IU/mL (for example, 1000-9000IU/mL, for example 6000IU/mL). mL) of IL-2 for about 3-14 days. Collect the cells in the culture medium and transfer them to a breathable culture bag, or Grex, or Xuri equipment. The cell serum-free culture medium can be added with the CD28 antibody, CD3 antibody and CD28 antibody of the present invention, and magnetic beads containing CD3 antibody and CD28 antibody (such as Dynabeads ) and/or a nanomatrix (eg transACT) comprising CD3 antibodies and CD28 antibodies, IL-2 at a concentration of 300-9000IU/mL (eg 1000-9000IU/mL, eg 6000IU/mL) and the expression and/or activity of at least 2 members of the a peptidase C64 family , ZC3H12 family, SSI family and the CBL family is reduced (selected from peptidase C64 family members may include TNFAIP3, ZC3H12 family members may include ZC3H12A, STAT-induced STAT Inhibitor (SSI) family members may include SOCS1 and CBL family members may include CBLB, for example, by carrying a ribonucleoprotein complex (RNP) formed by the gRNA and Cas protein of the present invention, or an LNP containing gRNA and Cas protein. , or the LNP containing the nucleic acid encoding gRNA and Cas protein is transduced so that the TIL encodes at least 2 families selected from the group consisting of peptidase C64 family, ZC3H12 family, STAT-induced STAT inhibitor (SSI) family and CBL family. The cell ratio of the gene of the member is about 95% or less), after activating the TIL of the present invention for a certain period of time, add irradiated PBMC (the ratio of TIL to PBMC is about 1:40 to about 1:400), and expand and culture for about 3- 14 days. Cell processing systems can be used to collect cells in the culture medium, wash, freeze, and detect. The CD3 ratio of the final product can be greater than 80%, the cell survival rate can be greater than 50%, and the cells greater than 80% can be memory effector cells and effector cells. IFN-γ can be secreted after stimulation, and/or can be characterized by an increase in the proportion of activated cells.

On the one hand, the present invention provides a cell, and the cell of the present invention can be cultured according to the culture method of the present invention. In one embodiment, the cells provided by the present invention may comprise one type or a batch of cells cultured by the culture method of the present invention. In one embodiment, the cells provided by the present invention may comprise multiple or multiple batches of cells cultured by the culture method of the present invention and combined in any proportion.

In some embodiments, cells expanded using the methods of the present invention can be administered to a patient as a pharmaceutical composition. In some embodiments, the pharmaceutical composition can be a suspension of cells in a sterile buffer. Cells expanded using the PBMCs of the invention may be administered by any suitable route known in the art. In some embodiments, cells can be administered in a single intra-arterial or intravenous infusion, which can last about 30 to 60 minutes. Other suitable routes of administration may include intraperitoneal, intrathecal and intralymphatic administration.

In some embodiments, any suitable dose of cells may be administered. In some embodiments, for example when the tumor is melanoma, approximately 1 × 10 ⁹ to approximately 13.7×10 ¹⁰ , preferably about 2.3×10 ⁹ to approximately 13.7×10 ¹⁰ cells. In some embodiments, about 1 x 10 ⁹ to approximately 12×10 ¹⁰ cells. In some embodiments, about 1.2 x 10 ¹⁰ to approximately 4.3×10 ¹⁰ cells. In some embodiments, about 3 x 10 ¹⁰ to approximately 12×10 ¹⁰ cells. In some embodiments, about 4 x 10 ¹⁰ to approximately 10×10 ¹⁰ cells. In some embodiments, about 5 x 10 ¹⁰ to approximately 8×10 ¹⁰ cells. In some embodiments, about 6 x 10 ¹⁰ to approximately 8×10 ¹⁰ cells. In some embodiments, about 7 x 10 ¹⁰ to approximately 8×10 ¹⁰ cells. In some embodiments, a therapeutically effective dose can be about 1 x 10 ⁹ to approximately 13.7×10 ¹⁰ , preferably about 2.3×10 ⁹ to approximately 13.7×10 ¹⁰ . In some embodiments, a therapeutically effective dose can be about 1 x 10 ⁹ to approximately 12×10 ¹⁰ cells. In some embodiments, the therapeutically effective dose can be about 1.2 x 10 ¹⁰ to approximately 4.3×10 ¹⁰ cells. In some embodiments, the therapeutically effective dose can be about 3 x 10 ¹⁰ to approximately 12×10 ¹⁰ cells. In some embodiments, a therapeutically effective dose can be about 4 x 10 ¹⁰ to approximately 10×10 ¹⁰ cells. In some embodiments, a therapeutically effective dose can be about 5 x 10 ¹⁰ to approximately 8×10 ¹⁰ cells. In some embodiments, a therapeutically effective dose can be about 6 x 10 ¹⁰ to approximately 8×10 ¹⁰ cells. In some embodiments, a therapeutically effective dose can be about 7 x 10 ¹⁰ to approximately 8×10 ¹⁰ cells.

In some embodiments, the number of cells provided in the compositions of the invention can be about 1 x 10⁶ -9×10 ¹³ , for example about 1×10 ⁶ , about 2×10 ⁶ , about 3×10 ⁶ , about 4×10 ⁶ , about 5×10 ⁶ , about 6×10 ⁶ , about 7×10 ⁶ , about 8×10 ⁶ , about 9×10 ⁶ , about 1×10 ⁷ , about 2×10 ⁷ , about 3×10 ⁷ , about 4×10 ⁷ , about 5×10 ⁷ , about 6×10 ⁷ , about 7×10 ⁷ , about 8×10 ⁷ , about 9×10 ⁷ , about 1×10 ⁸ , about 2×10 ⁸, about 3×10 ⁸ , about 4×10 ⁸ , about 5×10 ⁸ , about 6×10 ⁸ , about 7×10 ⁸ , about 8×10 ⁸ , about 9×10 ⁸ , about 1×10 ⁹ , about 2×10 ⁹ , about 3×10 ⁹ , about 4×10 ⁹ , about 5×10 ⁹ , about 6×10 ⁹ , about 7×10 ⁹ , about 8×10 ⁹ , about 9×10 ⁹ , about 1×10 ¹⁰ , about 2×10 ¹⁰ , about 3×10 ¹⁰ , about 4×10 ¹⁰ , about 5×10 ¹⁰ , about 6×10 ¹⁰ , about 7×10 ¹⁰ , about 8×10 ¹⁰ , about 9×10 ¹⁰ , about 1×10 ¹¹ , about 2×10 ¹¹ , about 3×10 ¹¹ , about 4×10 ¹¹ , about 5×10 ¹¹ , about 6×10 ¹¹ , about 7×10 ¹¹ , about 8×10 ¹¹ , about 9×10 ¹¹ , about 1×10 ¹² , about 2×10 ¹² , about 3×10 ¹² , about 4×10 ¹² , about 5×10 ¹² , about 6×10 ¹² , about 7×10 ¹² , about 8×10 ¹² , about 9×10 ¹² , about 1×10 ¹³ , about 2×10 ¹³ , about 3×10 ¹³ , about 4×10 ¹³ , about 5×10 ¹³ , about 6×10 ¹³ , about 7×10 ¹³ , about 8×10 ¹³ , or about 9×10 ¹³ .

In some embodiments, the number of cells provided in the compositions of the invention can range from about 1 x 10⁶ to 5×10 ⁶ , about 5×10 ⁶ to 1×10 ⁷ , about 1×10 ⁷ to 5×10⁷, about 5×10 ⁷ to 1×10 ⁸ , about 1×10 ⁸ to 5×10 ⁸ , about 5×10 ⁸ to 1×10 ⁹ , about 1×10 ⁹ to 5×10 ⁹ , about 5×10 ⁹ to 1×10 ¹⁰ , about 1×10 ¹⁰ to 5×10 ¹⁰ , about 5×10 ¹⁰ to 1×10 ¹¹ , about 5×10 ¹¹ to 1×10 ¹² , about 1×10 ¹² to 5×10 ¹² , about 5×10 ¹² to 1×10 ¹³ , about 1×10 ¹³ to 5×10 ¹³ , or about 5×10 ¹³ to 9×10 ¹³ .

In some embodiments, the concentration of cells provided in the compositions of the invention can be less than about 100-0.0001% w/w, w/v or v/v of the composition, such as about 100%, about 90%, about 80% , about 70%, about 60%, about 50%, about 40%, about 30%, about 20%, about 19%, about 18%, about 17%, about 16%, about 15%, about 14%, about 13%, about 12%, about 11%, about 10%, about 9%, about 8%, about 7%, about 6%, about 5%, about 4%, about 3%, about 2%, about 1% , about 0.9%, about 0.8%, about 0.7%, about 0.6%, about 0.5%, about 0.4%, about 0.3%, about 0.2%, about 0.1%, about 0.09%, about 0.08%, about 0.07%, about 0.06%, about 0.05%, about 0.04%, about 0.03%, about 0.02%, about 0.01%, about 0.009%, about 0.008%, about 0.007%, about 0.006%, about 0.005%, about 0.004%, about 0.003% , about 0.002%, about 0.001%, about 0.0009%, about 0.0008%, about 0.0007%, about 0.0006%, about 0.0005%, about 0.0004%, about 0.0003%, about 0.0002%, or about 0.0001% w/w, w /v or v/v.

In some embodiments, the concentration of cells provided in the compositions of the invention can be greater than about 90-0.0001% w/w, w/v or v/v of the composition, such as about 90%, about 80%, about 70% , about 60%, about 50%, about 40%, about 30%, about 20%, about 19.75%, about 19.50%, about 19.25%, about 19%, about 18.75%, about 18.50%, about 18.25%, about 18%, about 17.75%, about 17.50%, about 17.25%, about 17%, about 16.75%, about 16.50%, about 16.25%, about 16%, about 15.75%, about 15.50%, about 15.25%, about 15% , about 14.75%, about 14.50%, about 14.25%, about 14%, about 13.75%, about 13.50%, about 13.25%, about 13%, about 12.75%, about 12.50%, about 12.25%, about 12%, about 11.75%, about 11.50%, about 11.25%, about 11%, about 10.75%, about 10.50%, about 10.25%, about 10%, about 9.75%, about 9.50%, about 9.25

%, about 9%, about 8.75%, about 8.50%, about 8.25%, about 8%, about 7.75%, about 7.50%, about 7.25%, about 7%, about 6.75%, about 6.50%, about 6.25%, About 6%, about 5.75%, about 5.50%, about 5.25%, about 5%, about 4.75%, about 4.50%, about 4.25%, about 4%, about 3.75%, about 3.50%, about 3.25%, about 3 %, about 2.75%, about 2.50%, about 2.25%, about 2%, about 1.75%, about 1.50%, about 1.25%, about 1%, about 0.9%, about 0.8%, about 0.7%, about 0.6%, About 0.5%, about 0.4%, about 0.3%, about 0.2%, about 0.1%, about 0.09%, about 0.08%, about 0.07%, about 0.06%, about 0.05%, about 0.04%, about 0.03%, about 0.02 %, about 0.01%, about 0.009%, about 0.008%, about 0.007%, about 0.006%, about 0.005%, about 0.004%, about 0.003%, about 0.002%, about 0.001%, about 0.0009%, about 0.0008%, About 0.0007%, about 0.0006%, about 0.0005%, about 0.0004%, about 0.0003%, about or 0.0002%, or about 0.0001% w/w, w/v or v/v.

In some embodiments, the concentration of cells provided in the compositions of the invention may range from about 0.0001% to about 50%, from about 0.001% to about 40%, from about 0.01% to about 30%, from about 0.02% to about 0.02% of the composition. About 29%, about 0.03% to about 28%, about 0.04% to about 27%, about 0.05% to about 26%, about 0.06% to about 25%, about 0.07% to about 24%, about 0.08% to about 23 %, about 0.09% to about 22%, about 0.1% to about 21%, about 0.2% to about 20%, about 0.3% to about 19%, about 0.4% to about 18%, about 0.5% to about 17%, About 0.6% to about 16%, about 0.7% to about 15%, about 0.8% to about 14%, about 0.9% to about 12%, or about 1% to about 10% w/w, w/v or v/ v.

In some embodiments, the concentration of cells provided in the compositions of the invention may range from about 0.001% to about 10%, from about 0.01% to about 5%, from about 0.02% to about 4.5%, from about 0.03% to about 0.03% to about 10% of the composition. About 4%, about 0.04% to about 3.5%, about 0.05% to about 3%, about 0.06% to about 2.5%, about 0.07% to about 2%, about 0.08% to about 1.5%, about 0.09% to about 1 %, or about 0.1% to about 0.9% w/w, w/v or v/v.

In some embodiments, the amount of cells provided in the compositions of the present invention may be equal to or less than about 10-0.0001 g, such as about 10 g, about 9.5 g, about 9.0 g, about 8.5 g, about 8.0 g,

About 7.5g, about 7.0g, about 6.5g, about 6.0g, about 5.5g, about 5.0g, about 4.5g, about 4.0g, about 3.5g, about 3.0g, about 2.5g, about 2.0g, about 1.5 g, about 1.0g, about 0.95g, about 0.9g, about 0.85g, about 0.8g, about 0.75g, about 0.7g, about 0.65g, about 0.6g, about 0.55g, about 0.5g, about 0.45g, About 0.4g, about 0.35g, about 0.3g, about 0.25g, about 0.2g, about 0.15g, about 0.1g, about 0.09g, about 0.08g, about 0.07g, about 0.06g, about 0.05g, about 0.04 g, about 0.03g, about 0.02g, about 0.01g, about 0.009g, about 0.008g, about 0.007g, about 0.006g, about 0.005g, about 0.004g, about 0.003g, about 0.002g, about 0.001g, About 0.0009g, about 0.0008g, about 0.0007g, about 0.0006g, about 0.0005g, about 0.0004g, about 0.0003g, about 0.0002g, or about 0.0001g.

In some embodiments, the amount of cells provided in the compositions of the present invention can be greater than about 0.0001-10g, such as about 0.0001g, about 0.0002g, about 0.0003g, about 0.0004g, about 0.0005g, about 0.0006g, about 0.0007 g, about 0.0008g, about 0.0009g, about 0.001g, about 0.0015g, about 0.002g, about 0.0025g, about 0.003g, about 0.0035g, about 0.004g, about 0.0045g, about 0.005g, about 0.0055g, About 0.006g, about 0.0065g, about 0.007g, about 0.0075g, about 0.008g, about 0.0085g, about 0.009g, about 0.0095g, about 0.01g, about 0.015g, about 0.02g, about 0.025g, about 0.03 g, about 0.035g, about 0.04g, about 0.045g, about 0.05g, about 0.055g, about 0.06g, about 0.065g, about 0.07g, about 0.075g, about 0.08g, about 0.085g, about 0.09g, About 0.095g, about 0.1g, about 0.15g, about 0.2g, about 0.25g, about 0.3g, about 0.35g, about 0.4g, about 0.45g, about 0.5g, about 0.55g, about 0.6g, about 0.65 g, about 0.7g, about 0.75g, about 0.8g, about 0.85g, about 0.9g, about 0.95g, about 1g, about 1.5g, about 2g, about 2.5g, about 3g, about 3.5g, about 4g, About 4.5g, about 5g, about 5.5g, about 6g, about 6.5g, about 7g, about 7.5g, about 8g, about 8.5g, about 9g, about 9.5g, or about 10g.

In some embodiments, cells can be administered in a single dose. Such administration may be by injection, for example intravenously. In some embodiments, cells can be administered in multiple doses. Dosing may be once, twice, three, four, five, six, or more than six times per year. Dosing can be once monthly, once every two weeks, once a week, or once every 2 days. In some embodiments, administration of cells can be continuous.

In one aspect, the invention provides a pharmaceutical composition. In some embodiments, it may comprise a cell of the invention, and a pharmaceutically acceptable carrier.

On the one hand, the present invention provides a kit. The kit of the present invention may include cell activators, cell growth factors and/or feeder cells for the cell culture method of the present invention and instructions describing the steps of the cell culture method of the present invention. In one aspect, the invention provides a kit, which may comprise the cells of the invention and/or the pharmaceutical composition of the invention.

In one aspect, the invention provides a method of affecting the growth of cells, such as tumor cells, which may comprise administering to a subject a cell of the invention and/or a pharmaceutical composition of the invention. In some embodiments, affecting tumor growth may comprise reducing the volume of the tumor to about 99-0.1% of that before administration, such as about 99%, about 95%, about 90%, about 80%, about 70%, about 60%, About 50%, about 40%, about 30%, about 20%, about 19%, about 18%, about 17%, about 16%, about 15%, about 14%, about 13%, about 12%, about 11 %, about 10%, about 9%, about 8%, about 7%, about 6%, about 5%, about 4%, about 3%, about 2%, about 1%, about 0.9%, about 0.8%, About 0.7%, about 0.6%, about 0.5%, about 0.4%, about 0.3%, about 0.2%, or about 0.1%.

In one aspect, the present invention provides the use of the cells of the present invention and/or the pharmaceutical composition of the present invention in the preparation of medicaments, and the medicaments of the present invention can be used to prevent and/or treat diseases and/or symptoms. For example, diseases and/or conditions of the present invention may include tumors. In some embodiments, the tumor of the invention is selected from solid tumors. In some embodiments, the tumor of the invention can be selected from one or more of the following groups: melanoma, ovarian cancer, cervical cancer, lung cancer, bladder cancer, breast cancer, head and neck cancer, pancreatic cancer, liver cancer, gastric cancer, tuberculosis Rectal and kidney cancer.

In one aspect, the invention provides a method for preventing and/or treating diseases and/or symptoms, which may comprise administering to a subject the cells of the invention and/or the pharmaceutical composition of the invention. For example, diseases and/or conditions of the present invention may include tumors. In some embodiments, the tumor of the invention is selected from solid tumors. In some embodiments, tumors of the invention may be selected from the group consisting of: melanoma, ovarian cancer, cervical cancer, lung cancer, bladder cancer, breast cancer, head and neck cancer, pancreatic cancer, liver cancer, stomach cancer, colorectal cancer, and kidney cancer.

In one aspect, the invention provides a TIL of the invention and/or a pharmaceutical composition of the invention, which can be used to prevent and/or treat diseases and/or symptoms. For example, diseases and/or conditions of the present invention may include tumors. In some embodiments, the tumor of the invention is selected from solid tumors. In some embodiments, the tumor of the invention can be selected from one or more of the following groups: melanoma, ovarian cancer, cervical cancer, lung cancer, bladder cancer, breast cancer, head and neck cancer, pancreatic cancer, liver cancer, gastric cancer, tuberculosis Rectal and kidney cancer.

### EXAMPLES

Without being limited by any theory, the following examples were provided solely to illustrate the methods and uses of the present invention and were not intended to limit the scope of the present invention.

### Example 1

### (I) TIL Cell Culture

### 1.1 Receipt and Processing of Tumor Tissue

### 1.1.1 Receipt of Samples

Tumor tissue and blood samples from a donor were received. The sample information was checked and recorded, and corresponding sample labels were printed.

### 1.1.2 Tissue Processing and Culture

Sample tubes and blood collection tubes were disinfected using 75% alcohol and transferred to a biological safety cabinet. PBMC cells were isolated from blood samples and cryopreserved according to the above-described manual PBMC isolation and cryopreservation procedures. A culture bottle or culture bag with a breathable surface, for example, a culture bag (Origen), was taken, and 300 mL of rewarmed complete culture medium was added. The complete culture medium was, for example, X-vivo 15 culture medium or other commercially available T cell culture media, such as those from Stem Cell, Lonza, Thermo, or Miltenyi brands. The medium could be supplemented with essential amino acids and antibiotics, and was supplemented with IL-2 at a concentration of 300-9000 IU/mL (e.g., 1000-9000 IU/mL, e.g., 6000 IU/mL). Several 10 cm Petri dishes were taken, and an appropriate amount of culture medium was added to each. Tumor tissue was removed from the sample tube using sterile ophthalmic tweezers and placed into a 10 cm Petri dish. The tissue was washed, and the Petri dish was replaced. A preliminary cutting was performed using ophthalmic scissors and ophthalmic tweezers to remove adipose tissue and necrotic tissue. Each tissue piece was further minced to a size of approximately 27 cubic millimeters. A non-suspended tumor tissue block was taken. The internal plunger was removed from a 20 mL syringe, and the syringe was connected to the culture bag. Approximately 1 g of the tissue block was transferred into the culture bag through the syringe using a pipette. The culture bag was placed into a carbon dioxide incubator for cultivation. The scissors and tweezers were cleaned, preliminarily disinfected with 75% alcohol, ultrasonically cleaned, and then sterilized, whereby a first TIL population was obtained.

### 1.2 Step (A) In Vitro Expansion and Harvesting

### 1.2.1 Step (A) In Vitro Expansion

Depending on the growth status of the cells, fluid was added, or half of the medium was replaced, every 3-7 days to ensure cell nutrition. A complete culture medium was used. The complete culture medium was, for example, X-vivo 15 culture medium or other commercially available T cell culture media, such as those from Stem Cell, Lonza, Thermo, or Miltenyi brands. The medium could be supplemented with essential amino acids and antibiotics, and was supplemented with IL-2 (Shuanglu and/or Sihuan) at a concentration of 300-9000 IU/mL (e.g., 1000-9000 IU/mL, e.g., 6000 IU/mL). On days 3-14 of step (A), for example, on day 13 or 14, samples were taken for cell counting. If the cell count was between 5×10⁵ and 5×10⁸ cells, the process proceeded to the harvesting step of step (A).

### 1.2.2 Harvesting from Step (A)

Cells at the end of the in vitro expansion in step (A) were collected and centrifuged, and the culture medium was discarded. The cells were washed once with PBS or physiological saline to obtain TILs expanded in vitro in step (A) (referred to as the second TIL population). A sample was taken for cell counting, and approximately 5×10⁵ to 2×10⁸ cells were reserved for subsequent in vitro expansion steps. Approximately 5×10⁵ cells were used for quality control testing. The remaining cells were added to a cryopreservation solution and cryopreserved as preREP TIL in vitro cells.

### 1.3 Step (B) TIL Activation

The TILs (second TIL population) expanded in vitro in step (A) were further cultured, or the cryopreserved preREP TIL in vitro cells were thawed (cell recovery was performed), for TIL activation in step (B). A complete culture medium was used. The complete culture medium was, for example, X-vivo 15 culture medium or other commercially available T cell culture media, such as those from Stem Cell, Lonza, Thermo, or Miltenyi brands, and could be supplemented with essential amino acids and antibiotics. The cell density was adjusted to 5×10⁵ to 2×10⁶ cells/mL in a 24-well suspension culture plate, at 1 mL/well, and IL-2 was added to a

concentration of 300-9000 IU/mL (e.g., 1000-9000 IU/mL, e.g., 6000 IU/mL). T cell activators were also added to the culture medium of each TIL cell population; for example, a CD3 agonist and/or a CD28 agonist were added, such as approximately 30 ng/mL of CD3 antibody (Miltenyi Biotech, OKT3), and approximately 30 ng/mL of CD28 antibody (Merck, 15E8). Magnetic beads (Dynabeads, diameter approximately 1 to 10 µm, Thermo Fisher) were added at a ratio of magnetic beads to TILs of approximately 1:2 to 2:1, and/or TransAct (Miltenyi, diameter approximately 100 to 500 nm) was added at a ratio of TransAct to TILs of approximately 1:100 to 1:2000. After culturing for approximately 0-4 days, a third TIL population was obtained.

### 1.4 Step (C) TIL Cell Gene Editing

sgRNAs targeting various target sites, selected according to the present invention, were synthesized, thawed, and nuclease-free water was added to prepare a concentration of approximately 100 µM. Approximately 2 µL of gRNA (50 µM) was incubated at 95°C for 2 minutes for annealing and then added to P3 buffer. Subsequently, 0.3-1 µL of Cas9 (e.g., KAIKA, CUREBIO, or Acro, 10 mg/mL) was added, and the mixture was incubated at 25°C for 10 minutes to form a ribonucleoprotein (RNP) complex. In P3 buffer (Lonza), approximately 1×106 cells from the third TIL population were electroporated with the aforementioned RNP complex using a Lonza electroporator. For example, the electroporation program used was human T cell stim (EO115). The cells were cultured for approximately 0-4 days after gene editing by electroporation, yielding a fourth TIL population.

### 1.5 Step (D) Culture of TIL Cells After Gene Editing

Feeder cells (irradiated healthy donor PBMC T cells) were added to the fourth TIL cell population for culture. The contact between TILs and feeder cells was initiated at a time Tn after the TILs from step (B) were exposed to IL-2 and T cell activators (wherein Tn could range from 0 hours to 12 days, e.g., 24 hours or 48 hours). First, feeder cells, which were a mixture from 1-5 donors, were thawed. The activated TIL cells and feeder cells were mixed at a TIL cell to feeder cell ratio of approximately 1:200 and transferred into G-Rex100 culture flasks or breathable bags. Complete culture medium was added. Samples were taken for cell counting every 1-3 days, and medium was replenished or half of the medium was replaced according to the cell status, until the total cell number exceeded 1×10⁹ or the in vitro expansion culture of step (D) had been performed for approximately 5 to 14 days. The in vitro expansion culture of step (D) was then terminated.

### 1.6 Harvest of Tumor-Infiltrating Lymphocytes

Cells expanded in step (D) were taken and centrifuged, and the culture supernatant was discarded. The cells were washed three times with PBS, physiological saline, or a compound electrolyte solution to obtain TILs expanded in step (D) (fifth TIL population). During the third wash, a sample was taken for cell counting. Based on the counting results, the supernatant was discarded after a final centrifugation, and 3×106 cells were sent for quality control testing. All remaining cells were added to a cryopreservation solution, and the cell density was adjusted to 1-3×10⁸ cells/mL for cryopreservation.

### (II) TCR-T Cell Culture

### T Cell Activation:

T cells cryopreserved in liquid nitrogen were thawed and cultured. The cells were centrifuged and resuspended in T cell culture medium RPMI 1640 (Gibco) supplemented with 10% FBS (Bovogen) to a density of 5×10⁵ cells/mL. T cell TransAct (Miltenyi) was added at a 1: 100 dilution, and recombinant human IL-2 was added to a concentration of 30 IU/mL. The cells were then cultured for approximately 72 hours.

### TCR Transduction:

One day prior to transduction, 24-well suspension culture plates were coated with recombinant human fibronectin fragment (Retronectin, Takara) at a final concentration of 15 µg/mL, using 250 µL per well. The plates were protected from light and stored overnight at 4°C. The coated 24-well plates were taken out, the coating solution was aspirated and discarded, and 500 µL of blocking solution containing 2% BSA was added to each well for blocking at room temperature for 30 minutes. The blocking solution was aspirated and discarded. The plate was washed twice with 500 µL/well of washing solution containing 2.5% HEPES, and the washing solution was aspirated and discarded after each wash. The experimental group was transduced with a retrovirus carrying specific TCR nucleic acid fragments of an NY-ESO-1 antigen peptide. 0.1 to 1 mL of retrovirus solution was added to each well, and the plate was centrifuged at 32°C and 2000g for 2 hours. The supernatant was discarded from the 24-well plate, and thawed and activated T cells were added to each well at a volume of 500-1000 µL and a cell concentration of approximately 5×10⁵ cells/mL. The plate was centrifuged at 30-32°C and 1000g for 10 minutes. After centrifugation, the culture plate was placed in a 37°C, 5% CO₂ incubator for cultivation, yielding transduced cells. The cells were cultured for approximately 0-4 days after transduction to obtain a TCR-T cell population. After transduction, cell count, density, and viability were monitored every 1 to 3 days. Based on the count results, T cell culture medium was added, recombinant human IL-2 was added to a concentration of 30 to 100 IU/mL, and the initial culture cell density was adjusted to 0.5-2×10⁶ cells/mL. Cultivation was continued.

### TCR-T Gene Editing:

sgRNAs targeting various target sites, selected according to the present invention, were synthesized, thawed, and nuclease-free water was added to prepare a concentration of approximately 100 µM. Approximately 2 µL of gRNA (50 µM) was incubated at 95°C for 2 minutes for annealing and then added to P3 buffer. Subsequently, 0.3-1 µL of Cas9 (e.g., KAIKA, CUREBIO, Acro, 10 mg/mL) was added, and the mixture was incubated at 25°C for 10 minutes to form a ribonucleoprotein (RNP) complex. Approximately 1×10⁶ TCR-T cells were electroporated with the aforementioned RNP complex in P3 buffer (Lonza) using a Lonza electroporator. For example, the electroporation program used was human T cell stim (EO115). Recombinant human IL-2 was added to the electroporated T cells to a concentration of 100-300 IU/mL, and cultivation was continued to obtain the target gene-edited TCR-T cells of the present invention.

### (III) Knockout Efficiency Detection

The following reagents and materials were used for detecting the knockout efficiency of cells: DNA extraction solution (QuickExtract DNA extraction solution, Lucigen, QE09050), nuclease-free water (RNase/DNase free water, Tiangen), EDTA (Sangon, 0.5M), and Recombinant DNase I (RNase-free, TAKARA).

### Genomic DNA Extraction:

Approximately 2-7 days after T cell knockout (performed according to the gene editing method in step 1.4 of Example 1(I) or the TCR-T gene editing method of Example 1(II)), approximately 1×10⁵ to 2×10⁵ cells were taken. The cells were washed once with PBS, then resuspended in 44 µL of PBS. A 6 µL nuclease mixture (containing 1 µL DNase I and 5 µL 10× DNase I Buffer) was added, and the cells were incubated at 37°C for 5 minutes. 2.5 µL of 0.5 M EDTA was added to the sample, which was then incubated at 80°C for 10 minutes. After centrifugation and discarding the supernatant, 50 µL of DNA extraction solution was added to the cell pellet. After a brief centrifugation, the following program was run: 75°C for 10 minutes; 95°C for 5 minutes; maintained at 4°C. The DNA sample concentration was determined using a spectrophotometer (NanoDropTM).

### Sequencing:

PCR primers were designed for a region approximately 100 to 200 nucleotides upstream and downstream of the PAM site. The PCR reaction system was designed as follows:

| Reagent | Volume |
|---|---|
| 2×PCR Premix Buffer | 25 µL |
| DNA template | about 100-500 ng |
| forward primer (10 µM) | 1 µL |
| reverse primer (10 µM) | 1 µL |
| ddH₂O | Add to final volume of 50 µL |
| DMSO | 1.5 µL |

Amplification was performed using the following PCR program:

| Humidity | time | |
|---|---|---|
| 95°C | 4 min | |
| 95°C | 15 sec | repeat for 35 cycles |
| 58°C | 30 sec | |

| | | |
|---|---|---|
| 72°C | 30 sec | |
| 72°C | 7min | |
| 4°C | hold | |

The PCR products were subjected to Sanger sequencing analysis.

### Analysis of CRISPR-Cas9 Knockout Efficiency

The CRISPR-Cas9 knockout efficiency was analyzed based on Sanger sequencing data using the Tracking of Indels by DEcomposition (TIDE) method (see, e.g., Brinkman et al., Nucl. Acids Res. (2014) or shinyapps.datacurators.nl/tide/ for specific methods). The knockout efficiency analysis was performed by inputting the corresponding sgRNA sequence of the present invention, the control sequence before knockout, and the test sequence after CRISPR-Cas9 knockout, with the p-value threshold set to 0.001.

### (IV) Expansion Detection

### Experimental Preparation

On days 7-10 after gene editing as described in Example 1(I), the proliferation of TIL cells was assessed (IL-2 was withdrawn), and TIL cells from each group were harvested. Alternatively, TCR-transduced T cells obtained as described in Example 1(II) were used. The cells were washed once with PBS and resuspended in T cell culture medium (without IL-2). After counting, the cell density was adjusted to 1×10⁵ to 2×10⁶ cells/mL, and 100 µL/well was added to a flat-bottomed 96-well plate. For the no-stimulation medium group, no cell activating substances were added to the medium. For the CD3 antibody stimulation group, CD3 antibody (OKT3) was added at 30 ng/mL for stimulation. For the TransAct stimulation group, TransAct (diameter approximately 100 to 500 nm, Miltenyi) was added to achieve a working solution concentration of 1:1000 (v/v). For the Medium group, only an equivalent volume of cell culture medium was added. The fluorescence amount of T cells at the time of plating was analyzed using a CTG kit (CellTiter-Glo Luminescent Cell Viability Assay, Promega). After 3 days, the fluorescence amount of T cells was again analyzed using the CTG kit. The T cell expansion efficiency was characterized by the ratio of fluorescence amount on day 3 to the fluorescence amount at plating.

### (V) Cell Killing Ability Test

Beginning on the 6th day after gene editing, tumor target cells were plated in a 96-well flat-bottomed plate. On the next day, TIL cells from each group were co-cultured with target cells at different effector-to-target ratios (T cells: target cells, E:T). Alternatively, TCR-transduced T cells obtained as described in Example 1(II) were used. 100 µL each of target cells and T cells were used, with three replicate wells set for each group. A control group containing only target cells was also set. The target cells could be selected from Hey-T30 ovarian cancer cells and A375 melanoma cells. According to the instructions of the apoptosis detection reagent (Incucyte Caspase-3/7 Green Dye for Apoptosis, Sartorius), the apoptosis detection reagent was added at 0.2 µL/well, and Caspase 3/7 Green Dye was diluted with culture medium and added at 25 µL/well. The activity of Caspase 3/7 was recorded using an Incucyte live-cell analysis system (Sartorius) to analyze the killing ability of TIL cells against target cells. Recordings were performed every 3 hours for a total recording time of approximately 5 days.

### (VI) Cell Killing Ability Test (Flow Cytometry)

Flow cytometry was used to detect the expression of T cell exhaustion, stemness, and other related molecules in the TIL cells obtained on the 8th day after gene editing. Alternatively, TCR-transduced T cells obtained in Example 1(II) were detected. V-bottom 96-well plates (Corning, Cat. No. 3894) and flow cytometry tubes (Corning, Cat. No. 352052) were used. Flow cytometry antibodies were purchased from BD Biosciences or BioLegend.

### Cell Surface Molecule Detection:

For each group, 1×10⁵ to 5×10⁵ cell samples were added to flow cytometry tubes or V-bottom 96-well plates. The samples were centrifuged at 600g for 3 minutes, and the supernatant was discarded. Cells were washed once with PBS (1 mL/tube for flow cytometry tubes, 200 µL/well for 96-well plates), and the supernatant was discarded. Prepared antibody working solution was added for cell surface staining. The antibody (BD Biosciences or BioLegend) concentration was 1: 100 to 1:200, containing viability dye at 1:10000. Staining was performed with 100 µL/tube for flow cytometry tubes or 50 µL/well for 96-well plates, and incubated at 2-8°C in the dark for 30 minutes. After surface staining, cells were washed once with PBS (200 µL/wash for 96-well plates, 1 mL/wash for flow cytometry tubes), centrifuged at 600g for 3 minutes at room temperature, and the supernatant was discarded. Cells were resuspended in 100-500 µL PBS for flow cytometry analysis.

### Intracellular Molecule Detection:

An antibody mixture working solution was prepared for cell surface staining of CD3/CD4/CD8 (antibody concentration 1:100), with cell viability dye at a concentration of 1:10000. Staining was performed with 50 µL/well for 96-well plates or 100 µL/tube for flow cytometry tubes, and incubated at 2-8°C in the dark for 30 minutes. Cells were washed once with PBS (200 µL/wash for 96-well plates, 1 mL/wash for flow cytometry tubes), centrifuged at 600g for 3 minutes at room temperature, and the supernatant was discarded. 100 µL of Fixation/Permeabilization solution (BD Biosciences) was added to each well, and incubated at 2-8°C in the dark for 20-40 minutes. After fixation and permeabilization, cells were washed twice with 1× Perm/Wash Buffer (200 µL/wash for 96-well plates, 1 mL/wash for flow cytometry tubes), centrifuged at 600g for 3 minutes, and the supernatant was discarded. Intracellular molecular antibodies (e.g., TCF1) were prepared using 1× Perm/Wash Buffer, and TIL cells were resuspended (50 µL/well for 96-well plates, 100 µL/tube for flow cytometry tubes for staining), and incubated at 2-8°C in the dark for 30 minutes. After intracellular molecular staining, cells were washed 1-2 times with 1× Perm/Wash Buffer (200 µL/wash for 96-well plates, 1 mL/wash for flow cytometry tubes), centrifuged at 600g for 3 minutes, and the supernatant was discarded. Cells were resuspended in 100-500 µL PBS for flow cytometry analysis.

### (VII) Cytokine Expression Flow Cytometry Detection

The TIL cell populations obtained on the 7th or 8th day after gene editing in each experimental group were subjected to flow cytometry to detect cytokine expression. Alternatively, TCR-transduced T cells obtained in Example 1(II) were detected. Culture medium required for intracellular cytokine expression detection was prepared: TIL cell culture medium was taken, and Golgistop (0.7:1000 v/v), Golgiplug (1:1000 v/v), and CD107a antibody (1:500 v/v, i.e., 2 µL/mL) were added according to volume ratio. No interleukins were added.

### Detection Steps:

T cells from each experimental group were centrifuged, and then resuspended using the aforementioned culture medium required for intracellular cytokine expression detection. After counting, the cell density was adjusted to 1×106 cells/mL, and 200 µL/well was added to a 96-well plate. For the CD3 antibody stimulation group, CD3 antibody (OKT3) was added at 30 ng/mL for stimulation. For the TransAct stimulation group, TransAct (diameter approximately 100 to 500 nm, Miltenyi) was added to make the TransAct working solution concentration 1: 1000 (v/v). For the Medium group, only an equivalent volume of cell culture medium was added. The plate was placed in a 37°C incubator and incubated overnight. Sources of main reagents and materials for cytokine flow detection tests: V-bottom 96-well plates (Corning, Cat. No. 3894); flow cytometry tubes (Corning, Cat. No. 352052); flow cytometry antibodies were purchased from BD Biosciences or BioLegend. After incubation, cells were washed once with 200 µL/well PBS, centrifuged at 600g for 3 minutes, and the supernatant was discarded. An antibody mixture working solution was prepared for cell surface staining of CD3/CD4/CD8 (antibody concentration 1:100), with cell viability dye at a concentration of 1:10000. Staining was performed with 50 µL/well for 96-well plates or 100 µL/tube for flow cytometry tubes, and incubated at 2-8°C in the dark for 30 minutes. Cells were washed once with PBS (200 µL/wash for 96-well plates, 1 mL/wash for flow cytometry tubes), centrifuged at 600g for 3 minutes at room temperature, and the supernatant was discarded. 100 µL of Fixation/Permeabilization solution (BD Biosciences) was added to each well, and incubated at 2-8°C in the dark for 20-40 minutes. After fixation and permeabilization, cells were washed twice with 1× Perm/Wash Buffer (200 µL/wash for 96-well plates, 1 mL/wash for flow cytometry tubes), centrifuged at 600g for 3 minutes, and the supernatant was discarded. Cytokine detection antibodies (e.g., GZMB, TNF-α, IFN-γ) were prepared using 1× Perm/Wash Buffer, and TIL cells were resuspended (50 µL/well for 96-well plates, 100 µL/tube for flow cytometry tubes for staining), and incubated at 2-8°C in the dark for 30 minutes. After cytokine staining, cells were washed 1-2 times with 1× Perm/Wash Buffer (200 µL/wash for 96-well plates, 1 mL/wash for flow cytometry tubes), centrifuged at 600g for 3 minutes, and the supernatant was discarded. Cells were resuspended in 100-500 µL PBS for flow cytometry analysis.

### (VIII) Cell Apoptosis Detection

The TIL populations obtained on the 7th or 8th day after gene editing in each experimental group in Example 1 were subjected to apoptosis detection. Alternatively, TCR-transduced T cells obtained in Example 1(II) were detected. T cell apoptosis levels in TILs from the gene knockout group or the control group (NT, no treatment) were detected using an apoptosis detection kit (BD 559763 Annexin V PE Apoptosis kit).

### (IX) PDO Model Culture

Tumor tissue was obtained from surgery, transported and stored at 2 to 8°C after removal, and mechanically dissociated within 24 hours. The tissue was minced to approximately 0.5 mm³ in size, then digested with a special tissue digestion solution (bioGenous) at 37°C, after which the reaction was terminated using 10% FBS. The tissue suspension was filtered through a 100 µm filter and washed, then placed on ice and thoroughly mixed with Matrigel. The mixture was then spotted onto the bottom of a cell culture plate. The culture plate was placed in a 37°C incubator. After the Matrigel had fully solidified, complete culture medium was carefully added for cultivation. When the organoids grew to sufficient size and density, they were passaged or cryopreserved to obtain a PDO (Patient-Derived Organoid) model. The Matrigel and organoid mixture was scraped off using a pipette tip and transferred to a centrifuge tube containing basal culture medium. The mixture was pipetted to separate the organoids and Matrigel. After centrifugation, passaging tissue digestion solution was added, mixed well, and placed in a 37°C incubator for digestion. Basal culture medium was added to dilute the digestion solution, followed by centrifugation and washing. A small amount of cell suspension was taken, and 8 times its volume of digestion solution was added to digest it into single cells. FBS was used to terminate the reaction, followed by counting. Based on the counting result, the required volume of organoids was taken, centrifuged, and resuspended in T cell culture medium. The cells to be tested (e.g., TIL cells or TCR-T cells) were counted, and resuspended to the required density according to different effector-to-target ratios. In a flat-bottomed 96-well plate, 100 µL each of PDO target cells and cells to be tested were added, with three replicate wells set for each group, for PDO co-culture detection. Several different effector-to-target ratios were set. Simultaneously, a group containing only PDO target cells was set, and a control group (NT) was set for co-culturing unedited cells to be tested only with PDO target cells.

### Detection of Killing Ability in PDO Model

A PDO model was prepared according to the embodiments of the present invention and co-cultured with TIL cells or TCR-T cells. Caspase-3/7 substrate was added for apoptosis signal labeling, and then the experimental plate was placed in an Incucyte system for observation and recording.

### (X) Multi-Round Cell Killing Ability Detection

Changes in the fluorescence intensity of target cells A375-GFP during multiple rounds of killing were monitored using an IncuCyte system. A375-GFP cells were evenly plated in a multi-well plate. After culturing at 37°C for 4 hours, unedited cells or cells with the target of the present invention knocked out were taken. After adding to the corresponding wells, GFP fluorescence signal recording was started with the IncuCyte system, with 3 replicate wells in each group. After 24 hours of killing, the co-culture supernatant (20 µL/well) was taken for CBA detection. After 3 days of killing, a new multi-well plate evenly plated with fresh A375-GFP cells was used. After culturing at 37°C for 4 hours, the mixture of unedited cells or cells with the target of the present invention knocked out from the previous round of killing and A375-GFP cells was transferred to the new 96-well plate. The IncuCyte system was used to record the change curve of GFP fluorescence signal in the new round of killing.

### (XI) CBA Method for Detecting Cytokine Release

Gene-edited cells were co-cultured with autologous tumor cells. The co-culture supernatant was collected, and a CBA kit (BD Biosciences) was used to detect the cytokine release from unedited cells or cells with the target of the present invention knocked out.

### (XII) In Vivo Efficacy Testing of Gene-Edited Cells

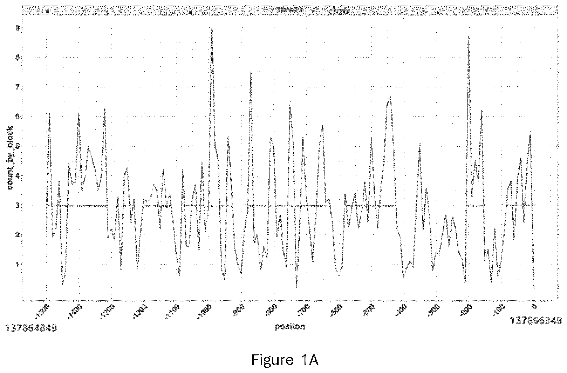

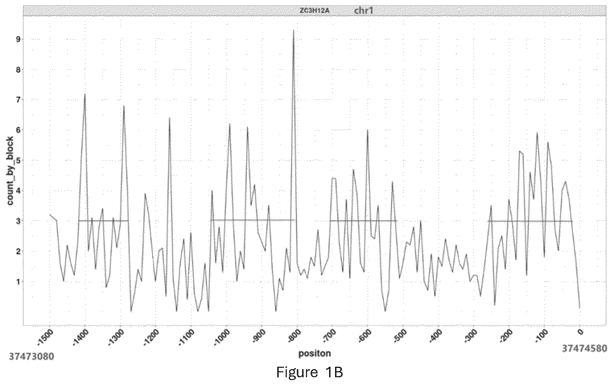

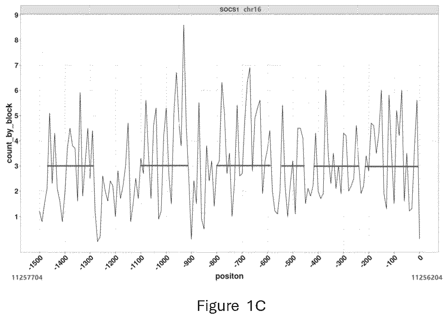

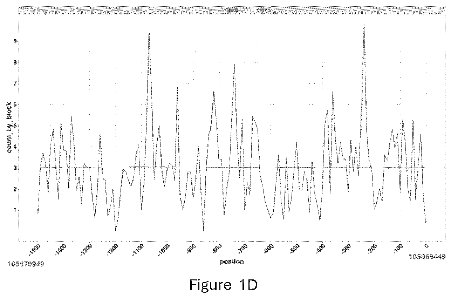

Tumor tissue samples were obtained from surgery and transferred to the laboratory under sterile conditions. Tumor necrotic tissue was removed on a sterile clean bench, and the tumor tissue was cut into 1-2 mm³ pieces. A small amount of Matrigel was added to the cut tumor tissue, which was then subcutaneously inoculated into NOG mice (Vital River, strain code 408) in an SPF animal facility. Each mouse was inoculated with 4-5 pieces of tissue. This batch of tumor tissue was designated as F0 generation. When the F0 generation tumor tissue grew to 800 mm³ under the skin of the mice, the F0 tumor tissue was surgically removed. The tumor tissue was again divided into 1-2 mm³ pieces; some were cryopreserved for later use, and some were subcutaneously inoculated into a second batch of NOG mice and continued to be passaged and expanded in the mice. This batch of tumor tissue was designated as F1 generation. This process was repeated until the tumor tissue was passaged to the F3 generation in mice. The tumor tissue size was measured regularly until the tumor volume grew to approximately 100 mm³, after which the mice were randomly grouped. Mice in the NT group were intravenously injected with unedited cells, and mice in the experimental group were intravenously injected with gene-edited cells of the present invention. The sequences of sgRNAs (single guide RNAs, or guides) targeting each target site were synthesized according to the sequences provided by the present invention. The region targeting each target could be selected from: the exon region of the target gene, an intron region approximately 100 bp or approximately 20 bp away from the exon of the target gene, and a region approximately 1500 bp upstream of the start codon of the target gene. When the region targeting each target was selected from the region approximately 1500 bp upstream of the start codon of the target gene, Figures 1A-1D show a continuous region provided by the present invention upstream of the start codon that has approximately 3 or more transcription factor binding sites (as indicated by the black line segment where the ordinate is 3 or above). The present invention also provided sgRNAs targeting the exon region of each target, or an intron region approximately 100 bp or approximately 20 bp away from the exon of the target gene, which, after knockout, enhanced cell function. Preferably, the sgRNA numbering information in the examples was as follows:

| sgRNA No. | sgRNA sequence | Target genomic region | SEQ ID NO: |
|---|---|---|---|
| TP-P1 | ACAAAGACAGTCTAACCGTG | chr6:137868162-137868189 | **7267** |
| TP-P2 | TAGACCTTGCAGAAGCGCTG | chr6:137867909-137867936 | **7268** |
| TP-P3 | ACCCGCATACAACTGAAACG | chr6:137867218-137867245 | **7269** |
| TP-P4 | AAATGCCCAGGTGACTCACG | chr6:137866958-137866985 | **7270** |
| TP-P5 | GAGGAGAACCGAAAACAAAG | chr6:137868271-137868298 | **7271** |
| TP-P6 | GTACCTGTTGCTGACAGACA | chr6:137870886-137870913 | **7272** |
| TP-P7 | AAATCCAGTGCTGTTCAGGG | chr6:137870822-137870849 | **7273** |
| TP-P8 | CTGTTGCTGACAGACAGGGT | chr6:137870890-137870917 | **7274** |
| TP-P9 | AGAGTGGGTACTGAGGAGGA | chr6:137870954-137870981 | **7275** |
| TP-P10 | CCAGTGTGAGTTAATCTCTG | chr6:137871028-137871055 | **7276** |
| TP-P11 | GTAATGACAAGATCAAACAC | chr6:137871104-137871131 | **7277** |
| TP-P12 | TTGTCATTACCCTGATCTGT | chr6:137871096-137871123 | **7278** |
| TP-P13 | GCAGGCGGGAGCTATCACCC | chr6:137871552-137871579 | **7279** |
| TP-P14 | CTGCATGTAAGACCCAGCAA | chr6:137874780-137874807 | **7280** |
| TP-P16 | TCTGTTAGTAGATAATTAGG | chr6:137875046-137875073 | **7281** |
| TP-P17 | CAGAATGACTTTTTAGTACA | chr6:137875615-137875642 | **7282** |
| TP-P18 | TTCATCATTCCAGTTCTAAG | chr6:137875680-137875707 | **7283** |
| TP-P19 | AATTCCACCCACTTTCAAAG | chr6:137876035-137876062 | **7284** |
| TP-P20 | TTCGCCCCTTTGAAAGTGGG | chr6:137876026-137876053 | **7285** |
| TP-P21 | CCACTTGTTAACAGAGACCG | chr6:137877085-137877112 | **7286** |
| TP-P22 | TGATGAGATGAGTTGTGCCA | chr6:137877224-137877251 | **7287** |
| TP-P23 | AAGACAGTTACAGATGACAC | chr6:137877282-137877309 | **7288** |
| TP-P24 | GGGGCACGCCCAGAATCCCA | chr6:137878538-137878565 | **7289** |
| TP-P25 | GGTGTTCACAGACATGAAGA | chr6:137878623-137878650 | **7290** |
| TP-P26 | CGTGAAGTTGCCGGGCGTTG | chr6:137878939-137878966 | **7291** |
| TP-P27 | GTTCCAAGTCAGATCCCTCG | chr6:137879125-137879152 | **7292** |
| TP-P28 | CTTCAAAAGGACTACAGCAG | chr6:137879057-137879084 | **7293** |
| TP-P29 | GGGCGTCGTTTCCAGCTCTG | chr6:137879182-137879209 | **7294** |
| TP-P30 | CCTCCCTGTGCAACGCATGG | chr6:137866305-137866332 | **7295** |
| TP-P31 | GAAAACAAACGTGAGTGAAG | chr6:137879337-137879364 | **7296** |
| TP-P32 | TAGATTTTGCTGCTGCCTCA | chr6:137880063-137880090 | **7297** |
| TP-P33 | TCAGAATCAGAGATTTCATG | chr6:137880202-137880229 | **7298** |
| TP-P34 | TTCAAACATGGTGCTTCCAA | chr6:137880155-137880182 | **7299** |
| TP-P35 | AAAAGCATCGAACACACGGG | chr6:137880280-137880307 | **7300** |
| TP-P36 | GAACCACACAAAGCACCTCA | chr6:137881058-137881085 | **7301** |
| TP-P37 | ATGGCTAACCGGAAACAGGT | chr6:137881307-137881334 | **7302** |
| TP-P38 | GCGCTGGCTCGATCTCTGAG | chr6:137881027-137881054 | **7303** |
| TP-P39 | AACCCGGGAGTGTTCCCAGG | chr6:137881483-137881510 | **7304** |
| TP-P40 | TGAAGCAGTCCTGATGCGCA | chr6:137881646-137881673 | **7305** |
| TP-P41 | GAAAAGACACACAAGTCGCG | chr6:137881683-137881710 | **7306** |
| TP-P42 | GCCAGATCCTCATGGCAGCG | chr6:137881764-137881791 | **7307** |
| TP-P43 | CCTGCCCGGTTCCTTTCCTG | chr6:137881865-137881892 | **7308** |
| TP-P44 | CTGATAATCAGTTCACCCCA | chr6:137866369-137866396 | **7309** |
| TP-P45 | AAATCGCTTCTAGTCCATCA | chr6:137866440-137866467 | **7310** |
| TP-P46 | AAAGAAGTTAGCTTGAACTG | chr6:137882061-137882088 | **7311** |
| TP-P47 | ATACCCTTACATTATGTATG | chr6:137882108-137882135 | **7312** |
| TP-P48 | GCCCTAGAAGTACAATAGGA | chr6:137882158-137882185 | **7313** |
| TP-P49 | AAGATATTTCTTCAGCTCTG | chr6:137882341-137882368 | **7314** |
| TP-P50 | AGAGGCCAATTTACTCCCTG | chr6:137882435-137882462 | **7315** |
| TP-P51 | CAAGGAGGTAGGACACTCAG | chr6:137882596-137882623 | **7316** |
| TP-P52 | AATGGTCACAGGGAAAGATG | chr6:137882650-137882677 | **7317** |
| TP-P53 | TCGTCTGGGAAAAACTTAGG | chr6:137883012-137883039 | **7318** |
| TP-P54 | GATAAAGGCTG CCATTTTGG | chr6:137882899-137882926 | **7319** |
| TP-P55 | TGGTGAGCAGAAATAGACAA | chr6:137866501-137866528 | **7320** |
| TP-P56 | GCATGAGACGTAGGTCCCAG | chr6:137866611-137866638 | **7321** |
| TP-P57 | TACCTAAGACTGGGGCCACG | chr6:137866575-137866602 | **7322** |
| TP-P58 | GAAAACCCTAAAAACCACCC | chr6:137866804-137866831 | **7323** |
| TP-P59 | CTGCAGAAAATCATCAACTG | chr6:137866854-137866881 | **7324** |
| ZC-P1 | ACGCAAAAGAGGCAAGTCCA | chr1:37479085-37479112 | **7325** |
| ZC-P2 | TGGGGTGAAGAGGCATTTGG | chr1:37479536-37479563 | **7326** |
| ZC-P3 | CATGTGTACATCTGTCCCTG | chr1:37479834-37479861 | **7327** |
| ZC-P4 | CACTACCACCACCAAAGCCA | chr1:37480166-37480193 | **7328** |
| ZC-P5 | AAGTTGTAGGTCACTCCCAG | chr1:37480498-37480525 | **7329** |
| ZC-P6 | AGCAGGTTAGGGAGCCCTGT | chr1:37481520-37481547 | **7330** |
| ZC-P7 | CGCAACCTCCCATGGCCACA | chr1:37481934-37481961 | **7331** |
| ZC-P8 | ACCCATAGAGCAGTTTTGCG | chr1:37482275-37482302 | **7332** |
| ZC-P9 | GCAGGGGCACTTCAGAACCA | chr1:37482686-37482713 | **7333** |
| ZC-P10 | CCACAAACCAAAGATACTGT | chr1:37483748-37483775 | **7334** |
| ZC-P11 | CAAACCGTCTTTTCTCTCAG | chr1:37483905-37483932 | **7335** |
| ZC-P12 | CACAGGTTCCCCCACCCATG | chr1:37484257-37484284 | **7336** |
| ZC-P13 | AAGAAGAGGGTGTAGACGGA | chr1:37484199-37484226 | **7337** |
| ZC-P14 | CTTTATTGATGTGTTACAGG | chr1:37484331-37484358 | **7338** |
| ZC-P15 | AAGACAATCACCTCTTGAAA | chr1:37484369-37484396 | **7339** |
| ZC-P16 | GTGGTGGTGAGGCACTGACG | chr1:37474511-37474538 | **7340** |
| ZC-P17 | GACGGCGGCGCCTTTATATG | chr1:37474548-37474575 | **7341** |
| ZC-P18 | CTCCCAAAACCACATCCAGC | chr1:37475384-37475411 | **7342** |
| ZC-P19 | CTCCTACTGAACAACCAACA | chr1:37475442-37475469 | **7343** |
| ZC-P20 | TCAGTAGGAGCTGTGGCGCG | chr1:37475450-37475477 | **7344** |
| ZC-P21 | GATGCGAACCTCATGTGTCC | chr1:37475959-37475986 | **7345** |
| SC-N2 | GTGATTCCGATTGTCGTGCG | chr16:11257289-11257316 | **7346** |
| SC-N3 | TGTCAGGGCGCGTCCCAAGA | chr16:11256538-11256565 | **7347** |
| SC-N4 | ATCGGGGTCGCCAAGTCCGA | chr16:11256476-11256503 | **7348** |
| SC-N6 | GCGACTGAGGGCGTCGACGG | chr16:11256714-11256741 | **7349** |
| SC-N7 | CGTCGACGGCGGGTGGAGCA | chr16:11256703-11256730 | **7350** |
| SC-N8 | CGGCTCTCGCGCATGCTCCG | chr16:11256135-11256162 | **7351** |
| SC-N9 | GCGTCCGGCGAGGACCGCTT | chr16:11256320-11256347 | **7352** |
| SC-N10 | GGCCACATAAAAACCTGGCT | chr16:11257384-11257411 | **7353** |
| SC-N11 | GCTTAACTGTATCTGGAGCC | chr16:11254587-11254614 | **7354** |
| SC-N12 | CTTGGTTACTGGCCTCACAC | chr16:11257414-11257441 | **7355** |
| SC-N14 | ATTTGCAATGGCTTGCAGAT | chr16:11256971-11256998 | **7356** |
| SC-N15 | AGAGCCGCCCCGGAGCGCCC | chr16:11256124-11256151 | **7357** |
| SC-N16 | CAGTATCTTTGCACAAACCA | chr16:11254522-11254549 | **7358** |
| SC-N17 | CCTCGTCTCCAGCCGAGGGC | chr16:11254683-11254710 | **7359** |
| SC-N18 | GAGGGTACCCACATGGTTCC | chr16:11254753-11254780 | **7360** |
| SC-N20 | CTCTCCCCAAAATGAAGACG | chr16:11257024-11257051 | **7361** |
| SC-N21 | ACCCCCTCAAGAGGTGAGAA | chr16:11254652-11254679 | **7362** |
| SC-N22 | GGTCATTGGTGTAGCCTCCT | chr16:11257651-11257677 | **7363** |
| SC-N24 | CTGGTTTGTGCAAAGATACT | chr16:11254519-11254546 | **7364** |
| SC-N25 | GGCGAGGCGCCTCCCGCCCT | chr16:11254700-11254727 | **7365** |
| SC-N26 | GACCCCTTCTCACCTCTTGA | chr16:11254660-11254687 | **7366** |
| SC-N27 | AGGCGCCTCCCGCCCTCGGC | chr16:11254696-11254723 | **7367** |
| SC-N28 | CGCCCAGGGCCCGGCTCACC | chr16:11256055-11256082 | **7368** |
| SC-N29 | GCGTCCCGCGCCCCGCCGCC | chr16:11256078-11256105 | **7369** |
| SC-N30 | CCGCCTCCTCGCCCAGGGCC | chr16:11256046-11256073 | **7370** |
| SC-N31 | CGCCCCGCCGCCAGGTGAGC | chr16:11256070-11256097 | **7371** |
| SC-N32 | GCCCCGCCGCCAGGTGAGCC | chr16:11256069-11256096 | **7372** |
| SC-N33 | AGCGTCCGGGGGTGCGCTGC | chr16:11255508-11255535 | **7373** |
| SC-N34 | GCGCTGCGGGAGAGACAAAG | chr16:11255521-11255548 | **7374** |
| SC-N35 | TAGCGTCCGGGGGTGCGCTG | chr16:11255507-11255534 | **7375** |
| CB-N1 | ACCAAATCTTGAGAAATGGA | chr3:105656295-105656322 | **7376** |
| CB-N2 | GGCATAGTTAGATCTCCCTG | chr3:105704010-105704037 | **7377** |
| CB-N3 | TGCCAACCGATTCATCATCA | chr3:105720054-105720081 | **7378** |
| CB-N4 | AGGAACTGAGCCCATAATCG | chr3:105720184-105720211 | **7379** |
| CB-N5 | GTTTAGGAGTCGGATGGTCA | chr3:105720234-105720261 | **7380** |
| CB-N6 | ACCCCAAAAAGTCTTTTTGG | chr3:105720341-105720368 | **7381** |
| CB-N7 | TAAATGCACTGAGAATAGGT | chr3:105724104-105724131 | **7382** |
| CB-N8 | AGAAGTTATTGAGTTTCTGT | chr3:105724165-105724192 | **7383** |
| CB-N9 | CAAAGATCTCTCCTGCTCTG | chr3:105724248-105724275 | **7384** |
| CB-N10 | ATACGTAATGGAATGAAAAG | chr3:105656481-105656508 | **7385** |
| CB-N11 | GTGCACATCAAATGCCCACA | chr3:105734026-105734053 | **7386** |
| CB-N12 | AATCTTACAGAGCTGAAAAG | chr3:105734082-105734109 | **7387** |
| CB-N13 | GTTATCTTTATCCTGATGGG | chr3:105737237-105737264 | **7388** |
| CB-N14 | AGATGCAGAGATTTTGCTGG | chr3:105656554-105656581 | **7389** |
| CB-N15 | AGCCCTGATTGATGGCAGCA | chr3:105740501-105740528 | **7390** |
| CB-N16 | GTTGCACTCGATTGGGACAG | chr3:105740592-105740619 | **7391** |
| CB-N17 | ACTTACTTAGGAACAAACCA | chr3:105745836-105745863 | **7392** |
| CB-N18 | AATATAGCACCAAACCCGGA | chr3:105745916-105745943 | **7393** |
| CB-N19 | AACCTGGACCTGAAATTTGG | chr3:105656625-105656652 | **7394** |
| CB-N20 | TGAGCAGTTAGGTTTAATGT | chr3:105749626-105749653 | **7395** |
| CB-N21 | AAATAAACAGTGCTATTCCA | chr3:105656851-105656878 | **7396** |
| CB-N22 | ACGTAGACTAAGTTGACCCT | chr3:105657189-105657216 | **7397** |
| CB-N23 | TACCATATCCAACTGGAGGG | chr3:105776314-105776341 | **7398** |
| CB-N24 | GATTTCGTCTGTAGGCACAA | chr3:105776530-105776557 | **7399** |
| CB-N25 | ATCAAAGCAATCTTTCCCAA | chr3:105776471-105776498 | **7400** |
| CB-N26 | TGAACATCTAGTAGATCTGG | chr3:105657234-105657261 | **7401** |
| CB-N27 | TTTACCCCAAAACAATTACA | chr3:105853309-105853336 | **7402** |
| CB-N28 | AAGACTCTTTAAAGAAGGCA | chr3:105853445-105853472 | **7403** |
| CB-N29 | AAAGACACCAATAGAGTGAA | chr3:105655647-105655674 | **7404** |
| CB-N30 | TTCTAAATTCTAAAGCCCTG | chr3:105657465-105657492 | **7405** |
| CB-N31 | ATTGTTGCCATAACAATGGT | chr3:105867297-105867324 | **7406** |
| CB-N32 | CCCCACTTCAAACTGGACGG | chr3:105868587-105868614 | **7407** |
| CB-N33 | CCTGATTTGATAAAGCAGTG | chr3:105657731-105657758 | **7408** |
| CB-N34 | ATTCACCACAGAAAACCCCA | chr3:105659148-105659175 | **7409** |
| CB-N35 | ATTTCTGGTGCAGTCCTGCG | chr3:105659164-105659191 | **7410** |
| CB-N36 | GACCCTTTTAAGTTGCAGTG | chr3:105655949-105655976 | **7411** |
| CB-N37 | TGTATGCTGAATGGAACACA | chr3:105681777-105681804 | **7412** |
| CB-N38 | TAGTCCCAAACACATCCCGA | chr3:105702244-105702271 | **7413** |
| CB1 | TTCCGCAAAATAGAGCCCCA | chr3:105746011-105746038 | **7414** |
| CB2 | TGTGGGATGTCGACTCCTAG | chr3:105702219-105702246 | **7415** |
| ZC3 | CAGCTCCCTCTAGTCCCGCG | chr1:37475802-37475829 | **7416** |
| ZC7 | GGTTCAGACCAGTACTCTCG | chr1:37483342-37483369 | **7417** |
| SC3 | ACGCCTGCGGATTCTACTG | chr16:11255234-11255261 | **7418** |
| TP4 | TATGCCATGAGTGCTCAGAG | chr6:137878648-137878675 | **7419** |
| TP2 | CTTGTGGCGCTGAAAACGAA | chr6:137871496-137871523 | **7420** |

### Example 2

According to the experimental methods in the examples, the knockout effect of sgRNA targeting TNFAIP3 was detected.

### (A) Knockout Efficiency

| sgRNA No. | Knockout efficiency (%) | sgRNA No. | Knockout efficiency (%) | sgRNA No. | Knockout efficiency (%) |
|---|---|---|---|---|---|
| TP-P1 | C | TP-P21 | A | TP-P40 | A |
| TP-P2 | A | TP-P22 | B | TP-P41 | C |
| TP-P3 | B | TP-P23 | C | TP-P42 | B |
| TP-P4 | B | TP-P24 | A | TP-P43 | B |
| TP-P5 | A | TP-P25 | A | TP-P44 | A |
| TP-P6 | A | TP-P26 | C | TP-P45 | A |
| TP-P7 | B | TP-P27 | A | TP-P46 | A |
| TP-P8 | B | TP-P28 | B | TP-P47 | A |
| TP-P9 | B | TP-P29 | B | TP-P48 | A |
| TP-P10 | C | TP-P30 | A | TP-P49 | A |
| TP-P11 | C | TP-P31 | A | TP-P50 | C |
| TP-P12 | A | TP-P32 | A | TP-P51 | A |
| TP-P13 | A | TP-P33 | A | TP-P52 | A |
| TP-P14 | A | TP-P34 | B | TP-P53 | A |
| TP-P16 | A | TP-P35 | B | TP-P54 | B |
| TP-P17 | A | TP-P36 | A | TP-P55 | A |
| TP-P18 | A | TP-P37 | B | TP-P56 | C |
| TP-P19 | C | TP-P38 | A | TP-P57 | B |
| TP-P20 | A | TP-P39 | A | TP-P58 | B |
| | | | | TP-P59 | A |

Wherein, A indicated that the knockout efficiency was greater than or equal to 70%; B indicated that the knockout efficiency was greater than or equal to 50% and less than 70%; C indicated that the knockout efficiency was greater than or equal to 10% and less than 50%; D indicated that the knockout efficiency was less than 10%; N/A indicated not detected. Donor 045 was a healthy donor, and donor 053 was a healthy donor; PBMC cells were isolated from them and transduced with TCR to serve as TCR-T cells.

### (B) Proliferation Ability

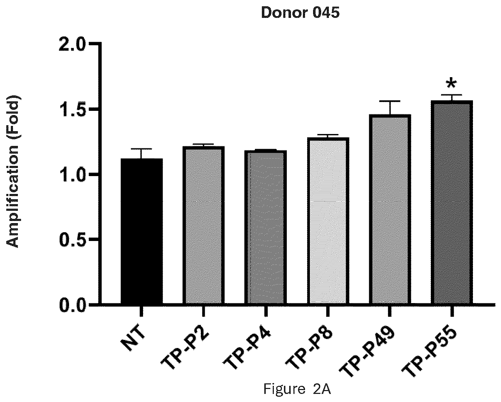

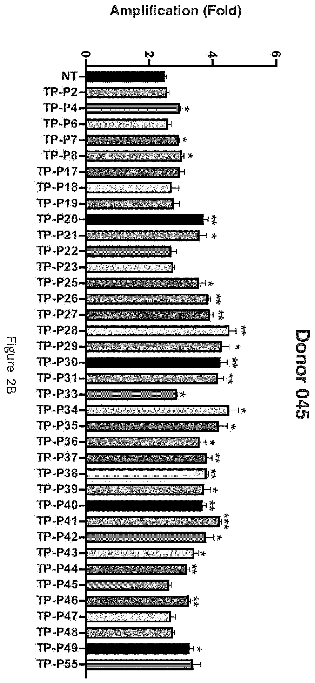

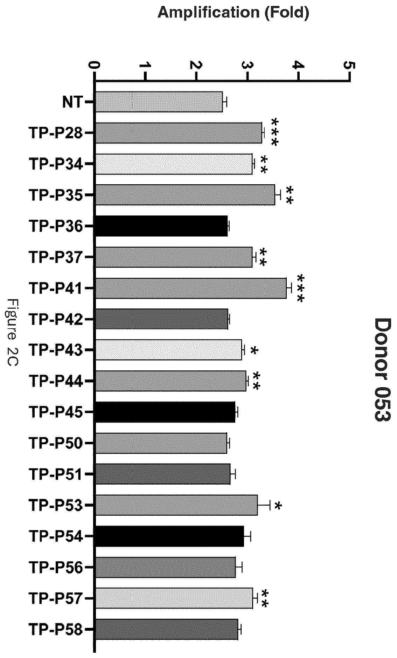

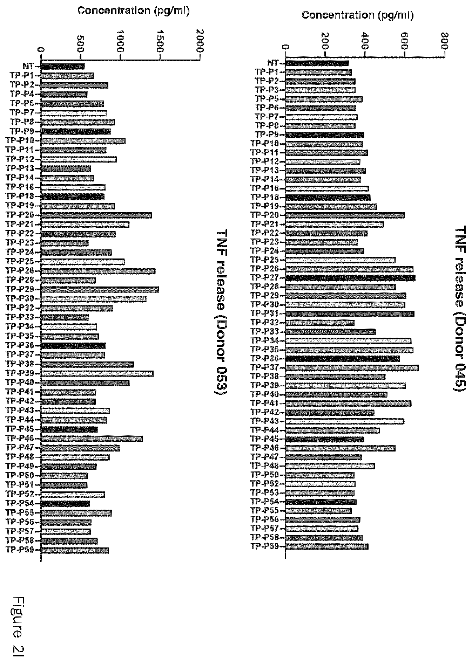

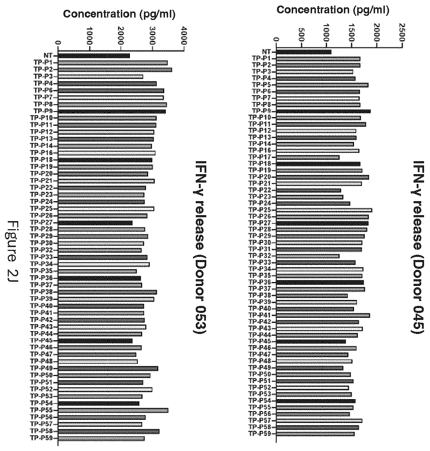

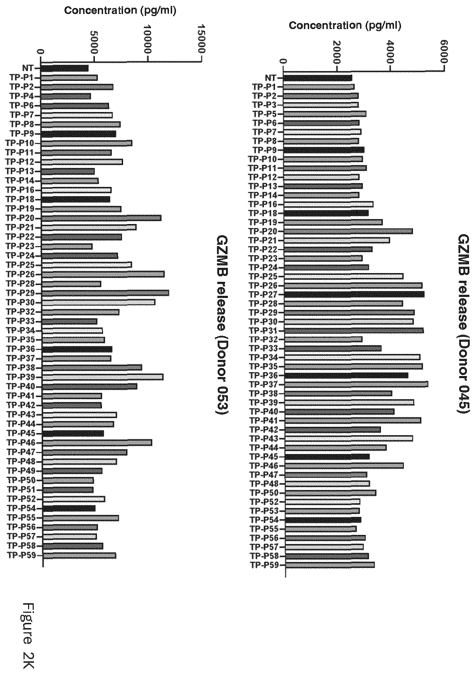

Figure 2A showed the TCR-T expansion fold of TNFAIP3 gene editing in the no-stimulation medium group. Figures 2B-2C showed the TCR-T expansion fold of TNFAIP3 gene editing in the TransAct stimulation group. Significant differences were relative to the unedited NT group: * indicated P<0.05, ** indicated P<0.01, *** indicated P<0.001, **** indicated P<0.0001. The results indicated that, compared to the non-gene-edited control group (NT), T cells edited for the target gene of the present invention could have significantly improved expansion ability.

### (C) Killing Ability

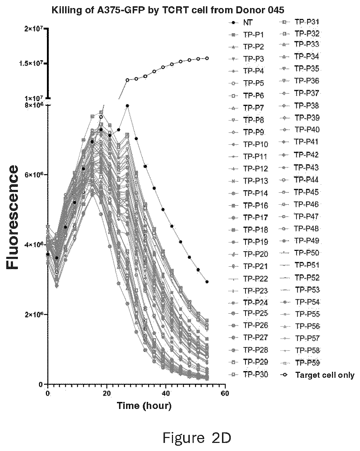

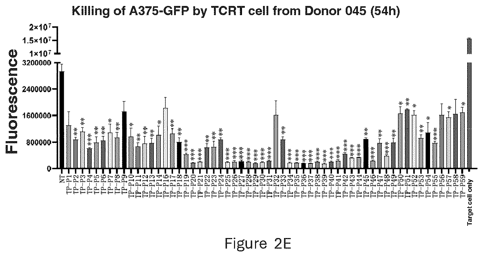

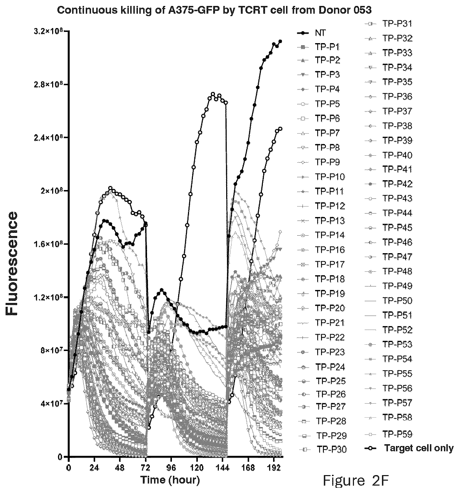

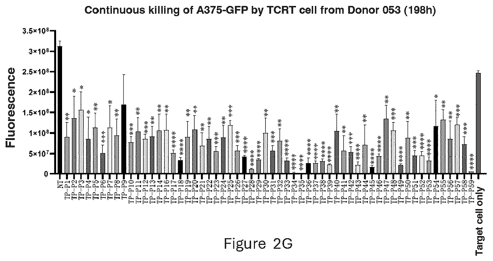

Figures 2D-2G showed the target cell killing ability of TCR-T cells derived from different donors after TNFAIP3 gene editing. Figures 2D and 2F showed the killing curves at each time point, and Figures 2E and 2G showed the killing status of each test group at the end of the experiment, all of which were higher than the unedited NT group. Significant differences were relative to the unedited NT group: * indicated P<0.05, ** indicated P<0.01, *** indicated P<0.001, **** indicated P<0.0001. The results indicated that, compared to the control group (NT), the target gene-edited T cells of the present invention could have significantly improved target cell killing ability and/or continuous killing ability.

### (D) Cytokine Release Ability

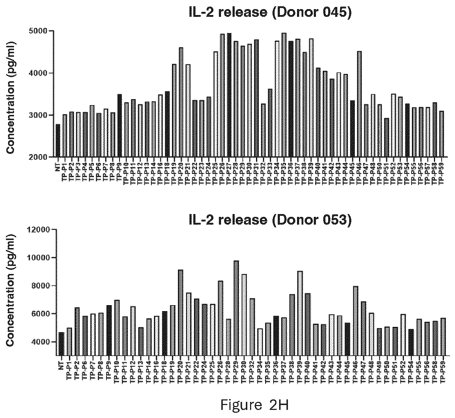

A CBA kit was used to detect the release of cytokines from unedited cells or cells with the target of the present invention knocked out. Figures 2H-2K showed the cytokine release ability of TNFAIP3 gene-edited TCR-T cells. The results indicated that, compared to the control group (NT), the T cells edited for the target gene of the present invention could have significantly improved cytokine release capacity.

### Example 3

According to the experimental methods in the examples, the knockout effect of the sgRNA targeting ZC3H12A was detected.

| sgRNA No. | knockout efficiency (%) | sgRNA No. | knockout efficiency (%) | sgRNA No. | knockout efficiency (%) |
|---|---|---|---|---|---|
| ZC-P1 | A | ZC-P8 | A | ZC-P15 | A |
| ZC-P2 | B | ZC-P9 | A | ZC-P16 | B |
| ZC-P3 | A | ZC-P10 | N/A | ZC-P17 | A |
| ZC-P4 | A | ZC-P11 | N/A | ZC-P18 | B |
| ZC-P5 | A | ZC-P12 | A | ZC-P19 | A |
| ZC-P6 | A | ZC-P13 | B | ZC-P20 | A |
| ZC-P7 | A | ZC-P14 | A | ZC-P21 | A |

Wherein, A indicated that the knockout efficiency was greater than or equal to 70%; B indicated that the knockout efficiency was greater than or equal to 50% and less than 70%; C indicated that the knockout efficiency was greater than or equal to 10% and less than 50%; D indicated that the knockout efficiency was less than 10%; N/A indicated not detected.

### (B) Proliferation Ability

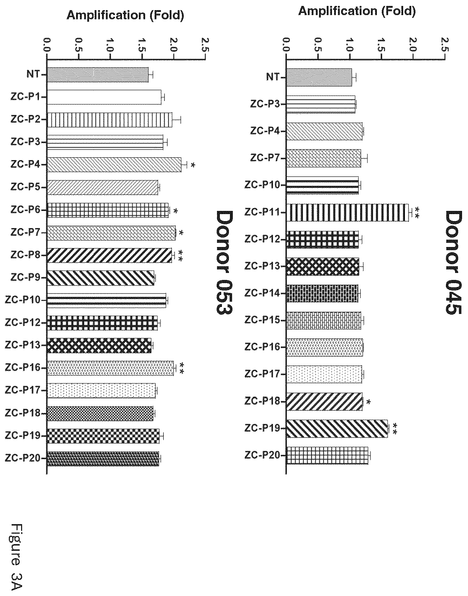

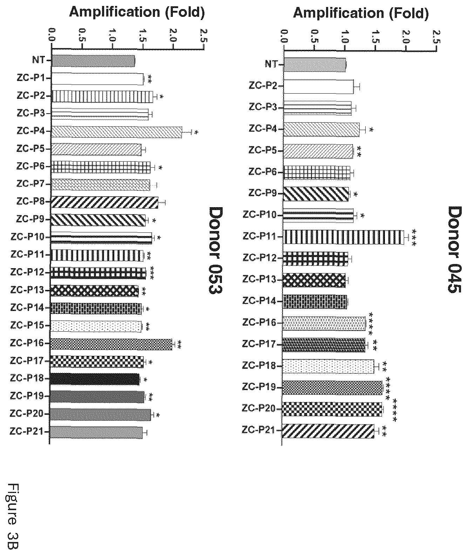

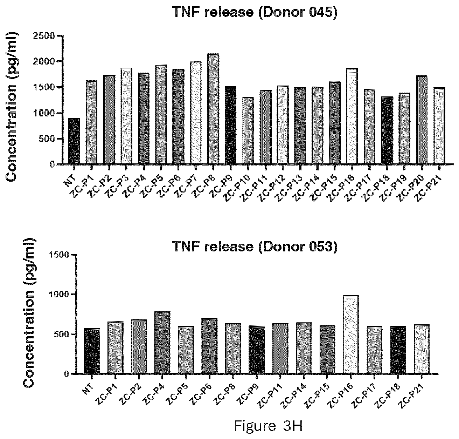

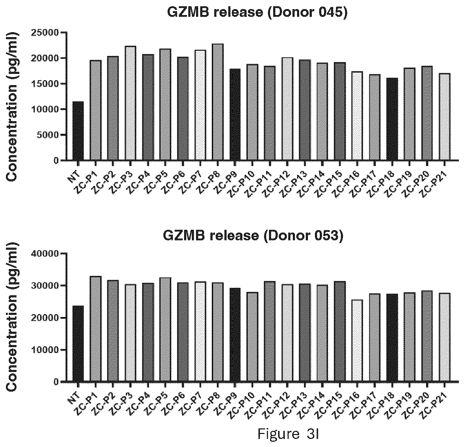

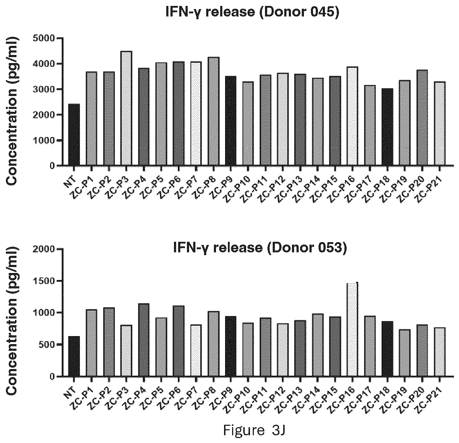

Figure 3A showed the TCR-T expansion fold of ZC3H12A gene editing in the no-stimulation medium group. Figure 3B showed the TCR-T expansion fold of ZC3H12A gene editing in the TransAct stimulation group. Significant differences were relative to the unedited NT group: * indicated P<0.05, ** indicated P<0.01, *** indicated P<0.001, **** indicated P<0.0001. The results indicated that, compared to the non-gene-edited control group (NT), T cells edited for the target gene of the present invention could have significantly improved expansion ability.

### (C) Killing Ability

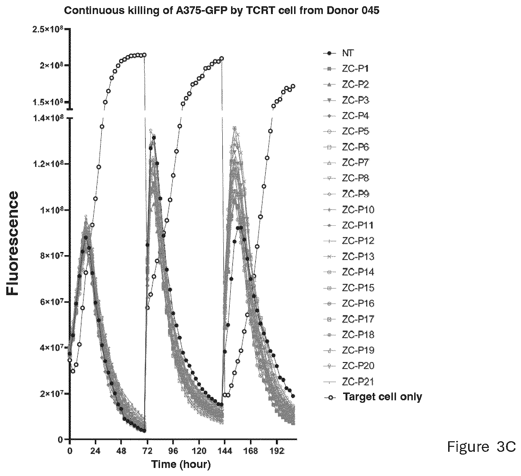

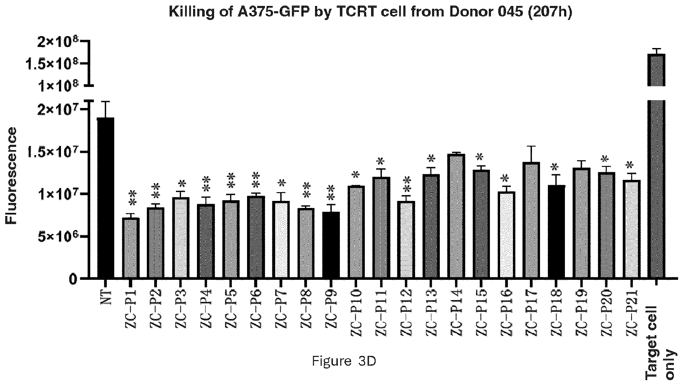

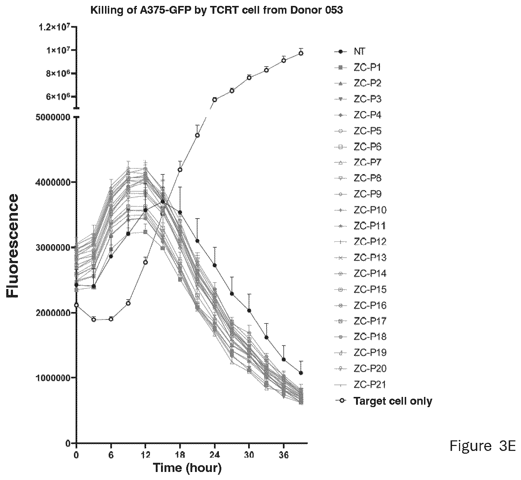

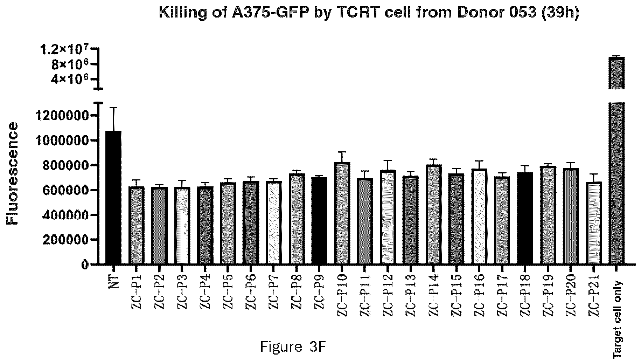

Figures 3C-3F showed the target cell killing ability of ZC3H12A gene-edited TCR-T cells. Figures 3C and 3E showed the killing curves at each time point, and Figures 3D and 3F showed the killing status of each test group at the end of the experiment, all of which were higher than the unedited NT group. Significant differences were relative to the unedited NT group: * indicated P<0.05, ** indicated P<0.01, *** indicated P<0.001, **** indicated P<0.0001. The results indicated that, compared to the control group (NT), the target gene-edited T cells of the present invention could have significantly improved target cell killing ability and/or continuous killing ability.

### (D) Cytokine Release Ability

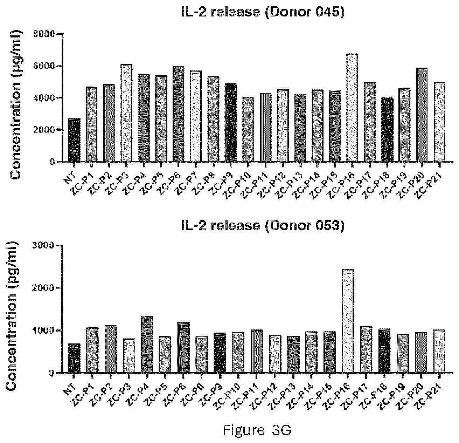

A CBA kit was used to detect the release of cytokines from unedited cells or cells with the target of the present invention knocked out. Figures 3G-3J showed the cytokine release ability of ZC3H12A gene-edited TCR-T cells. The results indicated that, compared to the control group (NT), the T cells edited for the target gene of the present invention could have significantly improved cytokine release capabilities.

### Example 4

According to the experimental methods in the examples, the knockout effect of sgRNA targeting SOCS1 was detected.

| sgRNA No. | knockout efficiency (%) | sgRNA No. | knockout efficiency (%) | sgRNA No. | knockout efficiency (%) |
|---|---|---|---|---|---|
| SC-N2 | A | SC-N14 | D | SC-N26 | C |
| SC-N3 | A | SC-N15 | A | SC-N27 | C |
| SC-N4 | C | SC-N16 | B | SC-N28 | A |
| SC-N6 | A | SC-N17 | B | SC-N29 | A |
| SC-N7 | A | SC-N18 | A | SC-N30 | A |
| SC-N8 | A | SC-N20 | A | SC-N31 | A |
| SC-N9 | A | SC-N21 | B | SC-N32 | A |
| SC-N10 | A | SC-N22 | A | SC-N33 | A |
| SC-N11 | B | SC-N24 | A | SC-N34 | A |
| SC-N12 | A | SC-N25 | B | SC-N35 | A |

Wherein, A indicated that the knockout efficiency was greater than or equal to 70%; B indicated that the knockout efficiency was greater than or equal to 50% and less than 70%; C indicated that the knockout efficiency was greater than or equal to 10% and less than 50%; D indicated that the knockout efficiency was less than 10%; N/A indicated not detected. Donor 005 was a healthy donor, donor 006 was a healthy donor, and donor 031 was a healthy donor; PBMC cells were isolated from them and transduced with TCR to serve as TCR-T cells. Donor 105 was a lung cancer donor, and donor 306 was a cervical cancer donor; TILs were isolated and provided from them.

### (B) Proliferation Ability

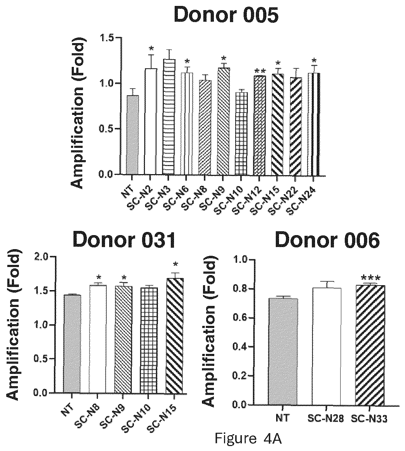

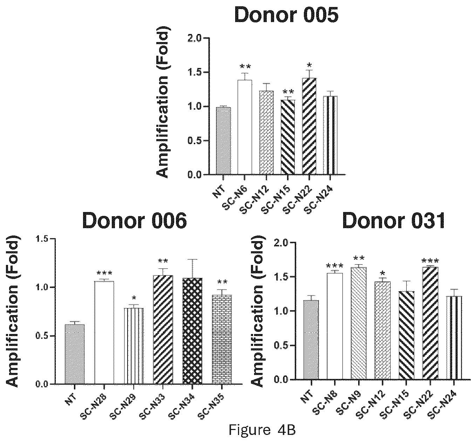

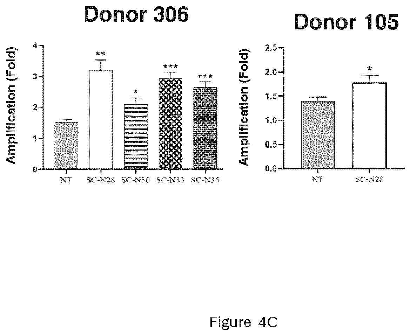

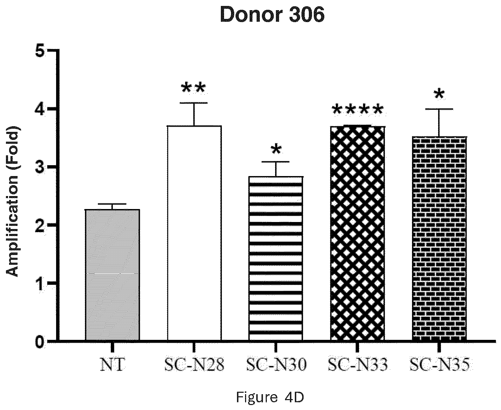

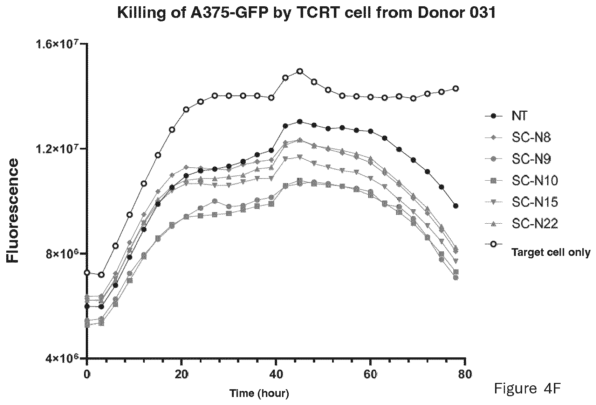

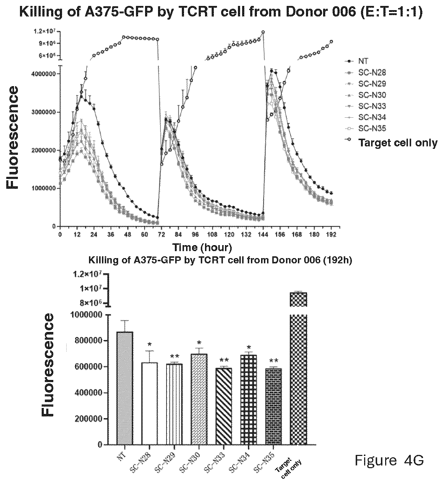

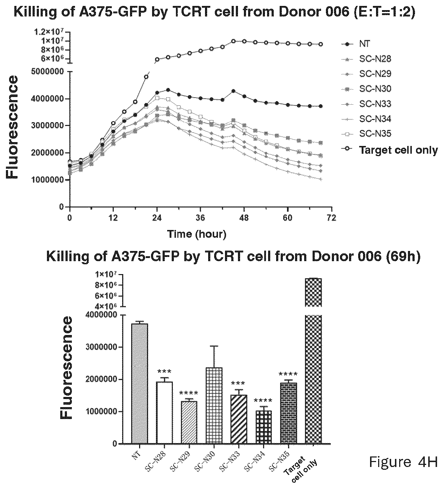

Figure 4A showed the TCR-T expansion fold of SOCS1 gene editing in the no-stimulation medium group. Figure 4B showed the TCR-T expansion fold of SOCS1 gene editing in the TransAct stimulation group. Figure 4C showed the TIL expansion fold of SOCS1 gene editing in the no-stimulation medium group. Figure 4D showed the TIL expansion fold of SOCS1 gene editing in the TransAct stimulation group. Significant differences were relative to the unedited NT group: * indicated P<0.05, ** indicated P<0.01, *** indicated P<0.001, **** indicated P<0.0001. The results indicated that, compared to the non-gene-edited control group (NT), T cells edited for the target gene of the present invention could have significantly improved expansion ability.

### (C) Killing Ability

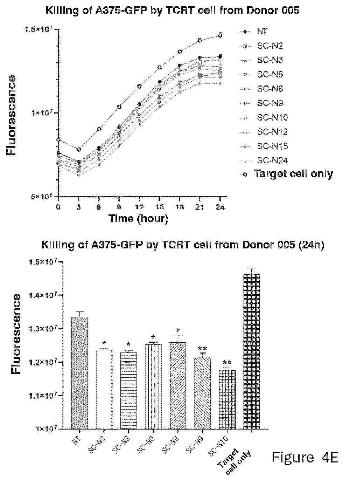

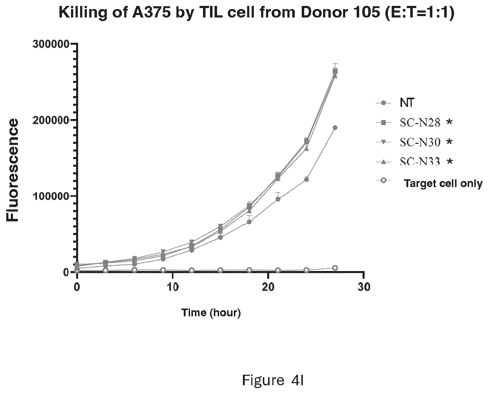

Figures 4E-4H showed the target cell killing ability of SOCS1 gene-edited TCR-T cells. Each figure showed the killing curve at each time point, as well as the killing situation of each test group at specific time points, which were higher than the unedited NT group. Figure 4I showed the target cell killing ability of SOCS1 gene-edited TIL cells. Significant differences were relative to the unedited NT group: * indicated P<0.05, ** indicated P<0.01, *** indicated P<0.001, **** indicated P<0.0001. The results indicated that, compared to the control group (NT), the target gene-edited T cells of the present invention could have significantly improved target cell killing ability and/or continuous killing ability.

### (D) Cytokine Expression and Release

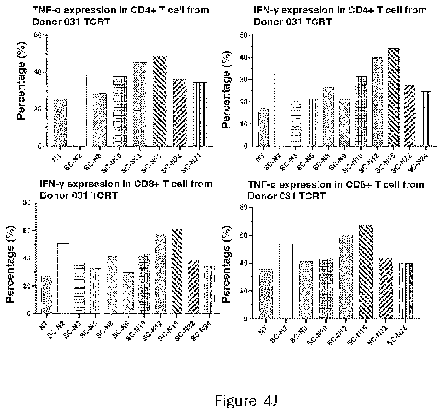

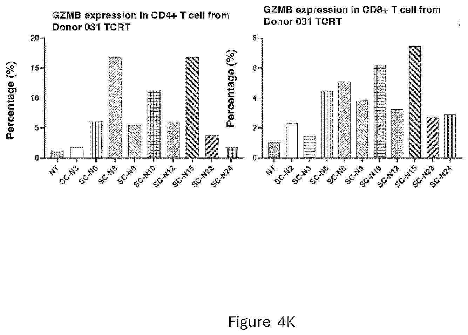

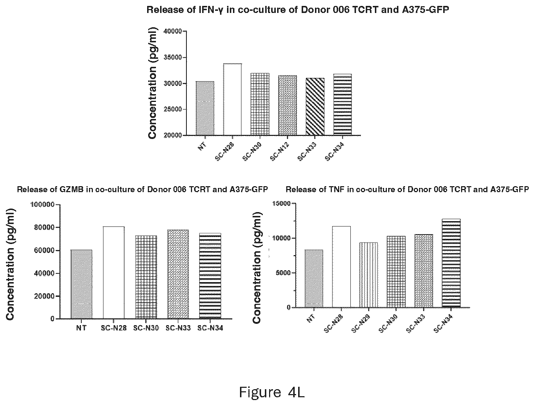

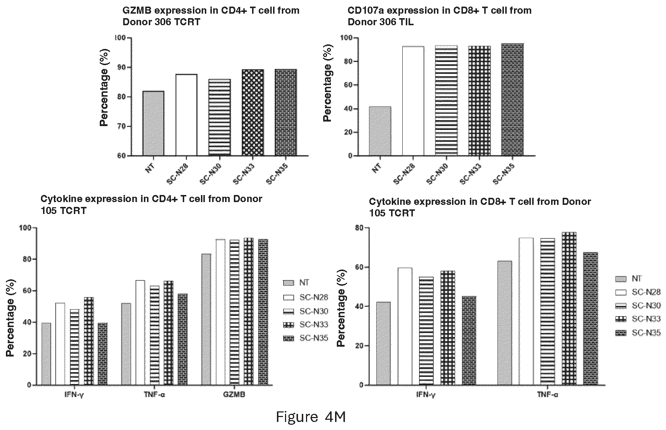

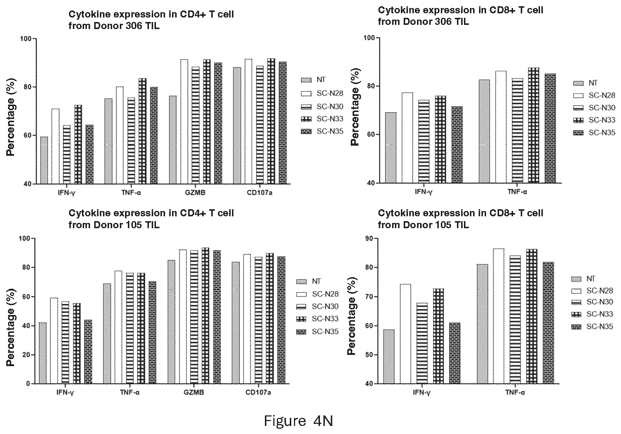

A flow cytometer was used to detect the cytokine expression of unedited cells or cells with the target of the present invention knocked out, and a CBA kit was used to detect the cytokine release ability. Figure 4J showed the cytokine expression of SOCS1 gene-edited TCR-T cells in the unstimulated group. Figure 4K showed the cytokine expression of SOCS1 gene-edited TCR-T cells in the CD3 antibody stimulation group. Figure 4L showed the cytokine release ability of SOCS1 gene-edited TCR-T cells co-cultured with A375 target cells. Figure 4M showed the cytokine expression of SOCS1 gene-edited TIL cells in the unstimulated group. Figure 4N showed the cytokine expression of SOCS1 gene-edited TIL cells in the TransAct stimulation group. The results indicated that, compared to the control group (NT), the T cells edited for the target gene of the present invention could have significantly improved cytokine expression and/or release capabilities.

### (E) Flow Cytometry Detection

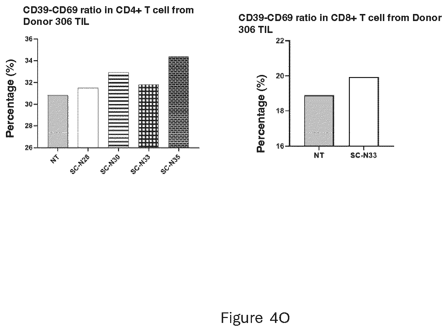

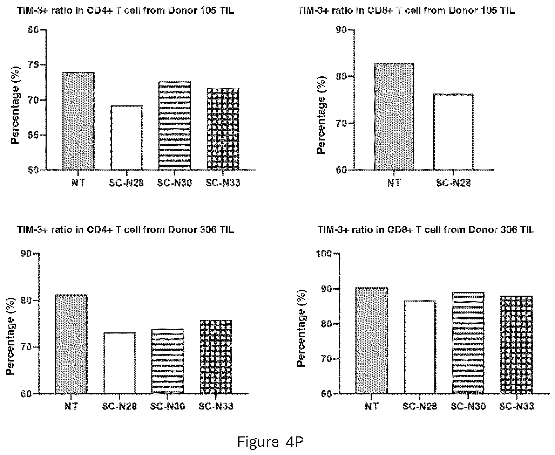

Figure 4O showed that TIL cells after SOCS1 gene editing had a higher proportion of stem-like cells. Figure 4P showed that TIL cells after SOCS1 gene editing had a lower proportion of exhausted T cells. For example, stem-like cells could be CD39 negative CD69 negative cells. For example, exhausted T cells could be PD-1 positive, LAG-3 positive, TIM-3 positive, CD38 positive and/or CD101 positive cells. The results indicated that, compared to the non-gene-edited control group (NT), the gene-edited TIL cells of the present invention could have more favorable cell phenotypic characteristics.

### Example 5

According to the experimental methods in the examples, the knockout effect of sgRNA targeting CBLB was detected.

| sgRNA No. | knockout efficiency (%) | sgRNA No. | knockout efficiency (%) | sgRNA No. | knockout efficiency (%) |
|---|---|---|---|---|---|
| CB-N1 | B | CB-N14 | C | CB-N27 | A |
| CB-N2 | A | CB-N15 | A | CB-N28 | A |
| CB-N3 | C | CB-N16 | N/A | CB-N29 | C |
| CB-N4 | B | CB-N17 | B | CB-N30 | C |
| CB-N5 | A | CB-N18 | B | CB-N31 | B |
| CB-N6 | B | CB-N19 | C | CB-N32 | N/A |
| CB-N7 | B | CB-N20 | A | CB-N33 | B |
| CB-N8 | C | CB-N21 | A | CB-N34 | N/A |
| CB-N9 | B | CB-N22 | A | CB-N35 | N/A |
| CB-N10 | C | CB-N23 | N/A | CB-N36 | N/A |
| CB-N11 | A | CB-N24 | N/A | CB-N37 | N/A |
| CB-N12 | A | CB-N25 | N/A | CB-N38 | N/A |
| CB-N13 | C | CB-N26 | C | | |

Wherein, A indicated that the knockout efficiency was greater than or equal to 70%; B indicated that the knockout efficiency was greater than or equal to 50% and less than 70%; C indicated that the knockout efficiency was greater than or equal to 10% and less than 50%; D indicated that the knockout efficiency was less than 10%; N/A indicated not detected.

### (B) Proliferation Ability

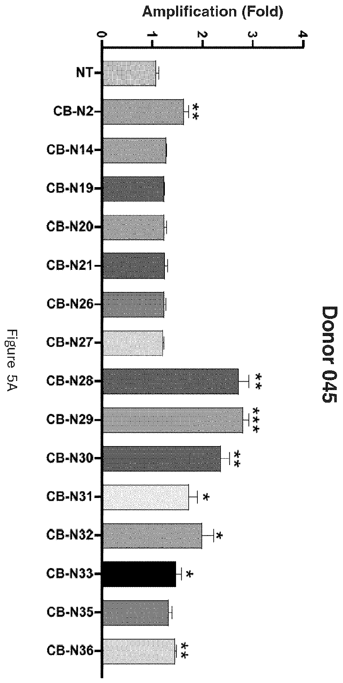

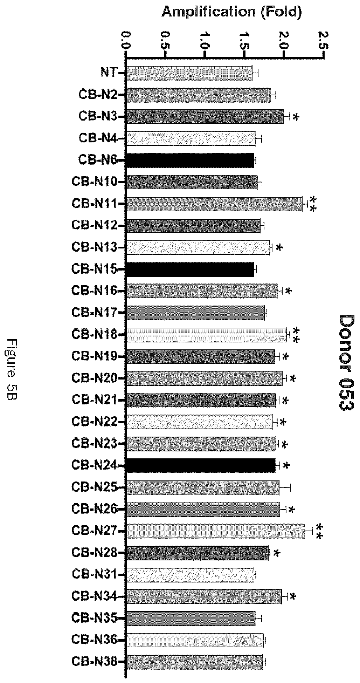

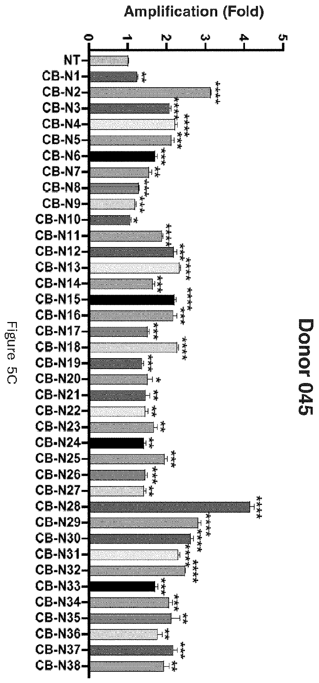

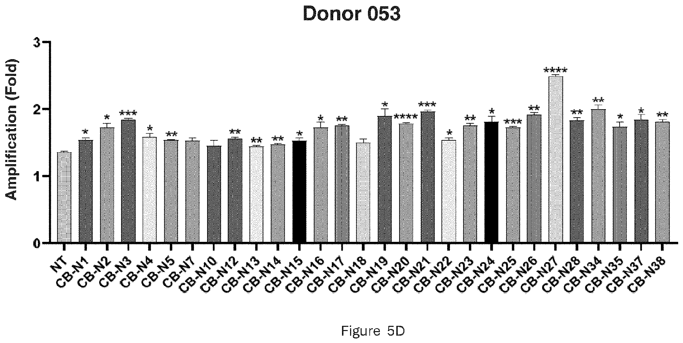

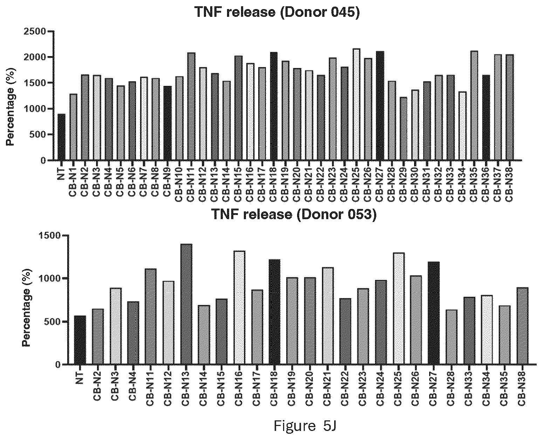

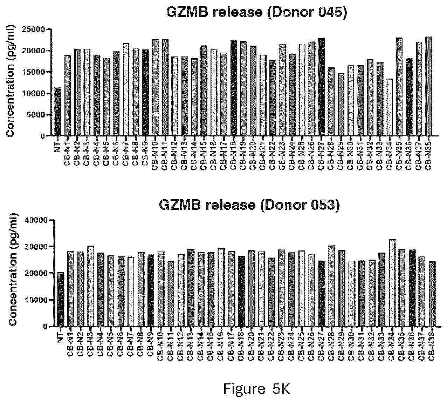

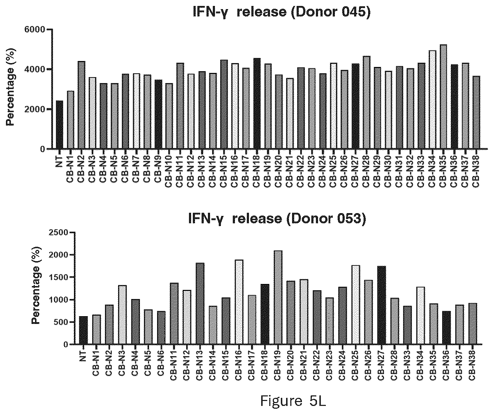

Figures 5A-5B showed the TCR-T expansion fold of CBLB gene editing in the no-stimulation medium group. Figures 5C-5D showed the TCR-T expansion fold of CBLB gene editing in the TransAct stimulation group. Significant differences were relative to the unedited NT group: * indicated P<0.05, ** indicated P<0.01, *** indicated P<0.001, **** indicated P<0.0001. The results indicated that, compared to the non-gene-edited control group (NT), T cells edited for the target gene of the present invention could have significantly improved expansion ability.

### (C) Killing Ability

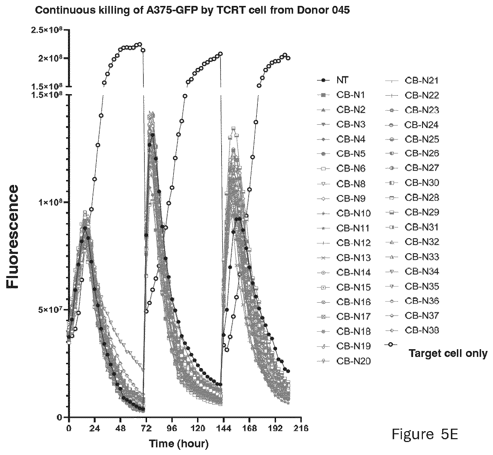

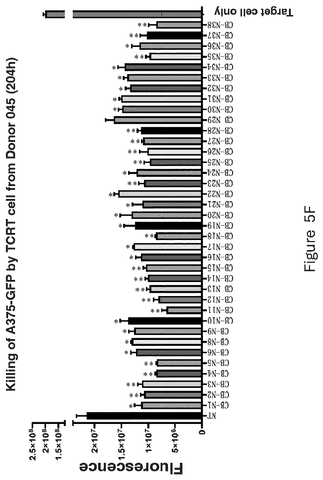

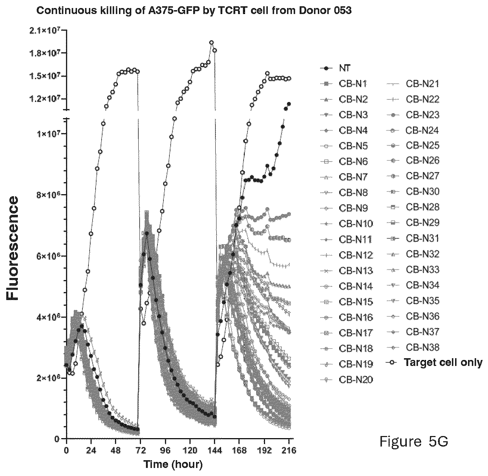

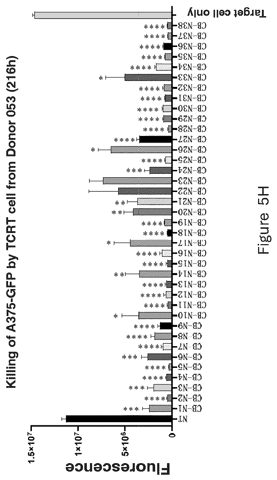

Figures 5E-5H showed the target cell killing ability of CBLB gene-edited TCR-T cells. Figures 5E and 5G showed the killing curves at each time point, and Figures 5F and 5H showed the killing status of each test group at the end of the experiment, all of which were higher than the unedited NT group. Significant differences were relative to the unedited NT group: * indicated P<0.05, ** indicated P<0.01, *** indicated P<0.001, **** indicated P<0.0001. The results indicated that, compared to the control group (NT), the target gene-edited T cells of the present invention could have significantly improved target cell killing ability and/or continuous killing ability.

### (D) Cytokine Release Ability

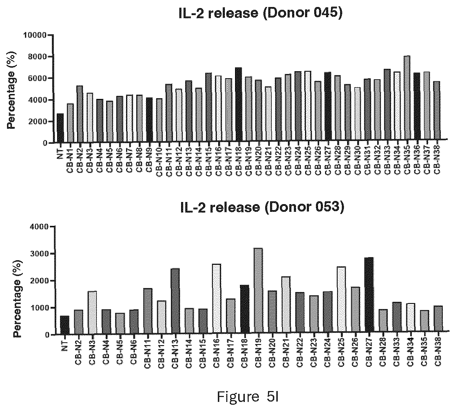

A CBA kit was used to detect the release of cytokines from unedited cells or cells with the target of the present invention knocked out. Figures 5I-5L showed the cytokine release ability of CBLB gene-edited TCR-T cells. The results indicated that, compared to the control group (NT), the T cells edited for the target gene of the present invention could have significantly improved cytokine release capacity.

### Example 6

According to the experimental methods in the embodiments, the knockout effect of sgRNAs targeting two or more selected from TNFAIP3, ZC3H12A, SOCS1, and CBLB was detected.

| Target combination | sgRNA No. | knockout efficiency (%) | Target combination | sgRNA No. | knockout efficiency (%) |
|---|---|---|---|---|---|
| CBLB+ ZC3H12A | CB1 | A | SOCS1+ ZC3H12A | SC3 | A |
| | ZC3 | A | | ZC7 | A |
| SOCS1+ CBLB | SC3 | A | TNFAIP3+ CBLB | TP4 | A |
| | CB1 | A | | CB1 | A |
| SOCS1+ CBLB | SC3 | A | TNFAIP3+ ZC3H12A | TP4 | A |
| | CB2 | A | | ZC3 | A |
| SOCS1+ TNFAIP3 | SC3 | A | TNFAIP3+ ZC3H12A | TP4 | A |
| | TP4 | A | | ZC7 | A |
| SOCS1+ ZC3H12A | SC3 | A | SOCS1+ TNFAIP3 | TP2 | A |
| | ZC3 | A | | SC-N30 | B |
| SOCS1+ TNFAIP3 | TP2 | A | SOCS1+ TNFAIP3 | TP2 | A |
| | SC-N28 | A | | SC-N33 | A |

Wherein, A indicated that the knockout efficiency was greater than or equal to 70%; B indicated that the knockout efficiency was greater than or equal to 50% and less than 70%; C indicated that the knockout efficiency was greater than or equal to 10% and less than 50%; D indicated that the knockout efficiency was less than 10%; N/A indicated not detected. Donor 713 was a cervical cancer patient, donor 316 was a malignant melanoma patient, and donors 504 and 607 were ovarian cancer patients. Donor 801 was a cervical cancer patient, and donor 312 was a cervical cancer patient.

### (B) Expansion Status

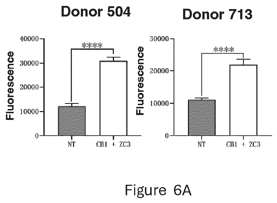

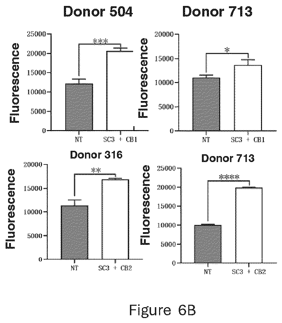

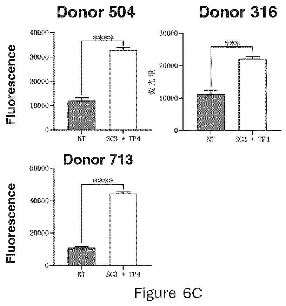

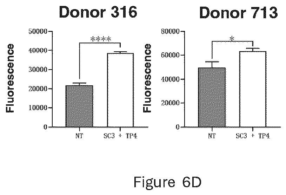

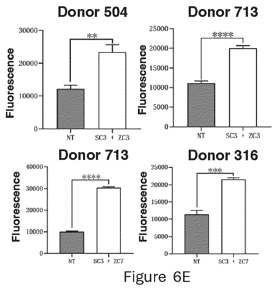

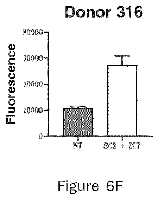

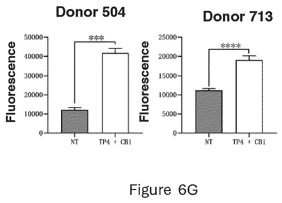

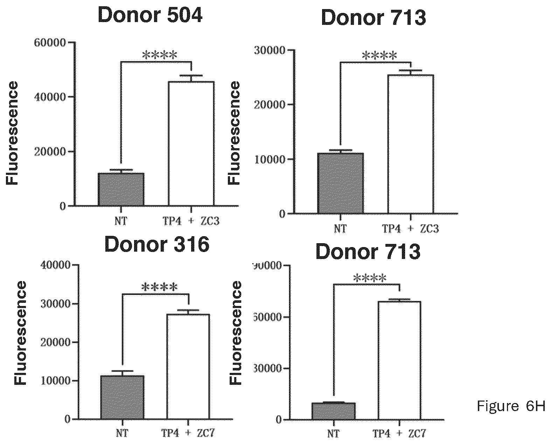

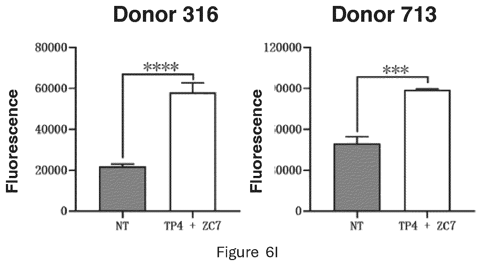

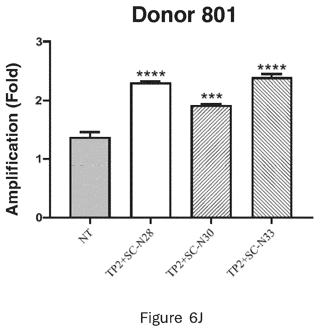

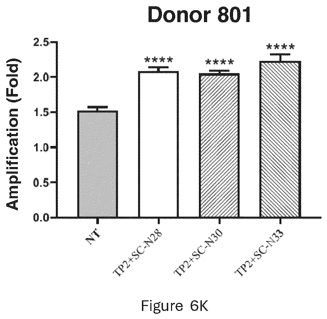

Figures 6A, 6B, 6C, 6E, 6G, 6H, and 6J showed the TIL expansion fold of combined gene editing in the no-stimulation medium group. Figures 6D, 6F, and 6I showed the TIL expansion fold of combined gene editing in the CD3 antibody stimulation group. Figure 6K showed the TIL expansion fold of combined gene editing in the TransAct antibody stimulation group. Significant differences were relative to the unedited NT group: * indicated P<0.05, ** indicated P<0.01, *** indicated P<0.001, **** indicated P<0.0001. The results indicated that, compared to the non-gene-edited control group (NT), TIL cells edited with the target combination of the present invention could have significantly improved expansion ability.

### (C) Killing Status

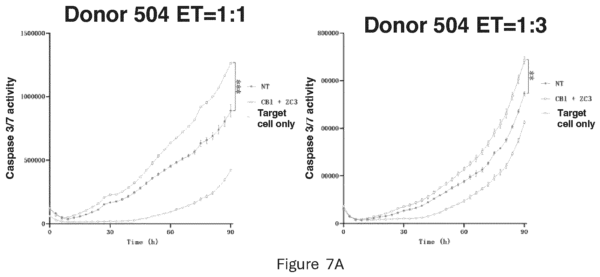

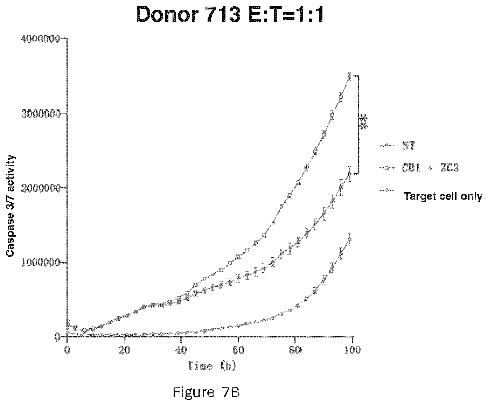

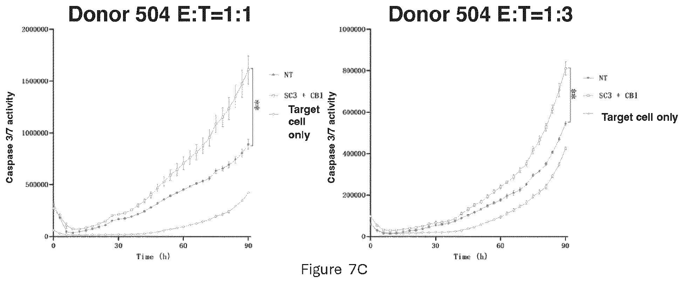

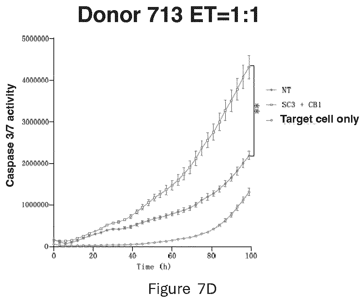

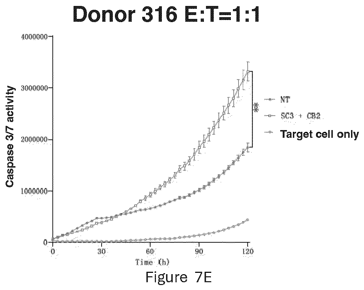

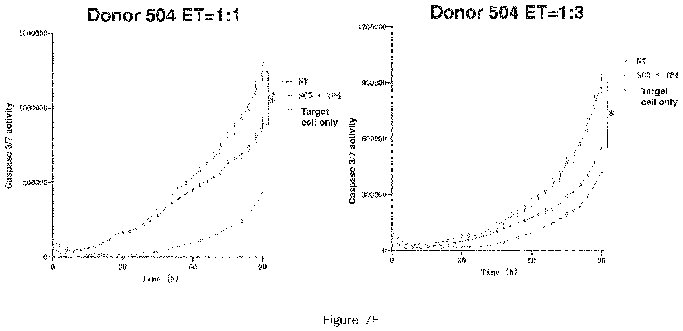

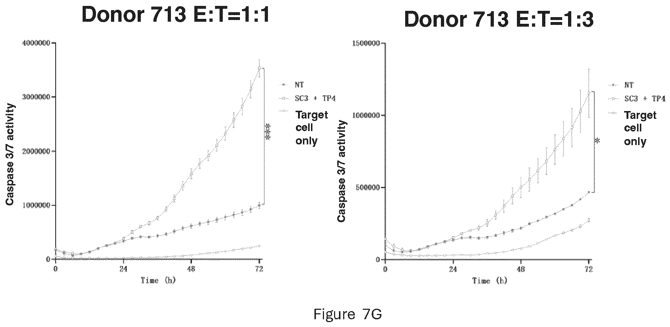

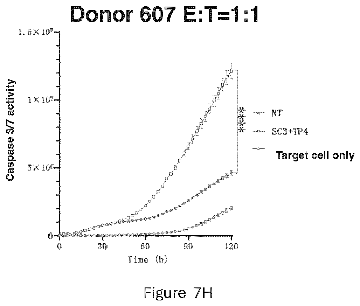

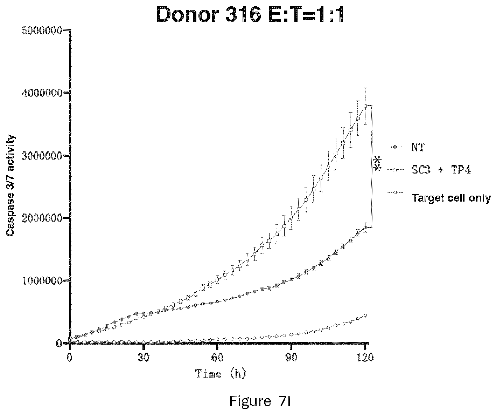

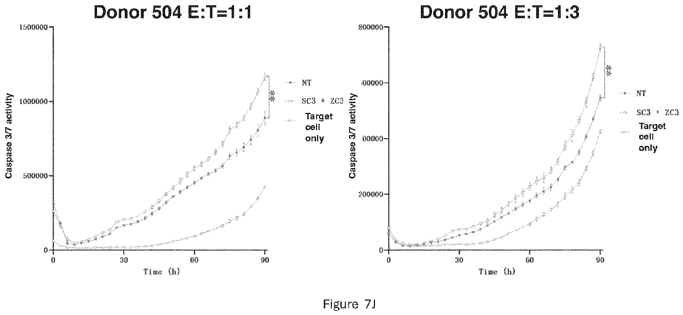

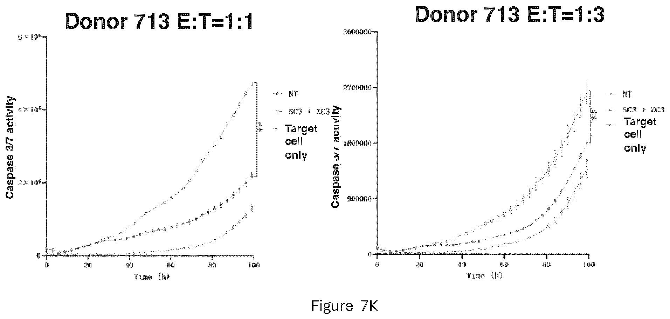

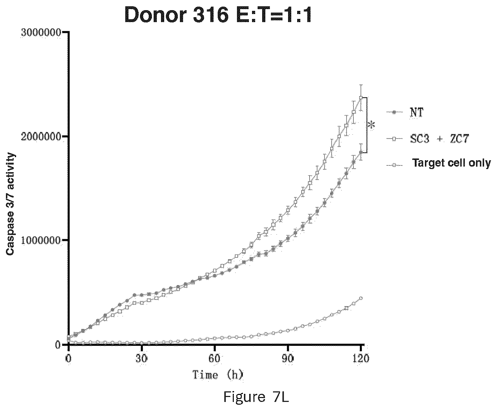

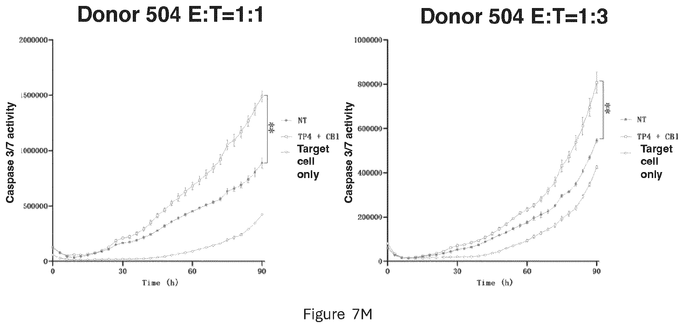

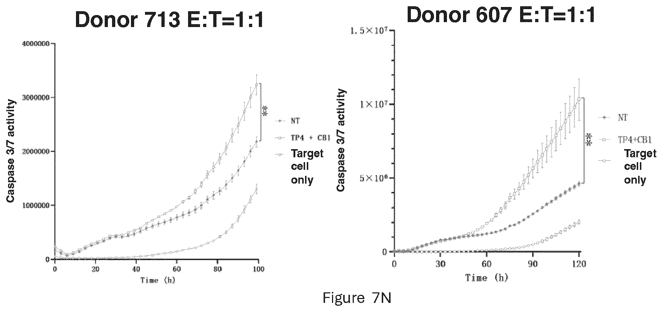

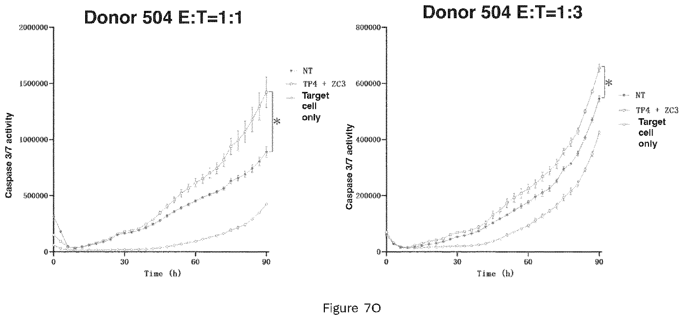

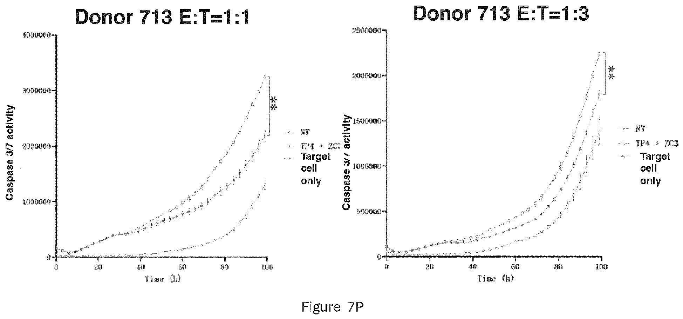

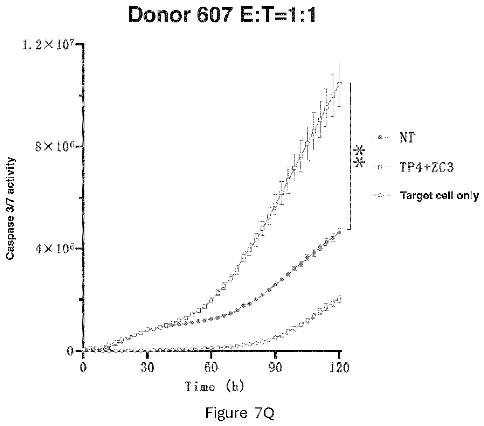

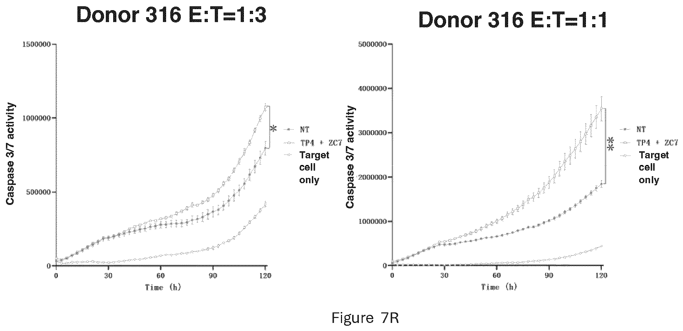

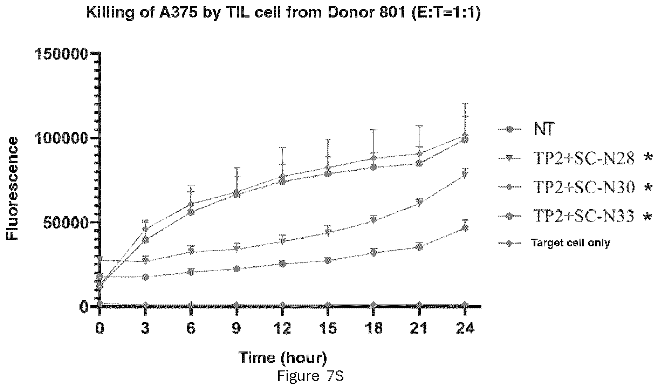

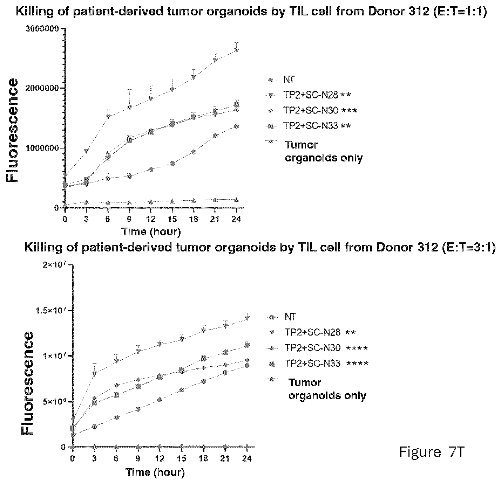

Figures 7A to 7S showed the target cell killing ability of combinatorially gene-edited TIL cells. Figure 7T showed the killing ability of combined gene-edited TIL cells against autologous tumor organoids. Significant differences were relative to the unedited NT group: * indicated P<0.05, ** indicated P<0.01, *** indicated P<0.001, **** indicated P<0.0001. The results indicated that, compared to the control group (NT), TIL cells edited with the target combination gene of the present invention could have significantly improved target cell killing ability.

### (D) Cell Flow Cytometry Detection

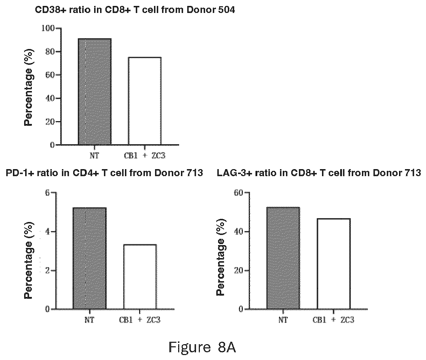

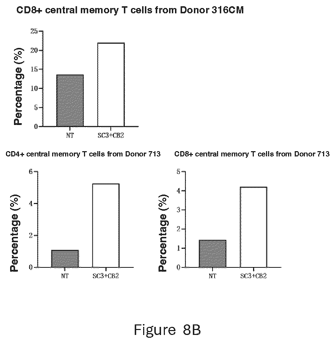

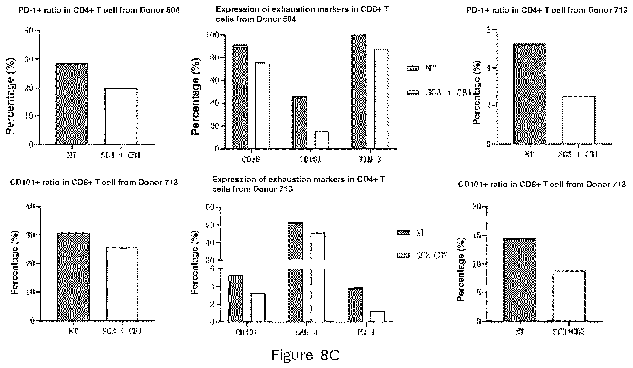

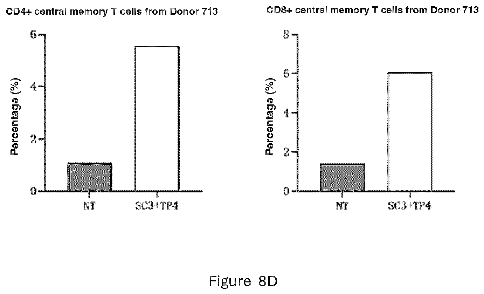

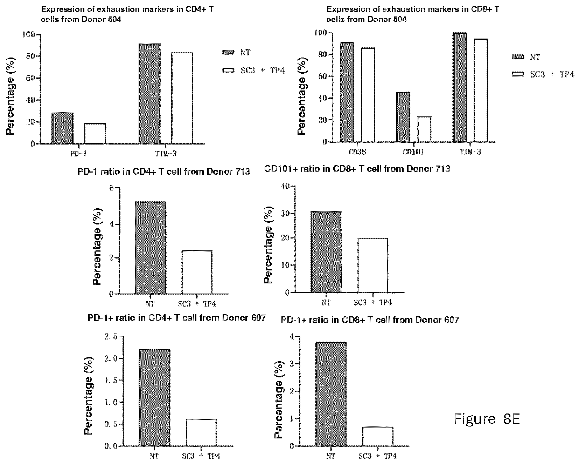

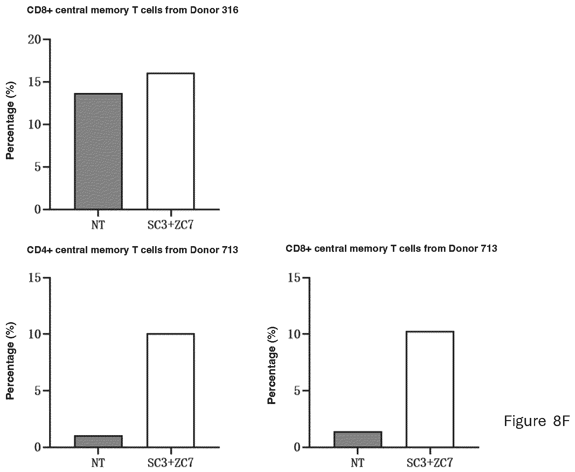

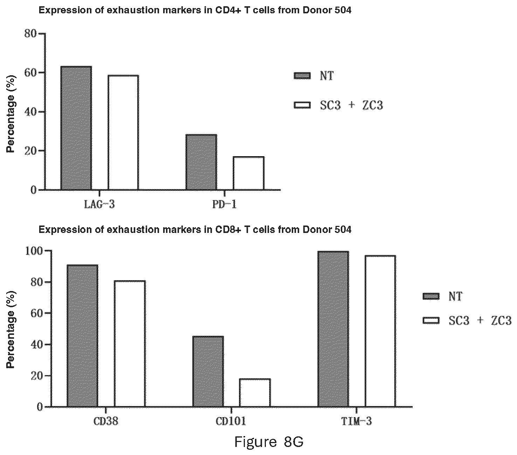

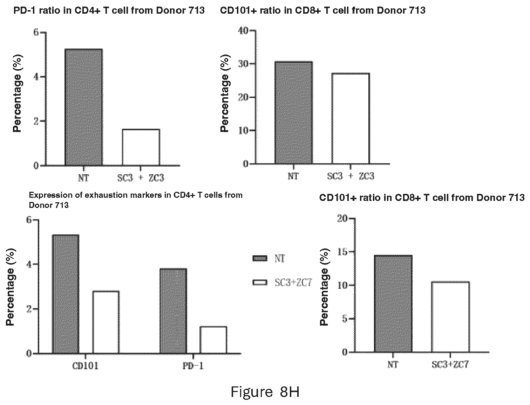

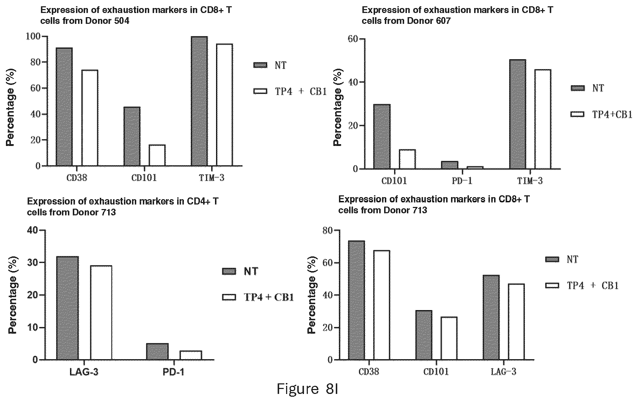

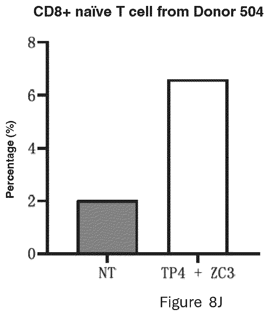

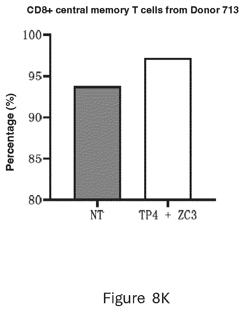

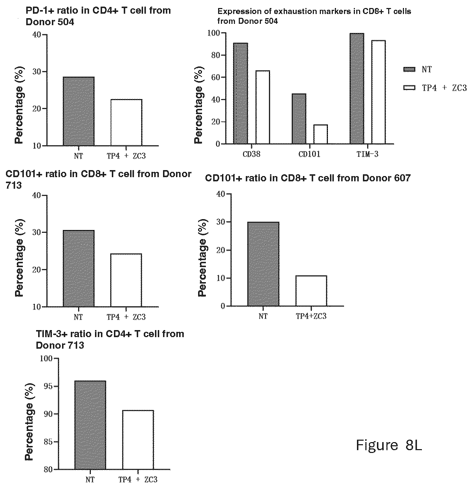

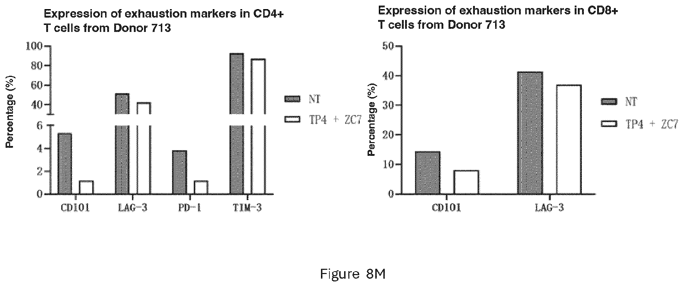

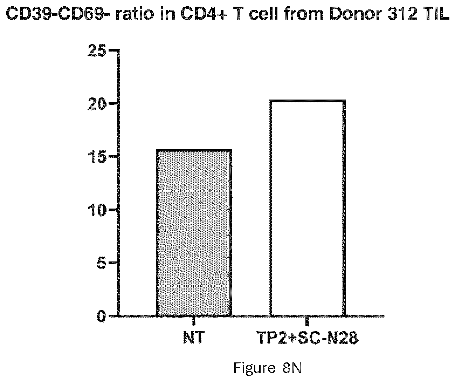

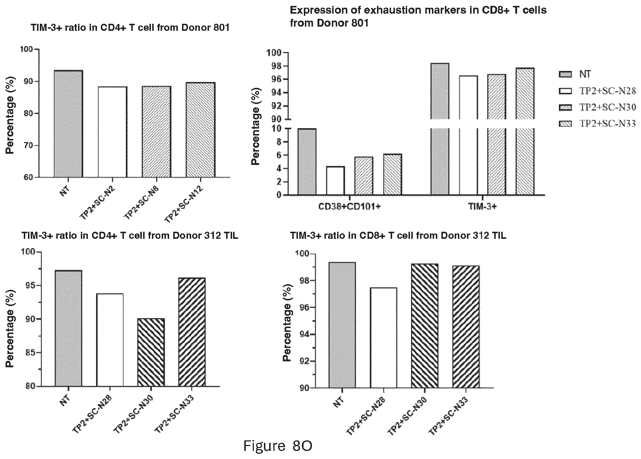

Figures 8A, 8C, 8E, 8G, 8H, 8I, 8L, 8M, and 8O showed that TIL cells after combined gene editing had a lower proportion of exhausted T cells. Figures 8B, 8D, 8F, and 8K showed that TIL cells after combined gene editing had a higher proportion of central memory T cells. Figure 8J showed that TIL cells edited with the combination of TNFAIP3 and ZC3H12A had a higher proportion of naive T cells. Figure 8N showed that TIL cells edited with the combination of TNFAIP3 and SOCS1 had a higher proportion of stem-like cells. For example, exhausted T cells could be PD-1 positive, LAG-3 positive, TIM-3 positive, CD38 positive and/or CD101 positive cells. For example, central memory T cells could be CD45RO positive CD62L positive cells. For example, stem-like cells could be CD39 negative CD69 negative cells. The results indicated that, compared to the non-gene-edited control group (NT), TIL cells edited with the target combination of the present invention could have more favorable cell phenotypic characteristics.

### (E) Cytokine Expression and Release

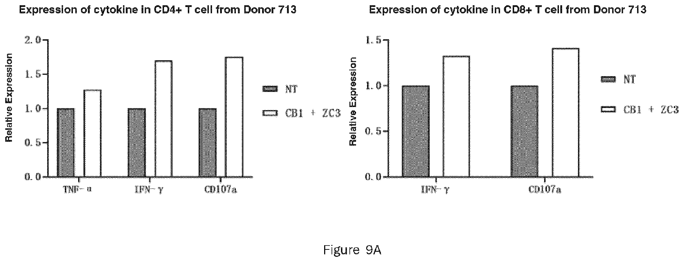

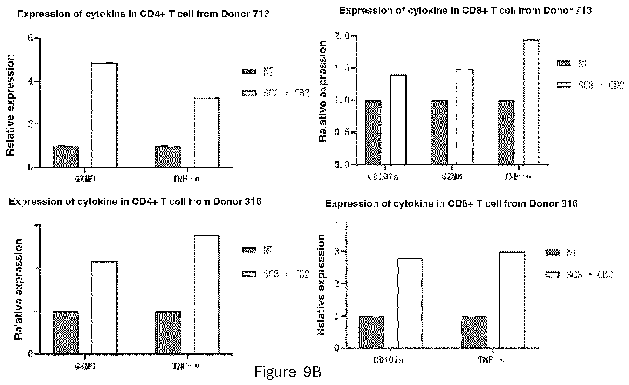

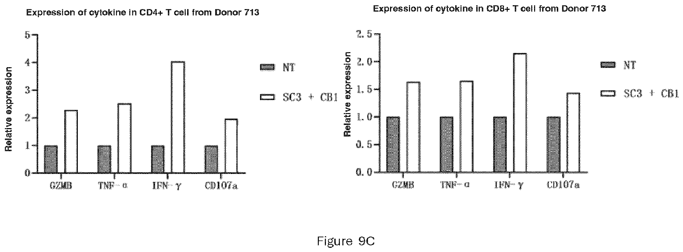

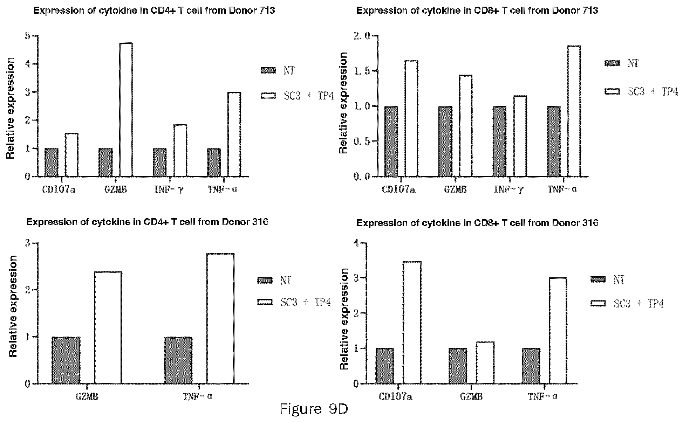

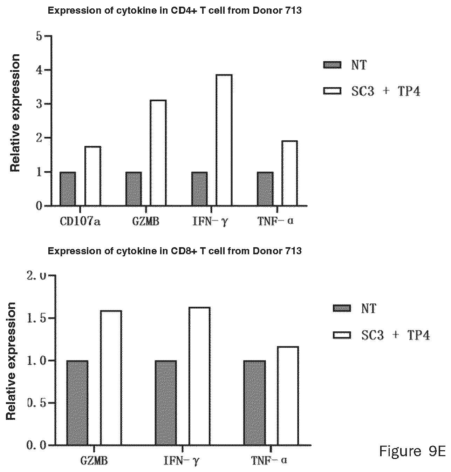

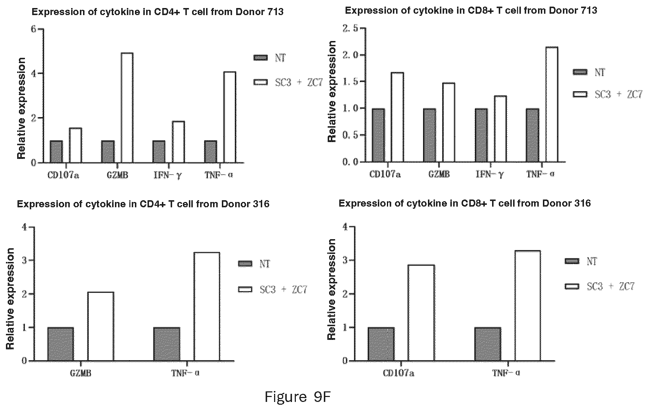

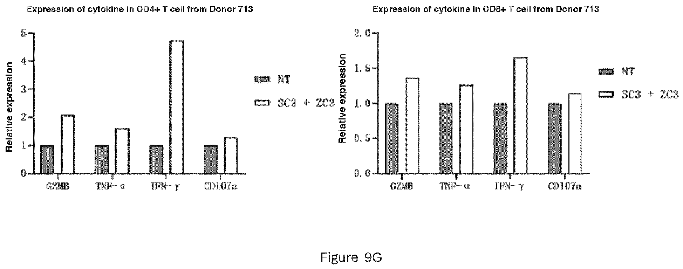

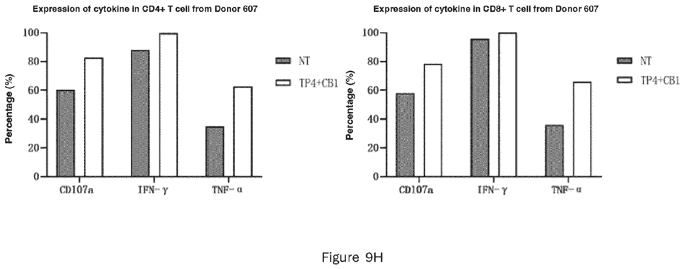

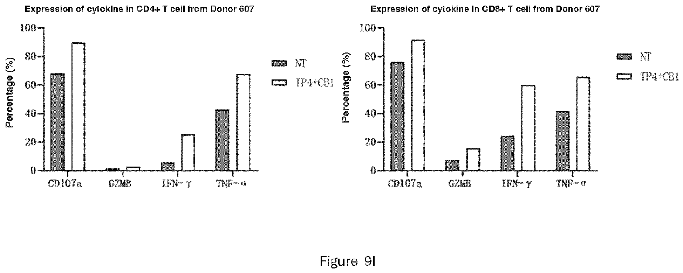

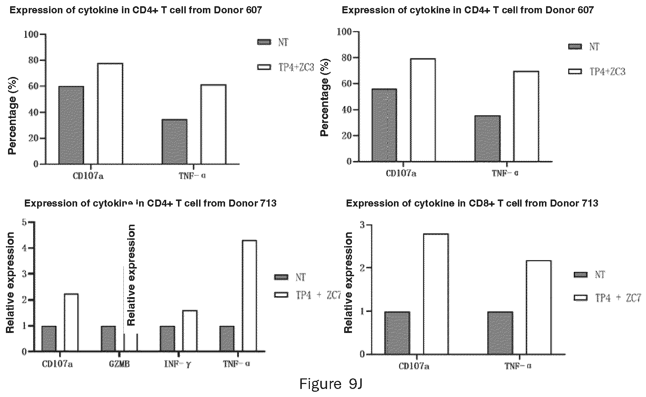

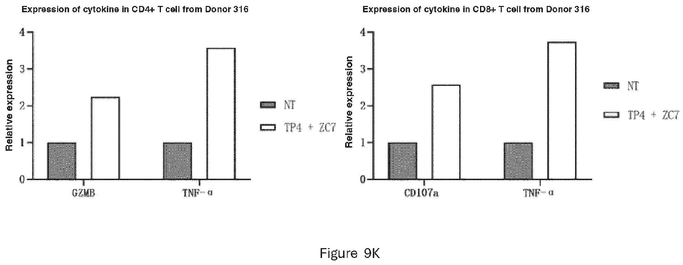

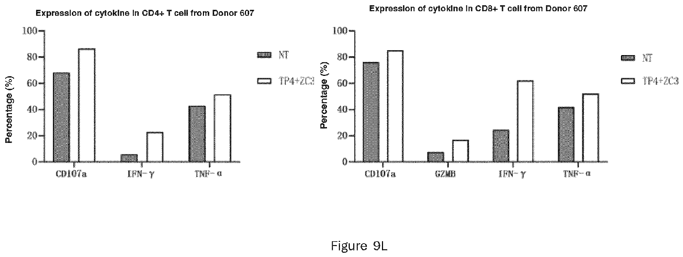

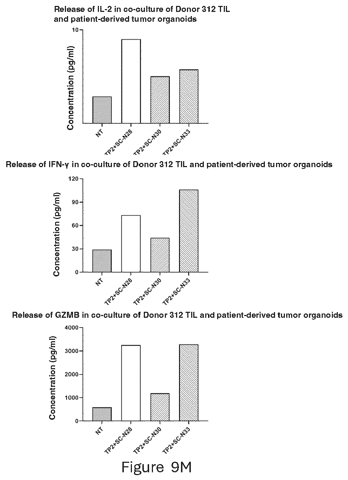

Figures 9A, 9C, 9E, 9G, 9I, and 9L showed that TIL cells after various combination gene edits in the CD3 antibody stimulation group had higher cytokine expression ratios. Figures 9B, 9D, 9F, 9H, 9J, and 9K showed that TIL cells after various combination gene edits in the no-stimulation Medium group had higher cytokine expression ratios. Figure 9M showed that TIL cells genetically edited with the combination of TNFAIP3 and SOCS1 co-cultured with autologous tumor organoids had higher cytokine release capacity. For example, cytokine expression ability included higher IFN-γ expression ability, higher TNF-α expression ability, higher CD107a expression ability, or higher GZMB expression ability. The results indicated that TIL cells edited with the target combination gene of the present invention had higher functional cytokine expression ability.

### (F) Apoptosis Detection

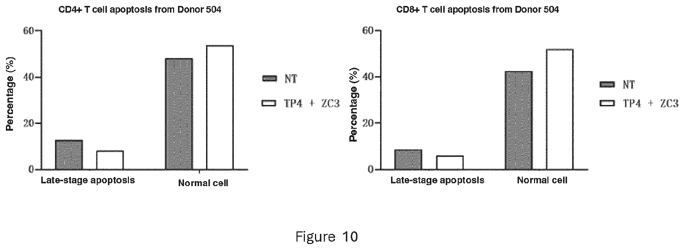

Figure 10 showed the results of apoptosis detection of TIL cells derived from donor 504. The results indicated that TIL cells edited with the target combination gene of the present invention could have a more significant anti-apoptotic ability.

### Example 7 In Vivo Efficacy Testing of Gene-Edited Cells

### (I) In Vivo Effects of Gene-Edited TCR-T Cells of the Present Invention

Immunodeficient mice (NOG mice, Vital River, strain code 408) were subcutaneously inoculated with A375 cells, at an inoculation quantity of approximately 1×10⁶ cells/mouse. Seven days after inoculation, when the tumor volume reached approximately 50-80 mm³, the mice were randomly grouped according to tumor volume based on the following protocol:

| Group | Cell number | IL-2 injection amount (IU) | mice number in each group |
|---|---|---|---|
| Blank control | 0 | - | 6 |
| unmodified cells | 2×10⁷ | 3E5*Bid*3 | 6 |
| SC3+TP4 knockout | 2×10⁷ | 0.6E5*Bid*3 | 6 |
| TP4+CB1 knockout | 2×10⁷ | 0.6E5*Bid*3 | 6 |
| TP4+ZC3 knockout | 2×10⁷ | 0.6E5*Bid*3 | 6 |

The day each group was injected into the tail vein with TCR-T cells gene-edited using the sgRNA numbers provided by the present invention was recorded as day 0. Simultaneously, IL-2 was administered by intraperitoneal injection, once every 12 hours, for 6 consecutive times. The injection dose of IL-2 was 3×10⁵ IU/dose for the NT group and 0.6×10⁵ IU/dose for each experimental group. Tumor volume was measured twice a week, and the tumor growth inhibition (TGI) rate on day 28 was calculated as follows:

| Group | TGI(%) | *P value* |
|---|---|---|
| Blank control | (tumor volume 3156.06 mm³) | - |
| Unmodified cells | 28.66% | 0.024 |
| SC3+TP4 knockout | A | 0.000078 |
| TP4+CB1 knockout | A | 0.00087 |
| TP4+ZC3 knockout | A | 0.000068 |

Data were analyzed using a T-test. Herein, A indicated TGI was greater than or equal to 50%.

### (II) In Vivo Effects of Gene-Edited TILs of the Present Invention

Immunodeficient mice (NOG mice, Vital River, strain code 408) were subcutaneously inoculated with HeyT-30 cells, at an inoculation quantity of approximately 1×10⁶ cells/mouse. Seven days after inoculation, when the tumor volume reached approximately 50-80 mm³, the mice were randomly grouped according to tumor volume based on the following protocol:

| Group | Cell number | IL-2 injection amount (IU) | mice number in each group |
|---|---|---|---|
| Blank control | 0 | - | 6 |
| Unmodified cells | 2.5×10⁷ | 3E5*Bid*3 | 6 |
| SC3+TP4 knockout | 2.5×10⁷ | 0.6E5*Bid*3 | 6 |
| TP4+ZC3 knockout | 2.5×10⁷ | 0.6E5*Bid*3 | 6 |

The day each group was injected into the tail vein with TIL cells gene-edited using the sgRNA numbers provided by the present invention was recorded as day 0. Simultaneously, IL-2 was administered by intraperitoneal injection, once every 12 hours, for 6 consecutive times. The injection dose of IL-2 was 3×10⁵ IU/dose for the NT group and 0.6×10⁵ IU/dose for each experimental group. Tumor volumes were measured twice weekly, and tumor growth inhibition rates were calculated as follows:

| Group | TGI(%) | *P value* |
|---|---|---|
| Blank control | (tumor volume 3682.13 mm³) | - |
| Unmodified cells | 8.11% | 0.262 |
| SC3+TP4 knockout | A | <0.000001 |
| TP4+ZC3 knockout | A | <0.000001 |

Data were analyzed using a T-test. Herein, A indicated that TGI was greater than or equal to 80%. The results showed that the mice in the experimental groups exhibited better tumor control effects and/or in vivo functional cytokine release compared to the NT group, demonstrating that the anti-tumor effect of cells knocked out with sgRNAs provided by the present invention was significantly stronger than that of the NT group.

The foregoing detailed description was provided by way of explanation and example and was not intended to limit the scope of the appended claims. Various modifications to the presently illustrated embodiments of the invention will be apparent to those skilled in the art and remain within the scope of the appended claims and their equivalents.

**Table 1A. Human TNFAIP3 selected target region genomic coordinate**

| Region No. | Subregion coordinate range |
|---|---|
| TNFAIP3 R_1 | chr6: 137864849-137866349 |
| TNFAIP3 R_2 | chr6: 137864869-137865029 |
| TNFAIP3 R_3 | chr6: 137865069-137865129 |
| TNFAIP3 R_4 | chr6: 137865269-137865419 |
| TNFAIP3 R_5 | chr6: 137865469-137865719 |
| TNFAIP3 R_6 | chr6: 137866139-137866199 |
| TNFAIP3 R_7 | chr6: 137866269-137866339 |
| TNFAIP3 R_8 | chr6: 137866349-137866972 |
| TNFAIP3 R_9 | chr6: 137867259-137868241 |
| TNFAIP3 R_10 | chr6: 137870833-137870912 |
| TNFAIP3 R_11 | chr6: 137871035-137871248 |
| TNFAIP3 R_12 | chr6: 137871269-137871361 |
| TNFAIP3 R_13 | chr6: 137874892-137874971 |
| TNFAIP3 R_14 | chr6: 137874992-137875035 |
| TNFAIP3 R_15 | chr6: 137875688-137875730 |
| TNFAIP3 R_16 | chr6: 137875770-137875819 |
| TNFAIP3 R_17 | chr6: 137875996-137876082 |
| TNFAIP3 R_18 | chr6: 137877076-137877172 |
| TNFAIP3 R_19 | chr6: 137877219-137877256 |
| TNFAIP3 R_20 | chr6: 137878467-137878494 |
| TNFAIP3 R_21 | chr6: 137878530-137878570 |
| TNFAIP3 R_22 | chr6: 137878593-137878652 |
| TNFAIP3 R_23 | chr6: 137878673-137878709 |
| TNFAIP3 R_24 | chr6: 137878866-137878900 |
| TNFAIP3 R_25 | chr6: 137878921-137878973 |
| TNFAIP3 R_26 | chr6: 137879022-137879269 |
| TNFAIP3 R_27 | chr6: 137879290-137879351 |
| TNFAIP3 R_28 | chr6: 137880071-137880091 |
| TNFAIP3 R_29 | chr6: 137880153-137880252 |
| TNFAIP3 R_30 | chr6: 137881035-137883312 |

**Table 1B. Human ZC3H12A selected target region genomic coordinate**

| Region No. | Subregion coordinate range |
|---|---|
| ZC3H12A R_1 | chr1: 37473080-37474580 |
| ZC3H12A R_2 | chr1: 37473160-37473310 |
| ZC3H12A R_3 | chr1: 37473540-37473780 |
| ZC3H12A R_4 | chr1: 37473860-37474050 |
| ZC3H12A R_5 | chr1: 37474310-37474560 |
| ZC3H12A R_6 | chr1: 37474580-37474629 |
| ZC3H12A R_7 | chr1: 37475459-37475481 |
| ZC3H12A R_8 | chr1: 37479151-37479925 |
| ZC3H12A R_9 | chr1: 37483639-37484377 |

**Table 1C. Human SOCS1 selected target region genomic coordinate**

| Region No. | Subregion coordinate range |
|---|---|
| SOCS1 R_1 | chr16: 11256204-11257704 |
| SOCS1 R_2 | chr16: 11257484-11257674 |
| SOCS1 R_3 | chr16: 11257124-11257304 |
| SOCS1 R_4 | chr16: 11256774-11257004 |
| SOCS1 R_5 | chr16: 11256664-11256754 |
| SOCS1 R_6 | chr16: 11256434-11256624 |
| SOCS1 R_7 | chr16: 11256214-11256424 |
| SOCS1 R_8 | chr16: 11256079-11256204 |
| SOCS1 R_9 | chr16: 11255501-11255528 |
| SOCS1 R_10 | chr16: 11254417-11254810 |

**Table 1D. Human CBLB selected target region genomic coordinate**

| Region No. | Subregion coordinate range |
|---|---|
| CBLB R_1 | chr3: 105869449-105870949 |
| CBLB R_2 | chr3: 105870699-105870939 |
| CBLB R_3 | chr3: 105870399-105870599 |
| CBLB R_4 | chr3: 105870099-105870299 |
| CBLB R_5 | chr3: 105869879-105870029 |
| CBLB R_6 | chr3: 105869659-105869839 |
| CBLB R_7 | chr3: 105869459-105869619 |
| CBLB R_8 | chr3: 105655461-105659229 |
| CBLB R_9 | chr3: 105670233-105670352 |
| CBLB R_10 | chr3: 105671021-105672462 |
| CBLB R_11 | chr3: 105675760-105675877 |
| CBLB R_12 | chr3: 105678431-105678571 |
| CBLB R_13 | chr3: 105681479-105681610 |
| CBLB R_14 | chr3: 105681724-105681818 |
| CBLB R_15 | chr3: 105685320-105685466 |
| CBLB R_16 | chr3: 105693494-105693540 |
| CBLB R_17 | chr3: 105693561-105693588 |
| CBLB R_18 | chr3: 105702094-105702459 |
| CBLB R_19 | chr3: 105703988-105704173 |
| CBLB R_20 | chr3: 105715200-105715313 |
| CBLB R_21 | chr3: 105720047-105720110 |
| CBLB R_22 | chr3: 105720131-105720159 |
| CBLB R_23 | chr3: 105720180-105720250 |
| CBLB R_24 | chr3: 105724026-105724189 |
| CBLB R_25 | chr3: 105734009-105734140 |
| CBLB R_26 | chr3: 105737171-105737258 |
| CBLB R_27 | chr3: 105740494-105740631 |
| CBLB R_28 | chr3: 105745917-105746038 |
| CBLB R_29 | chr3: 105749622-105749799 |
| CBLB R_30 | chr3: 105751473-105751618 |
| CBLB R_31 | chr3: 105754343-105754474 |
| CBLB R_32 | chr3: 105776396-105776542 |
| CBLB R_33 | chr3: 105824123-105824241 |
| CBLB R_34 | chr3: 105839423-105839470 |
| CBLB R_35 | chr3: 105853441-105853474 |
| CBLB R_36 | chr3: 105853534-105853599 |
| CBLB R_37 | chr3: 105853620-105853664 |
| CBLB R_38 | chr3: 105867469-105867528 |
| CBLB R_39 | chr3: 105867549-105867591 |
| CBLB R_40 | chr3: 105868169-105868381 |
| CBLB R_41 | chr3: 105868532-105868559 |
| CBLB R_42 | chr3: 105868736-105869012 |
| CBLB R_43 | chr3: 105869337-105869401 |
| CBLB R_44 | chr3: 105869425-105869449 |

**Table 2A. Human TNFAIP3 genomic coordinate for multiple knockout**

| Target No. | Target coordinate range | Target No. | Target coordinate range |
|---|---|---|---|
| TNFAIP3 1 | chr6:137883002-137883029 | TNFAIP3 54 | chr6:137881590-137881617 |
| TNFAIP3 2 | chr6:137881684-137881711 | TNFAIP3 55 | chr6:137881895-137881922 |
| TNFAIP3 3 | chr6:137882650-137882677 | TNFAIP3 56 | chr6:137881243-137881270 |
| TNFAIP3 4 | chr6:137882458-137882485 | TNFAIP3 57 | chr6:137881559-137881586 |
| TNFAIP3 5 | chr6:137881743-137881770 | TNFAIP3 58 | chr6:137881328-137881355 |
| TNFAIP3 6 | chr6:137882605-137882632 | TNFAIP3 59 | chr6:137881329-137881356 |
| TNFAIP3 7 | chr6:137881395-137881422 | TNFAIP3 60 | chr6:137882744-137882771 |
| TNFAIP3 8 | chr6:137882435-137882462 | TNFAIP3 61 | chr6:137882697-137882724 |
| TNFAIP3 9 | chr6:137882615-137882642 | TNFAIP3 62 | chr6:137881264-137881291 |
| TNFAIP3 10 | chr6:137881514-137881541 | TNFAIP3 63 | chr6:137881418-137881445 |
| TNFAIP3 11 | chr6:137882632-137882659 | TNFAIP3 64 | chr6:137882604-137882631 |
| TNFAIP3 12 | chr6:137882768-137882795 | TNFAIP3 65 | chr6:137882607-137882634 |
| TNFAIP3 13 | chr6:137881916-137881943 | TNFAIP3 66 | chr6:137881306-137881333 |
| TNFAIP3 14 | chr6:137881145-137881172 | TNFAIP3 67 | chr6:137881802-137881829 |
| TNFAIP3 15 | chr6:137881909-137881936 | TNFAIP3 68 | chr6:137881745-137881772 |
| TNFAIP3 16 | chr6:137881647-137881674 | TNFAIP3 69 | chr6:137881912-137881939 |
| TNFAIP3 17 | chr6:137882639-137882666 | TNFAIP3 70 | chr6:137881149-137881176 |
| TNFAIP3 18 | chr6:137881538-137881565 | TNFAIP3 71 | chr6:137881393-137881420 |
| TNFAIP3 19 | chr6:137881468-137881495 | TNFAIP3 72 | chr6:137881650-137881677 |
| TNFAIP3 20 | chr6:137881307-137881334 | TNFAIP3 73 | chr6:137881661-137881688 |
| TNFAIP3 21 | chr6:137881851-137881878 | TNFAIP3 74 | chr6:137882743-137882770 |
| TNFAIP3 22 | chr6:137881303-137881330 | TNFAIP3 75 | chr6:137882611-137882638 |
| TNFAIP3 23 | chr6:137882387-137882414 | TNFAIP3 76 | chr6:137883012-137883039 |
| TNFAIP3 24 | chr6:137881296-137881323 | TNFAIP3 77 | chr6:137882694-137882721 |
| TNFAIP3 25 | chr6:137882596-137882623 | TNFAIP3 78 | chr6:137881646-137881673 |
| TNFAIP3 26 | chr6:137882476-137882503 | TNFAIP3 79 | chr6:137882640-137882667 |
| TNFAIP3 27 | chr6:137881744-137881771 | TNFAIP3 80 | chr6:137881238-137881265 |
| TNFAIP3 28 | chr6:137881894-137881921 | TNFAIP3 81 | chr6:137882154-137882181 |
| TNFAIP3 29 | chr6:137881150-137881177 | TNFAIP3 82 | chr6:137882415-137882442 |
| TNFAIP3 30 | chr6:137881400-137881427 | TNFAIP3 83 | chr6:137881560-137881587 |
| TNFAIP3 31 | chr6:137881352-137881379 | TNFAIP3 84 | chr6:137882696-137882723 |
| TNFAIP3 32 | chr6:137882609-137882636 | TNFAIP3 85 | chr6:137881660-137881687 |
| TNFAIP3 33 | chr6:137882592-137882619 | TNFAIP3 86 | chr6:137881561-137881588 |
| TNFAIP3 34 | chr6:137882695-137882722 | TNFAIP3 87 | chr6:137881792-137881819 |
| TNFAIP3 35 | chr6:137881793-137881820 | TNFAIP3 88 | chr6:137881239-137881266 |
| TNFAIP3 36 | chr6:137882396-137882423 | TNFAIP3 89 | chr6:137881599-137881626 |
| TNFAIP3 37 | chr6:137881503-137881530 | TNFAIP3 90 | chr6:137881502-137881529 |
| TNFAIP3 38 | chr6:137881597-137881624 | TNFAIP3 91 | chr6:137881598-137881625 |
| TNFAIP3 39 | chr6:137882752-137882779 | TNFAIP3 92 | chr6:137881249-137881276 |
| TNFAIP3 40 | chr6:137883004-137883031 | TNFAIP3 93 | chr6:137878538-137878565 |
| TNFAIP3 41 | chr6:137881683-137881710 | TNFAIP3 94 | chr6:137871496-137871523 |
| TNFAIP3 42 | chr6:137882389-137882416 | TNFAIP3 95 | chr6:137878648-137878675 |
| TNFAIP3 43 | chr6:137881437-137881464 | TNFAIP3 96 | chr6:137871496-137871523 |
| TNFAIP3 44 | chr6:137882899-137882926 | TNFAIP3 97 | chr6:137871480-137871507 |
| TNFAIP3 45 | chr6:137881017-137881044 | TNFAIP3 98 | chr6:137874839-137874866 |
| TNFAIP3 46 | chr6:137882616-137882643 | TNFAIP3 99 | chr6:137878978-137879005 |
| TNFAIP3 47 | chr6:137881467-137881494 | TNFAIP3 100 | chr6:137879017-137879044 |
| TNFAIP3 48 | chr6:137881756-137881783 | TNFAIP3 101 | chr6:137874830-137874857 |
| TNFAIP3 49 | chr6:137881515-137881542 | TNFAIP3 102 | chr6:137877190-137877217 |
| TNFAIP3 50 | chr6:137881237-137881264 | TNFAIP3 103 | chr6:137878939-137878966 |
| TNFAIP3 51 | chr6:137882158-137882185 | TNFAIP3 104 | chr6:137871480-137871507 |
| TNFAIP3 52 | chr6:137882260-137882287 | TNFAIP3 105 | chr6:137871470-137871497 |
| TNFAIP3 53 | chr6:137882753-137882780 | TNFAIP3 106 | chr6:137876099-137876126 |

**Table 2B. Human ZC3H12A genomic coordinate for multiple knockout**

| Target No. | Target coordinate range | Target No. | Target coordinate range |
|---|---|---|---|
| ZC3H12A 1 | chr1:37483906-37483933 | ZC3H12A 35 | chr1:37484203-37484230 |
| ZC3H12A 2 | chr1:37484370-37484397 | ZC3H12A 36 | chr1:37484075-37484102 |
| ZC3H12A 3 | chr1:37484259-37484286 | ZC3H12A 37 | chr1:37484231-37484258 |
| ZC3H12A 4 | chr1:37483753-37483780 | ZC3H12A 38 | chr1:37482827-37482854 |
| ZC3H12A 5 | chr1:37483760-37483787 | ZC3H12A 39 | chr1:37484334-37484361 |
| ZC3H12A 6 | chr1:37484199-37484226 | ZC3H12A 40 | chr1:37483855-37483882 |
| ZC3H12A 7 | chr1:37484369-37484396 | ZC3H12A 41 | chr1:37484238-37484265 |
| ZC3H12A 8 | chr1:37484258-37484285 | ZC3H12A 42 | chr1:37482822-37482849 |
| ZC3H12A 9 | chr1:37483759-37483786 | ZC3H12A 43 | chr1:37482727-37482754 |
| ZC3H12A 10 | chr1:37484237-37484264 | ZC3H12A 44 | chr1:37484252-37484279 |
| ZC3H12A 11 | chr1:37484241-37484268 | ZC3H12A 45 | chr1:37482796-37482823 |
| ZC3H12A 12 | chr1:37483905-37483932 | ZC3H12A 46 | chr1:37484354-37484381 |
| ZC3H12A 13 | chr1:37484257-37484284 | ZC3H12A 47 | chr1:37482710-37482737 |
| ZC3H12A 14 | chr1:37482729-37482756 | ZC3H12A 48 | chr1:37482716-37482743 |
| ZC3H12A 15 | chr1:37482795-37482822 | ZC3H12A 49 | chr1:37484242-37484269 |
| ZC3H12A 16 | chr1:37483748-37483775 | ZC3H12A 50 | chr1:37483908-37483935 |
| ZC3H12A 17 | chr1:37482728-37482755 | ZC3H12A 51 | chr1:37482714-37482741 |
| ZC3H12A 18 | chr1:37483909-37483936 | ZC3H12A 52 | chr1:37483655-37483682 |
| ZC3H12A 19 | chr1:37483793-37483820 | ZC3H12A 53 | chr1:37484164-37484191 |
| ZC3H12A 20 | chr1:37483910-37483937 | ZC3H12A 54 | chr1:37482717-37482744 |
| ZC3H12A 21 | chr1:37483752-37483779 | ZC3H12A 55 | chr1:37484326-37484353 |
| ZC3H12A 22 | chr1:37482797-37482824 | ZC3H12A 56 | chr1:37483915-37483942 |
| ZC3H12A 23 | chr1:37483794-37483821 | ZC3H12A 57 | chr1:37481749-37481776 |
| ZC3H12A 24 | chr1:37484163-37484190 | ZC3H12A 58 | chr1:37481643-37481670 |
| ZC3H12A 25 | chr1:37484331-37484358 | ZC3H12A 59 | chr1:37481774-37481801 |
| ZC3H12A 26 | chr1:37484200-37484227 | ZC3H12A 60 | chr1:37481704-37481731 |
| ZC3H12A 27 | chr1:37482749-37482776 | ZC3H12A 61 | chr1:37481638-37481665 |
| ZC3H12A 28 | chr1:37484073-37484100 | ZC3H12A 62 | chr1:37475770-37475797 |
| ZC3H12A 29 | chr1:37484082-37484109 | ZC3H12A 63 | chr1:37475771-37475798 |
| ZC3H12A 30 | chr1:37482759-37482786 | ZC3H12A 64 | chr1:37475814-37475841 |
| ZC3H12A 31 | chr1:37482765-37482792 | ZC3H12A 65 | chr1:37475804-37475831 |
| ZC3H12A 32 | chr1:37484076-37484103 | ZC3H12A 66 | chr1:37481711-37481738 |
| ZC3H12A 33 | chr1:37482798-37482825 | ZC3H12A 67 | chr1:37481715-37481742 |
| ZC3H12A 34 | chr1:37483675-37483702 | ZC3H12A 68 | chr1:37483342-37483369 |

**Table 2C. Human SOCS1 genomic coordinate for multiple knockout**

| Target No. | Target coordinate range | Target No. | Target coordinate range |
|---|---|---|---|
| SOCS1 1 | chr16:11256040-11256067 | SOCS1 26 | chr16:11255529-11255556 |
| SOCS1 2 | chr16:11256041-11256068 | SOCS1 27 | chr16:11255530-11255557 |
| SOCS1 3 | chr16:11256046-11256073 | SOCS1 28 | chr16:11255531-11255558 |
| SOCS1 4 | chr16:11256055-11256082 | SOCS1 29 | chr16:11255539-11255566 |
| SOCS1 5 | chr16:11256047-11256074 | SOCS1 30 | chr16:11255540-11255567 |
| SOCS1 6 | chr16:11256050-11256077 | SOCS1 31 | chr16:11255541-11255568 |
| SOCS1 7 | chr16:11256051-11256078 | SOCS1 32 | chr16:11255507-11255534 |
| SOCS1 8 | chr16:11256054-11256081 | SOCS1 33 | chr16:11255508-11255535 |
| SOCS1 9 | chr16:11256057-11256084 | SOCS1 34 | chr16:11255569-11255596 |
| SOCS1 10 | chr16:11256062-11256089 | SOCS1 35 | chr16:11255494-11255521 |
| SOCS1 11 | chr16:11256063-11256090 | SOCS1 36 | chr16:11255495-11255522 |
| SOCS1 12 | chr16:11256069-11256096 | SOCS1 37 | chr16:11255496-11255523 |
| SOCS1 13 | chr16:11256070-11256097 | SOCS1 38 | chr16:11255497-11255524 |
| SOCS1 14 | chr16:11256078-11256105 | SOCS1 39 | chr16:11255492-11255519 |
| SOCS1 15 | chr16:11256034-11256061 | SOCS1 40 | chr16:11255496-11255523 |
| SOCS1 16 | chr16:11256035-11256062 | SOCS1 41 | chr16:11255509-11255536 |
| SOCS1 17 | chr16:11256036-11256063 | SOCS1 42 | chr16:11255518-11255545 |
| SOCS1 18 | chr16:11256039-11256066 | SOCS1 43 | chr16:11255287-11255314 |
| SOCS1 19 | chr16:11256040-11256067 | SOCS1 44 | chr16:11255225-11255252 |
| SOCS1 20 | chr16:11256043-11256070 | SOCS1 45 | chr16:11255234-11255261 |
| SOCS1 21 | chr16:11256046-11256073 | SOCS1 46 | chr16:11255235-11255262 |
| SOCS1 22 | chr16:11255545-11255572 | SOCS1 47 | chr16:11255236-11255263 |
| SOCS1 23 | chr16:11255546-11255573 | SOCS1 48 | chr16:11255246-11255273 |
| SOCS1 24 | chr16:11255550-11255577 | SOCS1 49 | chr16:11255460-11255487 |
| SOCS1 25 | chr16:11255521-11255548 | SOCS1 50 | chr16:11255411-11255438 |

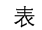 2D Human CBLB genomic coordinate for multiple knockout

| Target No. | Target coordinate range | Target No. | Target coordinate range |
|---|---|---|---|
| CBLB 1 | chr3:105659004-105659031 | CBLB 37 | chr3:105658523-105658550 |
| CBLB 2 | chr3:105656012-105656039 | CBLB 38 | chr3:105656558-105656585 |
| CBLB 3 | chr3:105656625-105656652 | CBLB 39 | chr3:105657893-105657920 |
| CBLB 4 | chr3:105657901-105657928 | CBLB 40 | chr3:105659058-105659085 |
| CBLB 5 | chr3:105658802-105658829 | CBLB 41 | chr3:105658960-105658987 |
| CBLB 6 | chr3:105658456-105658483 | CBLB 42 | chr3:105657111-105657138 |
| CBLB 7 | chr3:105657189-105657216 | CBLB 43 | chr3:105656000-105656027 |
| CBLB 8 | chr3:105657915-105657942 | CBLB 44 | chr3:105657144-105657171 |
| CBLB 9 | chr3:105658570-105658597 | CBLB 45 | chr3:105657102-105657129 |
| CBLB 10 | chr3:105658562-105658589 | CBLB 46 | chr3:105658584-105658611 |
| CBLB 11 | chr3:105658451-105658478 | CBLB 47 | chr3:105658526-105658553 |
| CBLB 12 | chr3:105659074-105659101 | CBLB 48 | chr3:105657847-105657874 |
| CBLB 13 | chr3:105657096-105657123 | CBLB 49 | chr3:105658840-105658867 |
| CBLB 14 | chr3:105656554-105656581 | CBLB 50 | chr3:105655702-105655729 |
| CBLB 15 | chr3:105658839-105658866 | CBLB 51 | chr3:105657258-105657285 |
| CBLB 16 | chr3:105658045-105658072 | CBLB 52 | chr3:105658790-105658817 |
| CBLB 17 | chr3:105656369-105656396 | CBLB 53 | chr3:105658845-105658872 |
| CBLB 18 | chr3:105657033-105657060 | CBLB 54 | chr3:105658192-105658219 |
| CBLB 19 | chr3:105656191-105656218 | CBLB 55 | chr3:105656181-105656208 |
| CBLB 20 | chr3:105658194-105658221 | CBLB 56 | chr3:105657497-105657524 |
| CBLB 21 | chr3:105659003-105659030 | CBLB 57 | chr3:105657501-105657528 |
| CBLB 22 | chr3:105657190-105657217 | CBLB 58 | chr3:105658842-105658869 |
| CBLB 23 | chr3:105658561-105658588 | CBLB 59 | chr3:105657035-105657062 |
| CBLB 24 | chr3:105657254-105657281 | CBLB 60 | chr3:105658527-105658554 |
| CBLB 25 | chr3:105657856-105657883 | CBLB 61 | chr3:105656621-105656648 |
| CBLB 26 | chr3:105656182-105656209 | CBLB 62 | chr3:105655999-105656026 |
| CBLB 27 | chr3:105657914-105657941 | CBLB 63 | chr3:105656201-105656228 |
| CBLB 28 | chr3:105656622-105656649 | CBLB 64 | chr3:105658575-105658602 |
| CBLB 29 | chr3:105658140-105658167 | CBLB 65 | chr3:105657038-105657065 |
| CBLB 30 | chr3:105655949-105655976 | CBLB 66 | chr3:105658172-105658199 |
| CBLB 31 | chr3:105657184-105657211 | CBLB 67 | chr3:105658972-105658999 |
| CBLB 32 | chr3:105658450-105658477 | CBLB 68 | chr3:105658040-105658067 |
| CBLB 33 | chr3:105657034-105657061 | CBLB 69 | chr3:105658171-105658198 |
| CBLB 34 | chr3:105658991-105659018 | CBLB 70 | chr3:105746011-105746038 |
| CBLB 35 | chr3:105658994-105659021 | CBLB 71 | chr3:105751603-105751630 |
| CBLB 36 | chr3:105657095-105657122 | | |

**Table 3A. Human TNFAIP3 genomic coordinate of the present invention**

| No. | Chromosomal coordinate range | No. | Chromosomal coordinate range |
|---|---|---|---|
| TP sk 1 | chr6:137864855-137864882 | TP sk 486 | chr6:137868162-137868189 |
| TP sk 2 | chr6:137864856-137864883 | TP sk 487 | chr6:137868163-137868190 |
| TP sk 3 | chr6:137864857-137864884 | TP sk 488 | chr6:137868164-137868191 |
| TP sk 4 | chr6:137864863-137864890 | TP sk 489 | chr6:137868189-137868216 |
| TP sk 5 | chr6:137864872-137864899 | TP sk 490 | chr6:137868198-137868225 |
| TP sk 6 | chr6:137864895-137864922 | TP sk 491 | chr6:137868199-137868226 |
| TP sk 7 | chr6:137864910-137864937 | TP sk 492 | chr6:137868205-137868232 |
| TP sk 8 | chr6:137864928-137864955 | TP sk 493 | chr6:137868208-137868235 |
| TP sk 9 | chr6:137864931-137864958 | TP sk 494 | chr6:137868209-137868236 |
| TP sk 10 | chr6:137864940-137864967 | TP sk 495 | chr6:137868212-137868239 |
| TP sk 11 | chr6:137864959-137864986 | TP sk 496 | chr6:137868217-137868244 |
| TP sk 12 | chr6:137864960-137864987 | TP sk 497 | chr6:137868220-137868247 |
| TP sk 13 | chr6:137864961-137864988 | TP sk 498 | chr6:137868224-137868251 |
| TP sk 14 | chr6:137864965-137864992 | TP sk 499 | chr6:137870815-137870842 |
| TP sk 15 | chr6:137864967-137864994 | TP sk 500 | chr6:137870818-137870845 |
| TP sk 16 | chr6:137864972-137864999 | TP sk 501 | chr6:137870819-137870846 |
| TP sk 17 | chr6:137864973-137865000 | TP sk 502 | chr6:137870822-137870849 |
| TP sk 18 | chr6:137865003-137865030 | TP sk 503 | chr6:137870823-137870850 |
| TP sk 19 | chr6:137865006-137865033 | TP sk 504 | chr6:137870824-137870851 |
| TP sk 20 | chr6:137865007-137865034 | TP sk 505 | chr6:137870825-137870852 |
| TP sk 21 | chr6:137865011-137865038 | TP sk 506 | chr6:137870831-137870858 |
| TP sk 22 | chr6:137865029-137865056 | TP sk 507 | chr6:137870838-137870865 |
| TP sk 23 | chr6:137865032-137865059 | TP sk 508 | chr6:137870839-137870866 |
| TP sk 24 | chr6:137865035-137865062 | TP sk 509 | chr6:137870850-137870877 |
| TP sk 25 | chr6:137865036-137865063 | TP sk 510 | chr6:137870851-137870878 |
| TP sk 26 | chr6:137865041-137865068 | TP sk 511 | chr6:137870859-137870886 |
| TP sk 27 | chr6:137865049-137865076 | TP sk 512 | chr6:137870885-137870912 |
| TP sk 28 | chr6:137865052-137865079 | TP sk 513 | chr6:137870886-137870913 |
| TP sk 29 | chr6:137865071-137865098 | TP sk 514 | chr6:137870890-137870917 |
| TP sk 30 | chr6:137865078-137865105 | TP sk 515 | chr6:137870894-137870921 |
| TP sk 31 | chr6:137865080-137865107 | TP sk 516 | chr6:137871026-137871053 |
| TP sk 32 | chr6:137865081-137865108 | TP sk 517 | chr6:137871027-137871054 |
| TP sk 33 | chr6:137865097-137865124 | TP sk 518 | chr6:137871028-137871055 |
| TP sk 34 | chr6:137865098-137865125 | TP sk 519 | chr6:137871033-137871060 |
| TP sk 35 | chr6:137865120-137865147 | TP sk 520 | chr6:137871044-137871071 |
| TP sk 36 | chr6:137865133-137865160 | TP sk 521 | chr6:137871045-137871072 |
| TP sk 37 | chr6:137865138-137865165 | TP sk 522 | chr6:137871053-137871080 |
| TP sk 38 | chr6:137865148-137865175 | TP sk 523 | chr6:137871056-137871083 |
| TP sk 39 | chr6:137865152-137865179 | TP sk 524 | chr6:137871067-137871094 |
| TP sk 40 | chr6:137865158-137865185 | TP sk 525 | chr6:137871068-137871095 |
| TP sk 41 | chr6:137865171-137865198 | TP sk 526 | chr6:137871081-137871108 |
| TP sk 42 | chr6:137865178-137865205 | TP sk 527 | chr6:137871082-137871109 |
| TP sk 43 | chr6:137865186-137865213 | TP sk 528 | chr6:137871096-137871123 |
| TP sk 44 | chr6:137865192-137865219 | TP sk 529 | chr6:137871104-137871131 |
| TP sk 45 | chr6:137865196-137865223 | TP sk 530 | chr6:137871105-137871132 |
| TP sk 46 | chr6:137865207-137865234 | TP sk 531 | chr6:137871106-137871133 |
| TP sk 47 | chr6:137865216-137865243 | TP sk 532 | chr6:137871117-137871144 |
| TP sk 48 | chr6:137865235-137865262 | TP sk 533 | chr6:137871129-137871156 |
| TP sk 49 | chr6:137865236-137865263 | TP sk 534 | chr6:137871137-137871164 |
| TP sk 50 | chr6:137865237-137865264 | TP sk 535 | chr6:137871147-137871174 |
| TP sk 51 | chr6:137865253-137865280 | TP sk 536 | chr6:137871159-137871186 |
| TP sk 52 | chr6:137865268-137865295 | TP sk 537 | chr6:137871186-137871213 |
| TP sk 53 | chr6:137865269-137865296 | TP sk 538 | chr6:137871192-137871219 |
| TP sk 54 | chr6:137865282-137865309 | TP sk 539 | chr6:137871199-137871226 |
| TP sk 55 | chr6:137865283-137865310 | TP sk 540 | chr6:137871204-137871231 |
| TP sk 56 | chr6:137865284-137865311 | TP sk 541 | chr6:137871266-137871293 |
| TP sk 57 | chr6:137865290-137865317 | TP sk 542 | chr6:137871267-137871294 |
| TP sk 58 | chr6:137865300-137865327 | TP sk 543 | chr6:137871303-137871330 |
| TP sk 59 | chr6:137865301-137865328 | TP sk 544 | chr6:137871304-137871331 |
| TP sk 60 | chr6:137865303-137865330 | TP sk 545 | chr6:137871305-137871332 |
| TP sk 61 | chr6:137865316-137865343 | TP sk 546 | chr6:137871321-137871348 |
| TP sk 62 | chr6:137865332-137865359 | TP sk 547 | chr6:137871470-137871497 |
| TP sk 63 | chr6:137865339-137865366 | TP sk 548 | chr6:137874883-137874910 |
| TP sk 64 | chr6:137865346-137865373 | TP sk 549 | chr6:137874891-137874918 |
| TP sk 65 | chr6:137865358-137865385 | TP sk 550 | chr6:137874895-137874922 |
| TP sk 66 | chr6:137865367-137865394 | TP sk 551 | chr6:137874916-137874943 |
| TP sk 67 | chr6:137865370-137865397 | TP sk 552 | chr6:137874973-137875000 |
| TP sk 68 | chr6:137865377-137865404 | TP sk 553 | chr6:137874988-137875015 |
| TP sk 69 | chr6:137865384-137865411 | TP sk 554 | chr6:137874989-137875016 |
| TP sk 70 | chr6:137865390-137865417 | TP sk 555 | chr6:137874990-137875017 |
| TP sk 71 | chr6:137865402-137865429 | TP sk 556 | chr6:137875008-137875035 |
| TP sk 72 | chr6:137865403-137865430 | TP sk 557 | chr6:137875009-137875036 |
| TP sk 73 | chr6:137865421-137865448 | TP sk 558 | chr6:137875013-137875040 |
| TP sk 74 | chr6:137865450-137865477 | TP sk 559 | chr6:137875666-137875693 |
| TP sk 75 | chr6:137865453-137865480 | TP sk 560 | chr6:137875673-137875700 |
| TP sk 76 | chr6:137865457-137865484 | TP sk 561 | chr6:137875674-137875701 |
| TP sk 77 | chr6:137865464-137865491 | TP sk 562 | chr6:137875675-137875702 |
| TP sk 78 | chr6:137865472-137865499 | TP sk 563 | chr6:137875678-137875705 |
| TP sk 79 | chr6:137865473-137865500 | TP sk 564 | chr6:137875679-137875706 |
| TP sk 80 | chr6:137865484-137865511 | TP sk 565 | chr6:137875680-137875707 |
| TP sk 81 | chr6:137865497-137865524 | TP sk 566 | chr6:137875681-137875708 |
| TP sk 82 | chr6:137865503-137865530 | TP sk 567 | chr6:137875682-137875709 |
| TP sk 83 | chr6:137865504-137865531 | TP sk 568 | chr6:137875697-137875724 |
| TP sk 84 | chr6:137865509-137865536 | TP sk 569 | chr6:137875789-137875816 |
| TP sk 85 | chr6:137865511-137865538 | TP sk 570 | chr6:137875830-137875857 |
| TP sk 86 | chr6:137865520-137865547 | TP sk 571 | chr6:137875975-137876002 |
| TP sk 87 | chr6:137865533-137865560 | TP sk 572 | chr6:137875989-137876016 |
| TP sk 88 | chr6:137865537-137865564 | TP sk 573 | chr6:137875996-137876023 |
| TP sk 89 | chr6:137865538-137865565 | TP sk 574 | chr6:137876022-137876049 |
| TP sk 90 | chr6:137865545-137865572 | TP sk 575 | chr6:137876023-137876050 |
| TP sk 91 | chr6:137865572-137865599 | TP sk 576 | chr6:137876026-137876053 |
| TP sk 92 | chr6:137865576-137865603 | TP sk 577 | chr6:137876035-137876062 |
| TP sk 93 | chr6:137865587-137865614 | TP sk 578 | chr6:137876036-137876063 |
| TP sk 94 | chr6:137865592-137865619 | TP sk 579 | chr6:137876037-137876064 |
| TP sk 95 | chr6:137865606-137865633 | TP sk 580 | chr6:137876048-137876075 |
| TP sk 96 | chr6:137865615-137865642 | TP sk 581 | chr6:137876146-137876173 |
| TP sk 97 | chr6:137865616-137865643 | TP sk 582 | chr6:137876147-137876174 |
| TP sk 98 | chr6:137865622-137865649 | TP sk 583 | chr6:137876163-137876190 |
| TP sk 99 | chr6:137865633-137865660 | TP sk 584 | chr6:137877070-137877097 |
| TP sk 100 | chr6:137865647-137865674 | TP sk 585 | chr6:137877080-137877107 |
| TP sk 101 | chr6:137865665-137865692 | TP sk 586 | chr6:137877083-137877110 |
| TP sk 102 | chr6:137865668-137865695 | TP sk 587 | chr6:137877084-137877111 |
| TP sk 103 | chr6:137865671-137865698 | TP sk 588 | chr6:137877085-137877112 |
| TP sk 104 | chr6:137865678-137865705 | TP sk 589 | chr6:137877090-137877117 |
| TP sk 105 | chr6:137865679-137865706 | TP sk 590 | chr6:137877107-137877134 |
| TP sk 106 | chr6:137865683-137865710 | TP sk 591 | chr6:137877141-137877168 |
| TP sk 107 | chr6:137865684-137865711 | TP sk 592 | chr6:137877202-137877229 |
| TP sk 108 | chr6:137865685-137865712 | TP sk 593 | chr6:137877206-137877233 |
| TP sk 109 | chr6:137865713-137865740 | TP sk 594 | chr6:137877207-137877234 |
| TP sk 110 | chr6:137865728-137865755 | TP sk 595 | chr6:137877208-137877235 |
| TP sk 111 | chr6:137865783-137865810 | TP sk 596 | chr6:137877212-137877239 |
| TP sk 112 | chr6:137865787-137865814 | TP sk 597 | chr6:137877224-137877251 |
| TP sk 113 | chr6:137865788-137865815 | TP sk 598 | chr6:137877250-137877277 |
| TP sk 114 | chr6:137865795-137865822 | TP sk 599 | chr6:137878405-137878432 |
| TP sk 115 | chr6:137865805-137865832 | TP sk 600 | chr6:137878409-137878436 |
| TP sk 116 | chr6:137865806-137865833 | TP sk 601 | chr6:137878417-137878444 |
| TP sk 117 | chr6:137865809-137865836 | TP sk 602 | chr6:137878451-137878478 |
| TP sk 118 | chr6:137865814-137865841 | TP sk 603 | chr6:137878517-137878544 |
| TP sk 119 | chr6:137865824-137865851 | TP sk 604 | chr6:137878518-137878545 |
| TP sk 120 | chr6:137865849-137865876 | TP sk 605 | chr6:137878519-137878546 |
| TP sk 121 | chr6:137865866-137865893 | TP sk 606 | chr6:137878538-137878565 |
| TP sk 122 | chr6:137865881-137865908 | TP sk 607 | chr6:137878577-137878604 |
| TP sk 123 | chr6:137865886-137865913 | TP sk 608 | chr6:137878578-137878605 |
| TP sk 124 | chr6:137865892-137865919 | TP sk 609 | chr6:137878579-137878606 |
| TP sk 125 | chr6:137865911-137865938 | TP sk 610 | chr6:137878613-137878640 |
| TP sk 126 | chr6:137865926-137865953 | TP sk 611 | chr6:137878614-137878641 |
| TP sk 127 | chr6:137865928-137865955 | TP sk 612 | chr6:137878621-137878648 |
| TP sk 128 | chr6:137865932-137865959 | TP sk 613 | chr6:137878622-137878649 |
| TP sk 129 | chr6:137865943-137865970 | TP sk 614 | chr6:137878623-137878650 |
| TP sk 130 | chr6:137865955-137865982 | TP sk 615 | chr6:137878657-137878684 |
| TP sk 131 | chr6:137865971-137865998 | TP sk 616 | chr6:137878855-137878882 |
| TP sk 132 | chr6:137865990-137866017 | TP sk 617 | chr6:137878863-137878890 |
| TP sk 133 | chr6:137866005-137866032 | TP sk 618 | chr6:137878869-137878896 |
| TP sk 134 | chr6:137866016-137866043 | TP sk 619 | chr6:137878924-137878951 |
| TP sk 135 | chr6:137866019-137866046 | TP sk 620 | chr6:137878939-137878966 |
| TP sk 136 | chr6:137866020-137866047 | TP sk 621 | chr6:137879008-137879035 |
| TP sk 137 | chr6:137866023-137866050 | TP sk 622 | chr6:137879013-137879040 |
| TP sk 138 | chr6:137866027-137866054 | TP sk 623 | chr6:137879017-137879044 |
| TP sk 139 | chr6:137866031-137866058 | TP sk 624 | chr6:137879019-137879046 |
| TP sk 140 | chr6:137866032-137866059 | TP sk 625 | chr6:137879020-137879047 |
| TP sk 141 | chr6:137866033-137866060 | TP sk 626 | chr6:137879031-137879058 |
| TP sk 142 | chr6:137866034-137866061 | TP sk 627 | chr6:137879044-137879071 |
| TP sk 143 | chr6:137866035-137866062 | TP sk 628 | chr6:137879057-137879084 |
| TP sk 144 | chr6:137866037-137866064 | TP sk 629 | chr6:137879085-137879112 |
| TP sk 145 | chr6:137866038-137866065 | TP sk 630 | chr6:137879088-137879115 |
| TP sk 146 | chr6:137866039-137866066 | TP sk 631 | chr6:137879091-137879118 |
| TP sk 147 | chr6:137866040-137866067 | TP sk 632 | chr6:137879095-137879122 |
| TP sk 148 | chr6:137866042-137866069 | TP sk 633 | chr6:137879103-137879130 |
| TP sk 149 | chr6:137866045-137866072 | TP sk 634 | chr6:137879107-137879134 |
| TP sk 150 | chr6:137866054-137866081 | TP sk 635 | chr6:137879110-137879137 |
| TP sk 151 | chr6:137866078-137866105 | TP sk 636 | chr6:137879111-137879138 |
| TP sk 152 | chr6:137866092-137866119 | TP sk 637 | chr6:137879114-137879141 |
| TP sk 153 | chr6:137866106-137866133 | TP sk 638 | chr6:137879118-137879145 |
| TP sk 154 | chr6:137866108-137866135 | TP sk 639 | chr6:137879125-137879152 |
| TP sk 155 | chr6:137866109-137866136 | TP sk 640 | chr6:137879133-137879160 |
| TP sk 156 | chr6:137866110-137866137 | TP sk 641 | chr6:137879134-137879161 |
| TP sk 157 | chr6:137866137-137866164 | TP sk 642 | chr6:137879144-137879171 |
| TP sk 158 | chr6:137866139-137866166 | TP sk 643 | chr6:137879145-137879172 |
| TP sk 159 | chr6:137866157-137866184 | TP sk 644 | chr6:137879158-137879185 |
| TP sk 160 | chr6:137866161-137866188 | TP sk 645 | chr6:137879164-137879191 |
| TP sk 161 | chr6:137866172-137866199 | TP sk 646 | chr6:137879165-137879192 |
| TP sk 162 | chr6:137866173-137866200 | TP sk 647 | chr6:137879166-137879193 |
| TP sk 163 | chr6:137866176-137866203 | TP sk 648 | chr6:137879169-137879196 |
| TP sk 164 | chr6:137866188-137866215 | TP sk 649 | chr6:137879170-137879197 |
| TP sk 165 | chr6:137866189-137866216 | TP sk 650 | chr6:137879171-137879198 |
| TP sk 166 | chr6:137866193-137866220 | TP sk 651 | chr6:137879182-137879209 |
| TP sk 167 | chr6:137866202-137866229 | TP sk 652 | chr6:137879184-137879211 |
| TP sk 168 | chr6:137866203-137866230 | TP sk 653 | chr6:137879202-137879229 |
| TP sk 169 | chr6:137866236-137866263 | TP sk 654 | chr6:137879203-137879230 |
| TP sk 170 | chr6:137866237-137866264 | TP sk 655 | chr6:137879204-137879231 |
| TP sk 171 | chr6:137866246-137866273 | TP sk 656 | chr6:137879206-137879233 |
| TP sk 172 | chr6:137866249-137866276 | TP sk 657 | chr6:137879214-137879241 |
| TP sk 173 | chr6:137866250-137866277 | TP sk 658 | chr6:137879215-137879242 |
| TP sk 174 | chr6:137866254-137866281 | TP sk 659 | chr6:137879216-137879243 |
| TP sk 175 | chr6:137866255-137866282 | TP sk 660 | chr6:137879221-137879248 |
| TP sk 176 | chr6:137866256-137866283 | TP sk 661 | chr6:137879225-137879252 |
| TP sk 177 | chr6:137866257-137866284 | TP sk 662 | chr6:137879226-137879253 |
| TP sk 178 | chr6:137866261-137866288 | TP sk 663 | chr6:137879227-137879254 |
| TP sk 179 | chr6:137866267-137866294 | TP sk 664 | chr6:137879237-137879264 |
| TP sk 180 | chr6:137866268-137866295 | TP sk 665 | chr6:137879274-137879301 |
| TP sk 181 | chr6:137866269-137866296 | TP sk 666 | chr6:137879282-137879309 |
| TP sk 182 | chr6:137866270-137866297 | TP sk 667 | chr6:137879283-137879310 |
| TP sk 183 | chr6:137866276-137866303 | TP sk 668 | chr6:137879297-137879324 |
| TP sk 184 | chr6:137866277-137866304 | TP sk 669 | chr6:137879337-137879364 |
| TP sk 185 | chr6:137866291-137866318 | TP sk 670 | chr6:137880057-137880084 |
| TP sk 186 | chr6:137866297-137866324 | TP sk 671 | chr6:137880062-137880089 |
| TP sk 187 | chr6:137866302-137866329 | TP sk 672 | chr6:137880063-137880090 |
| TP sk 188 | chr6:137866303-137866330 | TP sk 673 | chr6:137880134-137880161 |
| TP sk 189 | chr6:137866304-137866331 | TP sk 674 | chr6:137880135-137880162 |
| TP sk 190 | chr6:137866305-137866332 | TP sk 675 | chr6:137880152-137880179 |
| TP sk 191 | chr6:137866309-137866336 | TP sk 676 | chr6:137880155-137880182 |
| TP sk 192 | chr6:137866310-137866337 | TP sk 677 | chr6:137880156-137880183 |
| TP sk 193 | chr6:137866313-137866340 | TP sk 678 | chr6:137880167-137880194 |
| TP sk 194 | chr6:137866314-137866341 | TP sk 679 | chr6:137880186-137880213 |
| TP sk 195 | chr6:137866347-137866374 | TP sk 680 | chr6:137880202-137880229 |
| TP sk 196 | chr6:137866348-137866375 | TP sk 681 | chr6:137880210-137880237 |
| TP sk 197 | chr6:137866349-137866376 | TP sk 682 | chr6:137880226-137880253 |
| TP sk 198 | chr6:137866360-137866387 | TP sk 683 | chr6:137880230-137880257 |
| TP sk 199 | chr6:137866365-137866392 | TP sk 684 | chr6:137880238-137880265 |
| TP sk 200 | chr6:137866369-137866396 | TP sk 685 | chr6:137880241-137880268 |
| TP sk 201 | chr6:137866370-137866397 | TP sk 686 | chr6:137881017-137881044 |
| TP sk 202 | chr6:137866396-137866423 | TP sk 687 | chr6:137881027-137881054 |
| TP sk 203 | chr6:137866406-137866433 | TP sk 688 | chr6:137881043-137881070 |
| TP sk 204 | chr6:137866408-137866435 | TP sk 689 | chr6:137881058-137881085 |
| TP sk 205 | chr6:137866418-137866445 | TP sk 690 | chr6:137881059-137881086 |
| TP sk 206 | chr6:137866421-137866448 | TP sk 691 | chr6:137881066-137881093 |
| TP sk 207 | chr6:137866440-137866467 | TP sk 692 | chr6:137881073-137881100 |
| TP sk 208 | chr6:137866473-137866500 | TP sk 693 | chr6:137881074-137881101 |
| TP sk 209 | chr6:137866481-137866508 | TP sk 694 | chr6:137881078-137881105 |
| TP sk 210 | chr6:137866500-137866527 | TP sk 695 | chr6:137881086-137881113 |
| TP sk 211 | chr6:137866501-137866528 | TP sk 696 | chr6:137881087-137881114 |
| TP sk 212 | chr6:137866502-137866529 | TP sk 697 | chr6:137881095-137881122 |
| TP sk 213 | chr6:137866508-137866535 | TP sk 698 | chr6:137881096-137881123 |
| TP sk 214 | chr6:137866519-137866546 | TP sk 699 | chr6:137881097-137881124 |
| TP sk 215 | chr6:137866525-137866552 | TP sk 700 | chr6:137881102-137881129 |
| TP sk 216 | chr6:137866526-137866553 | TP sk 701 | chr6:137881105-137881132 |
| TP sk 217 | chr6:137866527-137866554 | TP sk 702 | chr6:137881110-137881137 |
| TP sk 218 | chr6:137866565-137866592 | TP sk 703 | chr6:137881118-137881145 |
| TP sk 219 | chr6:137866566-137866593 | TP sk 704 | chr6:137881122-137881149 |
| TP sk 220 | chr6:137866567-137866594 | TP sk 705 | chr6:137881123-137881150 |
| TP sk 221 | chr6:137866572-137866599 | TP sk 706 | chr6:137881127-137881154 |
| TP sk 222 | chr6:137866573-137866600 | TP sk 707 | chr6:137881145-137881172 |
| TP sk 223 | chr6:137866574-137866601 | TP sk 708 | chr6:137881149-137881176 |
| TP sk 224 | chr6:137866575-137866602 | TP sk 709 | chr6:137881150-137881177 |
| TP sk 225 | chr6:137866579-137866606 | TP sk 710 | chr6:137881156-137881183 |
| TP sk 226 | chr6:137866580-137866607 | TP sk 711 | chr6:137881157-137881184 |
| TP sk 227 | chr6:137866582-137866609 | TP sk 712 | chr6:137881158-137881185 |
| TP sk 228 | chr6:137866590-137866617 | TP sk 713 | chr6:137881161-137881188 |
| TP sk 229 | chr6:137866591-137866618 | TP sk 714 | chr6:137881162-137881189 |
| TP sk 230 | chr6:137866595-137866622 | TP sk 715 | chr6:137881166-137881193 |
| TP sk 231 | chr6:137866611-137866638 | TP sk 716 | chr6:137881167-137881194 |
| TP sk 232 | chr6:137866612-137866639 | TP sk 717 | chr6:137881168-137881195 |
| TP sk 233 | chr6:137866613-137866640 | TP sk 718 | chr6:137881178-137881205 |
| TP sk 234 | chr6:137866614-137866641 | TP sk 719 | chr6:137881179-137881206 |
| TP sk 235 | chr6:137866615-137866642 | TP sk 720 | chr6:137881186-137881213 |
| TP sk 236 | chr6:137866620-137866647 | TP sk 721 | chr6:137881187-137881214 |
| TP sk 237 | chr6:137866626-137866653 | TP sk 722 | chr6:137881190-137881217 |
| TP sk 238 | chr6:137866630-137866657 | TP sk 723 | chr6:137881200-137881227 |
| TP sk 239 | chr6:137866636-137866663 | TP sk 724 | chr6:137881204-137881231 |
| TP sk 240 | chr6:137866638-137866665 | TP sk 725 | chr6:137881205-137881232 |
| TP sk 241 | chr6:137866644-137866671 | TP sk 726 | chr6:137881206-137881233 |
| TP sk 242 | chr6:137866645-137866672 | TP sk 727 | chr6:137881207-137881234 |
| TP sk 243 | chr6:137866646-137866673 | TP sk 728 | chr6:137881208-137881235 |
| TP sk 244 | chr6:137866654-137866681 | TP sk 729 | chr6:137881209-137881236 |
| TP sk 245 | chr6:137866675-137866702 | TP sk 730 | chr6:137881214-137881241 |
| TP sk 246 | chr6:137866688-137866715 | TP sk 731 | chr6:137881215-137881242 |
| TP sk 247 | chr6:137866690-137866717 | TP sk 732 | chr6:137881216-137881243 |
| TP sk 248 | chr6:137866691-137866718 | TP sk 733 | chr6:137881217-137881244 |
| TP sk 249 | chr6:137866701-137866728 | TP sk 734 | chr6:137881218-137881245 |
| TP sk 250 | chr6:137866702-137866729 | TP sk 735 | chr6:137881219-137881246 |
| TP sk 251 | chr6:137866703-137866730 | TP sk 736 | chr6:137881231-137881258 |
| TP sk 252 | chr6:137866709-137866736 | TP sk 737 | chr6:137881232-137881259 |
| TP sk 253 | chr6:137866710-137866737 | TP sk 738 | chr6:137881237-137881264 |
| TP sk 254 | chr6:137866713-137866740 | TP sk 739 | chr6:137881238-137881265 |
| TP sk 255 | chr6:137866714-137866741 | TP sk 740 | chr6:137881239-137881266 |
| TP sk 256 | chr6:137866720-137866747 | TP sk 741 | chr6:137881240-137881267 |
| TP sk 257 | chr6:137866733-137866760 | TP sk 742 | chr6:137881243-137881270 |
| TP sk 258 | chr6:137866734-137866761 | TP sk 743 | chr6:137881249-137881276 |
| TP sk 259 | chr6:137866737-137866764 | TP sk 744 | chr6:137881264-137881291 |
| TP sk 260 | chr6:137866753-137866780 | TP sk 745 | chr6:137881288-137881315 |
| TP sk 261 | chr6:137866760-137866787 | TP sk 746 | chr6:137881296-137881323 |
| TP sk 262 | chr6:137866761-137866788 | TP sk 747 | chr6:137881303-137881330 |
| TP sk 263 | chr6:137866762-137866789 | TP sk 748 | chr6:137881306-137881333 |
| TP sk 264 | chr6:137866767-137866794 | TP sk 749 | chr6:137881307-137881334 |
| TP sk 265 | chr6:137866768-137866795 | TP sk 750 | chr6:137881320-137881347 |
| TP sk 266 | chr6:137866773-137866800 | TP sk 751 | chr6:137881327-137881354 |
| TP sk 267 | chr6:137866774-137866801 | TP sk 752 | chr6:137881328-137881355 |
| TP sk 268 | chr6:137866777-137866804 | TP sk 753 | chr6:137881329-137881356 |
| TP sk 269 | chr6:137866781-137866808 | TP sk 754 | chr6:137881338-137881365 |
| TP sk 270 | chr6:137866782-137866809 | TP sk 755 | chr6:137881341-137881368 |
| TP sk 271 | chr6:137866785-137866812 | TP sk 756 | chr6:137881352-137881379 |
| TP sk 272 | chr6:137866788-137866815 | TP sk 757 | chr6:137881357-137881384 |
| TP sk 273 | chr6:137866793-137866820 | TP sk 758 | chr6:137881386-137881413 |
| TP sk 274 | chr6:137866794-137866821 | TP sk 759 | chr6:137881392-137881419 |
| TP sk 275 | chr6:137866796-137866823 | TP sk 760 | chr6:137881393-137881420 |
| TP sk 276 | chr6:137866797-137866824 | TP sk 761 | chr6:137881394-137881421 |
| TP sk 277 | chr6:137866804-137866831 | TP sk 762 | chr6:137881395-137881422 |
| TP sk 278 | chr6:137866841-137866868 | TP sk 763 | chr6:137881396-137881423 |
| TP sk 279 | chr6:137866842-137866869 | TP sk 764 | chr6:137881400-137881427 |
| TP sk 280 | chr6:137866854-137866881 | TP sk 765 | chr6:137881401-137881428 |
| TP sk 281 | chr6:137866898-137866925 | TP sk 766 | chr6:137881405-137881432 |
| TP sk 282 | chr6:137866910-137866937 | TP sk 767 | chr6:137881413-137881440 |
| TP sk 283 | chr6:137866912-137866939 | TP sk 768 | chr6:137881418-137881445 |
| TP sk 284 | chr6:137866916-137866943 | TP sk 769 | chr6:137881437-137881464 |
| TP sk 285 | chr6:137866917-137866944 | TP sk 770 | chr6:137881454-137881481 |
| TP sk 286 | chr6:137866923-137866950 | TP sk 771 | chr6:137881459-137881486 |
| TP sk 287 | chr6:137866932-137866959 | TP sk 772 | chr6:137881467-137881494 |
| TP sk 288 | chr6:137866946-137866973 | TP sk 773 | chr6:137881468-137881495 |
| TP sk 289 | chr6:137866951-137866978 | TP sk 774 | chr6:137881480-137881507 |
| TP sk 290 | chr6:137866952-137866979 | TP sk 775 | chr6:137881483-137881510 |
| TP sk 291 | chr6:137866953-137866980 | TP sk 776 | chr6:137881490-137881517 |
| TP sk 292 | chr6:137866958-137866985 | TP sk 777 | chr6:137881491-137881518 |
| TP sk 293 | chr6:137866959-137866986 | TP sk 778 | chr6:137881502-137881529 |
| TP sk 294 | chr6:137866960-137866987 | TP sk 779 | chr6:137881503-137881530 |
| TP sk 295 | chr6:137866968-137866995 | TP sk 780 | chr6:137881509-137881536 |
| TP sk 296 | chr6:137866969-137866996 | TP sk 781 | chr6:137881511-137881538 |
| TP sk 297 | chr6:137866970-137866997 | TP sk 782 | chr6:137881514-137881541 |
| TP sk 298 | chr6:137866971-137866998 | TP sk 783 | chr6:137881515-137881542 |
| TP sk 299 | chr6:137867251-137867278 | TP sk 784 | chr6:137881538-137881565 |
| TP sk 300 | chr6:137867254-137867281 | TP sk 785 | chr6:137881559-137881586 |
| TP sk 301 | chr6:137867255-137867282 | TP sk 786 | chr6:137881560-137881587 |
| TP sk 302 | chr6:137867269-137867296 | TP sk 787 | chr6:137881561-137881588 |
| TP sk 303 | chr6:137867278-137867305 | TP sk 788 | chr6:137881564-137881591 |
| TP sk 304 | chr6:137867279-137867306 | TP sk 789 | chr6:137881568-137881595 |
| TP sk 305 | chr6:137867280-137867307 | TP sk 790 | chr6:137881569-137881596 |
| TP sk 306 | chr6:137867281-137867308 | TP sk 791 | chr6:137881571-137881598 |
| TP sk 307 | chr6:137867283-137867310 | TP sk 792 | chr6:137881576-137881603 |
| TP sk 308 | chr6:137867284-137867311 | TP sk 793 | chr6:137881580-137881607 |
| TP sk 309 | chr6:137867289-137867316 | TP sk 794 | chr6:137881586-137881613 |
| TP sk 310 | chr6:137867298-137867325 | TP sk 795 | chr6:137881590-137881617 |
| TP sk 311 | chr6:137867299-137867326 | TP sk 796 | chr6:137881592-137881619 |
| TP sk 312 | chr6:137867300-137867327 | TP sk 797 | chr6:137881597-137881624 |
| TP sk 313 | chr6:137867326-137867353 | TP sk 798 | chr6:137881598-137881625 |
| TP sk 314 | chr6:137867329-137867356 | TP sk 799 | chr6:137881599-137881626 |
| TP sk 315 | chr6:137867344-137867371 | TP sk 800 | chr6:137881604-137881631 |
| TP sk 316 | chr6:137867345-137867372 | TP sk 801 | chr6:137881610-137881637 |
| TP sk 317 | chr6:137867346-137867373 | TP sk 802 | chr6:137881632-137881659 |
| TP sk 318 | chr6:137867347-137867374 | TP sk 803 | chr6:137881638-137881665 |
| TP sk 319 | chr6:137867351-137867378 | TP sk 804 | chr6:137881639-137881666 |
| TP sk 320 | chr6:137867355-137867382 | TP sk 805 | chr6:137881640-137881667 |
| TP sk 321 | chr6:137867356-137867383 | TP sk 806 | chr6:137881646-137881673 |
| TP sk 322 | chr6:137867360-137867387 | TP sk 807 | chr6:137881647-137881674 |
| TP sk 323 | chr6:137867365-137867392 | TP sk 808 | chr6:137881650-137881677 |
| TP sk 324 | chr6:137867372-137867399 | TP sk 809 | chr6:137881660-137881687 |
| TP sk 325 | chr6:137867373-137867400 | TP sk 810 | chr6:137881661-137881688 |
| TP sk 326 | chr6:137867377-137867404 | TP sk 811 | chr6:137881683-137881710 |
| TP sk 327 | chr6:137867378-137867405 | TP sk 812 | chr6:137881684-137881711 |
| TP sk 328 | chr6:137867379-137867406 | TP sk 813 | chr6:137881688-137881715 |
| TP sk 329 | chr6:137867383-137867410 | TP sk 814 | chr6:137881721-137881748 |
| TP sk 330 | chr6:137867387-137867414 | TP sk 815 | chr6:137881727-137881754 |
| TP sk 331 | chr6:137867400-137867427 | TP sk 816 | chr6:137881732-137881759 |
| TP sk 332 | chr6:137867403-137867430 | TP sk 817 | chr6:137881735-137881762 |
| TP sk 333 | chr6:137867406-137867433 | TP sk 818 | chr6:137881738-137881765 |
| TP sk 334 | chr6:137867407-137867434 | TP sk 819 | chr6:137881739-137881766 |
| TP sk 335 | chr6:137867408-137867435 | TP sk 820 | chr6:137881740-137881767 |
| TP sk 336 | chr6:137867412-137867439 | TP sk 821 | chr6:137881743-137881770 |
| TP sk 337 | chr6:137867413-137867440 | TP sk 822 | chr6:137881744-137881771 |
| TP sk 338 | chr6:137867417-137867444 | TP sk 823 | chr6:137881745-137881772 |
| TP sk 339 | chr6:137867418-137867445 | TP sk 824 | chr6:137881746-137881773 |
| TP sk 340 | chr6:137867419-137867446 | TP sk 825 | chr6:137881756-137881783 |
| TP sk 341 | chr6:137867423-137867450 | TP sk 826 | chr6:137881764-137881791 |
| TP sk 342 | chr6:137867424-137867451 | TP sk 827 | chr6:137881770-137881797 |
| TP sk 343 | chr6:137867425-137867452 | TP sk 828 | chr6:137881776-137881803 |
| TP sk 344 | chr6:137867426-137867453 | TP sk 829 | chr6:137881785-137881812 |
| TP sk 345 | chr6:137867429-137867456 | TP sk 830 | chr6:137881792-137881819 |
| TP sk 346 | chr6:137867435-137867462 | TP sk 831 | chr6:137881793-137881820 |
| TP sk 347 | chr6:137867436-137867463 | TP sk 832 | chr6:137881794-137881821 |
| TP sk 348 | chr6:137867442-137867469 | TP sk 833 | chr6:137881795-137881822 |
| TP sk 349 | chr6:137867446-137867473 | TP sk 834 | chr6:137881802-137881829 |
| TP sk 350 | chr6:137867449-137867476 | TP sk 835 | chr6:137881832-137881859 |
| TP sk 351 | chr6:137867452-137867479 | TP sk 836 | chr6:137881851-137881878 |
| TP sk 352 | chr6:137867456-137867483 | TP sk 837 | chr6:137881865-137881892 |
| TP sk 353 | chr6:137867457-137867484 | TP sk 838 | chr6:137881866-137881893 |
| TP sk 354 | chr6:137867458-137867485 | TP sk 839 | chr6:137881870-137881897 |
| TP sk 355 | chr6:137867463-137867490 | TP sk 840 | chr6:137881871-137881898 |
| TP sk 356 | chr6:137867468-137867495 | TP sk 841 | chr6:137881874-137881901 |
| TP sk 357 | chr6:137867486-137867513 | TP sk 842 | chr6:137881875-137881902 |
| TP sk 358 | chr6:137867487-137867514 | TP sk 843 | chr6:137881881-137881908 |
| TP sk 359 | chr6:137867492-137867519 | TP sk 844 | chr6:137881886-137881913 |
| TP sk 360 | chr6:137867499-137867526 | TP sk 845 | chr6:137881894-137881921 |
| TP sk 361 | chr6:137867502-137867529 | TP sk 846 | chr6:137881895-137881922 |
| TP sk 362 | chr6:137867507-137867534 | TP sk 847 | chr6:137881909-137881936 |
| TP sk 363 | chr6:137867510-137867537 | TP sk 848 | chr6:137881912-137881939 |
| TP sk 364 | chr6:137867511-137867538 | TP sk 849 | chr6:137881916-137881943 |
| TP sk 365 | chr6:137867516-137867543 | TP sk 850 | chr6:137881996-137882023 |
| TP sk 366 | chr6:137867523-137867550 | TP sk 851 | chr6:137881997-137882024 |
| TP sk 367 | chr6:137867526-137867553 | TP sk 852 | chr6:137881998-137882025 |
| TP sk 368 | chr6:137867531-137867558 | TP sk 853 | chr6:137882017-137882044 |
| TP sk 369 | chr6:137867534-137867561 | TP sk 854 | chr6:137882018-137882045 |
| TP sk 370 | chr6:137867535-137867562 | TP sk 855 | chr6:137882061-137882088 |
| TP sk 371 | chr6:137867547-137867574 | TP sk 856 | chr6:137882108-137882135 |
| TP sk 372 | chr6:137867550-137867577 | TP sk 857 | chr6:137882109-137882136 |
| TP sk 373 | chr6:137867551-137867578 | TP sk 858 | chr6:137882116-137882143 |
| TP sk 374 | chr6:137867555-137867582 | TP sk 859 | chr6:137882117-137882144 |
| TP sk 375 | chr6:137867558-137867585 | TP sk 860 | chr6:137882154-137882181 |
| TP sk 376 | chr6:137867559-137867586 | TP sk 861 | chr6:137882158-137882185 |
| TP sk 377 | chr6:137867560-137867587 | TP sk 862 | chr6:137882164-137882191 |
| TP sk 378 | chr6:137867565-137867592 | TP sk 863 | chr6:137882165-137882192 |
| TP sk 379 | chr6:137867567-137867594 | TP sk 864 | chr6:137882184-137882211 |
| TP sk 380 | chr6:137867568-137867595 | TP sk 865 | chr6:137882194-137882221 |
| TP sk 381 | chr6:137867572-137867599 | TP sk 866 | chr6:137882195-137882222 |
| TP sk 382 | chr6:137867574-137867601 | TP sk 867 | chr6:137882196-137882223 |
| TP sk 383 | chr6:137867575-137867602 | TP sk 868 | chr6:137882200-137882227 |
| TP sk 384 | chr6:137867583-137867610 | TP sk 869 | chr6:137882251-137882278 |
| TP sk 385 | chr6:137867585-137867612 | TP sk 870 | chr6:137882252-137882279 |
| TP sk 386 | chr6:137867586-137867613 | TP sk 871 | chr6:137882260-137882287 |
| TP sk 387 | chr6:137867599-137867626 | TP sk 872 | chr6:137882267-137882294 |
| TP sk 388 | chr6:137867600-137867627 | TP sk 873 | chr6:137882289-137882316 |
| TP sk 389 | chr6:137867604-137867631 | TP sk 874 | chr6:137882339-137882366 |
| TP sk 390 | chr6:137867618-137867645 | TP sk 875 | chr6:137882340-137882367 |
| TP sk 391 | chr6:137867620-137867647 | TP sk 876 | chr6:137882341-137882368 |
| TP sk 392 | chr6:137867621-137867648 | TP sk 877 | chr6:137882375-137882402 |
| TP sk 393 | chr6:137867622-137867649 | TP sk 878 | chr6:137882387-137882414 |
| TP sk 394 | chr6:137867623-137867650 | TP sk 879 | chr6:137882389-137882416 |
| TP sk 395 | chr6:137867624-137867651 | TP sk 880 | chr6:137882395-137882422 |
| TP sk 396 | chr6:137867634-137867661 | TP sk 881 | chr6:137882396-137882423 |
| TP sk 397 | chr6:137867641-137867668 | TP sk 882 | chr6:137882400-137882427 |
| TP sk 398 | chr6:137867646-137867673 | TP sk 883 | chr6:137882414-137882441 |
| TP sk 399 | chr6:137867647-137867674 | TP sk 884 | chr6:137882415-137882442 |
| TP sk 400 | chr6:137867657-137867684 | TP sk 885 | chr6:137882425-137882452 |
| TP sk 401 | chr6:137867664-137867691 | TP sk 886 | chr6:137882430-137882457 |
| TP sk 402 | chr6:137867671-137867698 | TP sk 887 | chr6:137882435-137882462 |
| TP sk 403 | chr6:137867672-137867699 | TP sk 888 | chr6:137882453-137882480 |
| TP sk 404 | chr6:137867686-137867713 | TP sk 889 | chr6:137882458-137882485 |
| TP sk 405 | chr6:137867687-137867714 | TP sk 890 | chr6:137882466-137882493 |
| TP sk 406 | chr6:137867695-137867722 | TP sk 891 | chr6:137882476-137882503 |
| TP sk 407 | chr6:137867710-137867737 | TP sk 892 | chr6:137882479-137882506 |
| TP sk 408 | chr6:137867714-137867741 | TP sk 893 | chr6:137882480-137882507 |
| TP sk 409 | chr6:137867728-137867755 | TP sk 894 | chr6:137882481-137882508 |
| TP sk 410 | chr6:137867750-137867777 | TP sk 895 | chr6:137882502-137882529 |
| TP sk 411 | chr6:137867751-137867778 | TP sk 896 | chr6:137882542-137882569 |
| TP sk 412 | chr6:137867759-137867786 | TP sk 897 | chr6:137882554-137882581 |
| TP sk 413 | chr6:137867762-137867789 | TP sk 898 | chr6:137882592-137882619 |
| TP sk 414 | chr6:137867766-137867793 | TP sk 899 | chr6:137882596-137882623 |
| TP sk 415 | chr6:137867775-137867802 | TP sk 900 | chr6:137882604-137882631 |
| TP sk 416 | chr6:137867788-137867815 | TP sk 901 | chr6:137882605-137882632 |
| TP sk 417 | chr6:137867796-137867823 | TP sk 902 | chr6:137882607-137882634 |
| TP sk 418 | chr6:137867806-137867833 | TP sk 903 | chr6:137882609-137882636 |
| TP sk 419 | chr6:137867807-137867834 | TP sk 904 | chr6:137882611-137882638 |
| TP sk 420 | chr6:137867819-137867846 | TP sk 905 | chr6:137882614-137882641 |
| TP sk 421 | chr6:137867820-137867847 | TP sk 906 | chr6:137882615-137882642 |
| TP sk 422 | chr6:137867823-137867850 | TP sk 907 | chr6:137882616-137882643 |
| TP sk 423 | chr6:137867826-137867853 | TP sk 908 | chr6:137882626-137882653 |
| TP sk 424 | chr6:137867827-137867854 | TP sk 909 | chr6:137882632-137882659 |
| TP sk 425 | chr6:137867828-137867855 | TP sk 910 | chr6:137882639-137882666 |
| TP sk 426 | chr6:137867830-137867857 | TP sk 911 | chr6:137882640-137882667 |
| TP sk 427 | chr6:137867835-137867862 | TP sk 912 | chr6:137882650-137882677 |
| TP sk 428 | chr6:137867836-137867863 | TP sk 913 | chr6:137882663-137882690 |
| TP sk 429 | chr6:137867839-137867866 | TP sk 914 | chr6:137882678-137882705 |
| TP sk 430 | chr6:137867858-137867885 | TP sk 915 | chr6:137882692-137882719 |
| TP sk 431 | chr6:137867867-137867894 | TP sk 916 | chr6:137882693-137882720 |
| TP sk 432 | chr6:137867881-137867908 | TP sk 917 | chr6:137882694-137882721 |
| TP sk 433 | chr6:137867888-137867915 | TP sk 918 | chr6:137882695-137882722 |
| TP sk 434 | chr6:137867909-137867936 | TP sk 919 | chr6:137882696-137882723 |
| TP sk 435 | chr6:137867918-137867945 | TP sk 920 | chr6:137882697-137882724 |
| TP sk 436 | chr6:137867930-137867957 | TP sk 921 | chr6:137882701-137882728 |
| TP sk 437 | chr6:137867943-137867970 | TP sk 922 | chr6:137882702-137882729 |
| TP sk 438 | chr6:137867944-137867971 | TP sk 923 | chr6:137882715-137882742 |
| TP sk 439 | chr6:137867945-137867972 | TP sk 924 | chr6:137882722-137882749 |
| TP sk 440 | chr6:137867951-137867978 | TP sk 925 | chr6:137882730-137882757 |
| TP sk 441 | chr6:137867952-137867979 | TP sk 926 | chr6:137882734-137882761 |
| TP sk 442 | chr6:137867959-137867986 | TP sk 927 | chr6:137882735-137882762 |
| TP sk 443 | chr6:137867963-137867990 | TP sk 928 | chr6:137882736-137882763 |
| TP sk 444 | chr6:137867964-137867991 | TP sk 929 | chr6:137882737-137882764 |
| TP sk 445 | chr6:137867975-137868002 | TP sk 930 | chr6:137882743-137882770 |
| TP sk 446 | chr6:137867978-137868005 | TP sk 931 | chr6:137882744-137882771 |
| TP sk 447 | chr6:137867981-137868008 | TP sk 932 | chr6:137882752-137882779 |
| TP sk 448 | chr6:137867982-137868009 | TP sk 933 | chr6:137882753-137882780 |
| TP sk 449 | chr6:137867984-137868011 | TP sk 934 | chr6:137882768-137882795 |
| TP sk 450 | chr6:137867985-137868012 | TP sk 935 | chr6:137882775-137882802 |
| TP sk 451 | chr6:137867986-137868013 | TP sk 936 | chr6:137882782-137882809 |
| TP sk 452 | chr6:137867987-137868014 | TP sk 937 | chr6:137882788-137882815 |
| TP sk 453 | chr6:137867988-137868015 | TP sk 938 | chr6:137882815-137882842 |
| TP sk 454 | chr6:137867991-137868018 | TP sk 939 | chr6:137882853-137882880 |
| TP sk 455 | chr6:137867994-137868021 | TP sk 940 | chr6:137882856-137882883 |
| TP sk 456 | chr6:137867998-137868025 | TP sk 941 | chr6:137882857-137882884 |
| TP sk 457 | chr6:137867999-137868026 | TP sk 942 | chr6:137882858-137882885 |
| TP sk 458 | chr6:137868023-137868050 | TP sk 943 | chr6:137882884-137882911 |
| TP sk 459 | chr6:137868024-137868051 | TP sk 944 | chr6:137882896-137882923 |
| TP sk 460 | chr6:137868025-137868052 | TP sk 945 | chr6:137882897-137882924 |
| TP sk 461 | chr6:137868035-137868062 | TP sk 946 | chr6:137882898-137882925 |
| TP sk 462 | chr6:137868036-137868063 | TP sk 947 | chr6:137882899-137882926 |
| TP sk 463 | chr6:137868037-137868064 | TP sk 948 | chr6:137882912-137882939 |
| TP sk 464 | chr6:137868038-137868065 | TP sk 949 | chr6:137882915-137882942 |
| TP sk 465 | chr6:137868042-137868069 | TP sk 950 | chr6:137882940-137882967 |
| TP sk 466 | chr6:137868043-137868070 | TP sk 951 | chr6:137882958-137882985 |
| TP sk 467 | chr6:137868044-137868071 | TP sk 952 | chr6:137882966-137882993 |
| TP sk 468 | chr6:137868050-137868077 | TP sk 953 | chr6:137882974-137883001 |
| TP sk 469 | chr6:137868054-137868081 | TP sk 954 | chr6:137883002-137883029 |
| TP sk 470 | chr6:137868055-137868082 | TP sk 955 | chr6:137883003-137883030 |
| TP sk 471 | chr6:137868056-137868083 | TP sk 956 | chr6:137883004-137883031 |
| TP sk 472 | chr6:137868057-137868084 | TP sk 957 | chr6:137883012-137883039 |
| TP sk 473 | chr6:137868073-137868100 | TP sk 958 | chr6:137883013-137883040 |
| TP sk 474 | chr6:137868095-137868122 | TP sk 959 | chr6:137883014-137883041 |
| TP sk 475 | chr6:137868108-137868135 | TP sk 960 | chr6:137883015-137883042 |
| TP sk 476 | chr6:137868109-137868136 | TP sk 961 | chr6:137883026-137883053 |
| TP sk 477 | chr6:137868116-137868143 | TP sk 962 | chr6:137883027-137883054 |
| TP sk 478 | chr6:137868142-137868169 | TP sk 963 | chr6:137883052-137883079 |
| TP sk 479 | chr6:137868144-137868171 | TP sk 964 | chr6:137883061-137883088 |
| TP sk 480 | chr6:137868145-137868172 | TP sk 965 | chr6:137883134-137883161 |
| TP sk 481 | chr6:137868146-137868173 | TP sk 966 | chr6:137883208-137883235 |
| TP sk 482 | chr6:137868152-137868179 | TP sk 967 | chr6:137883209-137883236 |
| TP sk 483 | chr6:137868156-137868183 | TP sk 968 | chr6:137883211-137883238 |
| TP sk 484 | chr6:137868157-137868184 | TP sk 969 | chr6:137883221-137883248 |
| TP sk 485 | chr6:137868158-137868185 | TP sk 970 | chr6:137883224-137883251 |
| | | TP sk 971 | chr6:137876099-137876126 |

**Table 3B. Human ZC3H12A genomic coordinate of the present invention**

| No. | Chromosomal coordinate range | No. | Chromosomal coordinate range |
|---|---|---|---|
| ZC sk 1 | chr1:37473087-37473114 | ZC sk 292 | chr1:37479257-37479284 |
| ZC sk 2 | chr1:37473088-37473115 | ZC sk 293 | chr1:37479258-37479285 |
| ZC sk 3 | chr1:37473101-37473128 | ZC sk 294 | chr1:37479260-37479287 |
| ZC sk 4 | chr1:37473104-37473131 | ZC sk 295 | chr1:37479268-37479295 |
| ZC sk 5 | chr1:37473105-37473132 | ZC sk 296 | chr1:37479270-37479297 |
| ZC sk 6 | chr1:37473106-37473133 | ZC sk 297 | chr1:37479271-37479298 |
| ZC sk 7 | chr1:37473154-37473181 | ZC sk 298 | chr1:37479272-37479299 |
| ZC sk 8 | chr1:37473170-37473197 | ZC sk 299 | chr1:37479283-37479310 |
| ZC sk 9 | chr1:37473176-37473203 | ZC sk 300 | chr1:37479286-37479313 |
| ZC sk 10 | chr1:37473177-37473204 | ZC sk 301 | chr1:37479296-37479323 |
| ZC sk 11 | chr1:37473181-37473208 | ZC sk 302 | chr1:37479303-37479330 |
| ZC sk 12 | chr1:37473184-37473211 | ZC sk 303 | chr1:37479306-37479333 |
| ZC sk 13 | chr1:37473193-37473220 | ZC sk 304 | chr1:37479315-37479342 |
| ZC sk 14 | chr1:37473194-37473221 | ZC sk 305 | chr1:37479319-37479346 |
| ZC sk 15 | chr1:37473195-37473222 | ZC sk 306 | chr1:37479320-37479347 |
| ZC sk 16 | chr1:37473196-37473223 | ZC sk 307 | chr1:37479331-37479358 |
| ZC sk 17 | chr1:37473206-37473233 | ZC sk 308 | chr1:37479332-37479359 |
| ZC sk 18 | chr1:37473207-37473234 | ZC sk 309 | chr1:37479333-37479360 |
| ZC sk 19 | chr1:37473215-37473242 | ZC sk 310 | chr1:37479351-37479378 |
| ZC sk 20 | chr1:37473221-37473248 | ZC sk 311 | chr1:37479356-37479383 |
| ZC sk 21 | chr1:37473226-37473253 | ZC sk 312 | chr1:37479361-37479388 |
| ZC sk 22 | chr1:37473227-37473254 | ZC sk 313 | chr1:37479362-37479389 |
| ZC sk 23 | chr1:37473235-37473262 | ZC sk 314 | chr1:37479366-37479393 |
| ZC sk 24 | chr1:37473245-37473272 | ZC sk 315 | chr1:37479375-37479402 |
| ZC sk 25 | chr1:37473251-37473278 | ZC sk 316 | chr1:37479387-37479414 |
| ZC sk 26 | chr1:37473259-37473286 | ZC sk 317 | chr1:37479397-37479424 |
| ZC sk 27 | chr1:37473314-37473341 | ZC sk 318 | chr1:37479398-37479425 |
| ZC sk 28 | chr1:37473362-37473389 | ZC sk 319 | chr1:37479399-37479426 |
| ZC sk 29 | chr1:37473381-37473408 | ZC sk 320 | chr1:37479405-37479432 |
| ZC sk 30 | chr1:37473397-37473424 | ZC sk 321 | chr1:37479406-37479433 |
| ZC sk 31 | chr1:37473401-37473428 | ZC sk 322 | chr1:37479410-37479437 |
| ZC sk 32 | chr1:37473406-37473433 | ZC sk 323 | chr1:37479414-37479441 |
| ZC sk 33 | chr1:37473423-37473450 | ZC sk 324 | chr1:37479415-37479442 |
| ZC sk 34 | chr1:37473426-37473453 | ZC sk 325 | chr1:37479418-37479445 |
| ZC sk 35 | chr1:37473433-37473460 | ZC sk 326 | chr1:37479419-37479446 |
| ZC sk 36 | chr1:37473453-37473480 | ZC sk 327 | chr1:37479420-37479447 |
| ZC sk 37 | chr1:37473454-37473481 | ZC sk 328 | chr1:37479425-37479452 |
| ZC sk 38 | chr1:37473467-37473494 | ZC sk 329 | chr1:37479432-37479459 |
| ZC sk 39 | chr1:37473470-37473497 | ZC sk 330 | chr1:37479435-37479462 |
| ZC sk 40 | chr1:37473519-37473546 | ZC sk 331 | chr1:37479443-37479470 |
| ZC sk 41 | chr1:37473527-37473554 | ZC sk 332 | chr1:37479447-37479474 |
| ZC sk 42 | chr1:37473550-37473577 | ZC sk 333 | chr1:37479448-37479475 |
| ZC sk 43 | chr1:37473554-37473581 | ZC sk 334 | chr1:37479453-37479480 |
| ZC sk 44 | chr1:37473557-37473584 | ZC sk 335 | chr1:37479465-37479492 |
| ZC sk 45 | chr1:37473558-37473585 | ZC sk 336 | chr1:37479476-37479503 |
| ZC sk 46 | chr1:37473559-37473586 | ZC sk 337 | chr1:37479477-37479504 |
| ZC sk 47 | chr1:37473565-37473592 | ZC sk 338 | chr1:37479482-37479509 |
| ZC sk 48 | chr1:37473566-37473593 | ZC sk 339 | chr1:37479496-37479523 |
| ZC sk 49 | chr1:37473567-37473594 | ZC sk 340 | chr1:37479502-37479529 |
| ZC sk 50 | chr1:37473578-37473605 | ZC sk 341 | chr1:37479509-37479536 |
| ZC sk 51 | chr1:37473592-37473619 | ZC sk 342 | chr1:37479510-37479537 |
| ZC sk 52 | chr1:37473607-37473634 | ZC sk 343 | chr1:37479511-37479538 |
| ZC sk 53 | chr1:37473626-37473653 | ZC sk 344 | chr1:37479516-37479543 |
| ZC sk 54 | chr1:37473627-37473654 | ZC sk 345 | chr1:37479517-37479544 |
| ZC sk 55 | chr1:37473628-37473655 | ZC sk 346 | chr1:37479518-37479545 |
| ZC sk 56 | chr1:37473630-37473657 | ZC sk 347 | chr1:37479522-37479549 |
| ZC sk 57 | chr1:37473646-37473673 | ZC sk 348 | chr1:37479526-37479553 |
| ZC sk 58 | chr1:37473657-37473684 | ZC sk 349 | chr1:37479533-37479560 |
| ZC sk 59 | chr1:37473658-37473685 | ZC sk 350 | chr1:37479534-37479561 |
| ZC sk 60 | chr1:37473664-37473691 | ZC sk 351 | chr1:37479535-37479562 |
| ZC sk 61 | chr1:37473703-37473730 | ZC sk 352 | chr1:37479536-37479563 |
| ZC sk 62 | chr1:37473717-37473744 | ZC sk 353 | chr1:37479538-37479565 |
| ZC sk 63 | chr1:37473718-37473745 | ZC sk 354 | chr1:37479550-37479577 |
| ZC sk 64 | chr1:37473730-37473757 | ZC sk 355 | chr1:37479551-37479578 |
| ZC sk 65 | chr1:37473731-37473758 | ZC sk 356 | chr1:37479554-37479581 |
| ZC sk 66 | chr1:37473737-37473764 | ZC sk 357 | chr1:37479569-37479596 |
| ZC sk 67 | chr1:37473747-37473774 | ZC sk 358 | chr1:37479588-37479615 |
| ZC sk 68 | chr1:37473748-37473775 | ZC sk 359 | chr1:37479593-37479620 |
| ZC sk 69 | chr1:37473769-37473796 | ZC sk 360 | chr1:37479595-37479622 |
| ZC sk 70 | chr1:37473770-37473797 | ZC sk 361 | chr1:37479596-37479623 |
| ZC sk 71 | chr1:37473776-37473803 | ZC sk 362 | chr1:37479597-37479624 |
| ZC sk 72 | chr1:37473777-37473804 | ZC sk 363 | chr1:37479614-37479641 |
| ZC sk 73 | chr1:37473791-37473818 | ZC sk 364 | chr1:37479618-37479645 |
| ZC sk 74 | chr1:37473792-37473819 | ZC sk 365 | chr1:37479637-37479664 |
| ZC sk 75 | chr1:37473793-37473820 | ZC sk 366 | chr1:37479638-37479665 |
| ZC sk 76 | chr1:37473794-37473821 | ZC sk 367 | chr1:37479641-37479668 |
| ZC sk 77 | chr1:37473809-37473836 | ZC sk 368 | chr1:37479652-37479679 |
| ZC sk 78 | chr1:37473812-37473839 | ZC sk 369 | chr1:37479653-37479680 |
| ZC sk 79 | chr1:37473813-37473840 | ZC sk 370 | chr1:37479674-37479701 |
| ZC sk 80 | chr1:37473818-37473845 | ZC sk 371 | chr1:37479675-37479702 |
| ZC sk 81 | chr1:37473819-37473846 | ZC sk 372 | chr1:37479678-37479705 |
| ZC sk 82 | chr1:37473838-37473865 | ZC sk 373 | chr1:37479679-37479706 |
| ZC sk 83 | chr1:37473853-37473880 | ZC sk 374 | chr1:37479680-37479707 |
| ZC sk 84 | chr1:37473860-37473887 | ZC sk 375 | chr1:37479684-37479711 |
| ZC sk 85 | chr1:37473861-37473888 | ZC sk 376 | chr1:37479685-37479712 |
| ZC sk 86 | chr1:37473862-37473889 | ZC sk 377 | chr1:37479686-37479713 |
| ZC sk 87 | chr1:37473869-37473896 | ZC sk 378 | chr1:37479690-37479717 |
| ZC sk 88 | chr1:37473884-37473911 | ZC sk 379 | chr1:37479692-37479719 |
| ZC sk 89 | chr1:37473897-37473924 | ZC sk 380 | chr1:37479701-37479728 |
| ZC sk 90 | chr1:37473898-37473925 | ZC sk 381 | chr1:37479703-37479730 |
| ZC sk 91 | chr1:37473907-37473934 | ZC sk 382 | chr1:37479704-37479731 |
| ZC sk 92 | chr1:37473919-37473946 | ZC sk 383 | chr1:37479705-37479732 |
| ZC sk 93 | chr1:37473932-37473959 | ZC sk 384 | chr1:37479710-37479737 |
| ZC sk 94 | chr1:37473948-37473975 | ZC sk 385 | chr1:37479711-37479738 |
| ZC sk 95 | chr1:37473952-37473979 | ZC sk 386 | chr1:37479718-37479745 |
| ZC sk 96 | chr1:37473956-37473983 | ZC sk 387 | chr1:37479728-37479755 |
| ZC sk 97 | chr1:37473961-37473988 | ZC sk 388 | chr1:37479739-37479766 |
| ZC sk 98 | chr1:37473970-37473997 | ZC sk 389 | chr1:37479742-37479769 |
| ZC sk 99 | chr1:37473976-37474003 | ZC sk 390 | chr1:37479752-37479779 |
| ZC sk 100 | chr1:37473977-37474004 | ZC sk 391 | chr1:37479761-37479788 |
| ZC sk 101 | chr1:37473978-37474005 | ZC sk 392 | chr1:37479771-37479798 |
| ZC sk 102 | chr1:37473979-37474006 | ZC sk 393 | chr1:37479772-37479799 |
| ZC sk 103 | chr1:37473982-37474009 | ZC sk 394 | chr1:37479776-37479803 |
| ZC sk 104 | chr1:37473993-37474020 | ZC sk 395 | chr1:37479785-37479812 |
| ZC sk 105 | chr1:37473998-37474025 | ZC sk 396 | chr1:37479786-37479813 |
| ZC sk 106 | chr1:37473999-37474026 | ZC sk 397 | chr1:37479787-37479814 |
| ZC sk 107 | chr1:37474000-37474027 | ZC sk 398 | chr1:37479788-37479815 |
| ZC sk 108 | chr1:37474010-37474037 | ZC sk 399 | chr1:37479790-37479817 |
| ZC sk 109 | chr1:37474014-37474041 | ZC sk 400 | chr1:37479791-37479818 |
| ZC sk 110 | chr1:37474024-37474051 | ZC sk 401 | chr1:37479792-37479819 |
| ZC sk 111 | chr1:37474025-37474052 | ZC sk 402 | chr1:37479793-37479820 |
| ZC sk 112 | chr1:37474026-37474053 | ZC sk 403 | chr1:37479794-37479821 |
| ZC sk 113 | chr1:37474027-37474054 | ZC sk 404 | chr1:37479795-37479822 |
| ZC sk 114 | chr1:37474055-37474082 | ZC sk 405 | chr1:37479796-37479823 |
| ZC sk 115 | chr1:37474057-37474084 | ZC sk 406 | chr1:37479797-37479824 |
| ZC sk 116 | chr1:37474062-37474089 | ZC sk 407 | chr1:37479798-37479825 |
| ZC sk 117 | chr1:37474076-37474103 | ZC sk 408 | chr1:37479799-37479826 |
| ZC sk 118 | chr1:37474079-37474106 | ZC sk 409 | chr1:37479834-37479861 |
| ZC sk 119 | chr1:37474090-37474117 | ZC sk 410 | chr1:37479840-37479867 |
| ZC sk 120 | chr1:37474093-37474120 | ZC sk 411 | chr1:37479851-37479878 |
| ZC sk 121 | chr1:37474100-37474127 | ZC sk 412 | chr1:37479852-37479879 |
| ZC sk 122 | chr1:37474101-37474128 | ZC sk 413 | chr1:37479854-37479881 |
| ZC sk 123 | chr1:37474108-37474135 | ZC sk 414 | chr1:37479855-37479882 |
| ZC sk 124 | chr1:37474112-37474139 | ZC sk 415 | chr1:37479856-37479883 |
| ZC sk 125 | chr1:37474115-37474142 | ZC sk 416 | chr1:37479857-37479884 |
| ZC sk 126 | chr1:37474129-37474156 | ZC sk 417 | chr1:37479861-37479888 |
| ZC sk 127 | chr1:37474133-37474160 | ZC sk 418 | chr1:37479863-37479890 |
| ZC sk 128 | chr1:37474136-37474163 | ZC sk 419 | chr1:37479864-37479891 |
| ZC sk 129 | chr1:37474140-37474167 | ZC sk 420 | chr1:37479875-37479902 |
| ZC sk 130 | chr1:37474143-37474170 | ZC sk 421 | chr1:37479878-37479905 |
| ZC sk 131 | chr1:37474160-37474187 | ZC sk 422 | chr1:37479891-37479918 |
| ZC sk 132 | chr1:37474163-37474190 | ZC sk 423 | chr1:37479899-37479926 |
| ZC sk 133 | chr1:37474164-37474191 | ZC sk 424 | chr1:37479908-37479935 |
| ZC sk 134 | chr1:37474165-37474192 | ZC sk 425 | chr1:37479913-37479940 |
| ZC sk 135 | chr1:37474168-37474195 | ZC sk 426 | chr1:37479914-37479941 |
| ZC sk 136 | chr1:37474186-37474213 | ZC sk 427 | chr1:37479915-37479942 |
| ZC sk 137 | chr1:37474197-37474224 | ZC sk 428 | chr1:37479916-37479943 |
| ZC sk 138 | chr1:37474201-37474228 | ZC sk 429 | chr1:37479918-37479945 |
| ZC sk 139 | chr1:37474215-37474242 | ZC sk 430 | chr1:37479919-37479946 |
| ZC sk 140 | chr1:37474216-37474243 | ZC sk 431 | chr1:37479920-37479947 |
| ZC sk 141 | chr1:37474234-37474261 | ZC sk 432 | chr1:37479921-37479948 |
| ZC sk 142 | chr1:37474236-37474263 | ZC sk 433 | chr1:37479922-37479949 |
| ZC sk 143 | chr1:37474239-37474266 | ZC sk 434 | chr1:37480425-37480452 |
| ZC sk 144 | chr1:37474246-37474273 | ZC sk 435 | chr1:37480426-37480453 |
| ZC sk 145 | chr1:37474249-37474276 | ZC sk 436 | chr1:37483626-37483653 |
| ZC sk 146 | chr1:37474251-37474278 | ZC sk 437 | chr1:37483627-37483654 |
| ZC sk 147 | chr1:37474253-37474280 | ZC sk 438 | chr1:37483635-37483662 |
| ZC sk 148 | chr1:37474255-37474282 | ZC sk 439 | chr1:37483638-37483665 |
| ZC sk 149 | chr1:37474258-37474285 | ZC sk 440 | chr1:37483639-37483666 |
| ZC sk 150 | chr1:37474273-37474300 | ZC sk 441 | chr1:37483640-37483667 |
| ZC sk 151 | chr1:37474274-37474301 | ZC sk 442 | chr1:37483641-37483668 |
| ZC sk 152 | chr1:37474275-37474302 | ZC sk 443 | chr1:37483645-37483672 |
| ZC sk 153 | chr1:37474291-37474318 | ZC sk 444 | chr1:37483646-37483673 |
| ZC sk 154 | chr1:37474297-37474324 | ZC sk 445 | chr1:37483647-37483674 |
| ZC sk 155 | chr1:37474298-37474325 | ZC sk 446 | chr1:37483648-37483675 |
| ZC sk 156 | chr1:37474302-37474329 | ZC sk 447 | chr1:37483655-37483682 |
| ZC sk 157 | chr1:37474318-37474345 | ZC sk 448 | chr1:37483675-37483702 |
| ZC sk 158 | chr1:37474326-37474353 | ZC sk 449 | chr1:37483677-37483704 |
| ZC sk 159 | chr1:37474337-37474364 | ZC sk 450 | chr1:37483686-37483713 |
| ZC sk 160 | chr1:37474338-37474365 | ZC sk 451 | chr1:37483687-37483714 |
| ZC sk 161 | chr1:37474344-37474371 | ZC sk 452 | chr1:37483690-37483717 |
| ZC sk 162 | chr1:37474355-37474382 | ZC sk 453 | chr1:37483692-37483719 |
| ZC sk 163 | chr1:37474358-37474385 | ZC sk 454 | chr1:37483693-37483720 |
| ZC sk 164 | chr1:37474361-37474388 | ZC sk 455 | chr1:37483694-37483721 |
| ZC sk 165 | chr1:37474362-37474389 | ZC sk 456 | chr1:37483697-37483724 |
| ZC sk 166 | chr1:37474364-37474391 | ZC sk 457 | chr1:37483698-37483725 |
| ZC sk 167 | chr1:37474372-37474399 | ZC sk 458 | chr1:37483701-37483728 |
| ZC sk 168 | chr1:37474374-37474401 | ZC sk 459 | chr1:37483702-37483729 |
| ZC sk 169 | chr1:37474378-37474405 | ZC sk 460 | chr1:37483705-37483732 |
| ZC sk 170 | chr1:37474379-37474406 | ZC sk 461 | chr1:37483706-37483733 |
| ZC sk 171 | chr1:37474380-37474407 | ZC sk 462 | chr1:37483709-37483736 |
| ZC sk 172 | chr1:37474381-37474408 | ZC sk 463 | chr1:37483710-37483737 |
| ZC sk 173 | chr1:37474382-37474409 | ZC sk 464 | chr1:37483711-37483738 |
| ZC sk 174 | chr1:37474388-37474415 | ZC sk 465 | chr1:37483716-37483743 |
| ZC sk 175 | chr1:37474389-37474416 | ZC sk 466 | chr1:37483717-37483744 |
| ZC sk 176 | chr1:37474390-37474417 | ZC sk 467 | chr1:37483721-37483748 |
| ZC sk 177 | chr1:37474391-37474418 | ZC sk 468 | chr1:37483748-37483775 |
| ZC sk 178 | chr1:37474392-37474419 | ZC sk 469 | chr1:37483752-37483779 |
| ZC sk 179 | chr1:37474394-37474421 | ZC sk 470 | chr1:37483753-37483780 |
| ZC sk 180 | chr1:37474395-37474422 | ZC sk 471 | chr1:37483759-37483786 |
| ZC sk 181 | chr1:37474396-37474423 | ZC sk 472 | chr1:37483760-37483787 |
| ZC sk 182 | chr1:37474409-37474436 | ZC sk 473 | chr1:37483787-37483814 |
| ZC sk 183 | chr1:37474410-37474437 | ZC sk 474 | chr1:37483788-37483815 |
| ZC sk 184 | chr1:37474414-37474441 | ZC sk 475 | chr1:37483793-37483820 |
| ZC sk 185 | chr1:37474420-37474447 | ZC sk 476 | chr1:37483794-37483821 |
| ZC sk 186 | chr1:37474424-37474451 | ZC sk 477 | chr1:37483798-37483825 |
| ZC sk 187 | chr1:37474425-37474452 | ZC sk 478 | chr1:37483812-37483839 |
| ZC sk 188 | chr1:37474426-37474453 | ZC sk 479 | chr1:37483835-37483862 |
| ZC sk 189 | chr1:37474431-37474458 | ZC sk 480 | chr1:37483836-37483863 |
| ZC sk 190 | chr1:37474446-37474473 | ZC sk 481 | chr1:37483843-37483870 |
| ZC sk 191 | chr1:37474447-37474474 | ZC sk 482 | chr1:37483855-37483882 |
| ZC sk 192 | chr1:37474448-37474475 | ZC sk 483 | chr1:37483861-37483888 |
| ZC sk 193 | chr1:37474451-37474478 | ZC sk 484 | chr1:37483867-37483894 |
| ZC sk 194 | chr1:37474459-37474486 | ZC sk 485 | chr1:37483874-37483901 |
| ZC sk 195 | chr1:37474460-37474487 | ZC sk 486 | chr1:37483879-37483906 |
| ZC sk 196 | chr1:37474462-37474489 | ZC sk 487 | chr1:37483880-37483907 |
| ZC sk 197 | chr1:37474463-37474490 | ZC sk 488 | chr1:37483896-37483923 |
| ZC sk 198 | chr1:37474464-37474491 | ZC sk 489 | chr1:37483905-37483932 |
| ZC sk 199 | chr1:37474467-37474494 | ZC sk 490 | chr1:37483906-37483933 |
| ZC sk 200 | chr1:37474468-37474495 | ZC sk 491 | chr1:37483908-37483935 |
| ZC sk 201 | chr1:37474469-37474496 | ZC sk 492 | chr1:37483909-37483936 |
| ZC sk 202 | chr1:37474470-37474497 | ZC sk 493 | chr1:37483910-37483937 |
| ZC sk 203 | chr1:37474482-37474509 | ZC sk 494 | chr1:37483911-37483938 |
| ZC sk 204 | chr1:37474483-37474510 | ZC sk 495 | chr1:37483914-37483941 |
| ZC sk 205 | chr1:37474492-37474519 | ZC sk 496 | chr1:37483915-37483942 |
| ZC sk 206 | chr1:37474493-37474520 | ZC sk 497 | chr1:37483918-37483945 |
| ZC sk 207 | chr1:37474494-37474521 | ZC sk 498 | chr1:37483921-37483948 |
| ZC sk 208 | chr1:37474497-37474524 | ZC sk 499 | chr1:37483922-37483949 |
| ZC sk 209 | chr1:37474498-37474525 | ZC sk 500 | chr1:37483923-37483950 |
| ZC sk 210 | chr1:37474499-37474526 | ZC sk 501 | chr1:37483924-37483951 |
| ZC sk 211 | chr1:37474504-37474531 | ZC sk 502 | chr1:37483933-37483960 |
| ZC sk 212 | chr1:37474505-37474532 | ZC sk 503 | chr1:37483938-37483965 |
| ZC sk 213 | chr1:37474511-37474538 | ZC sk 504 | chr1:37483939-37483966 |
| ZC sk 214 | chr1:37474522-37474549 | ZC sk 505 | chr1:37483943-37483970 |
| ZC sk 215 | chr1:37474527-37474554 | ZC sk 506 | chr1:37483944-37483971 |
| ZC sk 216 | chr1:37474530-37474557 | ZC sk 507 | chr1:37483961-37483988 |
| ZC sk 217 | chr1:37474533-37474560 | ZC sk 508 | chr1:37483974-37484001 |
| ZC sk 218 | chr1:37474537-37474564 | ZC sk 509 | chr1:37483975-37484002 |
| ZC sk 219 | chr1:37474538-37474565 | ZC sk 510 | chr1:37483983-37484010 |
| ZC sk 220 | chr1:37474545-37474572 | ZC sk 511 | chr1:37483984-37484011 |
| ZC sk 221 | chr1:37474548-37474575 | ZC sk 512 | chr1:37483985-37484012 |
| ZC sk 222 | chr1:37474549-37474576 | ZC sk 513 | chr1:37483988-37484015 |
| ZC sk 223 | chr1:37474550-37474577 | ZC sk 514 | chr1:37483989-37484016 |
| ZC sk 224 | chr1:37474555-37474582 | ZC sk 515 | chr1:37483990-37484017 |
| ZC sk 225 | chr1:37474561-37474588 | ZC sk 516 | chr1:37483991-37484018 |
| ZC sk 226 | chr1:37474563-37474590 | ZC sk 517 | chr1:37483996-37484023 |
| ZC sk 227 | chr1:37474566-37474593 | ZC sk 518 | chr1:37483997-37484024 |
| ZC sk 228 | chr1:37474569-37474596 | ZC sk 519 | chr1:37483999-37484026 |
| ZC sk 229 | chr1:37474570-37474597 | ZC sk 520 | chr1:37484000-37484027 |
| ZC sk 230 | chr1:37474575-37474602 | ZC sk 521 | chr1:37484001-37484028 |
| ZC sk 231 | chr1:37474576-37474603 | ZC sk 522 | chr1:37484004-37484031 |
| ZC sk 232 | chr1:37474577-37474604 | ZC sk 523 | chr1:37484005-37484032 |
| ZC sk 233 | chr1:37474578-37474605 | ZC sk 524 | chr1:37484006-37484033 |
| ZC sk 234 | chr1:37474581-37474608 | ZC sk 525 | chr1:37484009-37484036 |
| ZC sk 235 | chr1:37474589-37474616 | ZC sk 526 | chr1:37484010-37484037 |
| ZC sk 236 | chr1:37474590-37474617 | ZC sk 527 | chr1:37484019-37484046 |
| ZC sk 237 | chr1:37474591-37474618 | ZC sk 528 | chr1:37484033-37484060 |
| ZC sk 238 | chr1:37474598-37474625 | ZC sk 529 | chr1:37484034-37484061 |
| ZC sk 239 | chr1:37474601-37474628 | ZC sk 530 | chr1:37484040-37484067 |
| ZC sk 240 | chr1:37474602-37474629 | ZC sk 531 | chr1:37484047-37484074 |
| ZC sk 241 | chr1:37474603-37474630 | ZC sk 532 | chr1:37484051-37484078 |
| ZC sk 242 | chr1:37474605-37474632 | ZC sk 533 | chr1:37484052-37484079 |
| ZC sk 243 | chr1:37474608-37474635 | ZC sk 534 | chr1:37484054-37484081 |
| ZC sk 244 | chr1:37474609-37474636 | ZC sk 535 | chr1:37484055-37484082 |
| ZC sk 245 | chr1:37474612-37474639 | ZC sk 536 | chr1:37484062-37484089 |
| ZC sk 246 | chr1:37474613-37474640 | ZC sk 537 | chr1:37484065-37484092 |
| ZC sk 247 | chr1:37474616-37474643 | ZC sk 538 | chr1:37484073-37484100 |
| ZC sk 248 | chr1:37474617-37474644 | ZC sk 539 | chr1:37484075-37484102 |
| ZC sk 249 | chr1:37474618-37474645 | ZC sk 540 | chr1:37484076-37484103 |
| ZC sk 250 | chr1:37474621-37474648 | ZC sk 541 | chr1:37484082-37484109 |
| ZC sk 251 | chr1:37474622-37474649 | ZC sk 542 | chr1:37484083-37484110 |
| ZC sk 252 | chr1:37474627-37474654 | ZC sk 543 | chr1:37484144-37484171 |
| ZC sk 253 | chr1:37474628-37474655 | ZC sk 544 | chr1:37484145-37484172 |
| ZC sk 254 | chr1:37475432-37475459 | ZC sk 545 | chr1:37484148-37484175 |
| ZC sk 255 | chr1:37475435-37475462 | ZC sk 546 | chr1:37484155-37484182 |
| ZC sk 256 | chr1:37475436-37475463 | ZC sk 547 | chr1:37484156-37484183 |
| ZC sk 257 | chr1:37475437-37475464 | ZC sk 548 | chr1:37484157-37484184 |
| ZC sk 258 | chr1:37475442-37475469 | ZC sk 549 | chr1:37484162-37484189 |
| ZC sk 259 | chr1:37475443-37475470 | ZC sk 550 | chr1:37484163-37484190 |
| ZC sk 260 | chr1:37475448-37475475 | ZC sk 551 | chr1:37484164-37484191 |
| ZC sk 261 | chr1:37475449-37475476 | ZC sk 552 | chr1:37484167-37484194 |
| ZC sk 262 | chr1:37475450-37475477 | ZC sk 553 | chr1:37484170-37484197 |
| ZC sk 263 | chr1:37479130-37479157 | ZC sk 554 | chr1:37484171-37484198 |
| ZC sk 264 | chr1:37479134-37479161 | ZC sk 555 | chr1:37484173-37484200 |
| ZC sk 265 | chr1:37479135-37479162 | ZC sk 556 | chr1:37484180-37484207 |
| ZC sk 266 | chr1:37479136-37479163 | ZC sk 557 | chr1:37484181-37484208 |
| ZC sk 267 | chr1:37479137-37479164 | ZC sk 558 | chr1:37484182-37484209 |
| ZC sk 268 | chr1:37479150-37479177 | ZC sk 559 | chr1:37484199-37484226 |
| ZC sk 269 | chr1:37479151-37479178 | ZC sk 560 | chr1:37484200-37484227 |
| ZC sk 270 | chr1:37479152-37479179 | ZC sk 561 | chr1:37484203-37484230 |
| ZC sk 271 | chr1:37479159-37479186 | ZC sk 562 | chr1:37484212-37484239 |
| ZC sk 272 | chr1:37479166-37479193 | ZC sk 563 | chr1:37484213-37484240 |
| ZC sk 273 | chr1:37479188-37479215 | ZC sk 564 | chr1:37484224-37484251 |
| ZC sk 274 | chr1:37479196-37479223 | ZC sk 565 | chr1:37484227-37484254 |
| ZC sk 275 | chr1:37479197-37479224 | ZC sk 566 | chr1:37484231-37484258 |
| ZC sk 276 | chr1:37479201-37479228 | ZC sk 567 | chr1:37484237-37484264 |
| ZC sk 277 | chr1:37479209-37479236 | ZC sk 568 | chr1:37484238-37484265 |
| ZC sk 278 | chr1:37479219-37479246 | ZC sk 569 | chr1:37484241-37484268 |
| ZC sk 279 | chr1:37479227-37479254 | ZC sk 570 | chr1:37484242-37484269 |
| ZC sk 280 | chr1:37479228-37479255 | ZC sk 571 | chr1:37484243-37484270 |
| ZC sk 281 | chr1:37479229-37479256 | ZC sk 572 | chr1:37484244-37484271 |
| ZC sk 282 | chr1:37479234-37479261 | ZC sk 573 | chr1:37484252-37484279 |
| ZC sk 283 | chr1:37479235-37479262 | ZC sk 574 | chr1:37484253-37484280 |
| ZC sk 284 | chr1:37479236-37479263 | ZC sk 575 | chr1:37484257-37484284 |
| ZC sk 285 | chr1:37479240-37479267 | ZC sk 576 | chr1:37484258-37484285 |
| ZC sk 286 | chr1:37479241-37479268 | ZC sk 577 | chr1:37484259-37484286 |
| ZC sk 287 | chr1:37479242-37479269 | ZC sk 578 | chr1:37484274-37484301 |
| ZC sk 288 | chr1:37479243-37479270 | ZC sk 579 | chr1:37484326-37484353 |
| ZC sk 289 | chr1:37479244-37479271 | ZC sk 580 | chr1:37484331-37484358 |
| ZC sk 290 | chr1:37479248-37479275 | ZC sk 581 | chr1:37484334-37484361 |
| ZC sk 291 | chr1:37479249-37479276 | ZC sk 582 | chr1:37484369-37484396 |
| | | ZC sk 583 | chr1:37484370-37484397 |

**Table 3C. Human SOCS1 genomic coordinate of the present invention**

| No. | Chromosomal coordinate range | No. | Chromosomal coordinate range |
|---|---|---|---|
| SC sk 1 | chr16:11257672-11257699 | SC sk 348 | chr16:11256310-11256337 |
| SC sk 2 | chr16:11257669-11257696 | SC sk 349 | chr16:11256305-11256332 |
| SC sk 3 | chr16:11257650-11257677 | SC sk 350 | chr16:11256298-11256325 |
| SC sk 4 | chr16:11257641-11257668 | SC sk 351 | chr16:11256298-11256325 |
| SC sk 5 | chr16:11257636-11257663 | SC sk 352 | chr16:11256295-11256322 |
| SC sk 6 | chr16:11257629-11257656 | SC sk 353 | chr16:11256292-11256319 |
| SC sk 7 | chr16:11257626-11257653 | SC sk 354 | chr16:11256291-11256318 |
| SC sk 8 | chr16:11257624-11257651 | SC sk 355 | chr16:11256290-11256317 |
| SC sk 9 | chr16:11257623-11257650 | SC sk 356 | chr16:11256289-11256316 |
| SC sk 10 | chr16:11257623-11257650 | SC sk 357 | chr16:11256286-11256313 |
| SC sk 11 | chr16:11257619-11257646 | SC sk 358 | chr16:11256285-11256312 |
| SC sk 12 | chr16:11257618-11257645 | SC sk 359 | chr16:11256284-11256311 |
| SC sk 13 | chr16:11257614-11257641 | SC sk 360 | chr16:11256280-11256307 |
| SC sk 14 | chr16:11257613-11257640 | SC sk 361 | chr16:11256280-11256307 |
| SC sk 15 | chr16:11257613-11257640 | SC sk 362 | chr16:11256276-11256303 |
| SC sk 16 | chr16:11257612-11257639 | SC sk 363 | chr16:11256275-11256302 |
| SC sk 17 | chr16:11257601-11257628 | SC sk 364 | chr16:11256274-11256301 |
| SC sk 18 | chr16:11257600-11257627 | SC sk 365 | chr16:11256273-11256300 |
| SC sk 19 | chr16:11257590-11257617 | SC sk 366 | chr16:11256270-11256297 |
| SC sk 20 | chr16:11257589-11257616 | SC sk 367 | chr16:11256269-11256296 |
| SC sk 21 | chr16:11257571-11257598 | SC sk 368 | chr16:11256268-11256295 |
| SC sk 22 | chr16:11257568-11257595 | SC sk 369 | chr16:11256256-11256283 |
| SC sk 23 | chr16:11257561-11257588 | SC sk 370 | chr16:11256240-11256267 |
| SC sk 24 | chr16:11257559-11257586 | SC sk 371 | chr16:11256237-11256264 |
| SC sk 25 | chr16:11257556-11257583 | SC sk 372 | chr16:11256236-11256263 |
| SC sk 26 | chr16:11257555-11257582 | SC sk 373 | chr16:11256234-11256261 |
| SC sk 27 | chr16:11257552-11257579 | SC sk 374 | chr16:11256233-11256260 |
| SC sk 28 | chr16:11257542-11257569 | SC sk 375 | chr16:11256227-11256254 |
| SC sk 29 | chr16:11257541-11257568 | SC sk 376 | chr16:11256223-11256250 |
| SC sk 30 | chr16:11257540-11257567 | SC sk 377 | chr16:11256222-11256249 |
| SC sk 31 | chr16:11257533-11257560 | SC sk 378 | chr16:11256221-11256248 |
| SC sk 32 | chr16:11257530-11257557 | SC sk 379 | chr16:11256221-11256248 |
| SC sk 33 | chr16:11257525-11257552 | SC sk 380 | chr16:11256218-11256245 |
| SC sk 34 | chr16:11257524-11257551 | SC sk 381 | chr16:11256215-11256242 |
| SC sk 35 | chr16:11257523-11257550 | SC sk 382 | chr16:11256215-11256242 |
| SC sk 36 | chr16:11257519-11257546 | SC sk 383 | chr16:11256214-11256241 |
| SC sk 37 | chr16:11257518-11257545 | SC sk 384 | chr16:11256213-11256240 |
| SC sk 38 | chr16:11257517-11257544 | SC sk 385 | chr16:11256210-11256237 |
| SC sk 39 | chr16:11257514-11257541 | SC sk 386 | chr16:11256210-11256237 |
| SC sk 40 | chr16:11257511-11257538 | SC sk 387 | chr16:11256209-11256236 |
| SC sk 41 | chr16:11257510-11257537 | SC sk 388 | chr16:11256187-11256214 |
| SC sk 42 | chr16:11257506-11257533 | SC sk 389 | chr16:11256181-11256208 |
| SC sk 43 | chr16:11257505-11257532 | SC sk 390 | chr16:11256179-11256206 |
| SC sk 44 | chr16:11257500-11257527 | SC sk 391 | chr16:11256170-11256197 |
| SC sk 45 | chr16:11257499-11257526 | SC sk 392 | chr16:11256163-11256190 |
| SC sk 46 | chr16:11257495-11257522 | SC sk 393 | chr16:11256159-11256186 |
| SC sk 47 | chr16:11257492-11257519 | SC sk 394 | chr16:11256157-11256184 |
| SC sk 48 | chr16:11257491-11257518 | SC sk 395 | chr16:11256140-11256167 |
| SC sk 49 | chr16:11257483-11257510 | SC sk 396 | chr16:11256135-11256162 |
| SC sk 50 | chr16:11257469-11257496 | SC sk 397 | chr16:11256134-11256161 |
| SC sk 51 | chr16:11257460-11257487 | SC sk 398 | chr16:11256134-11256161 |
| SC sk 52 | chr16:11257452-11257479 | SC sk 399 | chr16:11256133-11256160 |
| SC sk 53 | chr16:11257451-11257478 | SC sk 400 | chr16:11256124-11256151 |
| SC sk 54 | chr16:11257448-11257475 | SC sk 401 | chr16:11256115-11256142 |
| SC sk 55 | chr16:11257431-11257458 | SC sk 402 | chr16:11256112-11256139 |
| SC sk 56 | chr16:11257415-11257442 | SC sk 403 | chr16:11256111-11256138 |
| SC sk 57 | chr16:11257414-11257441 | SC sk 404 | chr16:11256110-11256137 |
| SC sk 58 | chr16:11257403-11257430 | SC sk 405 | chr16:11256102-11256129 |
| SC sk 59 | chr16:11257402-11257429 | SC sk 406 | chr16:11256101-11256128 |
| SC sk 60 | chr16:11257396-11257423 | SC sk 407 | chr16:11256100-11256127 |
| SC sk 61 | chr16:11257391-11257418 | SC sk 408 | chr16:11256100-11256127 |
| SC sk 62 | chr16:11257389-11257416 | SC sk 409 | chr16:11256094-11256121 |
| SC sk 63 | chr16:11257388-11257415 | SC sk 410 | chr16:11256093-11256120 |
| SC sk 64 | chr16:11257384-11257411 | SC sk 411 | chr16:11256092-11256119 |
| SC sk 65 | chr16:11257379-11257406 | SC sk 412 | chr16:11256091-11256118 |
| SC sk 66 | chr16:11257375-11257402 | SC sk 413 | chr16:11256090-11256117 |
| SC sk 67 | chr16:11257374-11257401 | SC sk 414 | chr16:11256087-11256114 |
| SC sk 68 | chr16:11257373-11257400 | SC sk 415 | chr16:11256083-11256110 |
| SC sk 69 | chr16:11257363-11257390 | SC sk 416 | chr16:11256082-11256109 |
| SC sk 70 | chr16:11257362-11257389 | SC sk 417 | chr16:11256079-11256106 |
| SC sk 71 | chr16:11257361-11257388 | SC sk 418 | chr16:11256078-11256105 |
| SC sk 72 | chr16:11257343-11257370 | SC sk 419 | chr16:11256078-11256105 |
| SC sk 73 | chr16:11257341-11257368 | SC sk 420 | chr16:11256070-11256097 |
| SC sk 74 | chr16:11257334-11257361 | SC sk 421 | chr16:11256069-11256096 |
| SC sk 75 | chr16:11257331-11257358 | SC sk 422 | chr16:11256069-11256096 |
| SC sk 76 | chr16:11257317-11257344 | SC sk 423 | chr16:11256068-11256095 |
| SC sk 77 | chr16:11257308-11257335 | SC sk 424 | chr16:11256063-11256090 |
| SC sk 78 | chr16:11257301-11257328 | SC sk 425 | chr16:11256063-11256090 |
| SC sk 79 | chr16:11257300-11257327 | SC sk 426 | chr16:11256062-11256089 |
| SC sk 80 | chr16:11257295-11257322 | SC sk 427 | chr16:11256062-11256089 |
| SC sk 81 | chr16:11257289-11257316 | SC sk 428 | chr16:11256061-11256088 |
| SC sk 82 | chr16:11257237-11257264 | SC sk 429 | chr16:11256058-11256085 |
| SC sk 83 | chr16:11257236-11257263 | SC sk 430 | chr16:11256057-11256084 |
| SC sk 84 | chr16:11257235-11257262 | SC sk 431 | chr16:11256055-11256082 |
| SC sk 85 | chr16:11257231-11257258 | SC sk 432 | chr16:11256054-11256081 |
| SC sk 86 | chr16:11257229-11257256 | SC sk 433 | chr16:11256051-11256078 |
| SC sk 87 | chr16:11257228-11257255 | SC sk 434 | chr16:11256050-11256077 |
| SC sk 88 | chr16:11257224-11257251 | SC sk 435 | chr16:11256047-11256074 |
| SC sk 89 | chr16:11257221-11257248 | SC sk 436 | chr16:11256046-11256073 |
| SC sk 90 | chr16:11257220-11257247 | SC sk 437 | chr16:11256046-11256073 |
| SC sk 91 | chr16:11257217-11257244 | SC sk 438 | chr16:11256043-11256070 |
| SC sk 92 | chr16:11257217-11257244 | SC sk 439 | chr16:11256041-11256068 |
| SC sk 93 | chr16:11257214-11257241 | SC sk 440 | chr16:11256040-11256067 |
| SC sk 94 | chr16:11257213-11257240 | SC sk 441 | chr16:11256040-11256067 |
| SC sk 95 | chr16:11257213-11257240 | SC sk 442 | chr16:11256039-11256066 |
| SC sk 96 | chr16:11257212-11257239 | SC sk 443 | chr16:11256036-11256063 |
| SC sk 97 | chr16:11257209-11257236 | SC sk 444 | chr16:11256035-11256062 |
| SC sk 98 | chr16:11257207-11257234 | SC sk 445 | chr16:11256034-11256061 |
| SC sk 99 | chr16:11257206-11257233 | SC sk 446 | chr16:11256026-11256053 |
| SC sk 100 | chr16:11257205-11257232 | SC sk 447 | chr16:11256025-11256052 |
| SC sk 101 | chr16:11257201-11257228 | SC sk 448 | chr16:11256017-11256044 |
| SC sk 102 | chr16:11257196-11257223 | SC sk 449 | chr16:11256011-11256038 |
| SC sk 103 | chr16:11257195-11257222 | SC sk 450 | chr16:11256010-11256037 |
| SC sk 104 | chr16:11257181-11257208 | SC sk 451 | chr16:11256003-11256030 |
| SC sk 105 | chr16:11257172-11257199 | SC sk 452 | chr16:11256000-11256027 |
| SC sk 106 | chr16:11257169-11257196 | SC sk 453 | chr16:11255999-11256026 |
| SC sk 107 | chr16:11257168-11257195 | SC sk 454 | chr16:11255998-11256025 |
| SC sk 108 | chr16:11257167-11257194 | SC sk 455 | chr16:11255997-11256024 |
| SC sk 109 | chr16:11257166-11257193 | SC sk 456 | chr16:11255994-11256021 |
| SC sk 110 | chr16:11257161-11257188 | SC sk 457 | chr16:11255986-11256013 |
| SC sk 111 | chr16:11257154-11257181 | SC sk 458 | chr16:11255985-11256012 |
| SC sk 112 | chr16:11257150-11257177 | SC sk 459 | chr16:11255984-11256011 |
| SC sk 113 | chr16:11257149-11257176 | SC sk 460 | chr16:11255978-11256005 |
| SC sk 114 | chr16:11257148-11257175 | SC sk 461 | chr16:11255978-11256005 |
| SC sk 115 | chr16:11257144-11257171 | SC sk 462 | chr16:11255953-11255980 |
| SC sk 116 | chr16:11257143-11257170 | SC sk 463 | chr16:11255950-11255977 |
| SC sk 117 | chr16:11257142-11257169 | SC sk 464 | chr16:11255942-11255969 |
| SC sk 118 | chr16:11257141-11257168 | SC sk 465 | chr16:11255942-11255969 |
| SC sk 119 | chr16:11257136-11257163 | SC sk 466 | chr16:11255941-11255968 |
| SC sk 120 | chr16:11257133-11257160 | SC sk 467 | chr16:11255940-11255967 |
| SC sk 121 | chr16:11257132-11257159 | SC sk 468 | chr16:11255935-11255962 |
| SC sk 122 | chr16:11257127-11257154 | SC sk 469 | chr16:11255934-11255961 |
| SC sk 123 | chr16:11257119-11257146 | SC sk 470 | chr16:11255929-11255956 |
| SC sk 124 | chr16:11257114-11257141 | SC sk 471 | chr16:11255925-11255952 |
| SC sk 125 | chr16:11257113-11257140 | SC sk 472 | chr16:11255925-11255952 |
| SC sk 126 | chr16:11257112-11257139 | SC sk 473 | chr16:11255924-11255951 |
| SC sk 127 | chr16:11257096-11257123 | SC sk 474 | chr16:11255924-11255951 |
| SC sk 128 | chr16:11257075-11257102 | SC sk 475 | chr16:11255923-11255950 |
| SC sk 129 | chr16:11257074-11257101 | SC sk 476 | chr16:11255923-11255950 |
| SC sk 130 | chr16:11257062-11257089 | SC sk 477 | chr16:11255920-11255947 |
| SC sk 131 | chr16:11257044-11257071 | SC sk 478 | chr16:11255919-11255946 |
| SC sk 132 | chr16:11257029-11257056 | SC sk 479 | chr16:11255914-11255941 |
| SC sk 133 | chr16:11257026-11257053 | SC sk 480 | chr16:11255911-11255938 |
| SC sk 134 | chr16:11257025-11257052 | SC sk 481 | chr16:11255910-11255937 |
| SC sk 135 | chr16:11257024-11257051 | SC sk 482 | chr16:11255909-11255936 |
| SC sk 136 | chr16:11257020-11257047 | SC sk 483 | chr16:11255908-11255935 |
| SC sk 137 | chr16:11257020-11257047 | SC sk 484 | chr16:11255907-11255934 |
| SC sk 138 | chr16:11257015-11257042 | SC sk 485 | chr16:11255898-11255925 |
| SC sk 139 | chr16:11257014-11257041 | SC sk 486 | chr16:11255893-11255920 |
| SC sk 140 | chr16:11257013-11257040 | SC sk 487 | chr16:11255892-11255919 |
| SC sk 141 | chr16:11257009-11257036 | SC sk 488 | chr16:11255881-11255908 |
| SC sk 142 | chr16:11256986-11257013 | SC sk 489 | chr16:11255874-11255901 |
| SC sk 143 | chr16:11256980-11257007 | SC sk 490 | chr16:11255872-11255899 |
| SC sk 144 | chr16:11256972-11256999 | SC sk 491 | chr16:11255870-11255897 |
| SC sk 145 | chr16:11256971-11256998 | SC sk 492 | chr16:11255869-11255896 |
| SC sk 146 | chr16:11256959-11256986 | SC sk 493 | chr16:11255867-11255894 |
| SC sk 147 | chr16:11256948-11256975 | SC sk 494 | chr16:11255864-11255891 |
| SC sk 148 | chr16:11256947-11256974 | SC sk 495 | chr16:11255863-11255890 |
| SC sk 149 | chr16:11256943-11256970 | SC sk 496 | chr16:11255855-11255882 |
| SC sk 150 | chr16:11256942-11256969 | SC sk 497 | chr16:11255855-11255882 |
| SC sk 151 | chr16:11256941-11256968 | SC sk 498 | chr16:11255854-11255881 |
| SC sk 152 | chr16:11256938-11256965 | SC sk 499 | chr16:11255849-11255876 |
| SC sk 153 | chr16:11256938-11256965 | SC sk 500 | chr16:11255847-11255874 |
| SC sk 154 | chr16:11256937-11256964 | SC sk 501 | chr16:11255844-11255871 |
| SC sk 155 | chr16:11256937-11256964 | SC sk 502 | chr16:11255841-11255868 |
| SC sk 156 | chr16:11256936-11256963 | SC sk 503 | chr16:11255840-11255867 |
| SC sk 157 | chr16:11256932-11256959 | SC sk 504 | chr16:11255837-11255864 |
| SC sk 158 | chr16:11256931-11256958 | SC sk 505 | chr16:11255836-11255863 |
| SC sk 159 | chr16:11256930-11256957 | SC sk 506 | chr16:11255835-11255862 |
| SC sk 160 | chr16:11256922-11256949 | SC sk 507 | chr16:11255834-11255861 |
| SC sk 161 | chr16:11256921-11256948 | SC sk 508 | chr16:11255833-11255860 |
| SC sk 162 | chr16:11256920-11256947 | SC sk 509 | chr16:11255832-11255859 |
| SC sk 163 | chr16:11256918-11256945 | SC sk 510 | chr16:11255830-11255857 |
| SC sk 164 | chr16:11256917-11256944 | SC sk 511 | chr16:11255827-11255854 |
| SC sk 165 | chr16:11256914-11256941 | SC sk 512 | chr16:11255822-11255849 |
| SC sk 166 | chr16:11256908-11256935 | SC sk 513 | chr16:11255821-11255848 |
| SC sk 167 | chr16:11256900-11256927 | SC sk 514 | chr16:11255820-11255847 |
| SC sk 168 | chr16:11256899-11256926 | SC sk 515 | chr16:11255816-11255843 |
| SC sk 169 | chr16:11256888-11256915 | SC sk 516 | chr16:11255812-11255839 |
| SC sk 170 | chr16:11256880-11256907 | SC sk 517 | chr16:11255808-11255835 |
| SC sk 171 | chr16:11256877-11256904 | SC sk 518 | chr16:11255798-11255825 |
| SC sk 172 | chr16:11256870-11256897 | SC sk 519 | chr16:11255796-11255823 |
| SC sk 173 | chr16:11256869-11256896 | SC sk 520 | chr16:11255792-11255819 |
| SC sk 174 | chr16:11256865-11256892 | SC sk 521 | chr16:11255778-11255805 |
| SC sk 175 | chr16:11256864-11256891 | SC sk 522 | chr16:11255772-11255799 |
| SC sk 176 | chr16:11256863-11256890 | SC sk 523 | chr16:11255772-11255799 |
| SC sk 177 | chr16:11256862-11256889 | SC sk 524 | chr16:11255762-11255789 |
| SC sk 178 | chr16:11256860-11256887 | SC sk 525 | chr16:11255761-11255788 |
| SC sk 179 | chr16:11256859-11256886 | SC sk 526 | chr16:11255759-11255786 |
| SC sk 180 | chr16:11256858-11256885 | SC sk 527 | chr16:11255756-11255783 |
| SC sk 181 | chr16:11256858-11256885 | SC sk 528 | chr16:11255755-11255782 |
| SC sk 182 | chr16:11256857-11256884 | SC sk 529 | chr16:11255755-11255782 |
| SC sk 183 | chr16:11256856-11256883 | SC sk 530 | chr16:11255754-11255781 |
| SC sk 184 | chr16:11256855-11256882 | SC sk 531 | chr16:11255753-11255780 |
| SC sk 185 | chr16:11256854-11256881 | SC sk 532 | chr16:11255752-11255779 |
| SC sk 186 | chr16:11256853-11256880 | SC sk 533 | chr16:11255739-11255766 |
| SC sk 187 | chr16:11256850-11256877 | SC sk 534 | chr16:11255738-11255765 |
| SC sk 188 | chr16:11256847-11256874 | SC sk 535 | chr16:11255731-11255758 |
| SC sk 189 | chr16:11256834-11256861 | SC sk 536 | chr16:11255724-11255751 |
| SC sk 190 | chr16:11256834-11256861 | SC sk 537 | chr16:11255724-11255751 |
| SC sk 191 | chr16:11256833-11256860 | SC sk 538 | chr16:11255723-11255750 |
| SC sk 192 | chr16:11256832-11256859 | SC sk 539 | chr16:11255721-11255748 |
| SC sk 193 | chr16:11256820-11256847 | SC sk 540 | chr16:11255718-11255745 |
| SC sk 194 | chr16:11256819-11256846 | SC sk 541 | chr16:11255715-11255742 |
| SC sk 195 | chr16:11256814-11256841 | SC sk 542 | chr16:11255710-11255737 |
| SC sk 196 | chr16:11256814-11256841 | SC sk 543 | chr16:11255710-11255737 |
| SC sk 197 | chr16:11256811-11256838 | SC sk 544 | chr16:11255709-11255736 |
| SC sk 198 | chr16:11256809-11256836 | SC sk 545 | chr16:11255708-11255735 |
| SC sk 199 | chr16:11256802-11256829 | SC sk 546 | chr16:11255707-11255734 |
| SC sk 200 | chr16:11256795-11256822 | SC sk 547 | chr16:11255704-11255731 |
| SC sk 201 | chr16:11256794-11256821 | SC sk 548 | chr16:11255703-11255730 |
| SC sk 202 | chr16:11256793-11256820 | SC sk 549 | chr16:11255700-11255727 |
| SC sk 203 | chr16:11256781-11256808 | SC sk 550 | chr16:11255688-11255715 |
| SC sk 204 | chr16:11256778-11256805 | SC sk 551 | chr16:11255674-11255701 |
| SC sk 205 | chr16:11256772-11256799 | SC sk 552 | chr16:11255673-11255700 |
| SC sk 206 | chr16:11256771-11256798 | SC sk 553 | chr16:11255672-11255699 |
| SC sk 207 | chr16:11256766-11256793 | SC sk 554 | chr16:11255666-11255693 |
| SC sk 208 | chr16:11256763-11256790 | SC sk 555 | chr16:11255656-11255683 |
| SC sk 209 | chr16:11256762-11256789 | SC sk 556 | chr16:11255655-11255682 |
| SC sk 210 | chr16:11256762-11256789 | SC sk 557 | chr16:11255650-11255677 |
| SC sk 211 | chr16:11256758-11256785 | SC sk 558 | chr16:11255636-11255663 |
| SC sk 212 | chr16:11256757-11256784 | SC sk 559 | chr16:11255628-11255655 |
| SC sk 213 | chr16:11256757-11256784 | SC sk 560 | chr16:11255627-11255654 |
| SC sk 214 | chr16:11256756-11256783 | SC sk 561 | chr16:11255623-11255650 |
| SC sk 215 | chr16:11256751-11256778 | SC sk 562 | chr16:11255622-11255649 |
| SC sk 216 | chr16:11256750-11256777 | SC sk 563 | chr16:11255618-11255645 |
| SC sk 217 | chr16:11256749-11256776 | SC sk 564 | chr16:11255614-11255641 |
| SC sk 218 | chr16:11256744-11256771 | SC sk 565 | chr16:11255613-11255640 |
| SC sk 219 | chr16:11256741-11256768 | SC sk 566 | chr16:11255609-11255636 |
| SC sk 220 | chr16:11256740-11256767 | SC sk 567 | chr16:11255608-11255635 |
| SC sk 221 | chr16:11256738-11256765 | SC sk 568 | chr16:11255607-11255634 |
| SC sk 222 | chr16:11256737-11256764 | SC sk 569 | chr16:11255606-11255633 |
| SC sk 223 | chr16:11256736-11256763 | SC sk 570 | chr16:11255599-11255626 |
| SC sk 224 | chr16:11256734-11256761 | SC sk 571 | chr16:11255598-11255625 |
| SC sk 225 | chr16:11256727-11256754 | SC sk 572 | chr16:11255593-11255620 |
| SC sk 226 | chr16:11256726-11256753 | SC sk 573 | chr16:11255590-11255617 |
| SC sk 227 | chr16:11256717-11256744 | SC sk 574 | chr16:11255588-11255615 |
| SC sk 228 | chr16:11256714-11256741 | SC sk 575 | chr16:11255584-11255611 |
| SC sk 229 | chr16:11256713-11256740 | SC sk 576 | chr16:11255581-11255608 |
| SC sk 230 | chr16:11256710-11256737 | SC sk 577 | chr16:11255578-11255605 |
| SC sk 231 | chr16:11256704-11256731 | SC sk 578 | chr16:11255577-11255604 |
| SC sk 232 | chr16:11256703-11256730 | SC sk 579 | chr16:11255574-11255601 |
| SC sk 233 | chr16:11256702-11256729 | SC sk 580 | chr16:11255569-11255596 |
| SC sk 234 | chr16:11256697-11256724 | SC sk 581 | chr16:11255564-11255591 |
| SC sk 235 | chr16:11256694-11256721 | SC sk 582 | chr16:11255560-11255587 |
| SC sk 236 | chr16:11256693-11256720 | SC sk 583 | chr16:11255550-11255577 |
| SC sk 237 | chr16:11256692-11256719 | SC sk 584 | chr16:11255546-11255573 |
| SC sk 238 | chr16:11256691-11256718 | SC sk 585 | chr16:11255545-11255572 |
| SC sk 239 | chr16:11256684-11256711 | SC sk 586 | chr16:11255541-11255568 |
| SC sk 240 | chr16:11256683-11256710 | SC sk 587 | chr16:11255540-11255567 |
| SC sk 241 | chr16:11256680-11256707 | SC sk 588 | chr16:11255539-11255566 |
| SC sk 242 | chr16:11256679-11256706 | SC sk 589 | chr16:11255531-11255558 |
| SC sk 243 | chr16:11256678-11256705 | SC sk 590 | chr16:11255530-11255557 |
| SC sk 244 | chr16:11256673-11256700 | SC sk 591 | chr16:11255529-11255556 |
| SC sk 245 | chr16:11256667-11256694 | SC sk 592 | chr16:11255521-11255548 |
| SC sk 246 | chr16:11256666-11256693 | SC sk 593 | chr16:11255518-11255545 |
| SC sk 247 | chr16:11256662-11256689 | SC sk 594 | chr16:11255509-11255536 |
| SC sk 248 | chr16:11256661-11256688 | SC sk 595 | chr16:11255508-11255535 |
| SC sk 249 | chr16:11256660-11256687 | SC sk 596 | chr16:11255507-11255534 |
| SC sk 250 | chr16:11256647-11256674 | SC sk 597 | chr16:11255497-11255524 |
| SC sk 251 | chr16:11256638-11256665 | SC sk 598 | chr16:11255496-11255523 |
| SC sk 252 | chr16:11256637-11256664 | SC sk 599 | chr16:11255496-11255523 |
| SC sk 253 | chr16:11256636-11256663 | SC sk 600 | chr16:11255495-11255522 |
| SC sk 254 | chr16:11256635-11256662 | SC sk 601 | chr16:11255494-11255521 |
| SC sk 255 | chr16:11256629-11256656 | SC sk 602 | chr16:11255492-11255519 |
| SC sk 256 | chr16:11256629-11256656 | SC sk 603 | chr16:11255481-11255508 |
| SC sk 257 | chr16:11256622-11256649 | SC sk 604 | chr16:11255480-11255507 |
| SC sk 258 | chr16:11256621-11256648 | SC sk 605 | chr16:11255479-11255506 |
| SC sk 259 | chr16:11256620-11256647 | SC sk 606 | chr16:11255477-11255504 |
| SC sk 260 | chr16:11256619-11256646 | SC sk 607 | chr16:11255476-11255503 |
| SC sk 261 | chr16:11256617-11256644 | SC sk 608 | chr16:11255475-11255502 |
| SC sk 262 | chr16:11256616-11256643 | SC sk 609 | chr16:11255475-11255502 |
| SC sk 263 | chr16:11256612-11256639 | SC sk 610 | chr16:11254808-11254835 |
| SC sk 264 | chr16:11256607-11256634 | SC sk 611 | chr16:11254807-11254834 |
| SC sk 265 | chr16:11256606-11256633 | SC sk 612 | chr16:11254806-11254833 |
| SC sk 266 | chr16:11256594-11256621 | SC sk 613 | chr16:11254805-11254832 |
| SC sk 267 | chr16:11256591-11256618 | SC sk 614 | chr16:11254802-11254829 |
| SC sk 268 | chr16:11256590-11256617 | SC sk 615 | chr16:11254776-11254803 |
| SC sk 269 | chr16:11256585-11256612 | SC sk 616 | chr16:11254776-11254803 |
| SC sk 270 | chr16:11256575-11256602 | SC sk 617 | chr16:11254766-11254793 |
| SC sk 271 | chr16:11256574-11256601 | SC sk 618 | chr16:11254765-11254792 |
| SC sk 272 | chr16:11256573-11256600 | SC sk 619 | chr16:11254753-11254780 |
| SC sk 273 | chr16:11256573-11256600 | SC sk 620 | chr16:11254753-11254780 |
| SC sk 274 | chr16:11256572-11256599 | SC sk 621 | chr16:11254748-11254775 |
| SC sk 275 | chr16:11256571-11256598 | SC sk 622 | chr16:11254747-11254774 |
| SC sk 276 | chr16:11256570-11256597 | SC sk 623 | chr16:11254746-11254773 |
| SC sk 277 | chr16:11256570-11256597 | SC sk 624 | chr16:11254743-11254770 |
| SC sk 278 | chr16:11256567-11256594 | SC sk 625 | chr16:11254740-11254767 |
| SC sk 279 | chr16:11256564-11256591 | SC sk 626 | chr16:11254739-11254766 |
| SC sk 280 | chr16:11256563-11256590 | SC sk 627 | chr16:11254735-11254762 |
| SC sk 281 | chr16:11256554-11256581 | SC sk 628 | chr16:11254734-11254761 |
| SC sk 282 | chr16:11256553-11256580 | SC sk 629 | chr16:11254734-11254761 |
| SC sk 283 | chr16:11256539-11256566 | SC sk 630 | chr16:11254731-11254758 |
| SC sk 284 | chr16:11256538-11256565 | SC sk 631 | chr16:11254730-11254757 |
| SC sk 285 | chr16:11256533-11256560 | SC sk 632 | chr16:11254730-11254757 |
| SC sk 286 | chr16:11256530-11256557 | SC sk 633 | chr16:11254729-11254756 |
| SC sk 287 | chr16:11256520-11256547 | SC sk 634 | chr16:11254729-11254756 |
| SC sk 288 | chr16:11256520-11256547 | SC sk 635 | chr16:11254725-11254752 |
| SC sk 289 | chr16:11256519-11256546 | SC sk 636 | chr16:11254723-11254750 |
| SC sk 290 | chr16:11256511-11256538 | SC sk 637 | chr16:11254722-11254749 |
| SC sk 291 | chr16:11256510-11256537 | SC sk 638 | chr16:11254721-11254748 |
| SC sk 292 | chr16:11256505-11256532 | SC sk 639 | chr16:11254716-11254743 |
| SC sk 293 | chr16:11256504-11256531 | SC sk 640 | chr16:11254700-11254727 |
| SC sk 294 | chr16:11256503-11256530 | SC sk 641 | chr16:11254696-11254723 |
| SC sk 295 | chr16:11256494-11256521 | SC sk 642 | chr16:11254688-11254715 |
| SC sk 296 | chr16:11256493-11256520 | SC sk 643 | chr16:11254686-11254713 |
| SC sk 297 | chr16:11256492-11256519 | SC sk 644 | chr16:11254683-11254710 |
| SC sk 298 | chr16:11256490-11256517 | SC sk 645 | chr16:11254682-11254709 |
| SC sk 299 | chr16:11256489-11256516 | SC sk 646 | chr16:11254679-11254706 |
| SC sk 300 | chr16:11256476-11256503 | SC sk 647 | chr16:11254678-11254705 |
| SC sk 301 | chr16:11256473-11256500 | SC sk 648 | chr16:11254661-11254688 |
| SC sk 302 | chr16:11256472-11256499 | SC sk 649 | chr16:11254660-11254687 |
| SC sk 303 | chr16:11256471-11256498 | SC sk 650 | chr16:11254660-11254687 |
| SC sk 304 | chr16:11256461-11256488 | SC sk 651 | chr16:11254659-11254686 |
| SC sk 305 | chr16:11256459-11256486 | SC sk 652 | chr16:11254658-11254685 |
| SC sk 306 | chr16:11256455-11256482 | SC sk 653 | chr16:11254653-11254680 |
| SC sk 307 | chr16:11256455-11256482 | SC sk 654 | chr16:11254652-11254679 |
| SC sk 308 | chr16:11256451-11256478 | SC sk 655 | chr16:11254651-11254678 |
| SC sk 309 | chr16:11256445-11256472 | SC sk 656 | chr16:11254643-11254670 |
| SC sk 310 | chr16:11256444-11256471 | SC sk 657 | chr16:11254643-11254670 |
| SC sk 311 | chr16:11256442-11256469 | SC sk 658 | chr16:11254631-11254658 |
| SC sk 312 | chr16:11256440-11256467 | SC sk 659 | chr16:11254630-11254657 |
| SC sk 313 | chr16:11256439-11256466 | SC sk 660 | chr16:11254629-11254656 |
| SC sk 314 | chr16:11256438-11256465 | SC sk 661 | chr16:11254626-11254653 |
| SC sk 315 | chr16:11256431-11256458 | SC sk 662 | chr16:11254625-11254652 |
| SC sk 316 | chr16:11256420-11256447 | SC sk 663 | chr16:11254624-11254651 |
| SC sk 317 | chr16:11256417-11256444 | SC sk 664 | chr16:11254623-11254650 |
| SC sk 318 | chr16:11256416-11256443 | SC sk 665 | chr16:11254621-11254648 |
| SC sk 319 | chr16:11256410-11256437 | SC sk 666 | chr16:11254620-11254647 |
| SC sk 320 | chr16:11256410-11256437 | SC sk 667 | chr16:11254611-11254638 |
| SC sk 321 | chr16:11256409-11256436 | SC sk 668 | chr16:11254610-11254637 |
| SC sk 322 | chr16:11256408-11256435 | SC sk 669 | chr16:11254601-11254628 |
| SC sk 323 | chr16:11256404-11256431 | SC sk 670 | chr16:11254600-11254627 |
| SC sk 324 | chr16:11256404-11256431 | SC sk 671 | chr16:11254599-11254626 |
| SC sk 325 | chr16:11256403-11256430 | SC sk 672 | chr16:11254598-11254625 |
| SC sk 326 | chr16:11256402-11256429 | SC sk 673 | chr16:11254594-11254621 |
| SC sk 327 | chr16:11256394-11256421 | SC sk 674 | chr16:11254587-11254614 |
| SC sk 328 | chr16:11256391-11256418 | SC sk 675 | chr16:11254564-11254591 |
| SC sk 329 | chr16:11256390-11256417 | SC sk 676 | chr16:11254558-11254585 |
| SC sk 330 | chr16:11256384-11256411 | SC sk 677 | chr16:11254546-11254573 |
| SC sk 331 | chr16:11256383-11256410 | SC sk 678 | chr16:11254543-11254570 |
| SC sk 332 | chr16:11256375-11256402 | SC sk 679 | chr16:11254539-11254566 |
| SC sk 333 | chr16:11256374-11256401 | SC sk 680 | chr16:11254523-11254550 |
| SC sk 334 | chr16:11256371-11256398 | SC sk 681 | chr16:11254522-11254549 |
| SC sk 335 | chr16:11256370-11256397 | SC sk 682 | chr16:11254521-11254548 |
| SC sk 336 | chr16:11256357-11256384 | SC sk 683 | chr16:11254519-11254546 |
| SC sk 337 | chr16:11256356-11256383 | SC sk 684 | chr16:11254518-11254545 |
| SC sk 338 | chr16:11256355-11256382 | SC sk 685 | chr16:11254517-11254544 |
| SC sk 339 | chr16:11256354-11256381 | SC sk 686 | chr16:11254516-11254543 |
| SC sk 340 | chr16:11256353-11256380 | SC sk 687 | chr16:11254515-11254542 |
| SC sk 341 | chr16:11256349-11256376 | SC sk 688 | chr16:11254514-11254541 |
| SC sk 342 | chr16:11256348-11256375 | SC sk 689 | chr16:11254511-11254538 |
| SC sk 343 | chr16:11256341-11256368 | SC sk 690 | chr16:11254510-11254537 |
| SC sk 344 | chr16:11256339-11256366 | SC sk 691 | chr16:11254503-11254530 |
| SC sk 345 | chr16:11256329-11256356 | SC sk 692 | chr16:11254500-11254527 |
| SC sk 346 | chr16:11256320-11256347 | SC sk 693 | chr16:11254451-11254478 |
| SC sk 347 | chr16:11256317-11256344 | SC sk 694 | chr16:11254448-11254475 |

**Table 3D. Human CBLB genomic coordinate of the present invention**

| No. | Chromosomal coordinate range | No. | Chromosomal coordinate range |
|---|---|---|---|
| CB sk 1 | chr3:105655489-105655516 | CB sk 546 | chr3:105702237-105702264 |
| CB sk 2 | chr3:105655515-105655542 | CB sk 547 | chr3:105702243-105702270 |
| CB sk 3 | chr3:105655528-105655555 | CB sk 548 | chr3:105702244-105702271 |
| CB sk 4 | chr3:105655581-105655608 | CB sk 549 | chr3:105702253-105702280 |
| CB sk 5 | chr3:105655584-105655611 | CB sk 550 | chr3:105702254-105702281 |
| CB sk 6 | chr3:105655611-105655638 | CB sk 551 | chr3:105702261-105702288 |
| CB sk 7 | chr3:105655612-105655639 | CB sk 552 | chr3:105702264-105702291 |
| CB sk 8 | chr3:105655613-105655640 | CB sk 553 | chr3:105702265-105702292 |
| CB sk 9 | chr3:105655635-105655662 | CB sk 554 | chr3:105702267-105702294 |
| CB sk 10 | chr3:105655647-105655674 | CB sk 555 | chr3:105702270-105702297 |
| CB sk 11 | chr3:105655648-105655675 | CB sk 556 | chr3:105702271-105702298 |
| CB sk 12 | chr3:105655679-105655706 | CB sk 557 | chr3:105702274-105702301 |
| CB sk 13 | chr3:105655684-105655711 | CB sk 558 | chr3:105702278-105702305 |
| CB sk 14 | chr3:105655685-105655712 | CB sk 559 | chr3:105702282-105702309 |
| CB sk 15 | chr3:105655687-105655714 | CB sk 560 | chr3:105702301-105702328 |
| CB sk 16 | chr3:105655702-105655729 | CB sk 561 | chr3:105702318-105702345 |
| CB sk 17 | chr3:105655720-105655747 | CB sk 562 | chr3:105702327-105702354 |
| CB sk 18 | chr3:105655752-105655779 | CB sk 563 | chr3:105702330-105702357 |
| CB sk 19 | chr3:105655790-105655817 | CB sk 564 | chr3:105702331-105702358 |
| CB sk 20 | chr3:105655834-105655861 | CB sk 565 | chr3:105702336-105702363 |
| CB sk 21 | chr3:105655846-105655873 | CB sk 566 | chr3:105702339-105702366 |
| CB sk 22 | chr3:105655899-105655926 | CB sk 567 | chr3:105702342-105702369 |
| CB sk 23 | chr3:105655926-105655953 | CB sk 568 | chr3:105702351-105702378 |
| CB sk 24 | chr3:105655927-105655954 | CB sk 569 | chr3:105702354-105702381 |
| CB sk 25 | chr3:105655949-105655976 | CB sk 570 | chr3:105702357-105702384 |
| CB sk 26 | chr3:105655950-105655977 | CB sk 571 | chr3:105702360-105702387 |
| CB sk 27 | chr3:105655956-105655983 | CB sk 572 | chr3:105702363-105702390 |
| CB sk 28 | chr3:105655957-105655984 | CB sk 573 | chr3:105702366-105702393 |
| CB sk 29 | chr3:105655979-105656006 | CB sk 574 | chr3:105702377-105702404 |
| CB sk 30 | chr3:105655980-105656007 | CB sk 575 | chr3:105702378-105702405 |
| CB sk 31 | chr3:105655987-105656014 | CB sk 576 | chr3:105702381-105702408 |
| CB sk 32 | chr3:105655988-105656015 | CB sk 577 | chr3:105702384-105702411 |
| CB sk 33 | chr3:105655999-105656026 | CB sk 578 | chr3:105702387-105702414 |
| CB sk 34 | chr3:105656000-105656027 | CB sk 579 | chr3:105702393-105702420 |
| CB sk 35 | chr3:105656012-105656039 | CB sk 580 | chr3:105702394-105702421 |
| CB sk 36 | chr3:105656024-105656051 | CB sk 581 | chr3:105702399-105702426 |
| CB sk 37 | chr3:105656028-105656055 | CB sk 582 | chr3:105702426-105702453 |
| CB sk 38 | chr3:105656078-105656105 | CB sk 583 | chr3:105702444-105702471 |
| CB sk 39 | chr3:105656099-105656126 | CB sk 584 | chr3:105703966-105703993 |
| CB sk 40 | chr3:105656100-105656127 | CB sk 585 | chr3:105703978-105704005 |
| CB sk 41 | chr3:105656101-105656128 | CB sk 586 | chr3:105703979-105704006 |
| CB sk 42 | chr3:105656117-105656144 | CB sk 587 | chr3:105703987-105704014 |
| CB sk 43 | chr3:105656132-105656159 | CB sk 588 | chr3:105703989-105704016 |
| CB sk 44 | chr3:105656145-105656172 | CB sk 589 | chr3:105703990-105704017 |
| CB sk 45 | chr3:105656176-105656203 | CB sk 590 | chr3:105703998-105704025 |
| CB sk 46 | chr3:105656177-105656204 | CB sk 591 | chr3:105703999-105704026 |
| CB sk 47 | chr3:105656179-105656206 | CB sk 592 | chr3:105704010-105704037 |
| CB sk 48 | chr3:105656181-105656208 | CB sk 593 | chr3:105704024-105704051 |
| CB sk 49 | chr3:105656182-105656209 | CB sk 594 | chr3:105704029-105704056 |
| CB sk 50 | chr3:105656189-105656216 | CB sk 595 | chr3:105704031-105704058 |
| CB sk 51 | chr3:105656190-105656217 | CB sk 596 | chr3:105704032-105704059 |
| CB sk 52 | chr3:105656191-105656218 | CB sk 597 | chr3:105704037-105704064 |
| CB sk 53 | chr3:105656201-105656228 | CB sk 598 | chr3:105704038-105704065 |
| CB sk 54 | chr3:105656229-105656256 | CB sk 599 | chr3:105704041-105704068 |
| CB sk 55 | chr3:105656291-105656318 | CB sk 600 | chr3:105704045-105704072 |
| CB sk 56 | chr3:105656295-105656322 | CB sk 601 | chr3:105704047-105704074 |
| CB sk 57 | chr3:105656301-105656328 | CB sk 602 | chr3:105704056-105704083 |
| CB sk 58 | chr3:105656312-105656339 | CB sk 603 | chr3:105704057-105704084 |
| CB sk 59 | chr3:105656334-105656361 | CB sk 604 | chr3:105704063-105704090 |
| CB sk 60 | chr3:105656369-105656396 | CB sk 605 | chr3:105704068-105704095 |
| CB sk 61 | chr3:105656391-105656418 | CB sk 606 | chr3:105704069-105704096 |
| CB sk 62 | chr3:105656405-105656432 | CB sk 607 | chr3:105704074-105704101 |
| CB sk 63 | chr3:105656421-105656448 | CB sk 608 | chr3:105704080-105704107 |
| CB sk 64 | chr3:105656422-105656449 | CB sk 609 | chr3:105704093-105704120 |
| CB sk 65 | chr3:105656481-105656508 | CB sk 610 | chr3:105704094-105704121 |
| CB sk 66 | chr3:105656493-105656520 | CB sk 611 | chr3:105704099-105704126 |
| CB sk 67 | chr3:105656516-105656543 | CB sk 612 | chr3:105704100-105704127 |
| CB sk 68 | chr3:105656551-105656578 | CB sk 613 | chr3:105704101-105704128 |
| CB sk 69 | chr3:105656554-105656581 | CB sk 614 | chr3:105704106-105704133 |
| CB sk 70 | chr3:105656558-105656585 | CB sk 615 | chr3:105704113-105704140 |
| CB sk 71 | chr3:105656611-105656638 | CB sk 616 | chr3:105704125-105704152 |
| CB sk 72 | chr3:105656612-105656639 | CB sk 617 | chr3:105704136-105704163 |
| CB sk 73 | chr3:105656613-105656640 | CB sk 618 | chr3:105704162-105704189 |
| CB sk 74 | chr3:105656614-105656641 | CB sk 619 | chr3:105715186-105715213 |
| CB sk 75 | chr3:105656615-105656642 | CB sk 620 | chr3:105715199-105715226 |
| CB sk 76 | chr3:105656618-105656645 | CB sk 621 | chr3:105715200-105715227 |
| CB sk 77 | chr3:105656619-105656646 | CB sk 622 | chr3:105715209-105715236 |
| CB sk 78 | chr3:105656620-105656647 | CB sk 623 | chr3:105715226-105715253 |
| CB sk 79 | chr3:105656621-105656648 | CB sk 624 | chr3:105715248-105715275 |
| CB sk 80 | chr3:105656622-105656649 | CB sk 625 | chr3:105715249-105715276 |
| CB sk 81 | chr3:105656625-105656652 | CB sk 626 | chr3:105715250-105715277 |
| CB sk 82 | chr3:105656626-105656653 | CB sk 627 | chr3:105715267-105715294 |
| CB sk 83 | chr3:105656627-105656654 | CB sk 628 | chr3:105715268-105715295 |
| CB sk 84 | chr3:105656628-105656655 | CB sk 629 | chr3:105720026-105720053 |
| CB sk 85 | chr3:105656641-105656668 | CB sk 630 | chr3:105720028-105720055 |
| CB sk 86 | chr3:105656658-105656685 | CB sk 631 | chr3:105720029-105720056 |
| CB sk 87 | chr3:105656666-105656693 | CB sk 632 | chr3:105720046-105720073 |
| CB sk 88 | chr3:105656745-105656772 | CB sk 633 | chr3:105720054-105720081 |
| CB sk 89 | chr3:105656777-105656804 | CB sk 634 | chr3:105720061-105720088 |
| CB sk 90 | chr3:105656787-105656814 | CB sk 635 | chr3:105720065-105720092 |
| CB sk 91 | chr3:105656815-105656842 | CB sk 636 | chr3:105720126-105720153 |
| CB sk 92 | chr3:105656825-105656852 | CB sk 637 | chr3:105720129-105720156 |
| CB sk 93 | chr3:105656828-105656855 | CB sk 638 | chr3:105720168-105720195 |
| CB sk 94 | chr3:105656851-105656878 | CB sk 639 | chr3:105720169-105720196 |
| CB sk 95 | chr3:105656853-105656880 | CB sk 640 | chr3:105720184-105720211 |
| CB sk 96 | chr3:105656873-105656900 | CB sk 641 | chr3:105720202-105720229 |
| CB sk 97 | chr3:105656901-105656928 | CB sk 642 | chr3:105720203-105720230 |
| CB sk 98 | chr3:105656902-105656929 | CB sk 643 | chr3:105720204-105720231 |
| CB sk 99 | chr3:105656903-105656930 | CB sk 644 | chr3:105720234-105720261 |
| CB sk 100 | chr3:105656954-105656981 | CB sk 645 | chr3:105720235-105720262 |
| CB sk 101 | chr3:105656977-105657004 | CB sk 646 | chr3:105720240-105720267 |
| CB sk 102 | chr3:105656978-105657005 | CB sk 647 | chr3:105720244-105720271 |
| CB sk 103 | chr3:105656992-105657019 | CB sk 648 | chr3:105720246-105720273 |
| CB sk 104 | chr3:105656994-105657021 | CB sk 649 | chr3:105720250-105720277 |
| CB sk 105 | chr3:105657015-105657042 | CB sk 650 | chr3:105724025-105724052 |
| CB sk 106 | chr3:105657016-105657043 | CB sk 651 | chr3:105724056-105724083 |
| CB sk 107 | chr3:105657033-105657060 | CB sk 652 | chr3:105724065-105724092 |
| CB sk 108 | chr3:105657034-105657061 | CB sk 653 | chr3:105724100-105724127 |
| CB sk 109 | chr3:105657035-105657062 | CB sk 654 | chr3:105724104-105724131 |
| CB sk 110 | chr3:105657038-105657065 | CB sk 655 | chr3:105724134-105724161 |
| CB sk 111 | chr3:105657050-105657077 | CB sk 656 | chr3:105724139-105724166 |
| CB sk 112 | chr3:105657061-105657088 | CB sk 657 | chr3:105724162-105724189 |
| CB sk 113 | chr3:105657074-105657101 | CB sk 658 | chr3:105724165-105724192 |
| CB sk 114 | chr3:105657095-105657122 | CB sk 659 | chr3:105724166-105724193 |
| CB sk 115 | chr3:105657096-105657123 | CB sk 660 | chr3:105724188-105724215 |
| CB sk 116 | chr3:105657102-105657129 | CB sk 661 | chr3:105733988-105734015 |
| CB sk 117 | chr3:105657111-105657138 | CB sk 662 | chr3:105733997-105734024 |
| CB sk 118 | chr3:105657123-105657150 | CB sk 663 | chr3:105734004-105734031 |
| CB sk 119 | chr3:105657124-105657151 | CB sk 664 | chr3:105734008-105734035 |
| CB sk 120 | chr3:105657132-105657159 | CB sk 665 | chr3:105734012-105734039 |
| CB sk 121 | chr3:105657144-105657171 | CB sk 666 | chr3:105734017-105734044 |
| CB sk 122 | chr3:105657167-105657194 | CB sk 667 | chr3:105734026-105734053 |
| CB sk 123 | chr3:105657168-105657195 | CB sk 668 | chr3:105734045-105734072 |
| CB sk 124 | chr3:105657184-105657211 | CB sk 669 | chr3:105734046-105734073 |
| CB sk 125 | chr3:105657189-105657216 | CB sk 670 | chr3:105734082-105734109 |
| CB sk 126 | chr3:105657190-105657217 | CB sk 671 | chr3:105734112-105734139 |
| CB sk 127 | chr3:105657234-105657261 | CB sk 672 | chr3:105734113-105734140 |
| CB sk 128 | chr3:105657237-105657264 | CB sk 673 | chr3:105734117-105734144 |
| CB sk 129 | chr3:105657245-105657272 | CB sk 674 | chr3:105734124-105734151 |
| CB sk 130 | chr3:105657254-105657281 | CB sk 675 | chr3:105734125-105734152 |
| CB sk 131 | chr3:105657258-105657285 | CB sk 676 | chr3:105734126-105734153 |
| CB sk 132 | chr3:105657264-105657291 | CB sk 677 | chr3:105734129-105734156 |
| CB sk 133 | chr3:105657274-105657301 | CB sk 678 | chr3:105734132-105734159 |
| CB sk 134 | chr3:105657290-105657317 | CB sk 679 | chr3:105734133-105734160 |
| CB sk 135 | chr3:105657293-105657320 | CB sk 680 | chr3:105734140-105734167 |
| CB sk 136 | chr3:105657294-105657321 | CB sk 681 | chr3:105737162-105737189 |
| CB sk 137 | chr3:105657311-105657338 | CB sk 682 | chr3:105737165-105737192 |
| CB sk 138 | chr3:105657360-105657387 | CB sk 683 | chr3:105737170-105737197 |
| CB sk 139 | chr3:105657361-105657388 | CB sk 684 | chr3:105737176-105737203 |
| CB sk 140 | chr3:105657368-105657395 | CB sk 685 | chr3:105737200-105737227 |
| CB sk 141 | chr3:105657369-105657396 | CB sk 686 | chr3:105737214-105737241 |
| CB sk 142 | chr3:105657372-105657399 | CB sk 687 | chr3:105737221-105737248 |
| CB sk 143 | chr3:105657435-105657462 | CB sk 688 | chr3:105737237-105737264 |
| CB sk 144 | chr3:105657445-105657472 | CB sk 689 | chr3:105737240-105737267 |
| CB sk 145 | chr3:105657446-105657473 | CB sk 690 | chr3:105737241-105737268 |
| CB sk 146 | chr3:105657462-105657489 | CB sk 691 | chr3:105740470-105740497 |
| CB sk 147 | chr3:105657463-105657490 | CB sk 692 | chr3:105740477-105740504 |
| CB sk 148 | chr3:105657464-105657491 | CB sk 693 | chr3:105740493-105740520 |
| CB sk 149 | chr3:105657465-105657492 | CB sk 694 | chr3:105740494-105740521 |
| CB sk 150 | chr3:105657466-105657493 | CB sk 695 | chr3:105740497-105740524 |
| CB sk 151 | chr3:105657478-105657505 | CB sk 696 | chr3:105740501-105740528 |
| CB sk 152 | chr3:105657485-105657512 | CB sk 697 | chr3:105740502-105740529 |
| CB sk 153 | chr3:105657486-105657513 | CB sk 698 | chr3:105740506-105740533 |
| CB sk 154 | chr3:105657497-105657524 | CB sk 699 | chr3:105740507-105740534 |
| CB sk 155 | chr3:105657501-105657528 | CB sk 700 | chr3:105740509-105740536 |
| CB sk 156 | chr3:105657543-105657570 | CB sk 701 | chr3:105740519-105740546 |
| CB sk 157 | chr3:105657609-105657636 | CB sk 702 | chr3:105740524-105740551 |
| CB sk 158 | chr3:105657619-105657646 | CB sk 703 | chr3:105740563-105740590 |
| CB sk 159 | chr3:105657650-105657677 | CB sk 704 | chr3:105740565-105740592 |
| CB sk 160 | chr3:105657685-105657712 | CB sk 705 | chr3:105740566-105740593 |
| CB sk 161 | chr3:105657689-105657716 | CB sk 706 | chr3:105740570-105740597 |
| CB sk 162 | chr3:105657693-105657720 | CB sk 707 | chr3:105740571-105740598 |
| CB sk 163 | chr3:105657696-105657723 | CB sk 708 | chr3:105740572-105740599 |
| CB sk 164 | chr3:105657731-105657758 | CB sk 709 | chr3:105740584-105740611 |
| CB sk 165 | chr3:105657736-105657763 | CB sk 710 | chr3:105740591-105740618 |
| CB sk 166 | chr3:105657847-105657874 | CB sk 711 | chr3:105740592-105740619 |
| CB sk 167 | chr3:105657856-105657883 | CB sk 712 | chr3:105740595-105740622 |
| CB sk 168 | chr3:105657884-105657911 | CB sk 713 | chr3:105740599-105740626 |
| CB sk 169 | chr3:105657893-105657920 | CB sk 714 | chr3:105740600-105740627 |
| CB sk 170 | chr3:105657901-105657928 | CB sk 715 | chr3:105740619-105740646 |
| CB sk 171 | chr3:105657914-105657941 | CB sk 716 | chr3:105745896-105745923 |
| CB sk 172 | chr3:105657915-105657942 | CB sk 717 | chr3:105745897-105745924 |
| CB sk 173 | chr3:105657926-105657953 | CB sk 718 | chr3:105745902-105745929 |
| CB sk 174 | chr3:105657927-105657954 | CB sk 719 | chr3:105745916-105745943 |
| CB sk 175 | chr3:105657957-105657984 | CB sk 720 | chr3:105745920-105745947 |
| CB sk 176 | chr3:105657970-105657997 | CB sk 721 | chr3:105745963-105745990 |
| CB sk 177 | chr3:105658004-105658031 | CB sk 722 | chr3:105745978-105746005 |
| CB sk 178 | chr3:105658005-105658032 | CB sk 723 | chr3:105745986-105746013 |
| CB sk 179 | chr3:105658011-105658038 | CB sk 724 | chr3:105746011-105746038 |
| CB sk 180 | chr3:105658040-105658067 | CB sk 725 | chr3:105746012-105746039 |
| CB sk 181 | chr3:105658045-105658072 | CB sk 726 | chr3:105746018-105746045 |
| CB sk 182 | chr3:105658055-105658082 | CB sk 727 | chr3:105746031-105746058 |
| CB sk 183 | chr3:105658056-105658083 | CB sk 728 | chr3:105746032-105746059 |
| CB sk 184 | chr3:105658058-105658085 | CB sk 729 | chr3:105746033-105746060 |
| CB sk 185 | chr3:105658059-105658086 | CB sk 730 | chr3:105749602-105749629 |
| CB sk 186 | chr3:105658060-105658087 | CB sk 731 | chr3:105749615-105749642 |
| CB sk 187 | chr3:105658089-105658116 | CB sk 732 | chr3:105749624-105749651 |
| CB sk 188 | chr3:105658140-105658167 | CB sk 733 | chr3:105749626-105749653 |
| CB sk 189 | chr3:105658171-105658198 | CB sk 734 | chr3:105749657-105749684 |
| CB sk 190 | chr3:105658172-105658199 | CB sk 735 | chr3:105749674-105749701 |
| CB sk 191 | chr3:105658192-105658219 | CB sk 736 | chr3:105749751-105749778 |
| CB sk 192 | chr3:105658193-105658220 | CB sk 737 | chr3:105749756-105749783 |
| CB sk 193 | chr3:105658194-105658221 | CB sk 738 | chr3:105749765-105749792 |
| CB sk 194 | chr3:105658196-105658223 | CB sk 739 | chr3:105749786-105749813 |
| CB sk 195 | chr3:105658204-105658231 | CB sk 740 | chr3:105751461-105751488 |
| CB sk 196 | chr3:105658227-105658254 | CB sk 741 | chr3:105751471-105751498 |
| CB sk 197 | chr3:105658228-105658255 | CB sk 742 | chr3:105751528-105751555 |
| CB sk 198 | chr3:105658261-105658288 | CB sk 743 | chr3:105751542-105751569 |
| CB sk 199 | chr3:105658266-105658293 | CB sk 744 | chr3:105751543-105751570 |
| CB sk 200 | chr3:105658280-105658307 | CB sk 745 | chr3:105751546-105751573 |
| CB sk 201 | chr3:105658281-105658308 | CB sk 746 | chr3:105751551-105751578 |
| CB sk 202 | chr3:105658282-105658309 | CB sk 747 | chr3:105751556-105751583 |
| CB sk 203 | chr3:105658302-105658329 | CB sk 748 | chr3:105751558-105751585 |
| CB sk 204 | chr3:105658328-105658355 | CB sk 749 | chr3:105751575-105751602 |
| CB sk 205 | chr3:105658335-105658362 | CB sk 750 | chr3:105751581-105751608 |
| CB sk 206 | chr3:105658336-105658363 | CB sk 751 | chr3:105751601-105751628 |
| CB sk 207 | chr3:105658359-105658386 | CB sk 752 | chr3:105751603-105751630 |
| CB sk 208 | chr3:105658434-105658461 | CB sk 753 | chr3:105751604-105751631 |
| CB sk 209 | chr3:105658438-105658465 | CB sk 754 | chr3:105751613-105751640 |
| CB sk 210 | chr3:105658444-105658471 | CB sk 755 | chr3:105751614-105751641 |
| CB sk 211 | chr3:105658450-105658477 | CB sk 756 | chr3:105754319-105754346 |
| CB sk 212 | chr3:105658451-105658478 | CB sk 757 | chr3:105754320-105754347 |
| CB sk 213 | chr3:105658456-105658483 | CB sk 758 | chr3:105754342-105754369 |
| CB sk 214 | chr3:105658461-105658488 | CB sk 759 | chr3:105754345-105754372 |
| CB sk 215 | chr3:105658462-105658489 | CB sk 760 | chr3:105754346-105754373 |
| CB sk 216 | chr3:105658463-105658490 | CB sk 761 | chr3:105754349-105754376 |
| CB sk 217 | chr3:105658467-105658494 | CB sk 762 | chr3:105754386-105754413 |
| CB sk 218 | chr3:105658473-105658500 | CB sk 763 | chr3:105754392-105754419 |
| CB sk 219 | chr3:105658502-105658529 | CB sk 764 | chr3:105754433-105754460 |
| CB sk 220 | chr3:105658507-105658534 | CB sk 765 | chr3:105754434-105754461 |
| CB sk 221 | chr3:105658523-105658550 | CB sk 766 | chr3:105754440-105754467 |
| CB sk 222 | chr3:105658526-105658553 | CB sk 767 | chr3:105754451-105754478 |
| CB sk 223 | chr3:105658527-105658554 | CB sk 768 | chr3:105754452-105754479 |
| CB sk 224 | chr3:105658561-105658588 | CB sk 769 | chr3:105754464-105754491 |
| CB sk 225 | chr3:105658562-105658589 | CB sk 770 | chr3:105754465-105754492 |
| CB sk 226 | chr3:105658570-105658597 | CB sk 771 | chr3:105754474-105754501 |
| CB sk 227 | chr3:105658575-105658602 | CB sk 772 | chr3:105776402-105776429 |
| CB sk 228 | chr3:105658584-105658611 | CB sk 773 | chr3:105776416-105776443 |
| CB sk 229 | chr3:105658622-105658649 | CB sk 774 | chr3:105776437-105776464 |
| CB sk 230 | chr3:105658655-105658682 | CB sk 775 | chr3:105776450-105776477 |
| CB sk 231 | chr3:105658666-105658693 | CB sk 776 | chr3:105776451-105776478 |
| CB sk 232 | chr3:105658671-105658698 | CB sk 777 | chr3:105776459-105776486 |
| CB sk 233 | chr3:105658685-105658712 | CB sk 778 | chr3:105776460-105776487 |
| CB sk 234 | chr3:105658686-105658713 | CB sk 779 | chr3:105776471-105776498 |
| CB sk 235 | chr3:105658740-105658767 | CB sk 780 | chr3:105776494-105776521 |
| CB sk 236 | chr3:105658749-105658776 | CB sk 781 | chr3:105776495-105776522 |
| CB sk 237 | chr3:105658750-105658777 | CB sk 782 | chr3:105776499-105776526 |
| CB sk 238 | chr3:105658755-105658782 | CB sk 783 | chr3:105776522-105776549 |
| CB sk 239 | chr3:105658786-105658813 | CB sk 784 | chr3:105776529-105776556 |
| CB sk 240 | chr3:105658790-105658817 | CB sk 785 | chr3:105776530-105776557 |
| CB sk 241 | chr3:105658802-105658829 | CB sk 786 | chr3:105824098-105824125 |
| CB sk 242 | chr3:105658821-105658848 | CB sk 787 | chr3:105824106-105824133 |
| CB sk 243 | chr3:105658839-105658866 | CB sk 788 | chr3:105824107-105824134 |
| CB sk 244 | chr3:105658840-105658867 | CB sk 789 | chr3:105824135-105824162 |
| CB sk 245 | chr3:105658842-105658869 | CB sk 790 | chr3:105824138-105824165 |
| CB sk 246 | chr3:105658845-105658872 | CB sk 791 | chr3:105824142-105824169 |
| CB sk 247 | chr3:105658893-105658920 | CB sk 792 | chr3:105824152-105824179 |
| CB sk 248 | chr3:105658898-105658925 | CB sk 793 | chr3:105824153-105824180 |
| CB sk 249 | chr3:105658914-105658941 | CB sk 794 | chr3:105824154-105824181 |
| CB sk 250 | chr3:105658924-105658951 | CB sk 795 | chr3:105824161-105824188 |
| CB sk 251 | chr3:105658939-105658966 | CB sk 796 | chr3:105824183-105824210 |
| CB sk 252 | chr3:105658943-105658970 | CB sk 797 | chr3:105824186-105824213 |
| CB sk 253 | chr3:105658960-105658987 | CB sk 798 | chr3:105824194-105824221 |
| CB sk 254 | chr3:105658961-105658988 | CB sk 799 | chr3:105824195-105824222 |
| CB sk 255 | chr3:105658962-105658989 | CB sk 800 | chr3:105824205-105824232 |
| CB sk 256 | chr3:105658969-105658996 | CB sk 801 | chr3:105824206-105824233 |
| CB sk 257 | chr3:105658972-105658999 | CB sk 802 | chr3:105824207-105824234 |
| CB sk 258 | chr3:105658975-105659002 | CB sk 803 | chr3:105824210-105824237 |
| CB sk 259 | chr3:105658976-105659003 | CB sk 804 | chr3:105824211-105824238 |
| CB sk 260 | chr3:105658980-105659007 | CB sk 805 | chr3:105824212-105824239 |
| CB sk 261 | chr3:105658991-105659018 | CB sk 806 | chr3:105824225-105824252 |
| CB sk 262 | chr3:105658994-105659021 | CB sk 807 | chr3:105824241-105824268 |
| CB sk 263 | chr3:105659003-105659030 | CB sk 808 | chr3:105839418-105839445 |
| CB sk 264 | chr3:105659004-105659031 | CB sk 809 | chr3:105839422-105839449 |
| CB sk 265 | chr3:105659024-105659051 | CB sk 810 | chr3:105839426-105839453 |
| CB sk 266 | chr3:105659025-105659052 | CB sk 811 | chr3:105839434-105839461 |
| CB sk 267 | chr3:105659027-105659054 | CB sk 812 | chr3:105839447-105839474 |
| CB sk 268 | chr3:105659037-105659064 | CB sk 813 | chr3:105839457-105839484 |
| CB sk 269 | chr3:105659058-105659085 | CB sk 814 | chr3:105839458-105839485 |
| CB sk 270 | chr3:105659074-105659101 | CB sk 815 | chr3:105853387-105853414 |
| CB sk 271 | chr3:105659091-105659118 | CB sk 816 | chr3:105853445-105853472 |
| CB sk 272 | chr3:105659092-105659119 | CB sk 817 | chr3:105853521-105853548 |
| CB sk 273 | chr3:105659097-105659124 | CB sk 818 | chr3:105853571-105853598 |
| CB sk 274 | chr3:105659098-105659125 | CB sk 819 | chr3:105853618-105853645 |
| CB sk 275 | chr3:105659119-105659146 | CB sk 820 | chr3:105853619-105853646 |
| CB sk 276 | chr3:105659126-105659153 | CB sk 821 | chr3:105853626-105853653 |
| CB sk 277 | chr3:105659127-105659154 | CB sk 822 | chr3:105853664-105853691 |
| CB sk 278 | chr3:105659128-105659155 | CB sk 823 | chr3:105867417-105867444 |
| CB sk 279 | chr3:105659131-105659158 | CB sk 824 | chr3:105867418-105867445 |
| CB sk 280 | chr3:105659137-105659164 | CB sk 825 | chr3:105867477-105867504 |
| CB sk 281 | chr3:105659138-105659165 | CB sk 826 | chr3:105867487-105867514 |
| CB sk 282 | chr3:105659140-105659167 | CB sk 827 | chr3:105867500-105867527 |
| CB sk 283 | chr3:105659147-105659174 | CB sk 828 | chr3:105867501-105867528 |
| CB sk 284 | chr3:105659148-105659175 | CB sk 829 | chr3:105867534-105867561 |
| CB sk 285 | chr3:105659149-105659176 | CB sk 830 | chr3:105867537-105867564 |
| CB sk 286 | chr3:105659164-105659191 | CB sk 831 | chr3:105867543-105867570 |
| CB sk 287 | chr3:105659170-105659197 | CB sk 832 | chr3:105867546-105867573 |
| CB sk 288 | chr3:105659176-105659203 | CB sk 833 | chr3:105867554-105867581 |
| CB sk 289 | chr3:105659177-105659204 | CB sk 834 | chr3:105867558-105867585 |
| CB sk 290 | chr3:105659178-105659205 | CB sk 835 | chr3:105867574-105867601 |
| CB sk 291 | chr3:105659179-105659206 | CB sk 836 | chr3:105868144-105868171 |
| CB sk 292 | chr3:105659183-105659210 | CB sk 837 | chr3:105868147-105868174 |
| CB sk 293 | chr3:105659184-105659211 | CB sk 838 | chr3:105868161-105868188 |
| CB sk 294 | chr3:105659188-105659215 | CB sk 839 | chr3:105868168-105868195 |
| CB sk 295 | chr3:105659218-105659245 | CB sk 840 | chr3:105868201-105868228 |
| CB sk 296 | chr3:105659226-105659253 | CB sk 841 | chr3:105868202-105868229 |
| CB sk 297 | chr3:105670218-105670245 | CB sk 842 | chr3:105868209-105868236 |
| CB sk 298 | chr3:105670224-105670251 | CB sk 843 | chr3:105868234-105868261 |
| CB sk 299 | chr3:105670232-105670259 | CB sk 844 | chr3:105868235-105868262 |
| CB sk 300 | chr3:105670260-105670287 | CB sk 845 | chr3:105868245-105868272 |
| CB sk 301 | chr3:105670269-105670296 | CB sk 846 | chr3:105868251-105868278 |
| CB sk 302 | chr3:105670284-105670311 | CB sk 847 | chr3:105868256-105868283 |
| CB sk 303 | chr3:105670287-105670314 | CB sk 848 | chr3:105868259-105868286 |
| CB sk 304 | chr3:105670292-105670319 | CB sk 849 | chr3:105868273-105868300 |
| CB sk 305 | chr3:105670293-105670320 | CB sk 850 | chr3:105868274-105868301 |
| CB sk 306 | chr3:105670300-105670327 | CB sk 851 | chr3:105868304-105868331 |
| CB sk 307 | chr3:105670322-105670349 | CB sk 852 | chr3:105868309-105868336 |
| CB sk 308 | chr3:105670323-105670350 | CB sk 853 | chr3:105868313-105868340 |
| CB sk 309 | chr3:105670328-105670355 | CB sk 854 | chr3:105868315-105868342 |
| CB sk 310 | chr3:105671001-105671028 | CB sk 855 | chr3:105868316-105868343 |
| CB sk 311 | chr3:105671014-105671041 | CB sk 856 | chr3:105868322-105868349 |
| CB sk 312 | chr3:105671015-105671042 | CB sk 857 | chr3:105868323-105868350 |
| CB sk 313 | chr3:105671016-105671043 | CB sk 858 | chr3:105868339-105868366 |
| CB sk 314 | chr3:105671020-105671047 | CB sk 859 | chr3:105868344-105868371 |
| CB sk 315 | chr3:105671023-105671050 | CB sk 860 | chr3:105868350-105868377 |
| CB sk 316 | chr3:105671054-105671081 | CB sk 861 | chr3:105868351-105868378 |
| CB sk 317 | chr3:105671063-105671090 | CB sk 862 | chr3:105868352-105868379 |
| CB sk 318 | chr3:105671064-105671091 | CB sk 863 | chr3:105868356-105868383 |
| CB sk 319 | chr3:105671124-105671151 | CB sk 864 | chr3:105868357-105868384 |
| CB sk 320 | chr3:105671136-105671163 | CB sk 865 | chr3:105868358-105868385 |
| CB sk 321 | chr3:105671143-105671170 | CB sk 866 | chr3:105868359-105868386 |
| CB sk 322 | chr3:105671144-105671171 | CB sk 867 | chr3:105868367-105868394 |
| CB sk 323 | chr3:105671148-105671175 | CB sk 868 | chr3:105868368-105868395 |
| CB sk 324 | chr3:105671149-105671176 | CB sk 869 | chr3:105868369-105868396 |
| CB sk 325 | chr3:105671200-105671227 | CB sk 870 | chr3:105868372-105868399 |
| CB sk 326 | chr3:105671201-105671228 | CB sk 871 | chr3:105868373-105868400 |
| CB sk 327 | chr3:105671212-105671239 | CB sk 872 | chr3:105868374-105868401 |
| CB sk 328 | chr3:105671234-105671261 | CB sk 873 | chr3:105868375-105868402 |
| CB sk 329 | chr3:105671251-105671278 | CB sk 874 | chr3:105868378-105868405 |
| CB sk 330 | chr3:105671268-105671295 | CB sk 875 | chr3:105868379-105868406 |
| CB sk 331 | chr3:105671269-105671296 | CB sk 876 | chr3:105868380-105868407 |
| CB sk 332 | chr3:105671272-105671299 | CB sk 877 | chr3:105868507-105868534 |
| CB sk 333 | chr3:105671281-105671308 | CB sk 878 | chr3:105868508-105868535 |
| CB sk 334 | chr3:105671291-105671318 | CB sk 879 | chr3:105868512-105868539 |
| CB sk 335 | chr3:105671322-105671349 | CB sk 880 | chr3:105868515-105868542 |
| CB sk 336 | chr3:105671327-105671354 | CB sk 881 | chr3:105868518-105868545 |
| CB sk 337 | chr3:105671328-105671355 | CB sk 882 | chr3:105868526-105868553 |
| CB sk 338 | chr3:105671332-105671359 | CB sk 883 | chr3:105868531-105868558 |
| CB sk 339 | chr3:105671336-105671363 | CB sk 884 | chr3:105868534-105868561 |
| CB sk 340 | chr3:105671337-105671364 | CB sk 885 | chr3:105868540-105868567 |
| CB sk 341 | chr3:105671372-105671399 | CB sk 886 | chr3:105868541-105868568 |
| CB sk 342 | chr3:105671396-105671423 | CB sk 887 | chr3:105868547-105868574 |
| CB sk 343 | chr3:105671399-105671426 | CB sk 888 | chr3:105868548-105868575 |
| CB sk 344 | chr3:105671405-105671432 | CB sk 889 | chr3:105868549-105868576 |
| CB sk 345 | chr3:105671417-105671444 | CB sk 890 | chr3:105868550-105868577 |
| CB sk 346 | chr3:105671467-105671494 | CB sk 891 | chr3:105868554-105868581 |
| CB sk 347 | chr3:105671479-105671506 | CB sk 892 | chr3:105868555-105868582 |
| CB sk 348 | chr3:105671521-105671548 | CB sk 893 | chr3:105868556-105868583 |
| CB sk 349 | chr3:105671566-105671593 | CB sk 894 | chr3:105868559-105868586 |
| CB sk 350 | chr3:105671567-105671594 | CB sk 895 | chr3:105868711-105868738 |
| CB sk 351 | chr3:105671593-105671620 | CB sk 896 | chr3:105868712-105868739 |
| CB sk 352 | chr3:105671598-105671625 | CB sk 897 | chr3:105868713-105868740 |
| CB sk 353 | chr3:105671599-105671626 | CB sk 898 | chr3:105868715-105868742 |
| CB sk 354 | chr3:105671624-105671651 | CB sk 899 | chr3:105868720-105868747 |
| CB sk 355 | chr3:105671662-105671689 | CB sk 900 | chr3:105868721-105868748 |
| CB sk 356 | chr3:105671673-105671700 | CB sk 901 | chr3:105868722-105868749 |
| CB sk 357 | chr3:105671677-105671704 | CB sk 902 | chr3:105868726-105868753 |
| CB sk 358 | chr3:105671709-105671736 | CB sk 903 | chr3:105868727-105868754 |
| CB sk 359 | chr3:105671713-105671740 | CB sk 904 | chr3:105868735-105868762 |
| CB sk 360 | chr3:105671727-105671754 | CB sk 905 | chr3:105868745-105868772 |
| CB sk 361 | chr3:105671740-105671767 | CB sk 906 | chr3:105868749-105868776 |
| CB sk 362 | chr3:105671753-105671780 | CB sk 907 | chr3:105868750-105868777 |
| CB sk 363 | chr3:105671760-105671787 | CB sk 908 | chr3:105868755-105868782 |
| CB sk 364 | chr3:105671836-105671863 | CB sk 909 | chr3:105868779-105868806 |
| CB sk 365 | chr3:105671839-105671866 | CB sk 910 | chr3:105868780-105868807 |
| CB sk 366 | chr3:105671868-105671895 | CB sk 911 | chr3:105868785-105868812 |
| CB sk 367 | chr3:105671874-105671901 | CB sk 912 | chr3:105868786-105868813 |
| CB sk 368 | chr3:105671878-105671905 | CB sk 913 | chr3:105868787-105868814 |
| CB sk 369 | chr3:105671885-105671912 | CB sk 914 | chr3:105868795-105868822 |
| CB sk 370 | chr3:105671919-105671946 | CB sk 915 | chr3:105868799-105868826 |
| CB sk 371 | chr3:105671939-105671966 | CB sk 916 | chr3:105868800-105868827 |
| CB sk 372 | chr3:105671951-105671978 | CB sk 917 | chr3:105868805-105868832 |
| CB sk 373 | chr3:105671960-105671987 | CB sk 918 | chr3:105868806-105868833 |
| CB sk 374 | chr3:105672005-105672032 | CB sk 919 | chr3:105868817-105868844 |
| CB sk 375 | chr3:105672006-105672033 | CB sk 920 | chr3:105868818-105868845 |
| CB sk 376 | chr3:105672020-105672047 | CB sk 921 | chr3:105868819-105868846 |
| CB sk 377 | chr3:105672065-105672092 | CB sk 922 | chr3:105868837-105868864 |
| CB sk 378 | chr3:105672067-105672094 | CB sk 923 | chr3:105868854-105868881 |
| CB sk 379 | chr3:105672068-105672095 | CB sk 924 | chr3:105868855-105868882 |
| CB sk 380 | chr3:105672069-105672096 | CB sk 925 | chr3:105868869-105868896 |
| CB sk 381 | chr3:105672110-105672137 | CB sk 926 | chr3:105868870-105868897 |
| CB sk 382 | chr3:105672111-105672138 | CB sk 927 | chr3:105868871-105868898 |
| CB sk 383 | chr3:105672122-105672149 | CB sk 928 | chr3:105868883-105868910 |
| CB sk 384 | chr3:105672170-105672197 | CB sk 929 | chr3:105868886-105868913 |
| CB sk 385 | chr3:105672188-105672215 | CB sk 930 | chr3:105868911-105868938 |
| CB sk 386 | chr3:105672229-105672256 | CB sk 931 | chr3:105868925-105868952 |
| CB sk 387 | chr3:105672252-105672279 | CB sk 932 | chr3:105868929-105868956 |
| CB sk 388 | chr3:105672267-105672294 | CB sk 933 | chr3:105868930-105868957 |
| CB sk 389 | chr3:105672270-105672297 | CB sk 934 | chr3:105868931-105868958 |
| CB sk 390 | chr3:105672319-105672346 | CB sk 935 | chr3:105868934-105868961 |
| CB sk 391 | chr3:105672336-105672363 | CB sk 936 | chr3:105868938-105868965 |
| CB sk 392 | chr3:105672388-105672415 | CB sk 937 | chr3:105868939-105868966 |
| CB sk 393 | chr3:105672389-105672416 | CB sk 938 | chr3:105868940-105868967 |
| CB sk 394 | chr3:105672397-105672424 | CB sk 939 | chr3:105868941-105868968 |
| CB sk 395 | chr3:105672398-105672425 | CB sk 940 | chr3:105868943-105868970 |
| CB sk 396 | chr3:105672409-105672436 | CB sk 941 | chr3:105868944-105868971 |
| CB sk 397 | chr3:105672424-105672451 | CB sk 942 | chr3:105868947-105868974 |
| CB sk 398 | chr3:105672435-105672462 | CB sk 943 | chr3:105868948-105868975 |
| CB sk 399 | chr3:105672444-105672471 | CB sk 944 | chr3:105868962-105868989 |
| CB sk 400 | chr3:105672445-105672472 | CB sk 945 | chr3:105868963-105868990 |
| CB sk 401 | chr3:105675740-105675767 | CB sk 946 | chr3:105868966-105868993 |
| CB sk 402 | chr3:105675753-105675780 | CB sk 947 | chr3:105868969-105868996 |
| CB sk 403 | chr3:105675754-105675781 | CB sk 948 | chr3:105868972-105868999 |
| CB sk 404 | chr3:105675755-105675782 | CB sk 949 | chr3:105868983-105869010 |
| CB sk 405 | chr3:105675756-105675783 | CB sk 950 | chr3:105869000-105869027 |
| CB sk 406 | chr3:105675759-105675786 | CB sk 951 | chr3:105869010-105869037 |
| CB sk 407 | chr3:105675762-105675789 | CB sk 952 | chr3:105869317-105869344 |
| CB sk 408 | chr3:105675766-105675793 | CB sk 953 | chr3:105869318-105869345 |
| CB sk 409 | chr3:105675767-105675794 | CB sk 954 | chr3:105869319-105869346 |
| CB sk 410 | chr3:105675768-105675795 | CB sk 955 | chr3:105869322-105869349 |
| CB sk 411 | chr3:105675785-105675812 | CB sk 956 | chr3:105869326-105869353 |
| CB sk 412 | chr3:105675788-105675815 | CB sk 957 | chr3:105869329-105869356 |
| CB sk 413 | chr3:105675794-105675821 | CB sk 958 | chr3:105869330-105869357 |
| CB sk 414 | chr3:105675807-105675834 | CB sk 959 | chr3:105869336-105869363 |
| CB sk 415 | chr3:105675834-105675861 | CB sk 960 | chr3:105869342-105869369 |
| CB sk 416 | chr3:105675835-105675862 | CB sk 961 | chr3:105869343-105869370 |
| CB sk 417 | chr3:105675843-105675870 | CB sk 962 | chr3:105869348-105869375 |
| CB sk 418 | chr3:105675853-105675880 | CB sk 963 | chr3:105869349-105869376 |
| CB sk 419 | chr3:105675857-105675884 | CB sk 964 | chr3:105869365-105869392 |
| CB sk 420 | chr3:105675876-105675903 | CB sk 965 | chr3:105869366-105869393 |
| CB sk 421 | chr3:105675877-105675904 | CB sk 966 | chr3:105869367-105869394 |
| CB sk 422 | chr3:105678410-105678437 | CB sk 967 | chr3:105869372-105869399 |
| CB sk 423 | chr3:105678422-105678449 | CB sk 968 | chr3:105869375-105869402 |
| CB sk 424 | chr3:105678423-105678450 | CB sk 969 | chr3:105869378-105869405 |
| CB sk 425 | chr3:105678426-105678453 | CB sk 970 | chr3:105869379-105869406 |
| CB sk 426 | chr3:105678430-105678457 | CB sk 971 | chr3:105869385-105869412 |
| CB sk 427 | chr3:105678436-105678463 | CB sk 972 | chr3:105869411-105869438 |
| CB sk 428 | chr3:105678440-105678467 | CB sk 973 | chr3:105869424-105869451 |
| CB sk 429 | chr3:105678444-105678471 | CB sk 974 | chr3:105869428-105869455 |
| CB sk 430 | chr3:105678451-105678478 | CB sk 975 | chr3:105869434-105869461 |
| CB sk 431 | chr3:105678452-105678479 | CB sk 976 | chr3:105869438-105869465 |
| CB sk 432 | chr3:105678457-105678484 | CB sk 977 | chr3:105869442-105869469 |
| CB sk 433 | chr3:105678458-105678485 | CB sk 978 | chr3:105869443-105869470 |
| CB sk 434 | chr3:105678461-105678488 | CB sk 979 | chr3:105869462-105869489 |
| CB sk 435 | chr3:105678468-105678495 | CB sk 980 | chr3:105869463-105869490 |
| CB sk 436 | chr3:105678481-105678508 | CB sk 981 | chr3:105869466-105869493 |
| CB sk 437 | chr3:105678494-105678521 | CB sk 982 | chr3:105869467-105869494 |
| CB sk 438 | chr3:105678497-105678524 | CB sk 983 | chr3:105869478-105869505 |
| CB sk 439 | chr3:105678500-105678527 | CB sk 984 | chr3:105869481-105869508 |
| CB sk 440 | chr3:105678501-105678528 | CB sk 985 | chr3:105869496-105869523 |
| CB sk 441 | chr3:105678502-105678529 | CB sk 986 | chr3:105869497-105869524 |
| CB sk 442 | chr3:105678503-105678530 | CB sk 987 | chr3:105869498-105869525 |
| CB sk 443 | chr3:105678506-105678533 | CB sk 988 | chr3:105869518-105869545 |
| CB sk 444 | chr3:105678509-105678536 | CB sk 989 | chr3:105869519-105869546 |
| CB sk 445 | chr3:105678510-105678537 | CB sk 990 | chr3:105869520-105869547 |
| CB sk 446 | chr3:105678513-105678540 | CB sk 991 | chr3:105869529-105869556 |
| CB sk 447 | chr3:105678518-105678545 | CB sk 992 | chr3:105869534-105869561 |
| CB sk 448 | chr3:105678521-105678548 | CB sk 993 | chr3:105869535-105869562 |
| CB sk 449 | chr3:105678522-105678549 | CB sk 994 | chr3:105869536-105869563 |
| CB sk 450 | chr3:105678525-105678552 | CB sk 995 | chr3:105869542-105869569 |
| CB sk 451 | chr3:105678526-105678553 | CB sk 996 | chr3:105869543-105869570 |
| CB sk 452 | chr3:105678561-105678588 | CB sk 997 | chr3:105869555-105869582 |
| CB sk 453 | chr3:105678571-105678598 | CB sk 998 | chr3:105869559-105869586 |
| CB sk 454 | chr3:105681458-105681485 | CB sk 999 | chr3:105869606-105869633 |
| CB sk 455 | chr3:105681461-105681488 | CB sk 1000 | chr3:105869622-105869649 |
| CB sk 456 | chr3:105681462-105681489 | CB sk 1001 | chr3:105869626-105869653 |
| CB sk 457 | chr3:105681478-105681505 | CB sk 1002 | chr3:105869627-105869654 |
| CB sk 458 | chr3:105681484-105681511 | CB sk 1003 | chr3:105869628-105869655 |
| CB sk 459 | chr3:105681485-105681512 | CB sk 1004 | chr3:105869669-105869696 |
| CB sk 460 | chr3:105681515-105681542 | CB sk 1005 | chr3:105869682-105869709 |
| CB sk 461 | chr3:105681516-105681543 | CB sk 1006 | chr3:105869683-105869710 |
| CB sk 462 | chr3:105681530-105681557 | CB sk 1007 | chr3:105869691-105869718 |
| CB sk 463 | chr3:105681532-105681559 | CB sk 1008 | chr3:105869695-105869722 |
| CB sk 464 | chr3:105681533-105681560 | CB sk 1009 | chr3:105869696-105869723 |
| CB sk 465 | chr3:105681538-105681565 | CB sk 1010 | chr3:105869716-105869743 |
| CB sk 466 | chr3:105681542-105681569 | CB sk 1011 | chr3:105869732-105869759 |
| CB sk 467 | chr3:105681545-105681572 | CB sk 1012 | chr3:105869748-105869775 |
| CB sk 468 | chr3:105681548-105681575 | CB sk 1013 | chr3:105869776-105869803 |
| CB sk 469 | chr3:105681549-105681576 | CB sk 1014 | chr3:105869799-105869826 |
| CB sk 470 | chr3:105681551-105681578 | CB sk 1015 | chr3:105869800-105869827 |
| CB sk 471 | chr3:105681556-105681583 | CB sk 1016 | chr3:105869816-105869843 |
| CB sk 472 | chr3:105681557-105681584 | CB sk 1017 | chr3:105869843-105869870 |
| CB sk 473 | chr3:105681561-105681588 | CB sk 1018 | chr3:105869850-105869877 |
| CB sk 474 | chr3:105681565-105681592 | CB sk 1019 | chr3:105869862-105869889 |
| CB sk 475 | chr3:105681590-105681617 | CB sk 1020 | chr3:105869865-105869892 |
| CB sk 476 | chr3:105681610-105681637 | CB sk 1021 | chr3:105869875-105869902 |
| CB sk 477 | chr3:105681721-105681748 | CB sk 1022 | chr3:105869888-105869915 |
| CB sk 478 | chr3:105681722-105681749 | CB sk 1023 | chr3:105869921-105869948 |
| CB sk 479 | chr3:105681723-105681750 | CB sk 1024 | chr3:105869940-105869967 |
| CB sk 480 | chr3:105681724-105681751 | CB sk 1025 | chr3:105869954-105869981 |
| CB sk 481 | chr3:105681748-105681775 | CB sk 1026 | chr3:105869968-105869995 |
| CB sk 482 | chr3:105681777-105681804 | CB sk 1027 | chr3:105869975-105870002 |
| CB sk 483 | chr3:105681786-105681813 | CB sk 1028 | chr3:105869978-105870005 |
| CB sk 484 | chr3:105681798-105681825 | CB sk 1029 | chr3:105869990-105870017 |
| CB sk 485 | chr3:105681804-105681831 | CB sk 1030 | chr3:105870001-105870028 |
| CB sk 486 | chr3:105681818-105681845 | CB sk 1031 | chr3:105870005-105870032 |
| CB sk 487 | chr3:105685300-105685327 | CB sk 1032 | chr3:105870006-105870033 |
| CB sk 488 | chr3:105685303-105685330 | CB sk 1033 | chr3:105870012-105870039 |
| CB sk 489 | chr3:105685307-105685334 | CB sk 1034 | chr3:105870026-105870053 |
| CB sk 490 | chr3:105685308-105685335 | CB sk 1035 | chr3:105870029-105870056 |
| CB sk 491 | chr3:105685319-105685346 | CB sk 1036 | chr3:105870044-105870071 |
| CB sk 492 | chr3:105685327-105685354 | CB sk 1037 | chr3:105870045-105870072 |
| CB sk 493 | chr3:105685340-105685367 | CB sk 1038 | chr3:105870070-105870097 |
| CB sk 494 | chr3:105685341-105685368 | CB sk 1039 | chr3:105870109-105870136 |
| CB sk 495 | chr3:105685348-105685375 | CB sk 1040 | chr3:105870113-105870140 |
| CB sk 496 | chr3:105685349-105685376 | CB sk 1041 | chr3:105870137-105870164 |
| CB sk 497 | chr3:105685352-105685379 | CB sk 1042 | chr3:105870138-105870165 |
| CB sk 498 | chr3:105685353-105685380 | CB sk 1043 | chr3:105870141-105870168 |
| CB sk 499 | chr3:105685360-105685387 | CB sk 1044 | chr3:105870153-105870180 |
| CB sk 500 | chr3:105685393-105685420 | CB sk 1045 | chr3:105870155-105870182 |
| CB sk 501 | chr3:105685394-105685421 | CB sk 1046 | chr3:105870193-105870220 |
| CB sk 502 | chr3:105685411-105685438 | CB sk 1047 | chr3:105870205-105870232 |
| CB sk 503 | chr3:105685429-105685456 | CB sk 1048 | chr3:105870236-105870263 |
| CB sk 504 | chr3:105685447-105685474 | CB sk 1049 | chr3:105870237-105870264 |
| CB sk 505 | chr3:105685448-105685475 | CB sk 1050 | chr3:105870305-105870332 |
| CB sk 506 | chr3:105685449-105685476 | CB sk 1051 | chr3:105870308-105870335 |
| CB sk 507 | chr3:105685458-105685485 | CB sk 1052 | chr3:105870313-105870340 |
| CB sk 508 | chr3:105685466-105685493 | CB sk 1053 | chr3:105870342-105870369 |
| CB sk 509 | chr3:105693474-105693501 | CB sk 1054 | chr3:105870364-105870391 |
| CB sk 510 | chr3:105693477-105693504 | CB sk 1055 | chr3:105870394-105870421 |
| CB sk 511 | chr3:105693478-105693505 | CB sk 1056 | chr3:105870401-105870428 |
| CB sk 512 | chr3:105693481-105693508 | CB sk 1057 | chr3:105870404-105870431 |
| CB sk 513 | chr3:105693484-105693511 | CB sk 1058 | chr3:105870413-105870440 |
| CB sk 514 | chr3:105693485-105693512 | CB sk 1059 | chr3:105870457-105870484 |
| CB sk 515 | chr3:105693488-105693515 | CB sk 1060 | chr3:105870467-105870494 |
| CB sk 516 | chr3:105693495-105693522 | CB sk 1061 | chr3:105870476-105870503 |
| CB sk 517 | chr3:105693498-105693525 | CB sk 1062 | chr3:105870518-105870545 |
| CB sk 518 | chr3:105693501-105693528 | CB sk 1063 | chr3:105870537-105870564 |
| CB sk 519 | chr3:105693504-105693531 | CB sk 1064 | chr3:105870557-105870584 |
| CB sk 520 | chr3:105693544-105693571 | CB sk 1065 | chr3:105870567-105870594 |
| CB sk 521 | chr3:105693554-105693581 | CB sk 1066 | chr3:105870595-105870622 |
| CB sk 522 | chr3:105693566-105693593 | CB sk 1067 | chr3:105870600-105870627 |
| CB sk 523 | chr3:105693575-105693602 | CB sk 1068 | chr3:105870608-105870635 |
| CB sk 524 | chr3:105693588-105693615 | CB sk 1069 | chr3:105870611-105870638 |
| CB sk 525 | chr3:105702084-105702111 | CB sk 1070 | chr3:105870614-105870641 |
| CB sk 526 | chr3:105702093-105702120 | CB sk 1071 | chr3:105870615-105870642 |
| CB sk 527 | chr3:105702094-105702121 | CB sk 1072 | chr3:105870619-105870646 |
| CB sk 528 | chr3:105702101-105702128 | CB sk 1073 | chr3:105870636-105870663 |
| CB sk 529 | chr3:105702120-105702147 | CB sk 1074 | chr3:105870637-105870664 |
| CB sk 530 | chr3:105702121-105702148 | CB sk 1075 | chr3:105870638-105870665 |
| CB sk 531 | chr3:105702133-105702160 | CB sk 1076 | chr3:105870693-105870720 |
| CB sk 532 | chr3:105702155-105702182 | CB sk 1077 | chr3:105870721-105870748 |
| CB sk 533 | chr3:105702156-105702183 | CB sk 1078 | chr3:105870728-105870755 |
| CB sk 534 | chr3:105702169-105702196 | CB sk 1079 | chr3:105870729-105870756 |
| CB sk 535 | chr3:105702173-105702200 | CB sk 1080 | chr3:105870768-105870795 |
| CB sk 536 | chr3:105702177-105702204 | CB sk 1081 | chr3:105870810-105870837 |
| CB sk 537 | chr3:105702183-105702210 | CB sk 1082 | chr3:105870811-105870838 |
| CB sk 538 | chr3:105702199-105702226 | CB sk 1083 | chr3:105870824-105870851 |
| CB sk 539 | chr3:105702200-105702227 | CB sk 1084 | chr3:105870835-105870862 |
| CB sk 540 | chr3:105702215-105702242 | CB sk 1085 | chr3:105870840-105870867 |
| CB sk 541 | chr3:105702216-105702243 | CB sk 1086 | chr3:105870863-105870890 |
| CB sk 542 | chr3:105702219-105702246 | CB sk 1087 | chr3:105870875-105870902 |
| CB sk 543 | chr3:105702220-105702247 | CB sk 1088 | chr3:105870896-105870923 |
| CB sk 544 | chr3:105702221-105702248 | CB sk 1089 | chr3:105870916-105870943 |
| CB sk 545 | chr3:105702236-105702263 | CB sk 1090 | chr3:105870923-105870950 |

## Claims

1. A method for culturing cells, the method comprising: reducing the expression and/or reducing the activity of peptidase C64 family member (s) and/or functionally active fragments thereof in the cells.

2. The method of claim 1, wherein the cells comprise immune cells.

3. The method of claim 2, wherein the immune cells comprise phagocytes, lymphocytes, neutrophils, eosinophils, and/or basophils.

4. The method of any one of claims 2-3, wherein the immune cells comprise monocytes, macrophages, and/or dendritic cells.

5. The method of any one of claims 2-4, wherein the immune cells are derived from stem cell-differentiated immune cells.

6. The method of claim 5, wherein the stem cells comprise induced pluripotent stem cells (iPSCs).

7. The method of any one of claims 2-6, wherein the immune cells comprise B cells, T cells, natural killer cells, and/or natural killer-like T cells (NKT).

8. The method of any one of claims 2-7, wherein the immune cells comprise αβ T cells and/or γδ T cells.

9. The method of any one of claims 2-8, wherein the immune cells comprise tumor-infiltrating lymphocytes (TILs).

10. The method of claim 9, wherein the TIL is a TIL derived from fragments of tumor tissue, pleural effusion, and/or peritoneal effusion, and/or a TIL derived from resuscitation after cryopreservation.

11. The method of claim 10, wherein the fragment has a volume from about 1 cubic millimeter to about 27 cubic millimeters.

12. The method of any one of claims 2-11, wherein the immune cells comprise engineered immune receptors displayed on the cell surface.

13. The method of claim 12, wherein the engineered immune receptor specifically binds to an antigen expressed on a target cell.

14. The method of any one of claims 2-13, wherein the immune cells comprise chimeric antigen receptors and/or T cell receptors.

15. The method of any one of claims 1-14, wherein reducing the expression and/or activity of peptidase C64 family member(s) in the cell comprises inhibiting a deubiquitinating enzyme and/or a zinc finger nuclease function.

16. The method of any one of claims 1-15, wherein the cells obtained with reduced expression of the peptidase C64 family member(s) and/or reduced activity show improved cellular properties compared to cells in which the expression and/or activity of the peptidase C64 family member is unchanged.

17. The method of claim 16, wherein the improved cell properties comprise one or more properties selected from the group consisting of: improved cell proliferation capacity, increased proportion of viable cells, improved proportion of cell subpopulations, increased ability to secrete cytokines, and improved killing ability of tumor cells.

18. The method of claim 17, wherein the improved cell subpopulation ratio comprises one or more properties selected from the group consisting of: increased activated cell ratio, reduced regulatory cell ratio, reduced exhausted cell ratio, increased proportion of central memory cells and/or naive cells, decreased proportion of apoptotic cells, and increased proportion of stem cell-like cells.

19. The method of any one of claims 1-18, wherein the peptidase C64 family member comprises a ubiquitin-binding domain.

20. The method of any one of claims 1-19, wherein the peptidase C64 family member comprises TNFAIP3.

21. The method of any one of claims 1-20, wherein reducing the expression and/or activity of a peptidase C64 family member in the cell comprises introducing a gene regulatory system into the cell.

22. The method of claim 21, wherein the gene regulatory system is capable of destroying the peptidase C64 family member at the DNA level.

23. The method of any one of claims 21-22, wherein the gene regulatory system comprises a guide nucleic acid molecule and an enzymatic protein.

24. The method of claim 23, wherein reducing the expression and/or activity of the peptidase C64 family member comprises: introducing a ribonucleoprotein complex (RNP) comprising the guide nucleic acid molecule and the enzymatic protein, an LNP comprising a gRNA and a Cas protein, or an LNP comprising a nucleic acid encoding a gRNA and a Cas protein into the cell.

25. The method of any one of claims 23-24, wherein the enzymatic protein comprises Cas protein, Cas protein homolog, or a functionally active fragment thereof.

26. The method of any one of claims 23-25, wherein the guide nucleic acid molecule comprises a guide RNA (gRNA).

27. The method of any one of claims 23-26, wherein the guide nucleic acid molecule is capable of binding to the sequence of the peptidase C64 family member.

28. The method of any one of claims 23-27, wherein the guide nucleic acid molecule is capable of binding to a region selected from the group defined by the genomic coordinates shown in Table 1A, or a fragment thereof.

29. The method of any one of claims 23-28, wherein the guide nucleic acid molecule is capable of binding to a region selected from the group consisting of: SEQ ID NOs: 107-212, 1562-2532, or fragments thereof.

30. The method of any one of claims 23-29, wherein the guide nucleic acid molecule is capable of binding to a nucleotide sequence located about 15 to about 25 nucleotides upstream of the 5' end of a protospacer adjacent motif (PAM), wherein the sequence is selected from the group consisting of: AGG, TGG, CGG, and GGG.

31. The method of any one of claims 23-30, wherein the guide nucleic acid molecule comprises a targeting domain comprising the sequence shown in any one of SEQ ID NOs: 1-106, 591-1561, 7267-7324, 7419, and 7420.

32. The method of any one of claims 1-31, wherein the proportion of cells expressing the target genes is reduced and/or the expression of target gene in a single cell is reduced in cells obtained by reducing the expression and/or activity of the peptidase C64 family member, compared to cells in which the expression and/or activity of the peptidase C64 family member is unchanged.

33. The method according to any one of claims 1-32, wherein among the cells obtained by reducing the expression and/or reducing the activity of the peptidase C64 family member, the proportion of cells expressing the target gene is about 95% or less.

34. A cell produced by the method of any one of claims 1-33.

35. A composition comprising the cell of claim 34.

36. A pharmaceutical composition comprising the cell of claim 34 and/or the composition of claim 35, and optionally a pharmaceutically acceptable carrier.

37. A method of affecting cell growth, comprising administering the cell of claim 34, the composition of claim 35, and/or the pharmaceutical composition of claim 36.

38. Use of the cell of claim 34, the composition of claim 35, and/or the pharmaceutical composition of claim 36 in the preparation of a medicament, wherein the medicament is used to prevent and/or treat diseases and/or conditions.

39. Use of claim 38, wherein the disease and/or condition comprises a tumor.

40. Use of any one of claims 38-39, wherein the disease and/or condition comprises a solid tumor.

41. Use of any one of claims 38-40, wherein the disease and/or condition is one or more selected from the group consisting of: melanoma, ovarian cancer, cervical cancer, lung cancer, bladder cancer, breast cancer, head and neck cancer, pancreatic cancer, liver cancer, stomach cancer, colorectal cancer, and kidney cancer.
